(19) Europäisches Patentamt
European Patent Office
Office européen des brevets

(11) EP 2 053 094 B1

(12) **FASCICULE DE BREVET EUROPEEN**

(45) Date de publication et mention de la délivrance du brevet:
**26.03.2014 Bulletin 2014/13**

(51) Int Cl.:
*C09B 67/34* (2006.01) *A61K 8/46* (2006.01)
*A61K 8/49* (2006.01) *A61K 8/84* (2006.01)

(21) Numéro de dépôt: **08164949.3**

(22) Date de dépôt: **23.09.2008**

(54) **Composition pour la coloration de fibres kératiniques comprenant au moins un colorant direct a fonction disulfure/thiol et au moins un polymère a fonction thiol et procédé utilisant la composition**

Zusammensetzung zum Färben von Keratinfasern, die mindestens einen Direktfarbstoff mit Disulfid/Thiol-Funktion enthält und mindestens ein Polymer mit Thiolfunktion und Verfahren, bei dem diese Zusammensetzung zum Einsatz kommt

Composition for dyeing keratinous fibres comprising at least one direct dye with disulphur/thiol function and at least one polymer with thiol function and method using the composition

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **24.09.2007 FR 0757806**

(43) Date de publication de la demande:
**29.04.2009 Bulletin 2009/18**

(73) Titulaire: **L'Oréal**
**75008 Paris (FR)**

(72) Inventeurs:
- **De Boni, Maxime**
**Tokyo (JP)**
- **Daubresse, Nicolas**
**78170 La Celles St Cloud (FR)**

(74) Mandataire: **Fevrier, Murielle Françoise E.**
**L'Oréal**
**D.I.P.I.**
**25-29 Quai Aulagnier**
**92600 Asnières-sur-Seine Cedex (FR)**

(56) Documents cités:
**EP-A- 1 647 580**
**WO-A-2005/097051**
**WO-A-2006/134043**
**WO-A-2006/136617**
**WO-A-2007/025889**
**WO-A-2007/039527**
**WO-A-2007/072521**
**FR-A- 2 761 691**
**US-A1- 2002 108 188**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 053 094 B1

## Description

L'invention a pour objet une composition comprenant au moins un colorant direct à fonction disulfure/thiol et au moins un polymère à fonction thiol, utilisée pour colorer les fibres kératiniques, notamment humaines telles que les cheveux. Elle a également pour objet le procédé de coloration des fibres kératiniques mettant en oeuvre ladite composition et l'utilisation de colorants directs à fonction disulfure associé au polymère à fonction thiol dans la coloration et l'éclaircissement des fibres kératiniques.

Il est connu de teindre les fibres kératiniques, par une coloration directe ou coloration semi-permanente. La coloration directe ou coloration semi-permanente consiste à amener la couleur par une molécule colorée qui s'adsorbe à la surface du cheveu ou pénètre dans le cheveu. Ainsi, le procédé classiquement utilisé en coloration directe consiste à appliquer sur les fibres kératiniques des colorants directs qui sont des molécules colorées et colorantes ayant une affinité pour les fibres, à laisser les fibres en contact avec les molécules colorantes puis à rincer les fibres. Généralement, cette technologie conduit à des colorations chromatiques.

Depuis plusieurs années, des recherches scientifiques sont menées pour modifier la couleur de matières kératiniques notamment des fibres kératiniques et en particulier pour masquer les fibres blanches, modifier la couleur des fibres de façon permanente ou fugace, et répondre à de nouvelles envies et besoins en termes de couleurs et de durabilité.

Dans les demande EP 1 647 580 ou WO 2005/097051 il a été proposé un colorant direct comprenant une fonction disulfure susceptible de se greffer aux cheveux de façon covalente, à l'aide d'un traitement réducteur. Les demandes de brevet internationales WO 2006/136617, WO 2006/134043 et WO 2007/039527 divulguent également des colorants thiols et disulfures, seuls ou en mélanges, qui sont utilisés notamment pour colorer les cheveux humains.

Ces colorants directs sont plus tenaces sur les fibres kératiniques vis-à-vis des shampooings. Les réducteurs décrits dans ces documents sont des réducteurs de petites tailles et de faible masse molaire moléculaire tels que l'acide thioglycolique, l'acide thiolactique et leurs sels associés, la cystéine, et les borohydrures, ont tendance à migrer à l'intérieur de la fibre ce qui peut altérer l'intégrité de cette fibre ayant pour effet de la rendre cassante. De plus ces documents ne mentionnent pas de composition comprenant en outre de polymères à fonction(s) thiol(s), thiol(s) protégé(s) ou disulfure(s).

Il a été par ailleurs décrit dans la demande de brevet US 2002/108188 des procédés de traitement des cheveux en deux temps dont la première étape consiste en l'application sur les cheveux d'agent polyéthylèneimine ou dendrimère à fonction thiols puis en l'application de colorants tels que le 4-(aminosulphonyl)-7-fluoro-2,1,3-benzoxadiazole ou le colorant de type « Reactive Orange 16 » qui fluorescent une fois fixé. Ces colorants ne contiennent pas de fonctions disulfures, thiols ou thiols protégés.

Aucun de ces documents ne mentionne l'utilisation de colorants thiols, thiols protégés ou disulfures en vue d'éclaircir les cheveux foncés.

Le but de la présente invention est de fournir de nouveaux systèmes de coloration capillaire permettant d'obtenir des colorations homogènes, puissantes, tenaces vis-à-vis des agents extérieurs et qui n'altèrent pas les propriétés cosmétiques des fibres kératiniques.

Un autre but de l'invention est de fournir des systèmes de coloration permettant d'obtenir des colorations visibles sur cheveux foncés et des effets éclaircissants sur des fibres kératiniques naturellement ou artificiellement foncées sans dégradation de la fibre.

Un autre objet de l'invention est de rendre possible un effacement de la coloration sur cheveu clair ou sur cheveu foncé, tout en limitant les altérations de la qualité de la fibre, au moment où cela est souhaité.

Ces buts sont atteint avec la présente invention qui a pour objet une composition tinctoriale, comprenant dans un milieu cosmétique approprié, i) un ou plusieurs colorants choisis parmi les colorants directs à fonction(s) disulfure(s), et fonction(s) thiol(s) protégé(s) et ii) au moins un ou plusieurs polymère(s) à fonction(s) thiol(s) de poids moléculaire supérieur à 500 notamment supérieur à 1000.

L'invention a aussi pour objet un procédé de coloration des fibres kératiniques notamment humaines, telles que les cheveux, consistant à appliquer sur les fibres la composition tinctoriale mentionnée précédemment.

Un autre objet de l'invention est l'utilisation cosmétique notamment pour la coloration des fibres kératiniques, notamment les cheveux, d'un ou plusieurs colorant(s) direct(s) à fonction(s) disulfure(s), ou à fonction(s) thiol(s) protégé(s) et d'un ou plusieurs polymère(s) à fonction(s) thiol(s) de poids moléculaire supérieur à 500 notamment supérieur à 1000.

La composition selon l'invention permet en particulier de colorer de façon puissante et homogène les fibres kératiniques humaines telles que les cheveux tout en respectant l'intégrité des fibres. Les polymères à fonction thiol utiles dans la présente invention, peuvent réduire les molécules de cystéine présentes à la surface des cheveux permettant ainsi l'apparition de fonctions thiols en surface du cheveu puis la fixation covalente des colorants directs à fonction disulfure, thiol ou thiol protégés directement au cheveu, tout en évitant de pénétrer à l'intérieur des cheveux. Ces polymères peuvent se fixer eux-mêmes par l'établissement de liaisons covalentes aux protéines riche en motifs

cystines de la surface des cheveux et établir d'autres liaisons covalentes avec les colorants à motifs disulfures thiols ou thiol protégés, par échanges de liaisons « soufre-soufre ». Le cas échéant, l'établissement de liaisons disulfures asymétriques entre la surface du cheveu les polymères et les chromophores colorants issus des colorants de l'invention peut être assistée par un agent oxydant qui fixe entre eux deux fonctions thiols appartenant à deux espèces différentes.

Les polymères semblent assurer un rôle de sous-couche et de mordançage pour la fixation en surface des colorants disulfures ou thiols ou thiols protégés. De façon inattendue, la dégradation des fibres kératiniques est très limitée.

Les colorations obtenues sont esthétiques et très tenaces vis-à-vis des agressions courantes tel que le soleil, la transpiration, le sébum, et aux autres traitements capillaires comme les shampoings successifs.

Un autre avantage de l'invention concerne l'effacement a discrétion de la coloration qui est rendu possible avec un minimum d'altération de la qualité de la fibre kératinique. Cet effacement semble se limiter à des réactions en périphérie de la fibre.

Au sens de la présente invention, et à moins qu'une indication différente ne soit donnée :

■ un « colorant direct à fonction(s) disulfure(s) » est un colorant direct comportant un ou plusieurs chromophores qui absorbe(nt) la lumière dans le spectre du visible, et comprenant une ou plusieurs liaisons disulfures : -S-S- entre deux atomes de carbones, directement ou indirectement reliées au(x) chromophore(s) du colorant. Préférentiellement une seule liaison disulfure est indirectement reliée au(x) chromophore(s) ; la liaison -S-S- étant susceptible d'être réduite dans un milieu cosmétiquement acceptable; lorsque le colorant direct contient plusieurs chromophores, les chromophores peuvent être identiques ou différents ;

■ un « colorant direct à fonction thiol » est un colorant direct comportant un chromophore, et comprenant une ou plusieurs fonctions thiols, directement ou indirectement relié audit chromophore, préférentiellement une fonction thiol est indirectement relié au chromophore ; la fonction thiol est de type SH ou SY" avec Y" représentant a) un métal alcalin ; b) un métal alcalino-terreux ; c) un groupement ammonium : $N^+R^\alpha R^\beta R^\gamma R^\delta$, An"- ou un groupement phosphonium : $P^+R^\alpha R^\beta R^\gamma R^\delta$, An"-avec $R^\alpha$, $R^\beta$, $R^\gamma$ et $R^\delta$, identiques ou différents, représentant un atome d'hydrogène ou un groupement $(C_1\text{-}C_4)$alkyle et , An"- représentant un contre-ion anionique ; ;

■ un « colorant direct à fonction thiol protégée » est un colorant direct comportant un chromophore, et comprenant une ou plusieurs fonctions thiols protégées -SY' où Y' est un groupement protecteur connu par l'homme du métier comme par exemple ceux décris dans les ouvrages « Protective Groups in Organic Synthesis », T. W. Greene, John Willey & Sons ed., NY, 1981, pp.193-217 ; « Protecting Groups », P. Kocienski, Thieme, 3ème ed., 2005, chap. 5 ;

■ un « chromophore » est un radical issu d'un colorant, c'est-à-dire un radical issu d'une molécule absorbant la lumière dans le domaine visible du rayonnement perceptible visuellement et physiologiquement par l'homme *i.e.* à une longueur d'onde d'absorption $\lambda_{abs}$ comprise inclusivement entre 400 et 800 nm ; le chromophore peut être fluorescent ie qu'il est capable d'absorber dans le rayonnement UV ou visible à une longueur d'onde $\lambda_{abs}$ comprise entre 250 et 800 nm et capable de réémettre dans le domaine du visible à une longueur d'onde d'émission $\lambda_{ém}$ comprise entre 400 et 800 nm ;

■ les colorants disulfures selon l'invention contiennent un ou plusieurs chromophores, et ces colorants disulfures sont capables d'absorber la lumière à une longueur d'onde $\lambda_{abs}$ particulièrement comprise inclusivement entre 400 et 700 nm ;

■ les colorants disulfures « fluorescents » selon l'invention sont des colorants contenant au moins un chromophore fluorescent, et ces colorants disulfures sont capables d'absorber dans le visible à une longueur d'onde $\lambda_{abs}$ particulièrement comprise entre 400 et 800 nm et de réémettre dans le visible à une plus grande longueur d'onde $\lambda_{ém}$ que celle absorbée comprise entre 400 et 800 nm. La différence de la longueur d'onde d'absorption et d'émission également appelée déplacement de Stoke ou Stoke's shift est comprise entre 1 nm et 100 nm. Plus préférentiellement les colorants fluorescents sont des colorants capables d'absorber à une longueur d'onde $\lambda_{abs}$ comprise entre 420 nm et 550 nm et de réémettre dans le visible à une longueur d'onde $\lambda_{ém}$ comprise entre 470 et 600 nm ;

■ les chromophores sont dits « différents » lorsqu'ils diffèrent par leur structure chimique et peuvent être des chromophores issus de familles différentes ou d'une même famille à la condition de présenter des structures chimiques différentes : par exemple, les chromophores peuvent être choisis dans la famille des colorants azoïques mais différer par la structure chimique des radicaux le constituant ou par la position respective de ces radicaux ;

■ une « chaîne alkylène » représente un chaîne divalente en $C_1\text{-}C_{18}$; particulièrement en $C_1\text{-}C_6$, plus particulièrement en $C_1\text{-}C_2$ lorsque la chaîne est linéaire ; éventuellement substituée par un ou plusieurs, identiques ou différents, groupements choisis parmi hydroxy, $(C_1\text{-}C_2)$alkoxy, (poly)hydroxy$(C_2\text{-}C_4)$alcoxy (di)$(C_1\text{-}C_2)$ (alkyl)amino, $R^a\text{-}Z^a\text{-}C(Z^b)\text{-}$, et $R^a\text{-}Z^a\text{-}S(O)_t\text{-}$ avec $Z^a$, $Z^b$, identiques ou différents, représentant un atome d'oxygène, de soufre, ou un groupe $NR^{a'}$, $R^a$, représentant un métal alcalin, un atome d'hydrogène, un groupement alkyle ou alors est absent si une autre partie de la molécule cationique et $R^{a'}$ représentant un atome d'hydrogène ou un groupement alkyle et t vaut 1 ou 2 ;

■ une « chaîne hydrocarbonée divalente en $C_1$-$C_{30}$, saturée ou insaturée, éventuellement substituée », représente une chaîne hydrocarbonée, particulièrement an $C_1$-$C_8$, comprenant éventuellement une ou plusieurs doubles liaisons $\pi$, conjuguée ou non, particulièrement la chaîne hydrocarbonée est saturée ; ladite chaîne est éventuellement substituée par un ou plusieurs, identiques ou différents, groupements choisis parmi hydroxy, ($C_1$-$C_2$)alkoxy, (poly)hydroxy($C_2$-$C_4$)alcoxy (di)($C_1$-$C_2$) (alkyl)amino, $R^a$-$Z^a$-C($Z^b$)-, et $R^a$-$Z^a$-S(O)$_t$- avec $Z^a$, $Z^b$, identiques ou différents, représentant un atome d'oxygène, de soufre, ou un groupe $NR^{a'}$, $R^a$, représentant un métal alcalin, un atome d'hydrogène, un groupement alkyle ou alors est absent si une autre partie de la molécule cationique et $R^{a'}$ représentant un atome d'hydrogène ou un groupement alkyle et t vaut 1 ou 2 ;

■ les radicaux « aryle » ou « hétéroaryle » ou la partie aryle ou hétéroaryle d'un radical peuvent être substitués par au moins un substituant porté par un atome de carbone, choisi parmi :

- un radical alkyle en $C_1$-$C_{16}$, de préférence en $C_1$-$C_8$, éventuellement substitué par un ou plusieurs radicaux choisis parmi les radicaux hydroxy, alkoxy en $C_1$-$C_2$, (poly)-hydroxyalkoxy en $C_2$-$C_4$, acylamino, amino substitué par deux radicaux alkyle, identiques ou différents, en $C_1$-$C_4$, éventuellement porteurs d'au moins un groupement hydroxy ou, les deux radicaux pouvant former avec l'atome d'azote auquel ils sont rattachés, un hétérocycle comprenant de 5 à 7 chaînons, de préférence de 5 ou 6 chaînons, saturé ou insaturé éventuellement substitué comprenant éventuellement un autre hétéroatome identique ou différent de l'azote ;
- un atome d'halogène ;
- un groupement hydroxy ;
- un radical alkoxy en $C_1$-$C_2$ ;
- un radical (poly)-hydroxyalkoxy en $C_2$-$C_4$ ;
- un radical amino ;
- un radical héterocycloalkyle à 5 ou 6 chaînons ;
- un radical hétéroaryle à 5 ou 6 chaînons éventuellement cationique, préférentiellement imidazolium, et éventuellement substitué par un radical ($C_1$-$C_4$) alkyle, préférentiellement méthyle ;
- un radical amino substitué par un ou deux radicaux alkyle, identiques ou différents, en $C_1$-$C_6$ éventuellement porteurs d'au moins:

    i) un groupement hydroxy,
    ii) un groupement amino éventuellement substitué par un ou deux radicaux alkyle en $C_1$-$C_3$ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote,
    iii) un groupement ammonium quaternaire $-N^+R'R''R'''$, $M^-$ pour lequel R',
    R'', R''', identiques ou différents représentent un atome d'hydrogène, ou un groupement alkyle en $C_1$-$C_4$; et $M^-$ représente le contre-ion de l'acide organique, minéral ou de l'halogénure correspondant,
    iv) ou un radical hétéroaryle à 5 ou 6 chaînons éventuellement cationique, préférentiellement imidazolium, et éventuellement substitué par un radical ($C_1$-$C_4$) alkyle, préférentiellement méthyle ;

- un radical acylamino (-NR-COR') dans lequel le radical R est un atome d'hydrogène, un radical alkyle en $C_1$-$C_4$ éventuellement porteur d'au moins un groupement hydroxy et le radical R' est un radical alkyle en $C_1$-$C_2$; un radical carbamoyle ($(R)_2N$-CO-) dans lequel les radicaux R, identiques ou non, représentent un atome d'hydrogène, un radical alkyle en $C_1$-$C_4$ éventuellement porteur d'au moins un groupement hydroxy ; un radical alkylsulfonylamino ($R'SO_2$-NR-) dans lequel le radical R représente un atome d'hydrogène, un radical alkyle en $C_1$-$C_4$ éventuellement porteur d'au moins un groupement hydroxy et le radical R' représente un radical alkyle en $C_1$-$C_4$, un radical phényle ; un radical aminosulfonyle ($(R)_2N$-$SO_2$-) dans lequel les radicaux R, identiques ou non, représentent un atome d'hydrogène, un radical alkyle en $C_1$-$C_4$ éventuellement porteur d'au moins un groupement hydroxy,
- un radical carboxylique sous forme acide ou salifiée (de préférence avec un métal alcalin ou un ammonium, substitué ou non) ;
- un groupement cyano ;
- un groupement nitro ou nitroso,
- un groupement polyhalogénoalkyle, préférentiellement le trifluorométhyle ; la partie cyclique ou hétérocyclique d'un radical non aromatique peut être substituée par au moins un substituant choisi parmi les groupements :

- hydroxy,
- alkoxy en $C_1$-$C_4$, (poly)hydroxyalkoxy en $C_2$-$C_4$,
- alkyle en $C_1$-$C_4$,

- alkylcarbonylamino (RCO-NR'-) dans lequel le radical R' est un atome d'hydrogène, un radical alkyle en $C_1$-$C_4$ éventuellement porteur d'au moins un groupement hydroxy et le radical R est un radical alkyle en $C_1$-$C_2$, amino éventuellement substitué par un ou deux groupements alkyle identiques ou différents en $C_1$-$C_4$ eux-mêmes éventuellement porteurs d'au moins un groupement hydroxy, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
- alkylcarbonyloxy (RCO-O-) dans lequel le radical R est un radical alkyle en $C_1$-$C_4$, groupement amino éventuellement substitué par un ou deux groupements alkyle identiques ou différents en $C_1$-$C_4$ eux-mêmes éventuellement porteurs d'au moins un groupement hydroxy, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
- alkoxycarbonyle (RG-CO-) dans lequel le radical R est un radical alkoxy en $C_1$-$C_4$, G est un atome d'oxygène, ou un groupement amino éventuellement substitué par un groupement alkyle en $C_1$-$C_4$ lui-même éventuellement porteurs d'au moins un groupement hydroxy, ledit radical alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;

■ un radical cyclique, hétérocyclique, ou une partie non aromatique d'un radical aryle ou hétéroaryle, peut également être substitué par un ou plusieurs groupements oxo ;

■ une chaîne hydrocarbonée est insaturée lorsqu'elle comporte une ou plusieurs liaisons doubles et/ou une ou plusieurs liaisons triples ;

■ un radical « aryle » représente un groupement carboné mono ou polycyclique, condensé ou non, comprenant de 6 à 22 atomes de carbones, et dont au moins un cycle est aromatique ; préférentiellement le radical aryle est un phényle, biphényle, naphtyle, indényle, anthracényle, ou tétrahydronaphtyle ;

■ un « radical hétéroaryle » représente un groupement mono ou polycyclique, condensé ou non, éventuellement cationique, comprenant de 5 à 22 chaînons, de 1 à 6 hétéroatomes choisis parmi l'atome d'azote, d'oxygène, de soufre et de sélénium, et dont au moins un cycle est aromatique ; préférentiellement un radical hétéroaryle est choisis parmi acridinyle, benzimidazolyle, benzobistriazolyle, benzopyrazolyle, benzopyridazinyle, benzoquinolyle, benzothiazolyle, benzotriazolyle, benzoxazolyle, pyridinyle, tetrazolyle, dihydrothiazolyle, imidazopyridinyle, imidazolyle, indolyle, isoquinolyle, naphthoimidazolyle, naphthooxazolyle, naphthopyrazolyle, oxadiazolyle, oxazolyle, oxazolopyridyle, phénazinyle, phénooxazolyle, pyrazinyle, pyrazolyle, pyrilyle, pyrazoyltriazyle, pyridyle, pyridinoimidazolyle, pyrrolyle, quinolyle, tétrazolyle, thiadiazolyle, thiazolyle, thiazolopyridinyle, thiazoylimidazolyle, thiopyrylyle, triazolyle, xanthylyle et son sel d'ammonium ;

■ un « radical cyclique » est un radical cycloalkyle non aromatique, mono ou polycyclique, condensé ou non, contenant de 5 à 22 atomes de carbone, pouvant comporter de une à plusieurs insaturations ;

■ un « radical hétérocyclique » est un radical non aromatique mono ou polycyclique, condensé ou non, contenant de 5 à 22 chaînons, comportant de 1 à 6 hétéroatomes choisis parmi l'atome d'azote, d'oxygène, de soufre et de sélénium ;

■ un « radical alkyle » est un radical hydrocarboné en $C_1$-$C_{16}$, linéaire ou ramifié, de préférence en $C_1$-$C_8$,

■ l'expression « éventuellement substitué » attribué au radical alkyle sous entend que ledit radical alkyle peut être substitué par un ou plusieurs radicaux choisis parmi les radicaux i) hydroxy, ii) alkoxy en $C_1$-$C_4$, iii) acylamino, iv) amino éventuellement substitué par un ou deux radicaux alkyle, identiques ou différents, en $C_1$-$C_4$, lesdits radicaux alkyles pouvant former avec l'atome d'azote qui les portent un hétérocyle comprenant de 5 à 7 chaînons, comprenant éventuellement un autre hétéroatome différent ou non de l'azote ; v) ou un groupement ammonium quaternaire $-N^+R'R''R'''$, $M^-$ pour lequel R', R'', R''', identiques ou différents représentent un atome d'hydrogène, ou un groupement alkyle en $C_1$-$C_4$, ou alors $-N^+R'R''R'''$ forme un hétéroaryle tel que imidazolium éventuellement substitué par un groupement $C_1$-$C_4$ alkyle, et $M^-$ représente le contre-ion de l'acide organique, minéral ou de l'halogénure correspondant ;

■ un « radical alkoxy » est un radical alkyle-oxy pour lequel le radical alkyle est un radical hydrocarboné, linéaire ou ramifié, en $C_1$-$C_{16}$ préférentiellement en $C_1$-$C_8$ ;

■ lorsque le groupement alkoxy est éventuellement substitué, cela sous entend que le groupe alkyle est éventuellement substitué tel que défini supra ;

■ la « hauteur de ton » est l'unité connue des professionnels de la coiffure, et publiée dans l'ouvrage « Science des traitements capillaires » de Charles ZVIAK 1988, Ed. Masson, p. 215 et 278, les hauteurs de ton s'échelonnent de 1 (noir) à 10 (blond clair clair), une unité correspondant à un ton, plus le chiffre est élevé et plus la nuance est claire ;

■ la fibre kératinique « foncée » est une fibre kératinique qui présente une luminescence L* chiffrée dans le système C.I.E.L*a*b*, inférieure ou égale à 45 et de préférence inférieure ou égale à 40, sachant par ailleurs que L*=0 équivaut au noir et L*=100 au blanc ;

■ les cheveux « naturellement ou artificiellement foncés », sont des cheveux dont la hauteur de ton est inférieure ou égale à 6 (blond foncé) et de préférence inférieure ou égale à 4 (châtain). Les cheveux colorés artificiellement sont des cheveux dont la couleur a été modifiée par un traitement de coloration par exemple une coloration avec des colorants directs ou des colorants d'oxydation.

*1.1. Colorants directs à fonction disulfure de l'invention :*

Selon un mode de réalisation de l'invention les colorants directs à fonction thiol protégé ou disulfure, sont choisis parmi les colorants de formule **(I) :**

$$\mathbf{A-(X)_p-C_{sat}-S\text{-}U} \qquad \mathbf{(I)}$$

leurs sels d'acide organique ou minéral, leurs isomères optiques, géométriques et leurs solvates tels que les hydrates, formule **(I),** dans laquelle :

- U représente un radical choisi parmi :

  i) **$-S-C'_{sat}-(X')_{p'}-A'$** ;
  ii) **$-S-C'_{sat}-(X')_{p'}-D$ ; et**
  **iii) -Y ;**

- **A** et **A',** identiques ou différents, représentent un radical contenant un ou plusieurs chromophore(s) cationique(s) ou non ;
- **Y** représente ; a) un métal alcalin ; b) un métal alcalino-terreux ; c) un groupement ammonium : $N^+R^{\alpha}R^{\beta}R^{\gamma}R^{\delta}$, An"- ou un groupement phosphonium : $P^+R^{\alpha}R^{\beta}R^{\gamma}R^{\delta}$, An"-avec $R^{\alpha}$, $R^{\beta}$, $R^{\gamma}$ et $R^{\delta}$, identiques ou différents, représentant un atome d'hydrogène

ou un groupement $(C_1\text{-}C_4)$alkyle et , An"- représentant un contre-ion anionique ; ou d) un groupement protecteur de fonction thiol ;

- **X** et **X',** identiques ou différents, représentent :

  a) une chaîne hydrocarbonée en $C_1$-$C_{30}$, particulièrement en $C_1$-$C_8$, linéaire ou ramifiée, saturée ou insaturée, éventuellement substituée, éventuellement interrompue et/ou éventuellement terminée à une ou aux deux extrémités de ladite chaine par un ou plusieurs groupes divalents ou leur combinaisons choisis parmi :

    i) -N(R)-; $-N^+(R)(R')$-, $Q^-$; -O-; -S-; -CO-; $-SO_2$- avec R, R', identiques ou différents, sont choisis parmi un hydrogène, un radical alkyle en $C_1$-$C_4$, hydroxyalkyle et aminoalkyle, $Q^-$ représente un contre-ion anionique ; et ii) un radical (hétéro)cyclique, aromatique ou non, saturé ou insaturé, condensé ou non, comprenant éventuellement un ou plusieurs hétéroatomes identiques ou non, éventuellement substitué ; ou
    b) un ou plusieurs groupes divalents ou leur combinaisons choisis parmi : $-SO_2$-; -O-; -S-; -N(R)-; $-N^+(R)(R°)$-; -CO-; avec R, R°, identiques ou différents, représentant un atome d'hydrogène, un radical alkyle en $C_1$-$C_4$, hydroxyalkyle en $C_1$-$C_4$ ou un aryl$(C_1\text{-}C_4)$alkyle; et un radical hétérocycloalkyle ou hétéroaryle, cationique ou non, préférentiellement monocyclique, contenant préférentiellement deux hétéroatomes (plus préférentiellement deux atomes d'azote) et comportant préférentiellement de 5 à 7 chaînons, plus préférentiellement imidazolium ;

  particulièrement X et X', identiques ou différents, représentent a) une chaîne hydrocarbonée en $C_1$-$C_{30}$, particulièrement en $C_1$-$C_8$, linéaire ou ramifiée, saturée ou insaturée, éventuellement substituée, éventuellement interrompue par un ou plusieurs groupes divalents ou leur combinaisons choisis parmi : i) et ii) tels que définis précédemment ;

- **p** et **p',** identiques ou différents, valent 0 ou 1 ;
- **$C_{sat}$** et **$C'_{sat}$,** identiques ou différents représentent une chaîne alkylène en $C_1$-$C_{18}$, linéaire ou ramifiée, éventuellement substituée, éventuellement cyclique ;
- **D** correspond à un radical choisi parmi les radicaux hydroxy, hydroxyalkyle, alkoxy, carboxyliques, carboxylates,

amino, alkylamino, dialkylamino.

Un mode particulier de l'invention concerne les compositions tinctoriales comprenant en tant que colorant à fonction disulfure, un colorant de formule (I) tel que défini supra.

*1.1.1.* ***Y*:**

Les colorants de l'invention de formule **(I)** avec **U** représentant iii) **-Y,** comportent une fonction SY pouvant se trouver sous la forme covalente -S-Y ou ionique $-S^{-}$ $Y^{+}$ selon la nature de Y et du pH du milieu.

Un mode particulier concerne les colorants thiols de formule **(I),** avec **Y** représentant un métal alcalin.

Conformément à un autre mode de réalisation particulier de l'invention, dans la formule **(I)** précitée, **Y** est un groupement protecteur connu par l'homme du métier comme par exemple ceux décrits dans les ouvrages « Protective Groups in Organic Synthesis », T. W. Greene, John Willey & Sons ed., NY, 1981, pp.193-217; « Protecting Groups », P. Kocienski, Thieme, 3ème ed., 2005, chap. 5. Etant entendu que lorsque U représente Y, et Y représente un groupe protecteur, il ne peut pas constituer avec l'atome de soufre auquel il est lié un colorant disulfure i.e. le radical U ne peut pas représenter : i) $-S-C'_{sat}-(X')_{p'}-A'$ ou ii)$-S-C'_{sat}-(X')_{p'}-$ D tels que définis précédemment. Le groupe protecteur ne peut pas représenter un groupe directement relié à l'atome de soufre du colorant thiol via un autre atome de soufre non oxydé.

Particulièrement, lorsque **Y** représente un groupement protecteur de la fonction thiol **Y'** qui est choisi parmi les radicaux suivants :

- ■ $(C_1-C_4)$alkylcarbonyle ;
- ■ $(C_1-C_4)$alkylthiocarbonyle ;
- ■ $(C_1-C_4)$alcoxycarbonyle ;
- ■ $(C_1-C_4)$alcoxythiocarbonyle ;
- ■ $(C_1-C_4)$alkylthio-thiocarbonyle ;
- ■ (di) $(C_1-C_4)$ (alkyl)aminocarbonyle ;
- ■ (di) $(C_1-C_4)$ (alkyl)aminothiocarbonyle;
- ■ arylcarbonyle comme phénylcarbonyle ;
- ■ aryloxycarbonyle ;
- ■ aryl$(C_1-C_4)$alkcoxycarbonyle ;
- ■ (di) $(C_1-C_4)$ (alkyl)aminocarbonyle comme diméthylaminocarbonyle ;
- ■ $(C_1-C_4)$(alkyl)arylaminocarbonyle ;
- ■ carboxy ;
- ■ $SO_3^-$; $M^+$ avec $M^+$ représentant un métal alcalin tel que le sodium ou le potassium, ou alors un contre-ion du chromophore cationique **A** et $M^+$ sont absents ;

- aryle éventuellement substitué tel que le phényle, dibenzosubéryle, ou 1,3,5-cycloheptatriényle ;
- hétéroaryle éventuellement substitué ; dont notamment l'hétéroaryle cationiques ou non, comprenant de 1 à 4 hétéroatomes suivants :

  i) monocycliques à 5, 6 ou 7 chaînons tels que furanyle ou furyle, pyrrolyle ou pyrryle, thiophényle ou thiényle, pyrazolyle, oxazolyle, oxazolium, isoxazolyle, isoxazolium, thiazolyle, thiazolium, isothiazolyle, isothiazolium, 1,2,4-triazolyle, 1,2,4-triazolium, 1,2,3-triazolyle, 1,2,3-triazolium, 1,2,4-oxazolyle, 1,2,4-oxazolium, 1,2,4-thiadiazolyle, 1,2,4-thiadiazolium, pyrylium, thiopyridyle, pyridinium, pyrimidinyle, pyrimidinium, pyrazinyle, pyrazinium, pyridazinyle, pyridazinium, triazinyle, triazinium, tétrazinyle, tétrazinium, azépine, azépinium, oxazépinyle, oxazépinium, thiépinyle, thiépinium, imidazolyle, imidazolium ;

  ii) bicycliques à 8 à 11 chaînons tels que indolyle, indolinium, benzoimidazolyle, benzoimidazolium, benzoxazolyle, benzoxazolium, dihydrobenzoxazolinyle, benzothiazolyle, benzothiazolium, pyridoimidazolyle, pyridoimidazolium, thiénocycloheptadiényle, ces groupes mono ou bicycliques étant éventuellement substitués par un ou plusieurs groupements tels que $(C_1-C_4)$alkyle comme méthyle, ou polyhalogéno$(C_1-C_4)$alkyle comme trifluorométhyle ;

  iii) ou tricyclique <u>ABC</u> suivant :

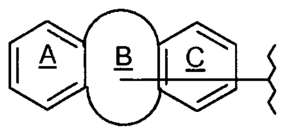

dans lequel les deux cycles A, C comportent éventuellement un hétéroatome, et le cycle B est un cycle à 5, 6 ou 7 chaînons particulièrement à 6 chaînons et contient au moins un hétéroatome comme pypéridyle, pyranyle ;

- hétérocycloalkyle éventuellement substitué, éventuellement cationique, le groupement hétérocycloalkyle représente notamment un groupement monocyclique saturé ou partiellement saturé à 5, 6 ou 7 chaînons comprenant de 1 à 4 hétéroatomes choisis parmi l'oxygène, le soufre et l'azote, tel que di/tétrahydrofuranyle, di/tétrahydrothiophényle, di/tétrahydropyrrolyle, di/tétrahydropyranyle, di/tétra/hexa-hydrothiopyranyle, dihydropyridyle, pipérazinyle, pipéridinyle, tétraméthylpipéridinyle, morpholinyle, di/tétra/hexahydroazépinyle, di/tétrahydropyrimidinyle ces groupes étant éventuellement substitués par un ou plusieurs groupes comme $(C_1-C_4)$ alkyle, oxo ou thioxo ; ou l'hétérocycle représente le groupement suivant :

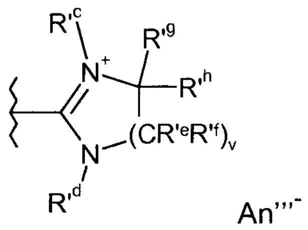

dans lequel $R'^c$, $R'^d$, $R'^e$, $R'^f$, $R'^g$ et $R'^h$, identiques ou différents représentent un atome d'hydrogène ou un groupement $(C_1-C_4)$ alkyle, ou alors deux groupement $R'^g$ avec $R'^h$, et/ou $R'^e$ avec $R'^f$ forment un groupement oxo ou thioxo, ou alors $R'^g$ avec $R'^e$ forment ensemble un cycloalkyle ; et v représente un entier compris inclusivement entre 1 et 3 ; préférentiellement $R'^c$ à $R'^h$ représentent un atome d'hydrogène ; et An'''⁻ représente un contre-ion ;
- isothiouronium $-C(NR'^cR'^d)=N^+R'^eR'^f$; An'''⁻ avec $R'^c$, $R'^d$, $R'^e$ et $R'^f$, identiques ou différents représentent un atome d'hydrogène ou un groupement $(C_1-C_4)$alkyle ; préférentiellement $R'^c$ à $R'^f$ représentent un atome d'hydrogène ; et An'''⁻ représente un contre-ion ;
- isothiourée $-C(NR'^cR'^d)=NR'^e$; avec $R'^c$, $R'^d$ et $R'^e$ sont tels que définis précédemment ;
- (di)aryl$(C_1-C_4)$alkyle éventuellement substitué tel que le 9-anthracénylméthyle, phénylméthyle ou diphénylméthyle éventuellement substitué par un plusieurs groupements notamment choisis parmi $(C_1-C_4)$ alkyle, $(C_1-C_4)$ alcoxy comme le méthoxy, hydroxy, alkylcarbonyle, (di)$(C_1-C_4)$(alkyl)amino comme le diméthylamino ;
- (di)hétéroaryl$(C_1-C_4)$akyle éventuellement substitué, le groupement hétéroaryle est notamment, cationique ou non, monocyclique, comprenant 5 ou 6 chaînons et de 1 à 4 hétéroatomes choisis parmi l'azote, l'oxygène et le soufre, tels que les groupes pyrrolyle, furanyle, thiophényle, pyridyle, pyridyle N-oxyde tels que le 4-pyridyle ou 2-pyridyl-N-oxyde, pyrylium, pyridinium, triazinyle, éventuellement substitué par un ou plusieurs groupement tel que alkyle particulièrement méthyle, avantageusement le (di)hétéroaryl$(C_1-C_4)$akyle est (di)hétéroarylméthyle ou (di)hétéroaryléthyle ;
- $CR^1R^2R^3$ avec $R^1$, $R^2$ et $R^3$ identiques ou différents, représentant un atome d'halogène ou un groupement choisi parmi : - $(C_1-C_4)$alkyle ;

  - $(C_1-C_4)$alcoxy;
  - aryle éventuellement substitué tel que phényle éventuellement substitué par un ou plusieurs groupements comme $(C_1-C_4)$alkyle, $(C_1-C_4)$alcoxy, hydroxy ;
  - hétéroaryle éventuellement substitué tel que thiophényle, furanyle, pyrrolyle, pyranyle, pyridyle, éventuellement substitué par un groupement $(C_1-C_4)$alkyle ;
  - $P(Z^1)R'^1R'^2R'^3$ avec $R'^1$ et $R'^2$ identiques ou différents représentent un groupement hydroxy, $(C_1-C_4)$alcoxy ou alkyle, $R'^3$ représente un groupement hydroxy ou $(C_1-C_4)$alcoxy, et $Z^1$ représente un atome d'oxygène ou de soufre ;

■ cyclique stériquement encombré ; et
■ alcoxyalkyle éventuellement substitué tels que le méthoxyméthyle (MOM), éthoxyéthyle (EOM) et l'isobutoxyméthyle.

Selon un mode de réalisation particulier les colorants fluorescents thiols protégés de formule **(I),** comportant un groupement **Y'** i) hétéroaryle monocyclique à 5 ou 6 chaînons aromatiques, cationiques comprenant de 1 à 4 hétéroatomes choisis parmi l'oxygène, le soufre et l'azote, tels que oxazolium, isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinyle, pyrazinium, pyridazinium, triazinium, tétrazinium, oxazépinium, thiépinyle, thiépinium, imidazolium ; ii) hétéroaryle bicyclique à 8 à 11 chaînons cationique tels que indolinium, benzoimidazolium, benzoxazolium, benzothiazolium, ces groupes hétéroaryle mono ou bicycliques étant éventuellement substitués par un ou plusieurs groupements tels que alkyle comme méthyle, ou polyhalogéno($C_1$-$C_4$)alkyle comme trifluorométhyle ; iii) ou hétérocyclique suivant :

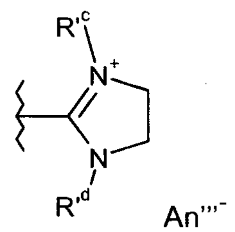

dans lequel R'[c] et R'[d], identiques ou différents, représentent un atome d'hydrogène ou un groupement ($C_1$-$C_4$)alkyle ; préférentiellement R'[c] à R'[d] représentent un groupement ($C_1$-$C_4$)alkyle tel que méthyle ; et An'''⁻ représente un contre-ion.

Particulièrement **Y** représente un groupement choisi parmi oxazolium, isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinium, pyridazinium, triazinium et imidazolium, benzoimidazolium, benzoxazolium, benzothiazolium, ces groupements étant éventuellement substitués par un ou plusieurs groupes ($C_1$-$C_4$) alkyle notamment méthyle. En particulier Y représente un métal alcalin ou un groupement protecteur tel que:

- ➢ ($C_1$-$C_4$)alkylcarbonyle comme méthylcarbonyle ou éthylcarbonyle ;
- ➢ arylcarbonyle comme phénylcarbonyle ;
- ➢ ($C_1$-$C_4$)alkoxycarbonyle ;
- ➢ aryloxycarbonyle ;
- ➢ aryl($C_1$-$C_4$)alkoxycarbonyle ;
- ➢ (di) ($C_1$-$C_4$) (alkyl)aminocarbonyle comme diméthylaminocarbonyle;
- ➢ ($C_1$-$C_4$)(alkyl)arylaminocarbonyle ;
- ➢ aryle éventuellement substitué tel que le phényle ;
- ➢ hétéroaryle monocyclique à 5 ou 6 chaînons tels que imidazolyle ou pyridyle ;
- ➢ hétéroaryle monocyclique cationique à 5, 6 chaînons tels que pyrylium, pyridinium, pyrimidinium, pyrazinium, pyridazinium, triazinium, imidazolium ; ces groupements étant éventuellement substitués par un ou plusieurs, identiques ou différents, groupes ($C_1$-$C_4$)alkyle tel que méthyle ;
- ➢ hétéroaryle bicyclique cationique à 8 à 11 chaînons tels que benzoimidazolium, ou le benzoxazolium ; ces groupements étant éventuellement substitué par un ou plusieurs, identiques ou différents, groupes ($C_1$-$C_4$)alkyle tel que méthyle ;
- ➢ hétérocycle cationique de formule suivante :

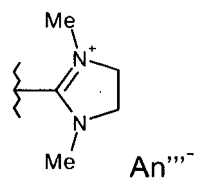

- ➢ isothiouronium $-C(NH_2)=N^+H_2$; An'''⁻;
- ➢ isothiourée $-C(NH_2)=NH$ ;
- ➢ $SO_3^-$, $M^+$ avec $M^+$ représentant un métal alcalin tel que le sodium ou le potassium.

*1.1.2.* ***$C_{sat}$*** *et* ***$C'_{sat}$*** *:*

Comme indiqué auparavant, dans les formules (I), $C_{sat}$ et $C'_{sat}$, indépendamment l'un de l'autre, représentent une chaîne alkylène en $C_1$-$C_{18}$, linéaire ou ramifiée, éventuellement substituée, éventuellement cyclique.

A titre de substituant, on peut citer les groupements amino, ($C_1$-$C_4$)alkylamino, ($C_1$-$C_4$)dialkylamino, ou le

groupement $R^a$-$Z^a$-C($Z^b$)- (dans laquelle $Z^a$, $Z^b$, identiques ou différents, représentent un atome d'oxygène, de soufre, ou un groupe NR $^{a'}$, et $R^a$, représente un métal alcalin, un atome d'hydrogène, ou un groupement $C_1$-$C_4$ alkyle et $R^{a'}$ représente un atome d'hydrogène ou un groupement $C_1$-$C_4$ alkyle) présents de préférence sur le carbone en position béta ou gamma des atomes de soufre.

De préférence, dans le cas des formules (I), $C_{sat}$ et $C'_{sat}$, représentent une chaîne $-(CH_2)_k-$ avec k entier, compris inclusivement entre 1 et 8.

*1.1.3.* ***X*** *et* ***X'****:*

Conformément à un mode de réalisation particulier de l'invention, dans les formules (I), précitées, lorsque p est égal à 1, X et X', identiques ou différents, représentent la séquence suivante :

$$-(T)_t-(Z)_z-(T')_{t'}-$$

ladite séquence étant reliée dans les formules (I) de façon symétrique comme suit: - $C_{sat}$ (ou $C'_{sat}$)-$(T)_t$-$(Z)_z$(A ou A') ; dans laquelle

**T** et **T',** identiques ou différents, représentent un ou plusieurs radicaux ou leurs combinaisons choisis parmi : -O- ; -S- ; -N(R)- ; $-N^+(R)(R°)-$ ;-SO- ; $-SO_2-$ ; -CO- ; avec R, R°, identiques ou différents, représentant un atome d'hydrogène, un radical alkyle en $C_1$-$C_4$, hydroxyalkyle en $C_1$-$C_4$ ou un aryl($C_1$-$C_4$)alkyle ; et un radical hétérocycloalkyle ou hétéroaryle, cationique ou non, préférentiellement monocyclique, contenant préférentiellement deux hétéroatomes (plus préférentiellement deux atomes d'azote) et comportant préférentiellement de 5 à 7 chaînons, plus préférentiellement imidazolium ; les indices **t** et **t',** identiques ou différents, valent 0 ou 1 ; **Z** représente :

- $-(CH_2)_m-$ avec m entier compris entre 1 et 8 ;
- $-(CH_2CH_2O)_q-$ ou $-(OCH_2CH_2)_q-$ dans lesquelles q est un entier compris inclusivement entre 1 et 5 ;
- un radical aryle, alkylaryle ou arylalkyle dont le radical alkyle est en $C_1$-$C_4$ et le radical aryle est de préférence en $C_6$, étant éventuellement substitué par au moins un groupement $SO_3M$ avec M représentant un atome d'hydrogène, un métal alcalin ou un groupement ammonium substitué par un ou plusieurs radicaux alkyle, identiques ou non, linéaires ou ramifiés, en $C_1$-$C_{18}$ éventuellement porteurs d'au moins un hydroxy **z** vaut 0 ou 1.

Par ailleurs, selon un mode de réalisation particulier de l'invention, Z représente :

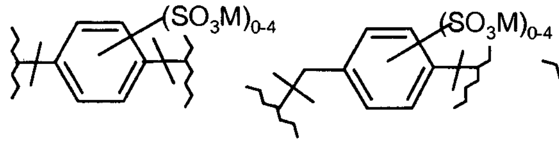
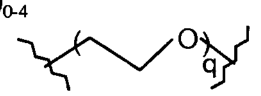
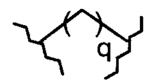

où M représente un atome d'hydrogène, un métal alcalin ou un groupement ammonium ou un groupement ammonium substitué par un ou plusieurs radicaux alkyle, identiques ou non, linéaires ou ramifiés, en $C_1$-$C_{10}$ éventuellement porteurs d'au moins un hydroxy et 0-4 représente un nombre entier compris inclusivement entre 0 et 4.

*1.1.4.* ***A*** *et* ***A'****:*

Les radicaux A et/ou A' des formules (I) peuvent contenir un ou plusieurs chromophores, identiques ou différents

A titre de chromophores A et/ou A' utiles dans la présente invention, on peut citer les radicaux issus des colorants acridines, acridones, anthranthrones, anthrapyrimidines, anthraquinones, azines, azoïques, azométhines, benzanthrones, benzimidazoles, benzimidazolones, benzindoles, benzoxazoles, benzopyranes, benzothiazoles, benzoquinones, bisazines, bis isoindolines, carboxanilides, coumarines, cyanines (comme les azacarbocyanines, diazacarbocyanines, diazahémicyanines, hémicyanines, tétraazacarbocyanines), diazines, dicétopyrrolopyrroles, dioxazines, diphénylamines, diphénylméthanes, dithiazines, flavonoïdes tels que flavanthrones et flavones, fluorindines, formazans, hydrazones, en particulier arylhydrazones, hydroxycétones, indamines, indanthrones, indigoides et pseudo-indigoïdes, indophénols, indoanilines, isoindolines, isoindolinones, isoviolanthrones, lactones, méthines, naphthalimides, naphthanilides, naphtholactames, naphthoquinones, nitro, notamment les nitro(hétéro)aromatiques, oxadiazoles, oxazines, périlones, périnones, pérylènes, phénazines, phénothiazines, phthalocyanine, polyènes/caroténoides, porphyrines, pyranthrones, pyrazolanthrones, pyrazolones, pyrimidinoanthrones, pyronines, quinacridones, quinolines, quinophthalones, squaranes, stilbènes, tétrazoliums, thiazines, thioindigo, thiopyronines, triarylméthanes, xanthènes.

A titre de chromophores fluorescents utiles A et/ou A' dans la présente invention, on peut citer les radicaux

issus des colorants acridines, acridones, benzanthrones, benzimidazoles, benzimidazolones, benzindoles, benzoxazoles, benzopyranes, benzothiazoles, coumarines, difluoro{2-[(2*H*-pyrrol-2-ylidène-kN)méthyl]-1H-pyrrolato-kN}bores (BODIPY®), dicétopyrrolo-pyrroles, fluorindines, (poly)méthines (notamment cyanines et styryles/hémicyanines), naphthalimides, naphthanilides, naphthylamine (comme les dansyles), oxadiazoles, oxazines, périlones, périnones, pérylènes, polyènes/caroténoides, squaranes, stilbènes, xanthènes.

On peut également citer les colorants fluorescents A et/ou A' décrits dans les documents EP 1133975, WO 03/029359, EP 860636, WO 95/01772, WO 95/15144, EP 714954 et ceux listés dans l'encyclopédie "The chemistry of synthetic dye" de K. VENKATARAMAN, 1952, Academic press vol 1 à 7, dans l'encyclopédie "Kirk Othmer" "Chemical technology", chapitre "dyes and Dye intermediate", 1993, Wiley and sons, et dans divers chapitres de l'encyclopédie "ULLMANN's ENCYCLOPEDIA of Industrial chemistry" 7th édition, Wiley and sons, dans The Handbook-A Guide to Fluorescent Probes and Labeling Technologies, 10th Ed Molecular Probes/Invitrogen - Oregon 2005 diffusé par Internet ou dans les éditions précédentes imprimées.

Parmi les chromophores nitro A et/ou A' utilisables selon l'invention, on peut citer de manière non limitative les radicaux issus des colorants suivants:

- 1,4-diamino-2-nitrobenzène
- 1-amino-2 nitro-4-β- hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)( β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-βγ-dihydroxypropyloxy-3-méthylamino-4-nitrobenzène
- 1-β-hydroxyéthylamino-4-β,γ-dihydroxypropyloxy-2-nitrobenzène
- 1-βγ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les chromophores azoïques A et/ou A' utilisables selon l'invention, on peut citer les radicaux issus des colorants azoïques cationiques décrits dans les demandes de brevets WO 95/15144, WO 95/01772 et EP-714954.

On peut également citer parmi les chromophores azoïques ceux décrits dans le Colour Index International 3$^{e}$ édition, et notamment les composés suivants :

- Disperse Red 17
- Acid Yellow 9
- Acid Black 1
- Basic Red 22
- Basic Red 76
- Basic Yellow 57

- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les chromophores quinoniques A et/ou A', ceux mentionnés dans le Colour Index International précité conviennent, et parmi ceux-ci, on peut citer entre autres, les radicaux issus des colorants suivants :

- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99 ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(βγ-dihydroxypropylamino)-anthraquinone.

Parmi les chromophores aziniques A et/ou A', conviennent ceux listés dans le Colour Index International et par exemple les radicaux issus des colorants suivants :

- Basic Blue 17
- Basic Red 2.

Parmi les chromophores triarylméthaniques A et/ou A' utilisables selon l'invention, on peut citer, outre ceux listés dans le Colour Index, les radicaux issus des colorants suivants :

- Basic Green 1
- Acid blue 9
- Basic Violet 3
- Basic Violet 14
- Basic Blue 7
- Acid Violet 49
- Basic Blue 26
- Acid Blue 7

Parmi les chromophores indoaminiques A et/ou A' utilisables selon l'invention, on peut citer les radicaux issus des colorants suivants :

- 2-β-hydroxyéthylamino-5-[bis-(β-4'-hydroxyéthyl)amino]anilino-1,4-benzoquinone

- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

On peut aussi citer les chromophores décrits dans les documents US 5888252, EP 1133975, WO 03/029359, EP 860636, WO 95/01772, WO 95/15144, EP 714954. On peut aussi citer ceux listés dans l'encyclopédie "The chemistry of synthetic dye" de K. VENKATARAMAN, 1952, Academic press vol 1 à 7, dans l'encyclopédie "Kirk Othmer" "Chemical technology", chapitre "dyes and Dye intermediate", 1993, Wiley and sons, et dans divers chapitre de l'encyclopédie "ULLMANN's ENCYCLOPEDIA of Industrial chemistry" 7th édition, Wiley and sons.

De préférence, les chromophores A et/ou A' sont choisis parmi ceux issus de colorants de type azoïque, anthraquinone et hydrazone.

De préférence, les chromophores fluorescents A et/ou A' sont choisis parmi ceux issus de colorants de type coumarines, (poly)méthines (notamment cyanines et styryl/hémicyanines) et naphthalimides.

Selon une variante, A et/ou A' des formules (I) contiennent au moins un radical cationique porté par ou inclus dans au moins un des chromophores.

De préférence, le radical cationique est un ammonium quaternaire.

Ces radicaux cationiques sont par exemple un radical alkylammonium, acridinium, benzimidazolium, benzobistriazolium, benzopyrazolium, benzopyridazinium, benzoquinolium, benzothiazolium, benzotriazolium, benzoxazolium, bi-pyridinium, bis-tétrazolium, dihydrothiazolium, imidazopyridinium, imidazolium, indolium, isoquinolium, naphthoimidazolium, naphthooxazolium, naphthopyrazolium, oxadiazolium, oxazolium, oxazolopyridinium, oxonium, phénazinium, phénooxazolium, pyrazinium, pyrazolium, pyrazoyltriazolium, pyridinium, pyridinoimidazolium, pyrrolium, pyrylium, quinolium, tétrazolium, thiadiazolium, thiazolium, thiazolopyridinium, thiazoylimidazolium, thiopyrylium, triazolium ou xanthylium.

Des exemples de chromophores cationiques utiles dans la présente invention ont été cités précédemment. D'autres exemples sont donnés dans les demandes de brevet WO 95/01772, WO 95/15144, EP 714954, EP 318294, WO 03/029359,

Selon un mode de réalisation particulier, les radicaux A, A' dans les formules (I) ou (II), comprennent au moins un chromophore azoïque cationique, décrit par exemple dans EP 850636, FR 2788433, EP 920856, WO 9948465, FR 2757385, EP 850637, EP 918053, WO 9744004, FR 2570946, FR 2285851, DE 2538363, FR 2189006, FR 1560664, FR 1540423, FR 1567219, FR 1516943, FR 1221122, DE 4220388, DE 4137005, WO 0166646, US 5708151, WO 9501772, WO 515144, GB 1195386, US 3524842, US 5879413, EP 1062940, EP 1133976, GB 738585, DE 2527638, FR 2275462, GB 1974-27645, Acta Histochem. (1978), 61(1), 48-52 ; Tsitologiya (1968), 10(3), 403-5 ; Zh. Obshch. Khim. (1970), 40(1), 195-202 ; Ann. Chim. (Rome) (1975), 65(5-6), 305-14 ; Journal of the Chinese Chemical Society (Taipei) (1998), 45(1), 209-211 ; Rev. Roum. Chim. (1988), 33(4), 377-83 ; Text. Res. J. (1984), 54(2), 105-7 ; Chim. Ind. (Milan) (1974), 56(9), 600-3 ; Khim. Tekhnol. (1979), 22(5), 548-53 ; Ger. Monatsh. Chem. (1975), 106(3), 643-8 ; MRL Bull. Res. Dev. (1992), 6(2), 21-7 ; Lihua Jianyan, Huaxue Fence (1993), 29(4), 233-4 ; Dyes Pigm. (1992), 19(1), 69-79 ; Dyes Pigm. (1989), 11(3), 163-72.

A titre d'exemple on peut citer les colorants suivants :

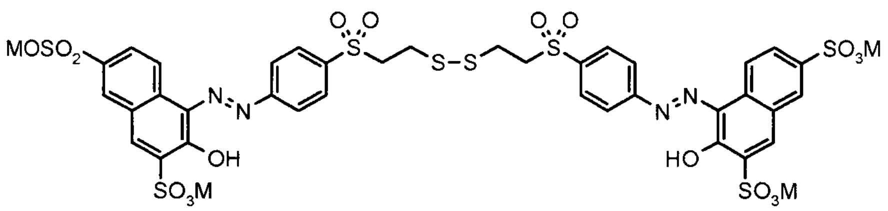

avec M représentant : un atome d'hydrogène, un métal alcalin ou un groupement ammonium ou un groupement ammonium substitué par un ou plusieurs radicaux alkyle, identiques ou non, linéaires ou ramifiés, en $C_1$-$C_{10}$ éventuellement porteurs d'au moins un hydroxy.

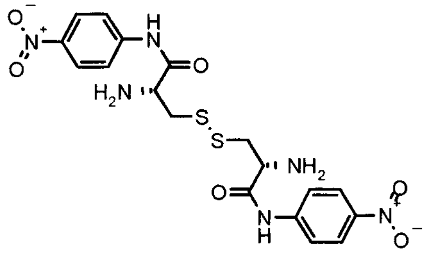

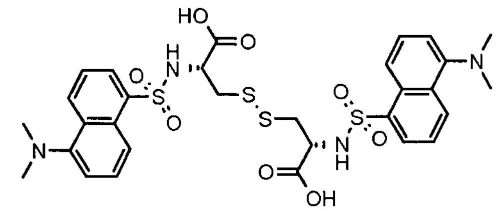

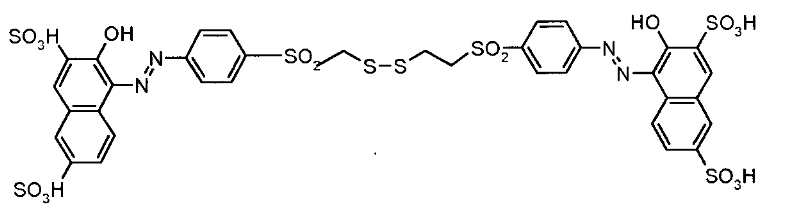

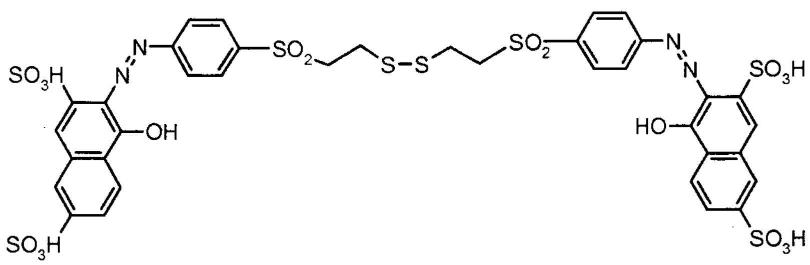

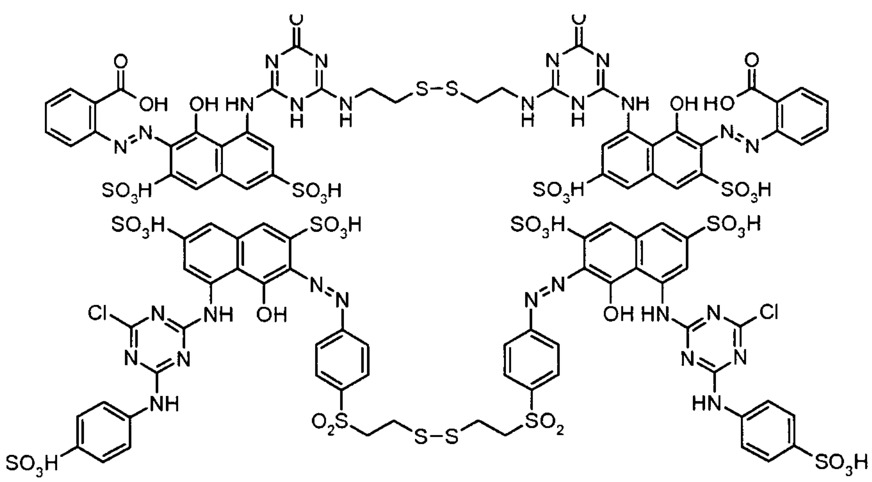

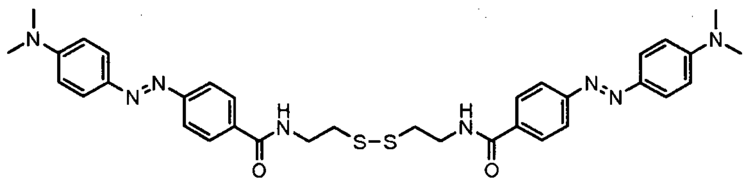

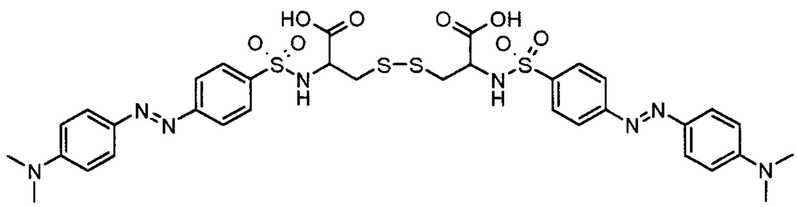

Ces composés particuliers, indépendamment de tout choix de forme libre ou salifiée, ainsi que leur mode de préparation sont connus de la technique.

A titre d'exemples particuliers on peut également citer les composés suivants, nouveaux et dont une préparation est décrite dans la présente invention

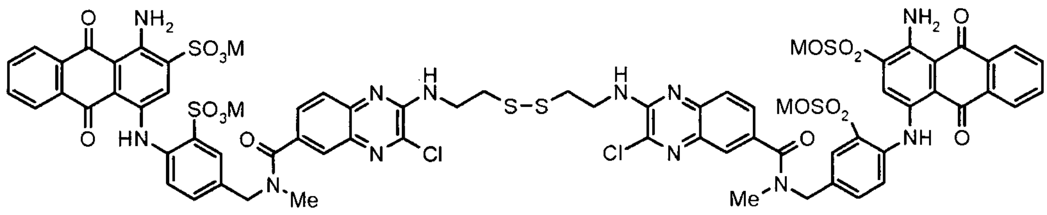

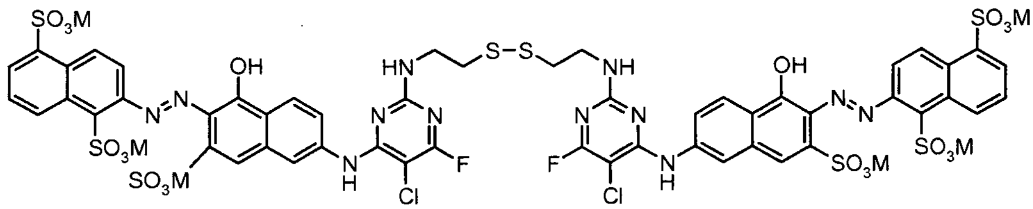

M ayant la même signification que précédemment.

Selon une autre variante de l'invention, le colorant direct à fonction disulfure est un colorant cationique comprenant au moins un radical ammonium quaternaire et tel que, dans la formule (I) avec p et p' égaux à 1 ; **A** et/ou **A'**, identiques ou différents, plus préférentiellement identiques, représentent W-N=N-Ar- ou Ar-N=N-W-, avec W représentant un hétéroaryle condensé ou non, comprenant un ammonium quaternaire, éventuellement substitué de préférence par un ou plusieurs groupe alkyle ; Ar représente un radical (hétéro)aryle à 5 ou 6 chaînons de type phényle, ou un bicycle (hétéro)aromatique de type naphtyle, benzopyrydyle, indolinyle, ou benzoindolinyle, éventuellement substitués par un ou plusieurs atomes d'halogène, de préférence chlore, fluor ; par un ou plusieurs groupements alkyle, de préférence en $C_1$-$C_4$ ; par un ou plusieurs groupements hydroxy ; par un ou plusieurs groupements alkoxy, par un ou plusieurs groupements hydroxyalkyle, par un ou plusieurs groupements amino ou (di)alkylamino, de préférence avec la partie alkyle en $C_1$-$C_4$

Selon une variante p=1, z=t'=0, t =1 et T représente -N(R)-, de préférence en position para sur Ar par rapport à la fonction azoïque.

Particulièrement dans une variante p=1, z=t'=0, t =1 et T représente -N(R)-, de préférence en position para sur Ar par rapport à la fonction azoïque et T' représente un groupement -N(R)-, $-N^+(R)(R°)-$ ou un imidazolium.

Selon un autre mode de réalisation particulier de l'invention, le colorant disulfure est un colorant fluorescent cationique comprenant au moins un chromophore fluorescent et un radical ammonium quaternaire tel que défini dans

la formule (I) avec p et p' égaux à 1 : **A** et/ou **A',** identiques ou différents, plus préférentiellement identiques, représentent W-C($R^c$)=C($R^d$)-Ar- ou -W-C($R^c$)=C($R^d$)-Ar, avec W représentant un hétérocycle ou un hétéroaryle, comprenant un ammonium quaternaire ; Ar représente un radical (hétéro)aryle à 5 ou 6 chaînons de type phényle ou pyridium, ou un bicycle (hétéro)aromatique de type naphtyle, benzopyrydinium, indolinyle, ou benzoindolinyle, éventuellement substitués par un ou plusieurs atomes d'halogène, de préférence chlore, fluor ; par un ou plusieurs groupements alkyle, de préférence en $C_1$-$C_4$ ; par un ou plusieurs groupements hydroxy ; par un ou plusieurs groupements alkoxy, par un ou plusieurs groupements hydroxyalkyle, par un ou plusieurs groupements amino ou (di)alkylamino, de préférence avec la partie alkyle en $C_1$-$C_4$, par un ou plusieurs groupements acylamino; par un ou plusieurs groupements hétérocycloalkyle ou hétéroaryle à 5 ou 6 chaînons préférentiellement choisi parmi pyrrolidinyle, pypérazinyle, pypéridinyle et imidazolinyle; $R^c$, $R^d$, identiques ou différents, représentent un atome d'hydrogène ou un groupement alkyle en $C_1$-$C_4$.

Selon une autre variante le colorant disulfure est un colorant fluorescent cationique comprenant au moins un radical ammonium quaternaire et tel que, dans la formule (I) avec p et p' égaux à 1 et A et/ou A' représentant un radical naphthalimidyle de formule :

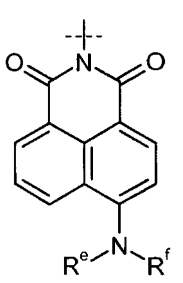

ou

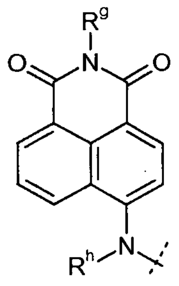

avec représentant la liaison avec le groupement X ou X', $C_{sat}$ ou $C'_{sat}$

dans lequel $R^e$, $R^f$, $R^g$,et $R^h$, identiques ou différents, représentent un atome d'hydrogène ou un groupement alkyle en $C_1$-$C_6$ éventuellement substitué.

Selon un mode de réalisation de l'invention p=1, z=t'=0, t =1 et T représente -N(R)-, de préférence en position para sur Ar par rapport à la fonction oléfine -C($R^c$)=C($R^d$)-.

Particulièrement dans une variante p=1, z=t'=0, t =1 et T représente -N(R)-, de préférence en position para sur Ar par rapport à la fonction oléfine -C($R^c$)=C($R^d$)- et T' représente un groupement -N(R)-, -$N^+$(R)(R°)- ou un imidazolium.

De préférence, W est un imidazolium, pyridinium, benzimidazolium, pyrazolium, benzothiazolium et quinolinium éventuellement substitués par un ou plusieurs radicaux alkyle, identiques ou non, en $C_1$-$C_4$.

A titre d'exemple de colorants de l'invention on peut citer les colorants disulfures choisis parmi les formules **(III)** à **(XIX)** suivantes:

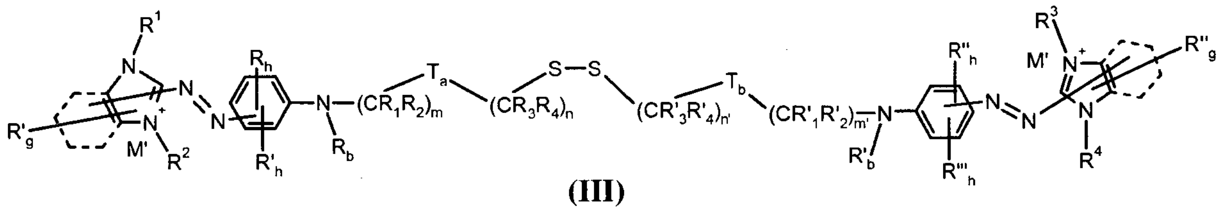

**(III)**

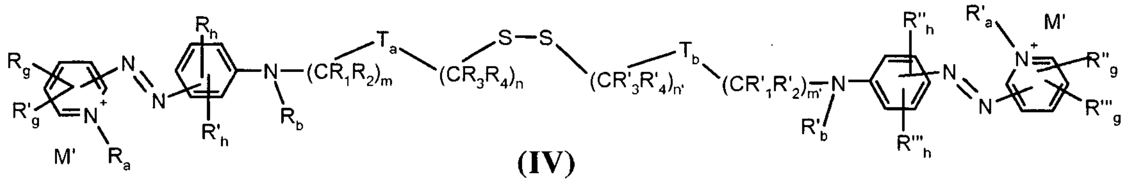

(IV)

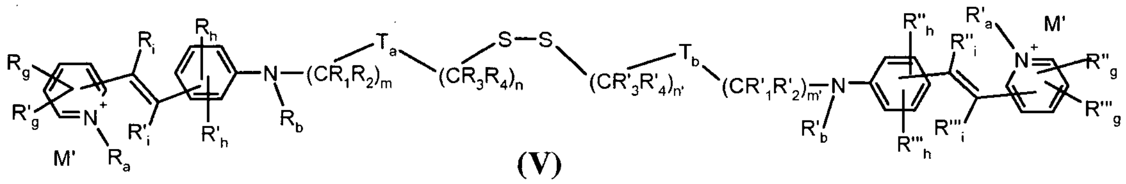

(V)

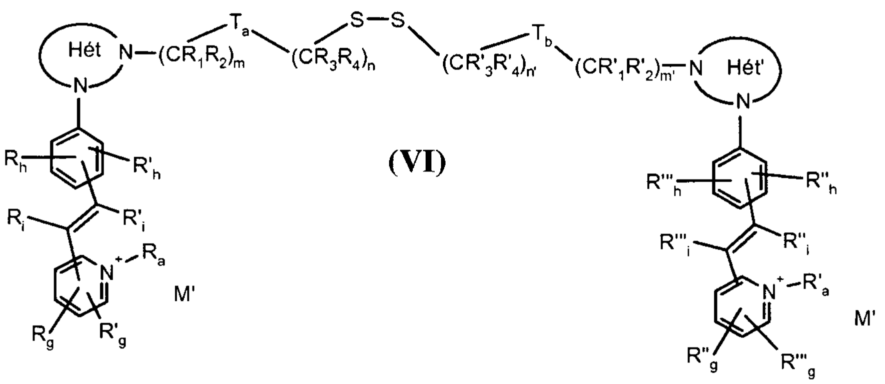

(VI)

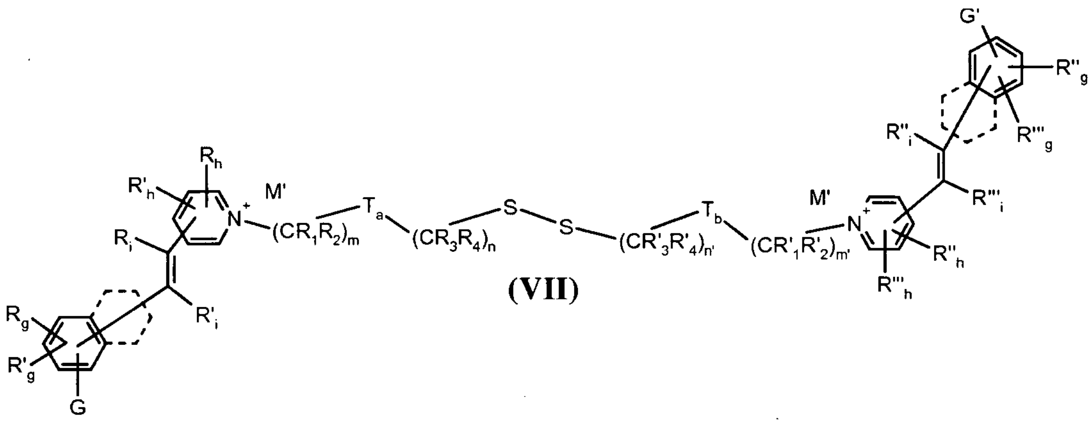

(VII)

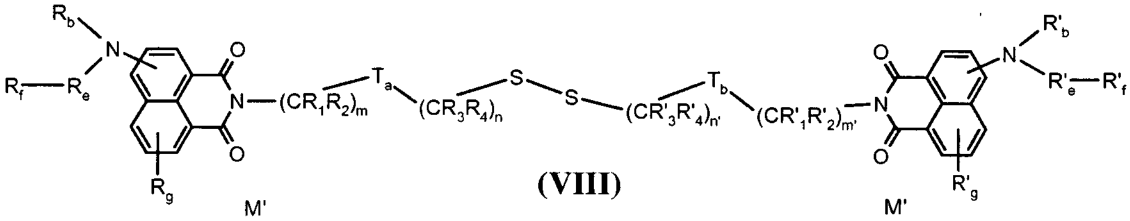

**(VIII)**

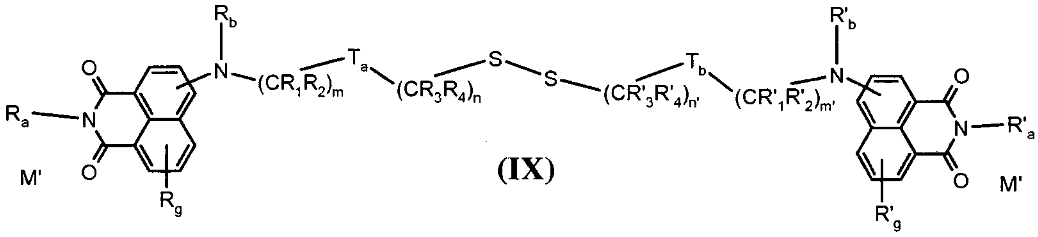

**(IX)**

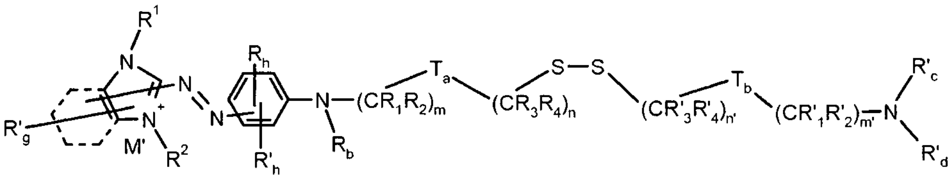

**(X)**

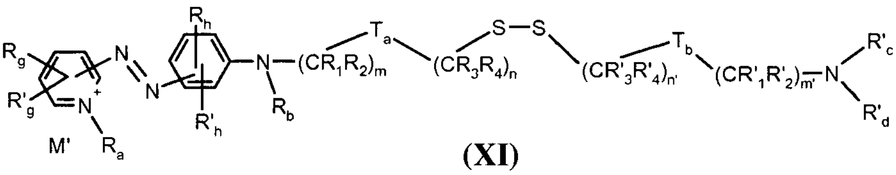

**(XI)**

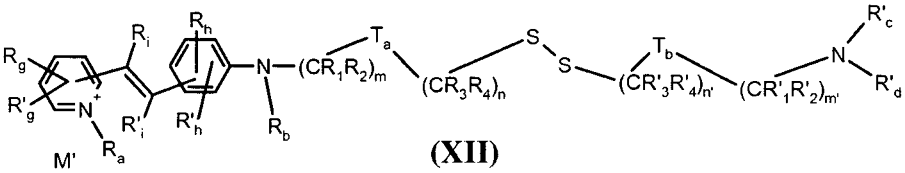

**(XII)**

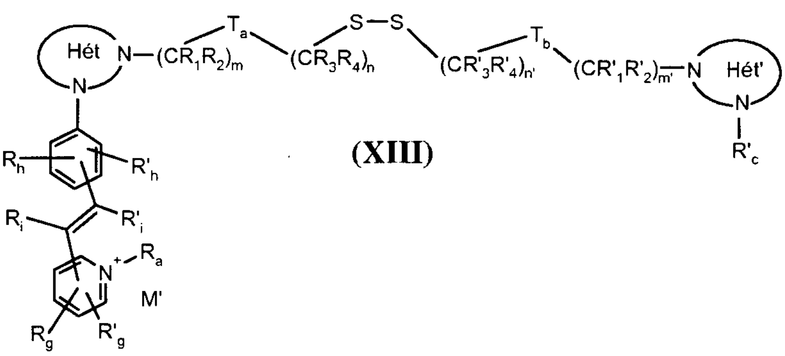

**(XIII)**

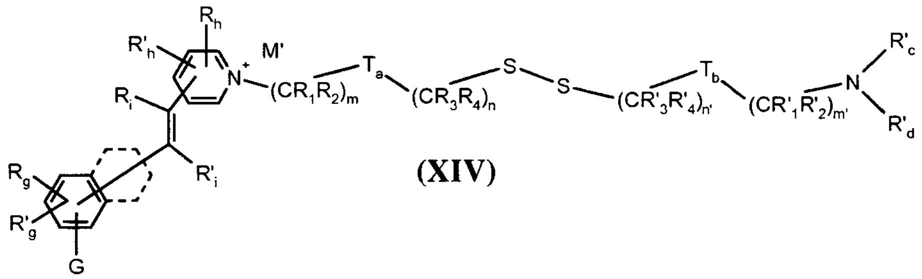

(XIV)

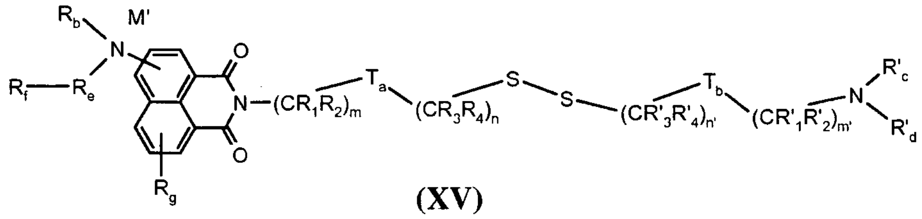

(XV)

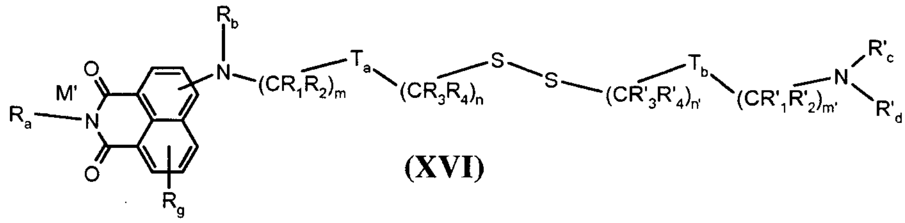

(XVI)

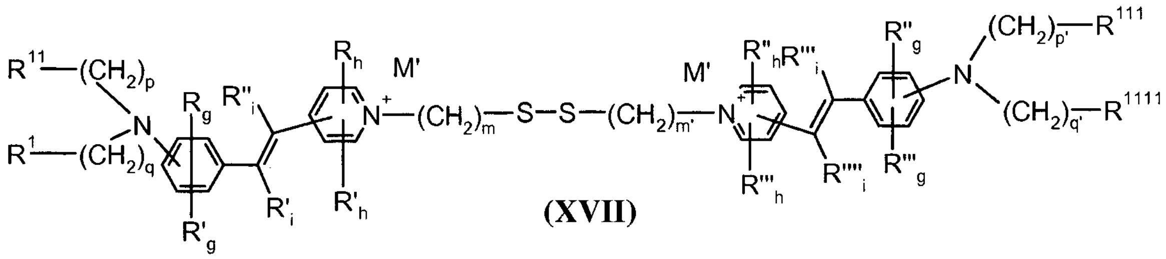

(XVII)

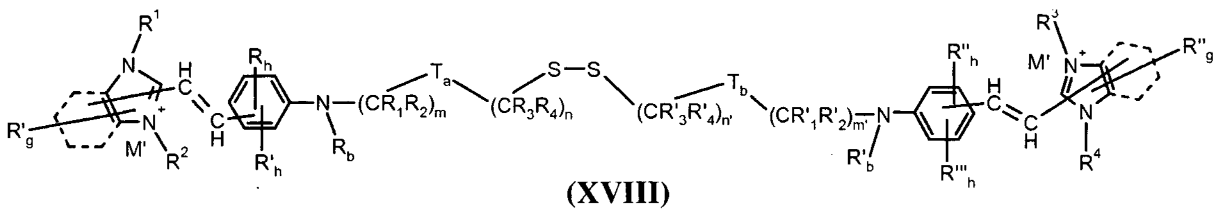

(XVIII)

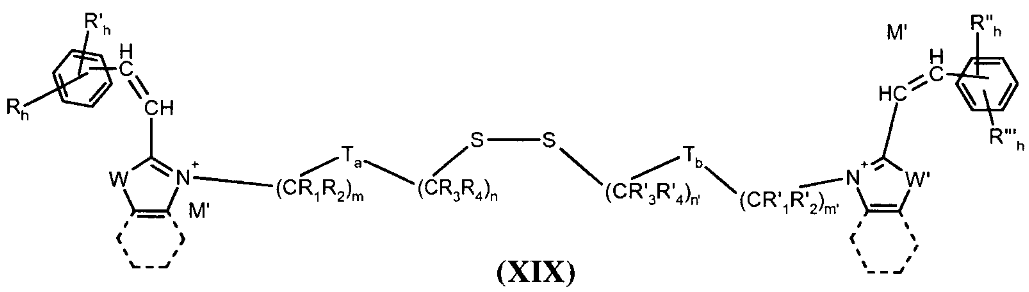

(XIX)

dans lequel

- G et G', identiques ou différents, représentent un groupement $-NR_cR_d$, $-NR'_cR'_d$, ou $C_1$-$C_6$ alkoxy éventuellement substitué, préférentiellement non substitué ; préférentiellement G et G' représentent un groupement $-NR_cR_d$ et $-NR'_cR'_d$ respectivement ;
- W et W', identiques ou différents, représentent un atome d'oxygène ou un groupement N-R1, avec R1 tel que défini ci après ;
- $R^1$, $R^2$, $R^3$ et $R^4$, identiques ou différents, représentent un atome d'hydrogène ou ub groupement alkyle $C_1$-$C_6$ alkyle ;
- $R_a$ et $R'_a$, identiques ou différents, représentent un groupement aryl($C_1$-$C_4$)alkyle ou un groupement $C_1$-$C_6$ alkyle éventuellement substitué par un groupement hydroxy ou amino, $C_1$-$C_4$ alkylamino, $C_1$-$C_4$ dialkylamino, lesdits radicaux alkyles pouvant former avec l'atome d'azote qui les portent un hétérocycle comprenant de 5 à 7 chaînons, comprenant éventuellement un autre hétéroatome différent ou non de l'azote ; préférentiellement $R_a$ et $R'_a$ représentent un groupement $C_1$-$C_3$ alkyle éventuellement substitué par un groupement hydroxy, ou un groupement benzyle ;
- $R_b$, $R'_b$, identiques ou différents, représentent un atome d'hydrogène, un groupement aryl($C_1$-$C_4$)alkyle ou un groupement $C_1$-$C_6$ alkyle éventuellement substitué; préférentiellement $R_b$, $R'_b$ représentent un atome d'hydrogène ou un groupement $C_1$-$C_3$ alkyle ou benzyle ;
- $R_c$, $R'_c$, $R_d$ et $R'_d$, identiques ou différents, représentent un atome d'hydrogène, un groupement aryl($C_1$-$C_4$)alkyle, $C_1$-$C_6$ alkoxy ou un groupement $C_1$-$C_6$ alkyle éventuellement substitué ; $R_c$, $R'_c$, $R_d$ et $R'_d$ représentent préférentiellement un atome d'hydrogène, un groupement hydroxy, $C_1$-$C_3$ alkoxy, amino, $C_1$-$C_3$ (di)alkylamino, ou un groupement $C_1$-$C_3$ alkyle éventuellement substitué par i) un groupement hydroxy, + ii) amino, iii) $C_1$-$C_3$ (di)alkylamino, ou iv) ammonium quaternaire (R")(R''')(R'''')$N^+$-;

ou alors deux radicaux adjacents $R_c$ et $R_d$, $R'_c$ et $R'_d$ portés par le même atome d'azote forment ensemble un groupe hétérocyclique ou hétéroaryle ; préférentiellement l'hétérocycle ou l'hétéroaryle est monocyclique et comprend entre 5 et 7 chaînons ; plus préférentiellement les groupements sont choisis parmi l'imidazolyle et le pyrrolidinyle ;

- $R_e$ et $R'_e$, identiques ou différents, représentent une chaîne hydrocarbonée en $C_1$-$C_6$ alkylènyle divalente, linéaire ou ramifiée, éventuellement insaturée ;
- $R_f$ et $R'_f$, identiques ou différents, représentent un groupement ammonium + quaternaire (R")(R''')(R'''')$N^+$- où R", R''' et R'''', identiques ou différents, représentent un atome d'hydrogène ou un groupement $C_1$-$C_4$ alkyle ou alors + (R")(R''')(R'''')N - représente un groupement hétéroaryle cationique éventuellement substitué préférentiellement un groupement imidazolinium éventuellement substitué par un groupement $C_1$-$C_3$ alkyle ;
- Rg, R'g, $R''_g$, $R'''_g$, $R_h$, $R'_h$, $R''_h$, et $R'''_h$, identiques ou différents, représentent un atome d'hydrogène, un atome d'halogène, un groupement amino, $C_1$-$C_4$ alkylamino, $C_1$-$C_4$ dialkylamino, cyano, carboxy, hydroxy, trifluorométhyle, un radical acylamino, alcoxy en $C_1$-$C_4$, (poly)hydroxyalcoxy en $C_2$-$C_4$, alkylcarbonyloxy alcoxycarbonyle, alkylcarbonylamino, un radical acylamino, carbamoyle , alkylsulfonylamino, un radical amino-sulfonyle, ou un radical $C_1$-$C_{16}$ alkyle éventuellement substitué par un groupement choisi parmi $C_1$-$C_{12}$ alcoxy, hydroxy, cyano, carboxy, amino, $C_1$-$C_4$ alkylamino et $C_1$-$C_4$ dialkylamino, ou alors les deux radicaux alkyles portés par l'atome d'azote du groupement amino forment un hétérocycle comprenant de 5 à 7 chaînons et comprenant éventuellement un autre hétéroatome identique ou différent à celui de l'atome d'azote ; préférentiellement Rg, R'g, $R''_g$, $R'''_g$, $R_h$, $R'_h$, $R''_h$, et $R'''_h$ représentent un atome d'hydrogène, d'halogène ou un groupement $C_1$-$C_3$ alkyle ;
- ou alors deux groupements Rg et R'g; R"g et R'''g ; $R_h$, et $R'_h$ ; $R''_h$ et $R'''_h$ portés par deux atomes de carbone adjacents, forment ensembles une cycle benzo, indéno, un groupement hétérocycloalkyle fusionné ou hétéroaryle fusionné; le cycle benzo, indéno, hétérocycloalkyle ou hétéroaryle étant éventuellement substitué par un atome d'halogène, un groupement amino, $C_1$-$C_4$ alkylamino, $C_1$-$C_4$ dialkylamino, nitro, cyano, carboxy, hydroxy, trifluorométhyle, un radical acylamino, alcoxy en $C_1$-$C_4$, (poly)hydroxyalcoxy en $C_2$-$C_4$, alkylcarbonyloxy alcoxycarbonyle, alkylcarbonylamino, un radical acylamino, carbamoyle , alkylsulfonylamino, un radical amino-sulfonyle, ou un radical $C_1$-$C_{16}$ alkyle éventuellement substitué par : un groupement choisi parmi $C_1$-$C_{12}$ alcoxy, hydroxy, cyano, carboxy, amino, $C_1$-$C_4$ alkylamino, $C_1$-$C_4$ dialkylamino, ou alors les deux radicaux alkyles portés par l'atome d'azote du groupement amino forment un hétérocycle comprenant de 5 à 7 chaînons et comprenant éventuellement un autre hétéroatome identique ou différent à celui de l'atome d'azote ; préférentiellement Rg et R'g; R"g et R'''g forment ensemble un groupement benzo ;
- ou alors lorsque G représente $-NR_cR_d$ et G' représente $-NR'_cR'_d$ deux groupements $R_c$ et R'g ; $R'_c$ et R"g ; $R_d$ et Rg ; $R'_d$ et R'''g forment ensemble un hétéroaryle ou hétérocycle saturé, éventuellement substitué par un ou plusieurs groupement $C_1$-$C_6$ alkyle, préférentiellement un l'hétérocycle contenant un ou deux hétéroatomes choisis parmi l'azote et l'oxygène et comprenant entre 5 et 7 chaînons ; plus préférentiellement l'hétérocycle est choisi parmi les groupement morpholinyle, pypérazinyle, pypéridinyle et pyrrolidinyle ;

- $R_i$, $R'_i$, $R''_i$, et $R'''_i$, identiques ou différents, représentent un atome d'hydrogène, ou un groupement $C_1$-$C_4$ alkyle ;
- $R_1$, $R_2$, $R_3$, $R_4$, $R'_1$, $R'_2$, $R'_3$, et $R'_4$, identiques ou différents, représentent un atome d'hydrogène ou un groupement $C_1$-$C_4$ alkyle, $C_1$-$C_{12}$ alkoxy, hydroxy, cyano, carboxy, amino, $C_1$-$C_4$ alkylamino, $C_1$-$C_4$ dialkylamino, lesdits radicaux alkyles pouvant former avec l'atome d'azote qui les portent un hétérocyle comprenant de 5 à 7 chaînons, comprenant éventuellement un autre hétéroatome différent ou non de l'azote ; préférentiellement $R_1$, $R_2$, $R_3$, $R_4$, $R'_1$, $R'_2$, $R'_3$, et $R'_4$ sont des atomes d'hydrogène ou un groupement amino ; plus préférentiellement $R_1$, $R_2$, $R_3$, $R_4$, $R'_1$, $R'_2$, $R'_3$, et $R'_4$ représentent un atome d'hydrogène;
- $T_a$, $T_b$, identiques ou différents, représentent i) soit une liaison covalente $\sigma$, ii) soit un ou plusieurs radicaux ou leurs combinaisons choisis parmi $-SO_2-$, -O-, -S-, - N(R)-, $-N^+(R)(R°)-$, -CO-, avec R, R°, identiques ou différents, représentant un atome d'hydrogène, un radical alkyle en $C_1$-$C_4$, hydroxyalkyle en $C_1$-$C_4$; ou un aryl($C_1$-$C_4$)alkyle, préférentiellement $T_a$ est identique à $T_b$ et représentent une liaison covalente $\sigma$ ou un groupement choisi parmi -N(R)-, -C(O)-N(R)-, -N(R)-C(O)-, -O-C(O)-, -C(O)-O- et $-N^+(R)(R°)-$, avec R, R° identiques ou différents représentant un atome d'hydrogène ou un groupement $C_1$-$C_4$ alkyle ; plus préférentiellement $T_a$ et $T_b$ représentent une liaison $\sigma$ ; iii) soit un radical hétérocycloalkyle ou hétéroaryle, cationique ou non, préférentiellement monocycliques, préférentiellement identiques, contenant préférentiellement deux hétéroatomes (plus préférentiellement deux atomes d'azote) et comportant préférentiellement de 5 à 7 chaînons tel que l'imidazolium; •

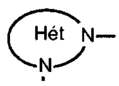 ou 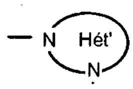

identiques ou différents, représentent un groupement hétérocyclique éventuellement substitué ; préférentiellement les hétérocycles sont identiques, monocycliques, saturés, et comprennent au total deux atomes d'azote et de 5 à 8 chaînons ; •

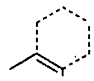

représentent un groupement aryle ou hétéroaryle fusionné au cycle imidazolium ou phényle ; ou alors est absent du cycle imidazolium ou phényle; préférentiellement lorsque le cycle est présent le cycle est un benzo ;
- $R^1$ et $R^{1111}$, identiques ou différents, représentent un groupement hydroxy, un groupement amino ($NR_aR_b$) ou ammonium ($N^+R_aR_bR_c$), $An^-$ ; avec $R_a$, $R_b$ et $R_c$, identiques ou différents, représentant un atome d'hydrogène ou un groupement ($C_1$-$C_4$)alkyle ; ou alors deux groupes alkyle $R_a$ et $R_b$ du groupement amino ou ammonium forment un hétérocycle comprenant de 5 à 7 chaînons et comprenant éventuellement un autre hétéroatome identique ou différent à celui de l'atome d'azote tel que morpholinyle, pipérazinyle, pipéridinyle, pyrrolyle, morpholinium, pipérazinium, pipéridinium, pyrrolinium, et $An^-$ représentant un contre-ion anionique ;
- $R^{11}$ et $R^{111}$, identiques ou différents, représentent un atome d'hydrogène ou un groupement tel que défini pour $R^1$ et $R^{1111}$ respectivement ;
- m, m', n et n', identiques ou différents, représentent un entier compris inclusivement entre 0 et 6 avec m+n, m'+n', identiques ou différents, représente un entier compris inclusivement entre 1 et 10; préférentiellement m+n=m'+n'=un entier compris inclusivement entre 2 et 4 ; plus préférentiellement m+n=m'+n'= un entier égal à 2; M' représentant contre-ion anionique, issu de sel d'acide organique ou minéral, ou d'une base organique ou minérale assurant l'électroneutralité de la molécule.

A titre d'exemple les colorants directs disulfures de l'invention, il est à mentionner ceux de structures chimiques suivantes:

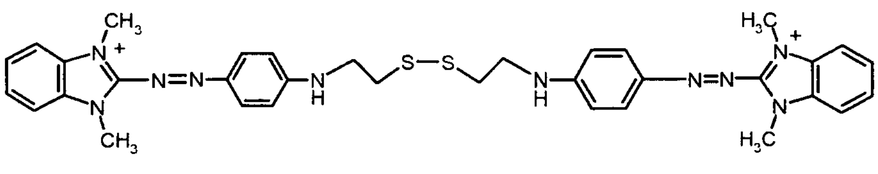

2M'

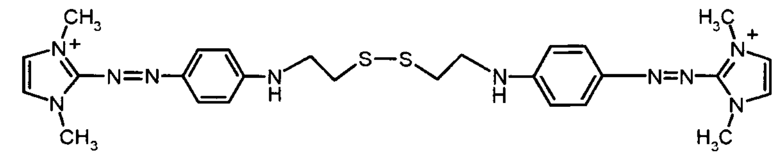

2M'

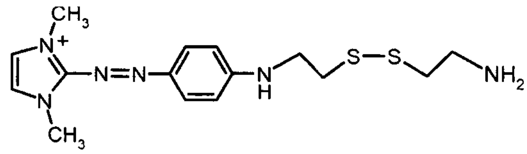

M'

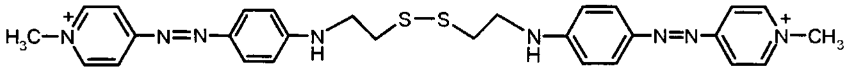

2M'

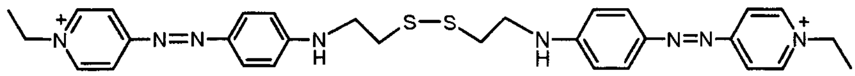

2M'

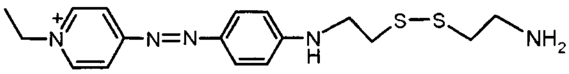

M'

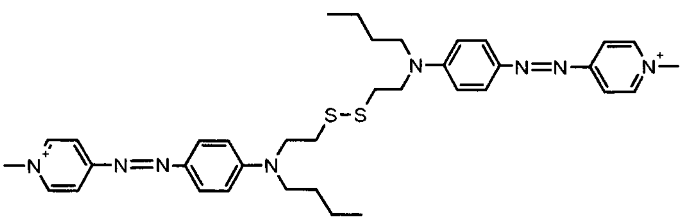

2M'

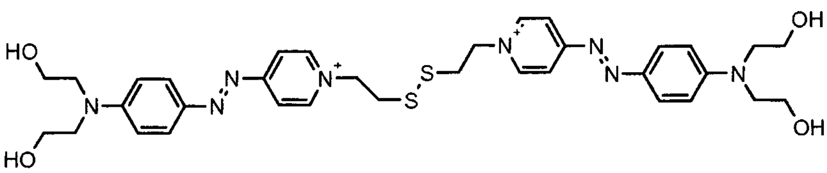

2M'

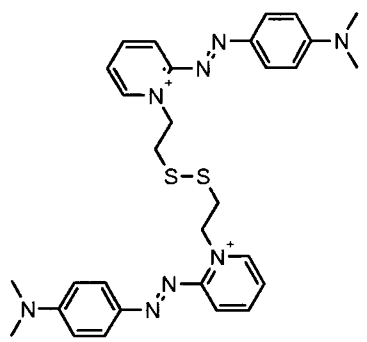

2M'

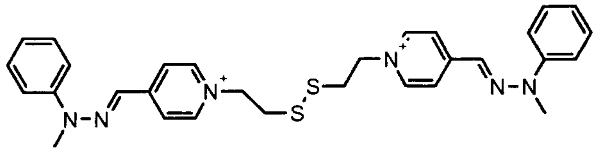

2M'

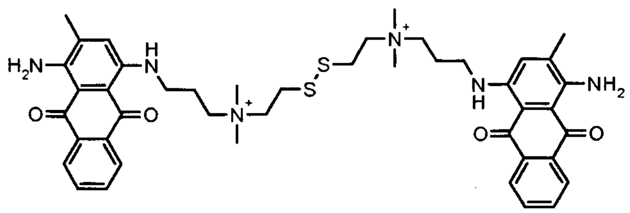

2M'

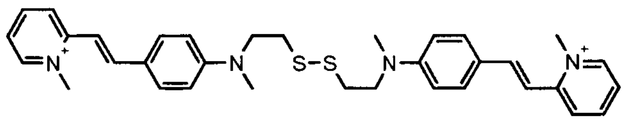

2M'

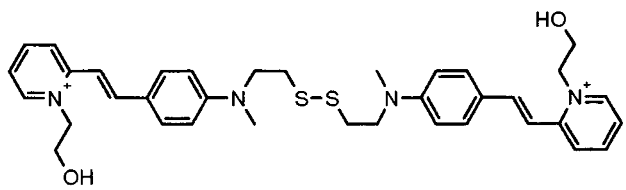

2M'

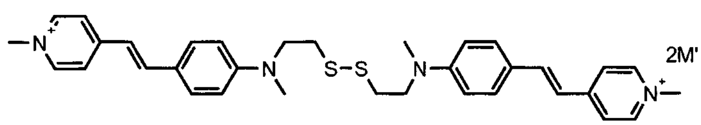

2M'

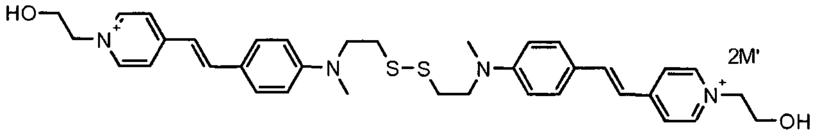

2M'

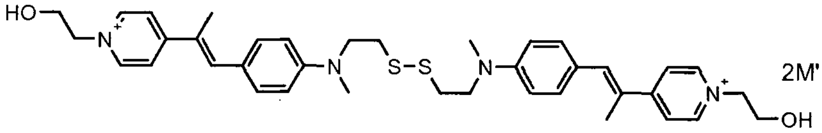

2M'

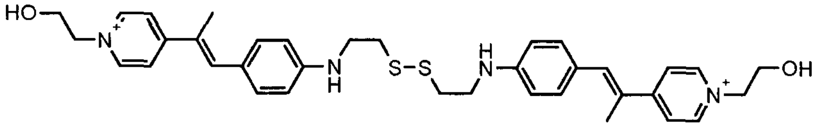

2M'

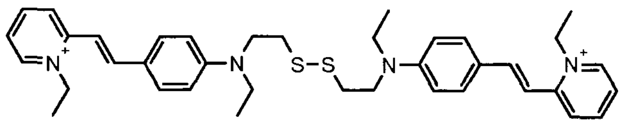

2M'

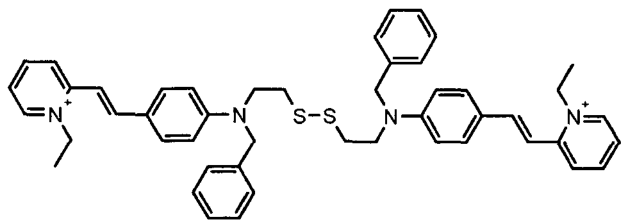

2M'

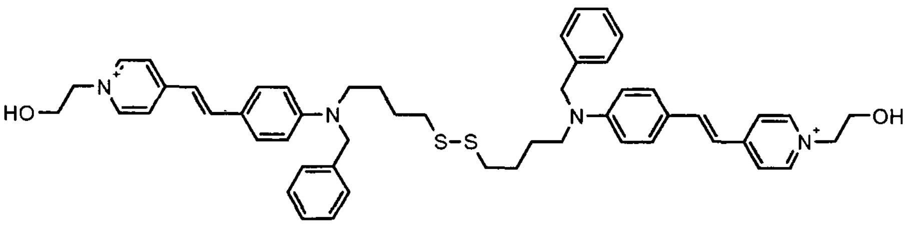

2M'

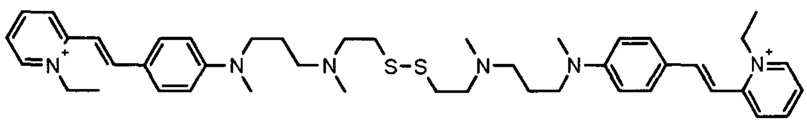

2M'

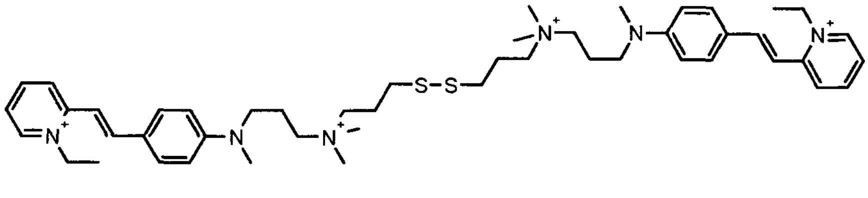

4M'

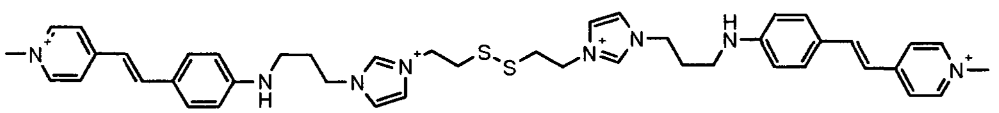

4M'

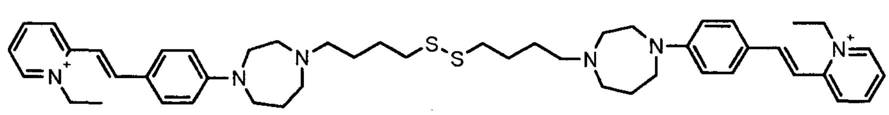

2M'

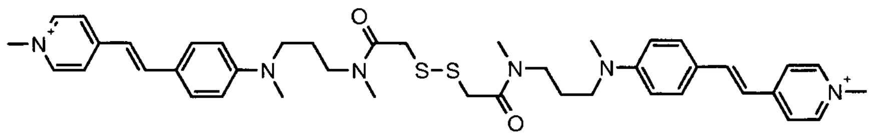

2M'

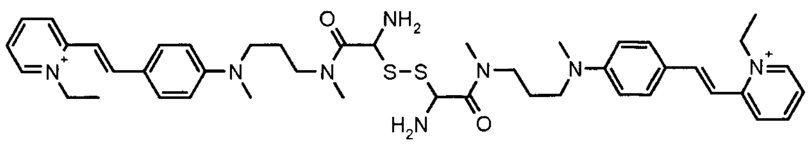

2M'

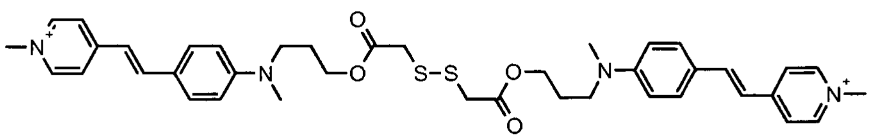

2M'

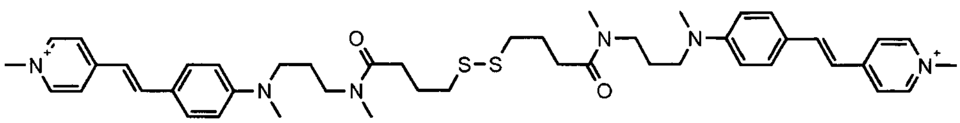

2M'

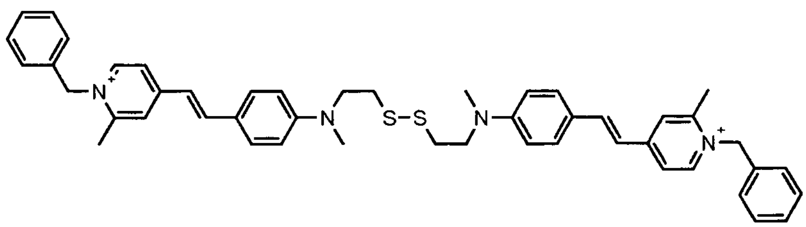

2M'

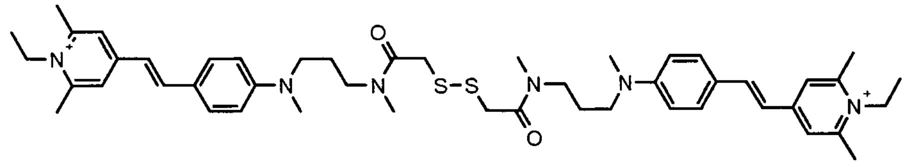

2M'

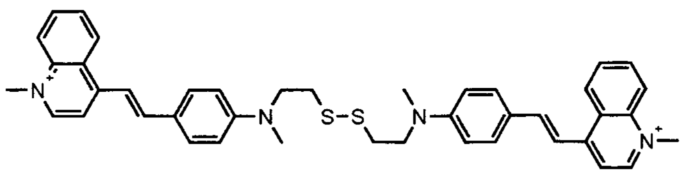

2M'

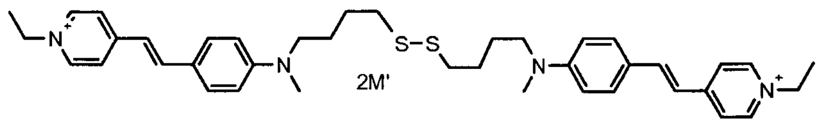

2M'

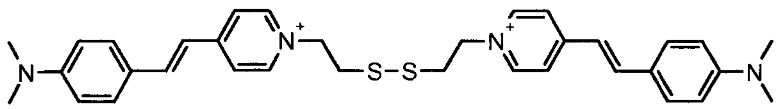

2M'

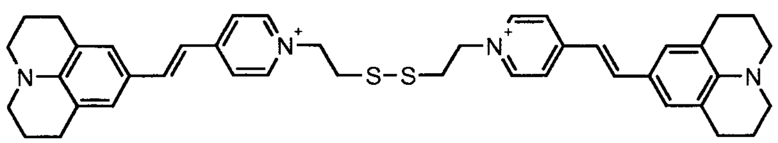

2M'

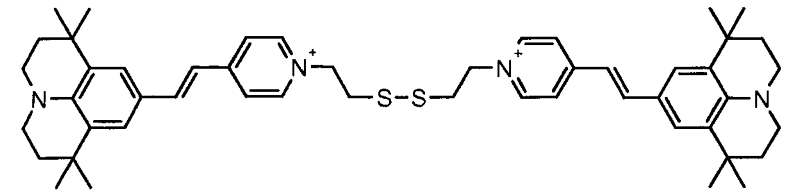

2M'

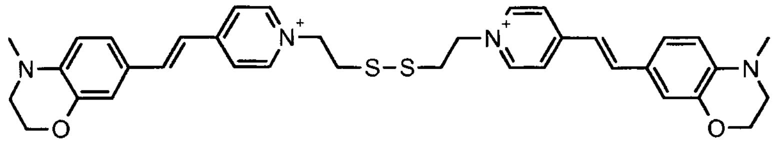

2M'

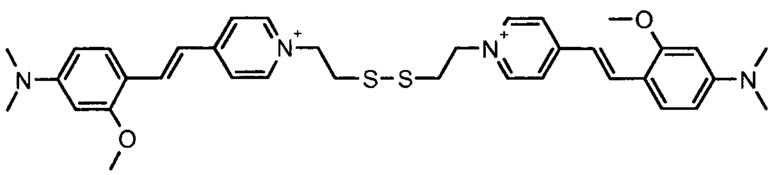

2M'

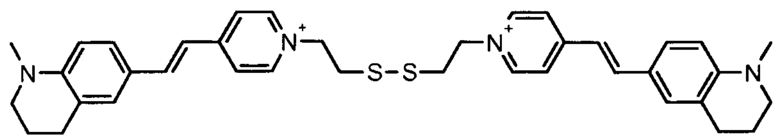

2M'

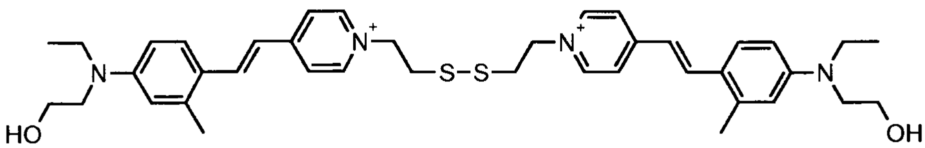

2M'

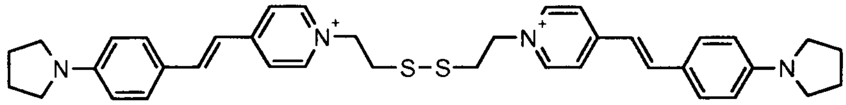 2M'

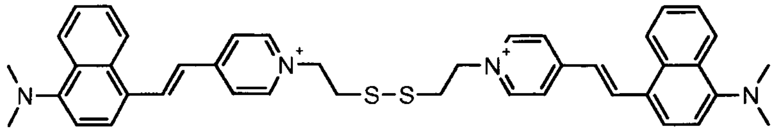 2M'

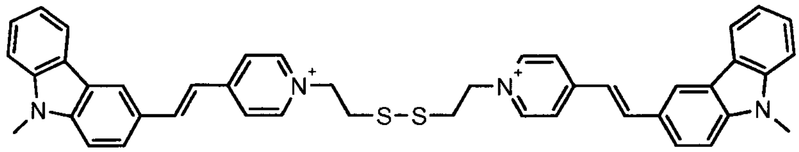 2M'

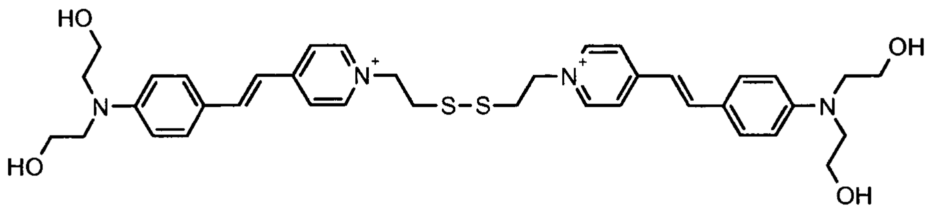 2M'

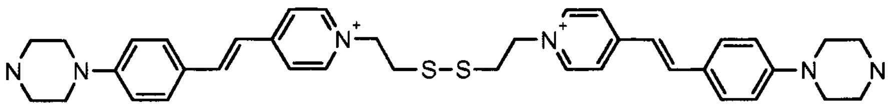 2M'

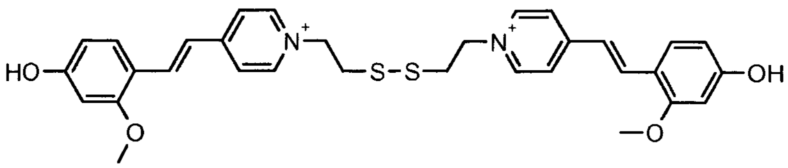 2M'

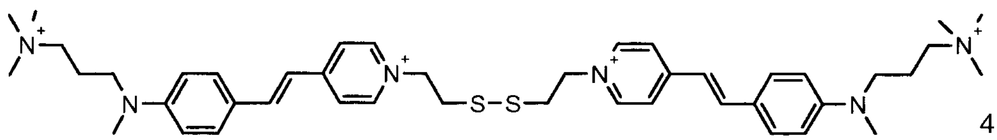 4M'

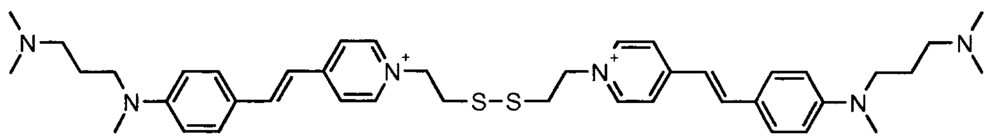 2M'

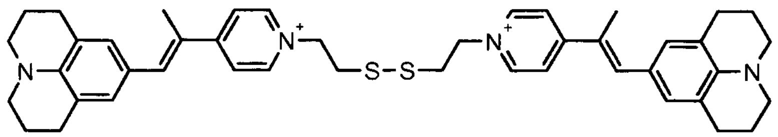

2M'

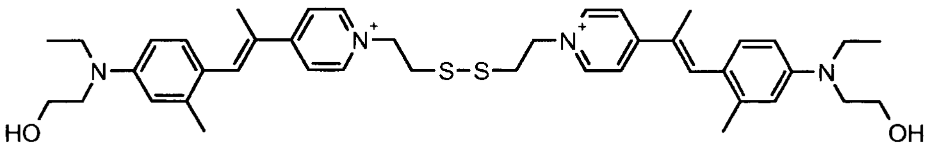

2M'

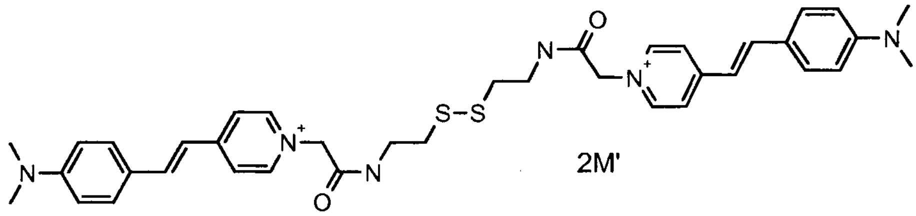

2M'

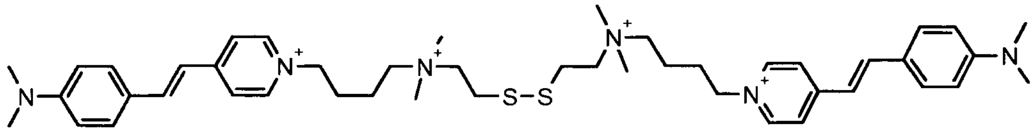

4M'

2M'

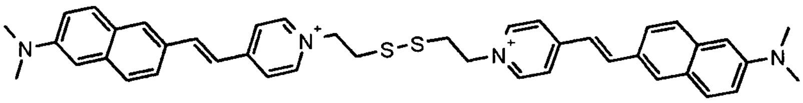

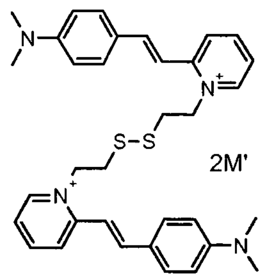

2M'

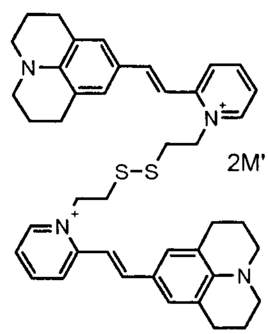

2M'

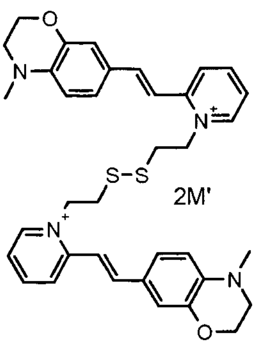

2M'

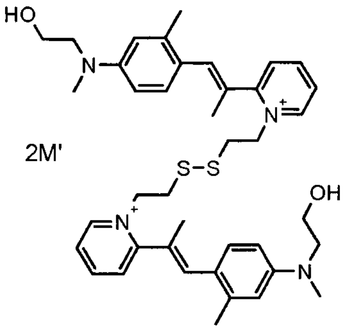

2M'

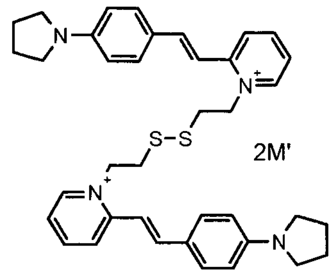

2M'

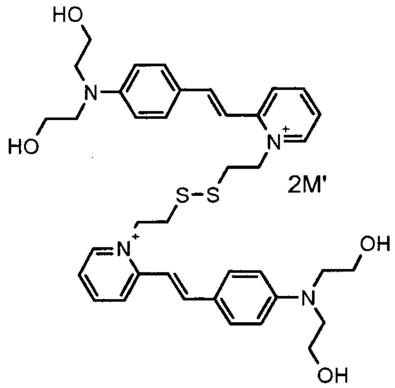

2M'

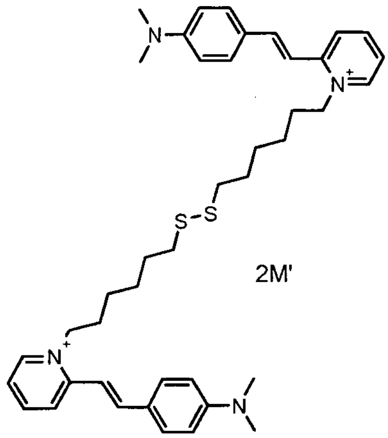

2M'

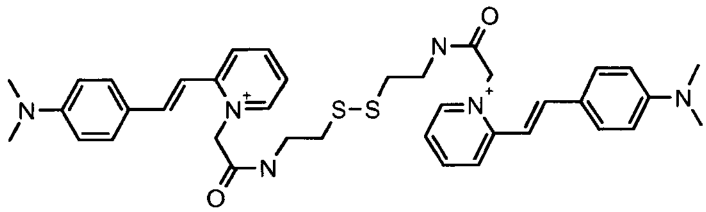

2M'

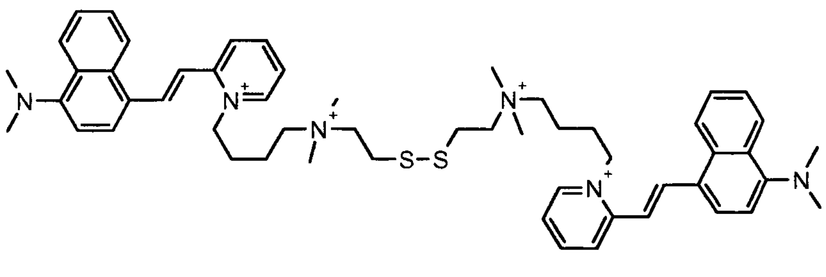

4M'

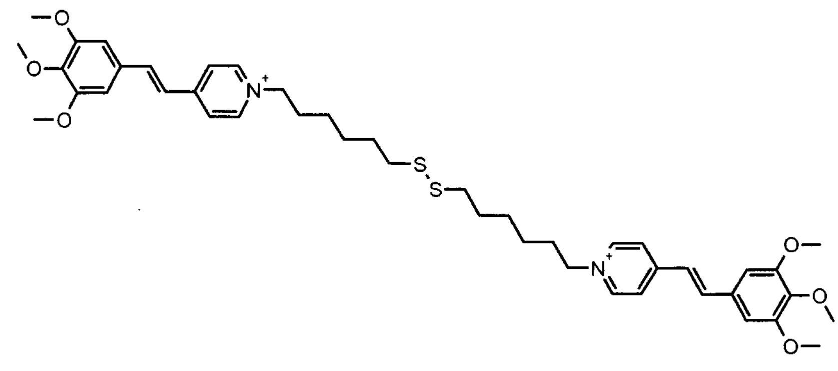

An$^-$

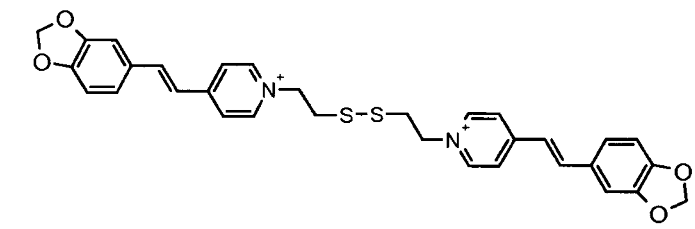

An$^-$

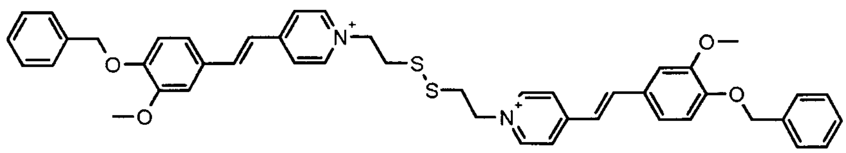

An$^-$

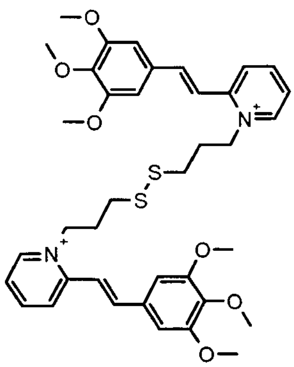

An$^-$

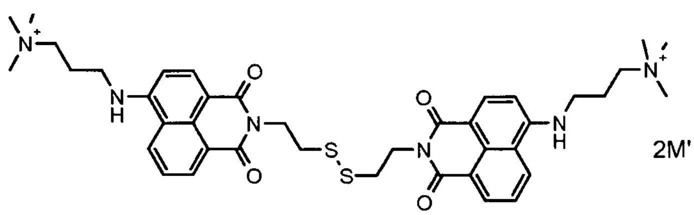

2M'

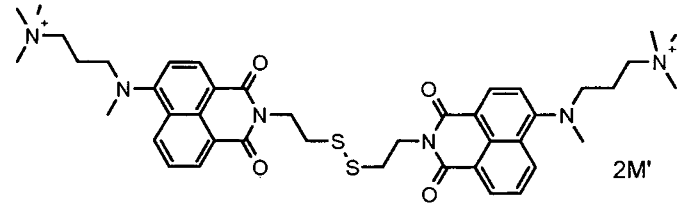

2M'

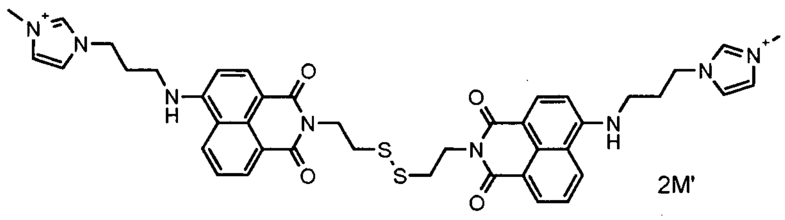

2M'

M'

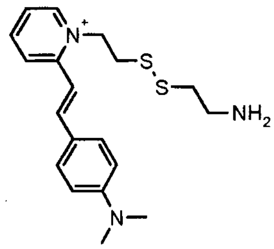

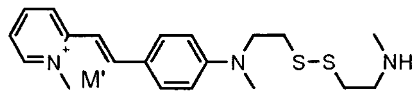

M'

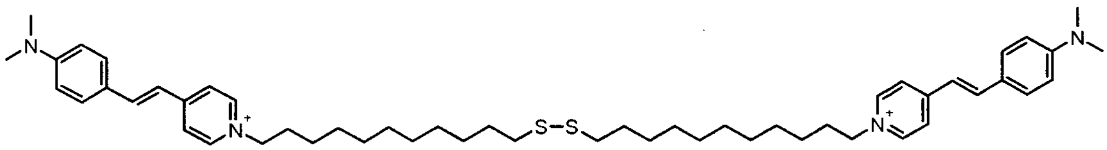

2M'

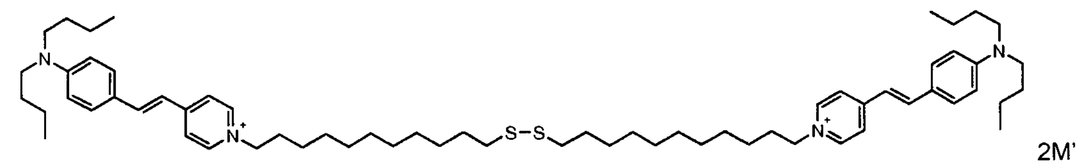

2M'

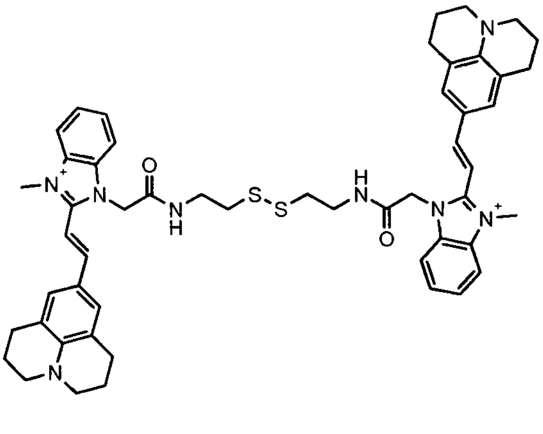

2M'

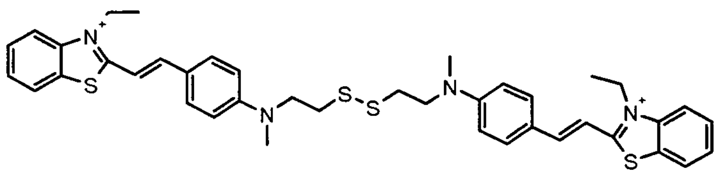

2M'

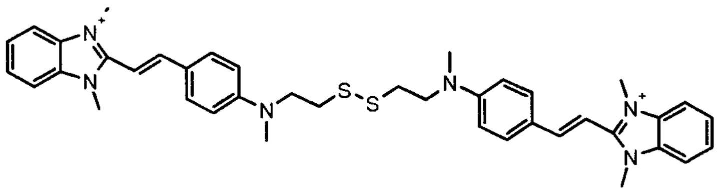

2M’

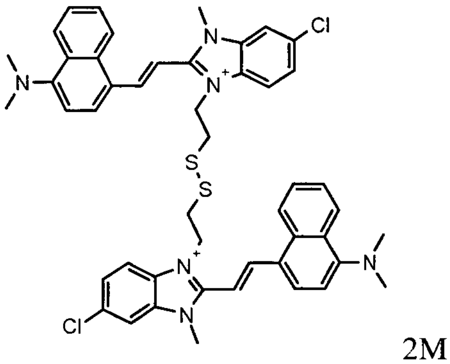

2M’

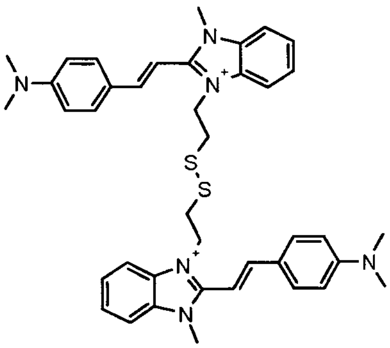

2M’

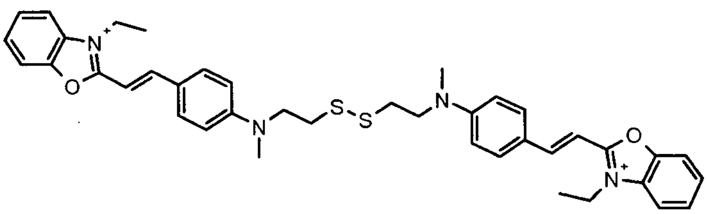

2M’

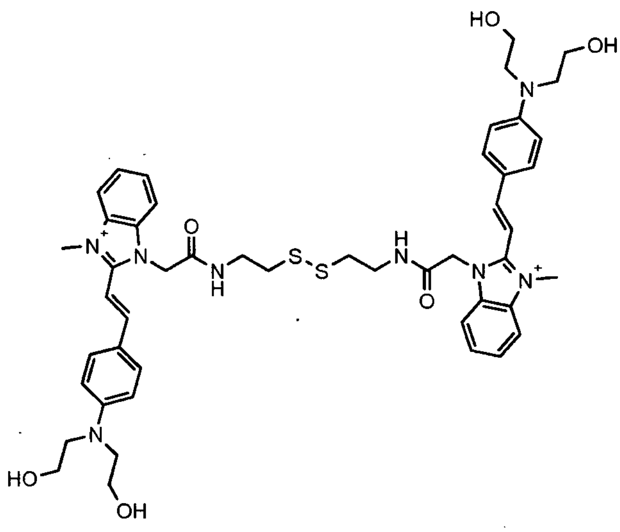

2M'

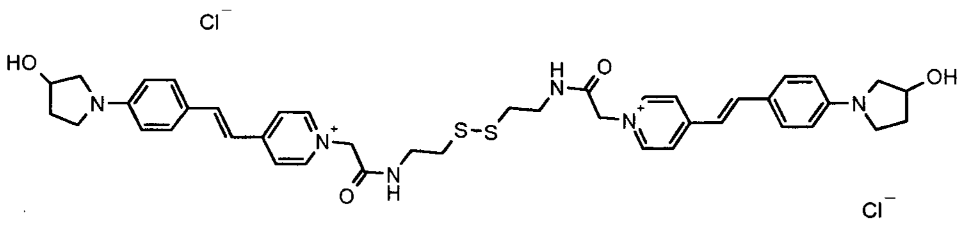

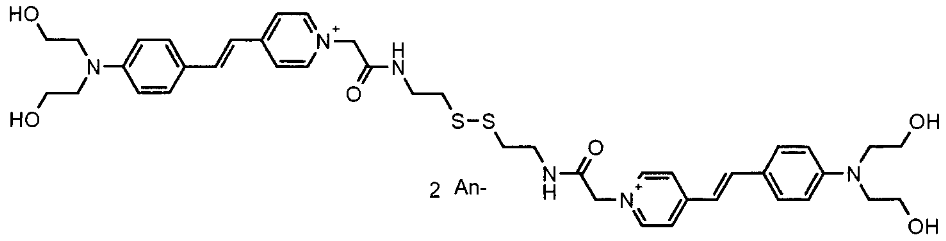

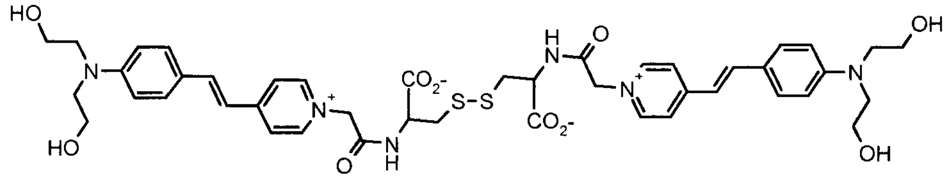

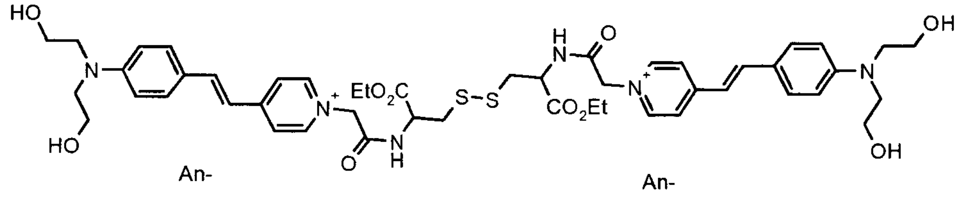

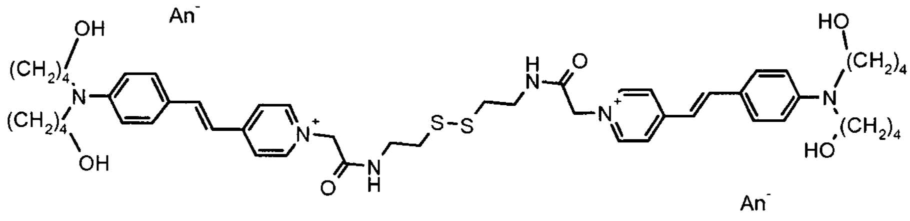

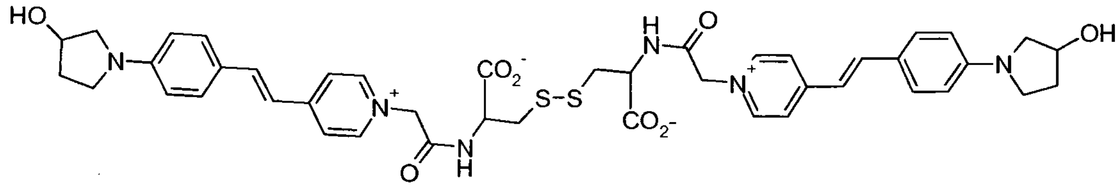

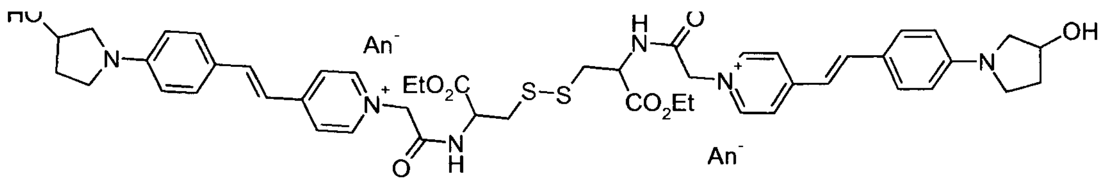

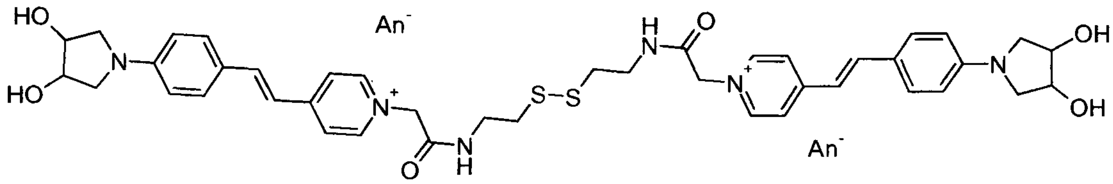

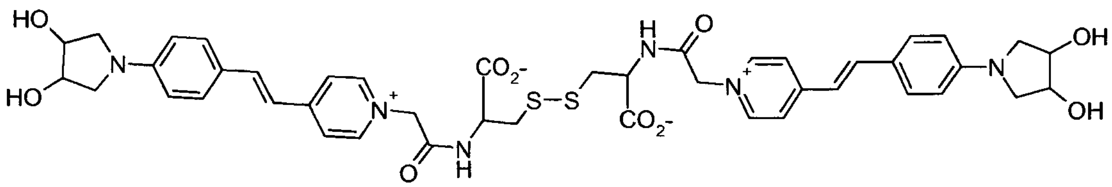

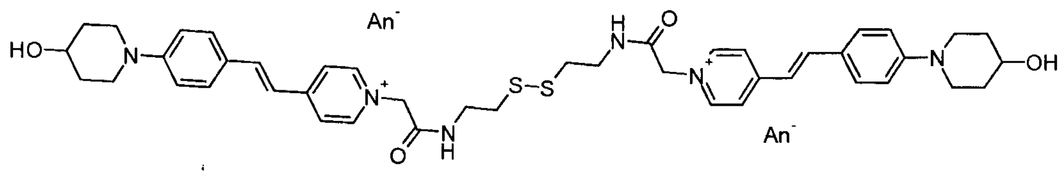

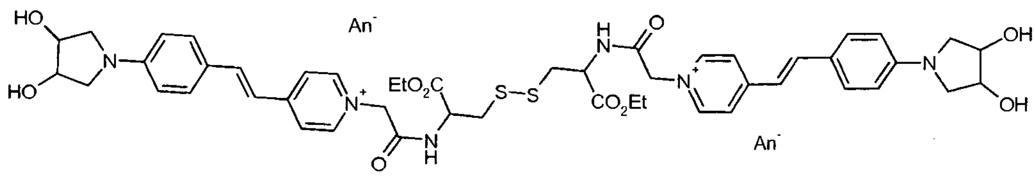

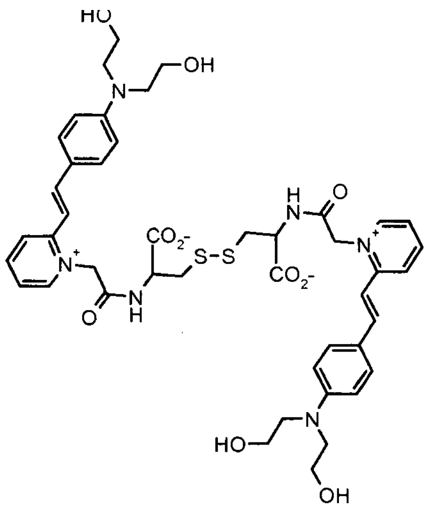

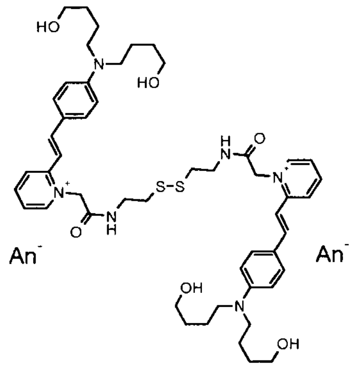

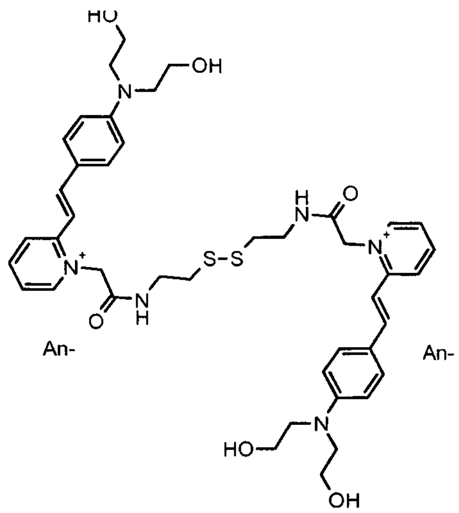

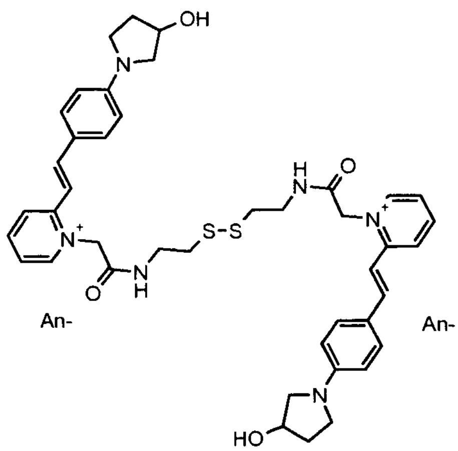

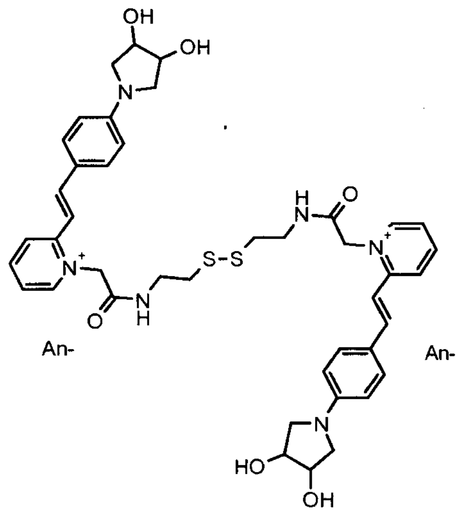

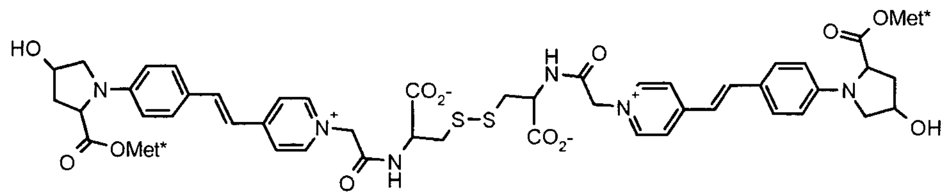

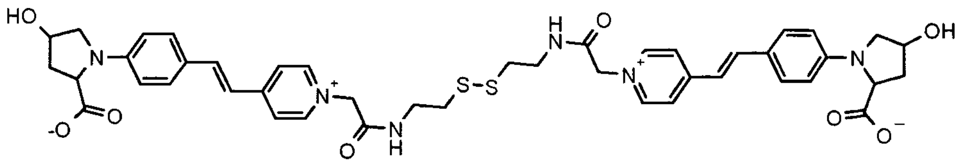

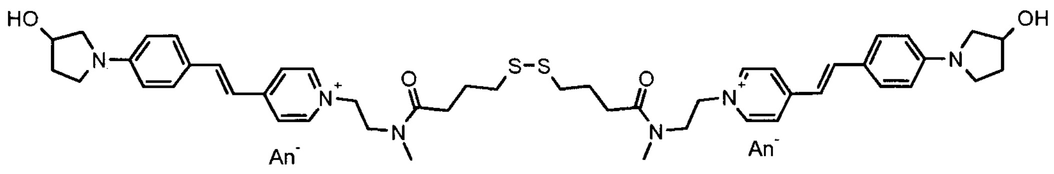

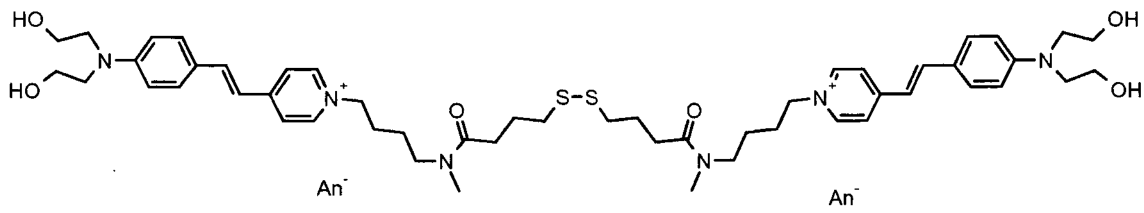

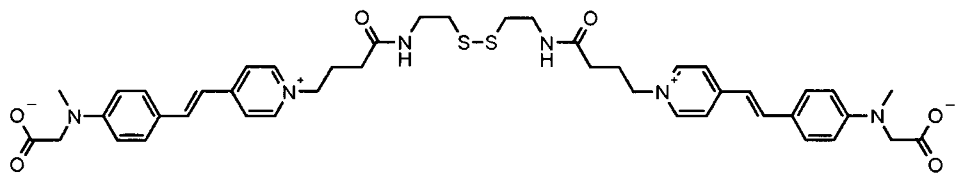

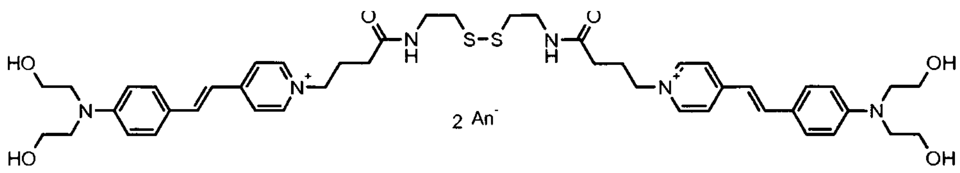

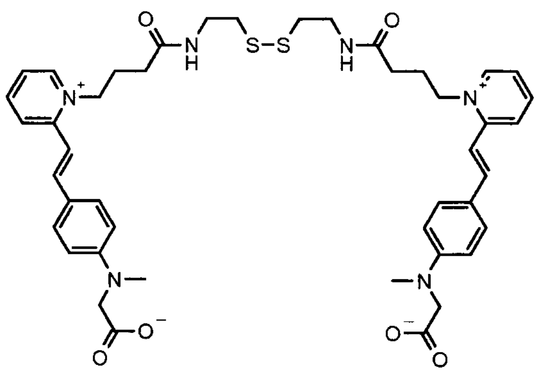

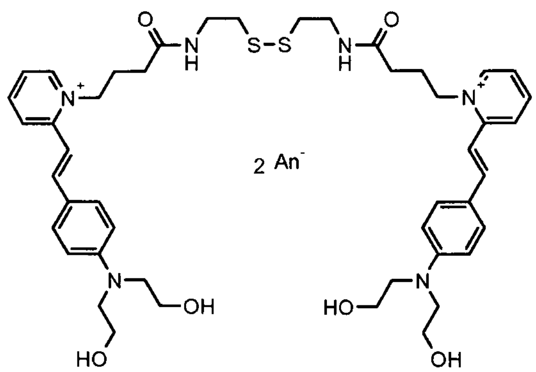

Avec M' et An⁻, représentant un contre-ion anionique.

Parmi les colorants thiols protégés de formule (I) avec U représentant Y où Y est un groupement protecteur, on peut en particulier retenir les composés de formules suivantes :

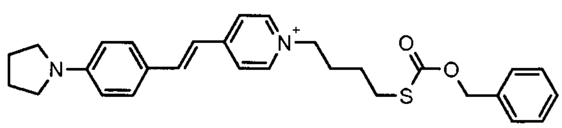
M'
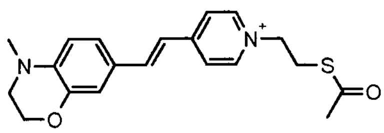
M'

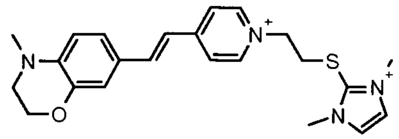
M'
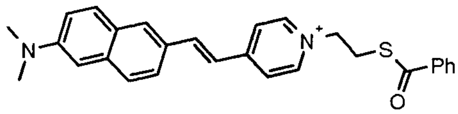
M'

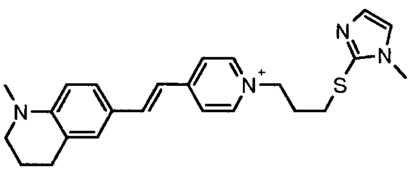
M'
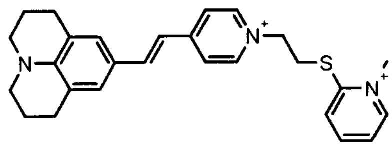
M'

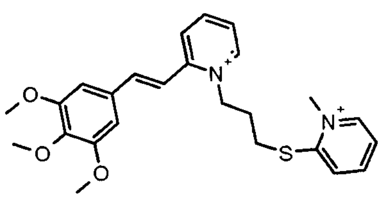
$An^-$
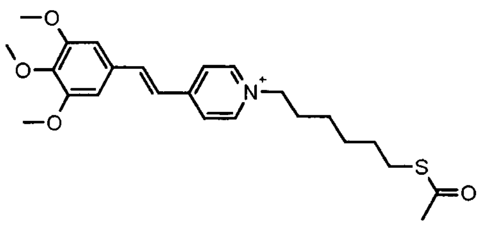
$An^-$

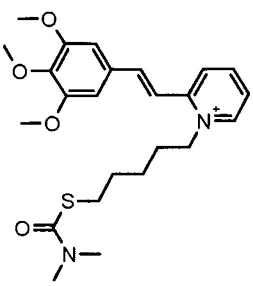
$An^-$
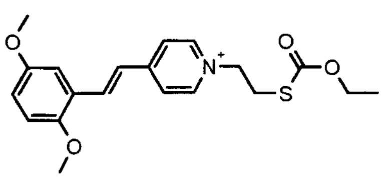
$An^-$

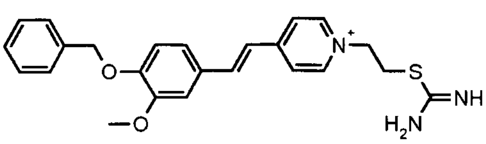
$An^-$
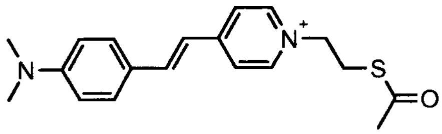
$An^-$

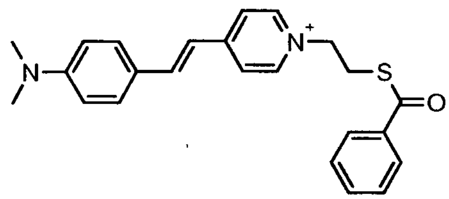

An

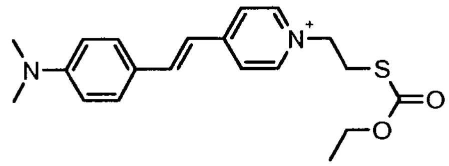

An$^-$

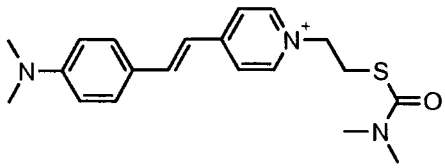

An$^-$

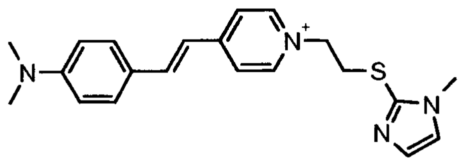

An$^-$

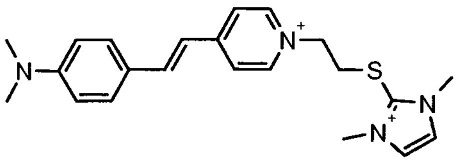

2An$^-$

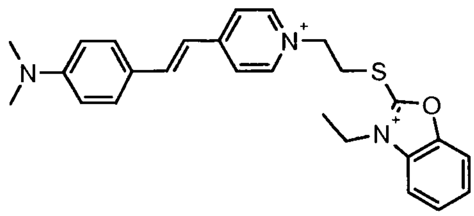

2An$^-$

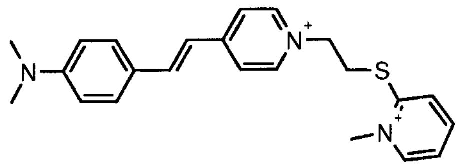

2An$^-$

avec An$^-$, M' représentant des contre-ions anioniques.

La composition tinctoriale utile dans l'invention contient dans un milieu cosmétique approprié, tel que défini précédemment, une quantité de colorant(s) à fonction(s) thiol(s) disulfure(s) en général comprise entre 0,001 et 30% par rapport au poids total de la composition. De préférence, cette quantité est comprise entre 0,01 et 5 % en poids par rapport au poids total de la composition. A titre d'exemple le ou le(s) colorant(s) se trouve dans une quantité comprise entre 0,01 % et 2%.

Etant entendu que dans le milieu l'interaction entre polymère(s) à fonction(s) thiol(s) selon l'invention et le(s) colorant(s) disulfure(s) peut générer le(s) colorant(s) par coupure réductrice de la ou les fonctions disulfures -S-S- en -SH.

*1.5 Le sel d'acide organique ou minéral et contre-ion des colorants de l'invention cosmétiquement acceptable :*

- un « sel d'acide organique ou minéral » est plus particulièrement choisi parmi un sel dérivé i) d'acide chlorhydrique HCl, ii) d'acide bromhydrique HBr, iii) d'acide sulfurique $H_2SO_4$, iv) d'acides alkylsulfoniques : Alk-S(O)$_2$OH tels que d'acide méthylsulfonique et d'acide éthylsulfonique ; v) d'acides arylsulfoniques : Ar-S(O)$_2$OH tel que d'acide benzène sulfonique et d'acide toluène sulfonique ; vi) d'acide citrique ; vii) d'acide succinique ; viii) d'acide tartrique ; ix) d'acide lactique, x) d'acides alkoxysulfiniques : Alk-O-S(O)OH tels que d'acide méthoxysulfinique et d'acide éthoxysulfinique ; xi) d'acides aryloxysulfiniques tels que d'acide toluèneoxysulfinique et d'acide phénoxysulfinique ; xii) d'acide phosphorique $H_3PO_4$; xiii) d'acide acétique $CH_3C(O)OH$ ; xiv) d'acide triflique $CF_3SO_3H$ et xv) d'acide tétrafluoroborique $HBF_4$;
- un « contre-ion anionique » est un anion ou un groupement anionique associé à la charge cationique du colorant ; plus particulièrement le contre-ion anionique est choisi parmi i) les halogénures tels que le chlorure, le bromure ;

ii) les nitrates ; iii) les sulfonates parmi lesquels les $C_1$-$C_6$ alkylsulfonates : Alk-$S(O)_2O^-$ tels que le méthylsulfonate ou mésylate et l'éthylsulfonate ; iv) les arylsulfonates : Ar-$S(O)_2O^-$ tel que le benzènesulfonate et le toluènesulfonate ou tosylate ; v) le citrate ; vi) le succinate ; vii) le tartrate ; viii) le lactate ; ix) les alkylsulfates : Alk-O-S(O)$O^-$ tels que le méthysulfate et l'éthylsulfate ; x) les arylsulfates : Ar-O-S(O)$O^-$ tels que le benzènesulfate et le toluènesulfate ; xi) les alkoxysulfates : Alk-O-$S(O)_2O^-$ tel que le méthoxy sulfate et l'éthoxysulfate ; xii) les aryloxysulfates : Ar-O-$S(O)_2O^-$, xiii) le phosphate ; xiv) l'acétate ; xv) le triflate ; et xvi) les borates tels que le tétrafluoroborate.

Par ailleurs, les sels d'addition utilisables dans le cadre de l'invention sont notamment choisis parmi les sels d'addition avec une base cosmétiquement acceptable telles que les hydroxydes de métal alcalin comme la soude, la potasse, l'ammoniaque, les amines ou les alkanolamines.

*2. Polymère à fonction thiol :*

Selon l'invention les polymères à fonction(s) thiol(s) regroupent les polymères linéaires à fonction(s) thiol(s) tels que les $\alpha$, $\omega$ -SH, les polymères non linéaires à fonction(s) thiol(s) dont les polymères ramifiés à fonction(s) thiol(s), les polymères hyperbranchés à fonction(s) thiol(s), les dendrimères à fonction(s) thiol(s) et les polymères réticulés à fonction(s) thiol(s).

Etant entendu que les polymères à fonction(s) thiol(s) comportent dans leur structure au moins un groupement -SH.

Les polymères à fonction thiols ont préférentiellement des masses molaires moléculaires élevées ie comprises inclusivement entre 8. $10^2$ et 5. $10^6$ g/mol. Leurs masses sont déterminées par les méthodes classiques par exemple de gel sur perméation, et dans le cadre des molécules fractales ou dendrimères par voie spectroscopique et/ou spectrométrique classiques pour chaque génération voir par exemple les méthodes mentionnées dans l'ouvrage « The Synthesis and Characterization of Dendritic Molecules », H.M. Janssen, E.W. Meijer, Material Science and Treatment, Synthesis of polymers, Ed. A-Dieter Schlüter, Wiley-VCH Verlag GmbH, 1999.

*2.1. Polymères linéaires et réticulés :*

A titre d'exemple de polymères linéaires et réticulés on peut citer les polyamines, les motifs amines étant inclus ou non dans la chaîne polymère,

Parmi les polyamines, on peut citer les polyamines naturelles telles que le chitosane et les protéines riches en motifs lysines, les polyamines de synthèse telles que les polylysines (poly-$\alpha$ lysines et poly-$\varepsilon$ lysines), les polyvinylamines, les polyallylamines, certaines polyéthylène imines.

Les modes de préparation des polymères thiolés suivant l'invention sont connus de l'homme de l'art, plusieurs modes sont rapportés ci-après de façon non limitative.

Les polymères thiols suivant l'invention peuvent être obtenus par polymérisation, polycondensation de motifs monomères à fonctions thiols ou thiols protégés, éventuellement en co-polymérisation ou co-polycondensation de motifs monomères dépourvus de fonctions thiols ou thiols protégés.

Alternativement, les polymères thiolés suivant l'invention peuvent être obtenus par additions de sulfure d'hydrogène, de ses sels tels que l'hydrogénosulfure de sodium ou le sulfure de potassium ou encore un groupement apte à former une liaison carbone-soufre tel que les dérivés de thiourées, le thiosulfate, sur un polymère comportant au moins une double liaison.

Les polymères thiolés suivant l'invention peuvent aussi être obtenus par substitution nucléophile d'un groupement partant présent sur une chaîne de polymère (par exemple halogène tel que le chlore, le brome, un ester sulfonique tel que mésylate ou tosyltate) par un composé comportant au moins un atome de soufre tel que ceux précités

Les polymères thiolés suivant l'invention peuvent aussi être obtenus par réaction de polymères comportant des groupements nucléophiles tels que des amines sur des composés électrophiles comportant un atome de soufre tels que l'acide 2-oxo-4-thiazolidine carboxylique également connu sous le nom de procystéine :

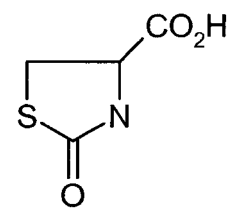

la N-acétyl homocystéine-thiolactone :

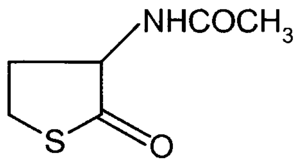

la γ -thiobutyrolactone :

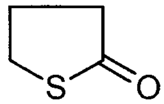

l'imino thiolane :

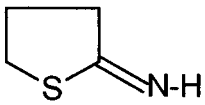

Préférentiellement, les polymères thiolés suivant l'invention sont des polymères solubles dans les milieux cosmétiques, particulièrement dans les milieux aqueux ou hydroalcoolique.
Ils sont plus préférentiellement choisis parmi les polymères aminés et leurs sels d'ammonium ou les polymères polyhydroxylés.

*2.2. Polymères hyperbranchés et dendrimères :*

Les polymères hyperbranchés sont des constructions moléculaires ayant une structure ramifiée, en général autour d'un coeur. Leur structure est en règle générale exempte de symétrie. En effet, les unités de base ou monomères ayant servi à la construction du polymère hyperbranché peuvent être de natures différentes et leur répartition est irrégulière. Les branches du polymère peuvent être de natures et de longueurs différentes. Le nombre d'unités de base, ou monomères, peut être différent suivant les différentes ramifications. Tout en étant asymétriques, les polymères hyperbranchés peuvent posséder une structure extrêmement ramifiée, autour d'un coeur ; des couches ou générations successives de ramifications ; une couche de chaînes terminales.
Les polymères hyperbranchés sont généralement issus de la polycondensation d'un ou plusieurs monomères ABx, A et B étant des groupements réactifs susceptibles de réagir ensemble, x étant un entier supérieur ou égal à 2, mais d'autres procédés de préparation peuvent être envisagés.
Les polymères hyperbranchés se caractérisent par leur degré de polymérisation DP = 1-b, b étant le pourcentage de fonctions, non terminales, de B qui n'ont pas réagi avec un groupement A.
La condensation étant non systématique, au contraire de la synthèse de dendrimères (voir infra), le degré de polymérisation est inférieur à 100%. On peut faire réagir un groupement terminal T sur le polymère hyperbranché pour obtenir une fonction particulière en extrémité de chaînes.
Plusieurs polymères hyperbranchés peuvent être associés entre eux, par une liaison covalente ou un autre type de liaison, par l'intermédiaire de leurs groupes terminaux. De tels polymères, dits pontés, entrent dans la définition des polymères hyperbranchés selon la présente invention.
De nombreux polymères hyperbranchés et dendrimères ont déjà été décrits. On peut se reporter par exemple à : D. A. Tomalia et al, Angew. Chem. Int. Engl. 29 (1990) 138-175 ; N. Ardoin et D. Astruc, Bull. Soc. Chim. Fr. (1995) 132, 875-909 ; B. I. Voit, Acta Polymer, 46, 87-99 (1995).
De tels polymères sont décrits en particulier dans B.I.Voit, Acta Polymer., 46, 87-99 (1995) ; EP-682059 ; WO-9614346 ; WO-9614345 ; WO-9612754.
Plusieurs polymères hyperbranchés peuvent être associés entre eux, par une liaison covalente ou un autre type de liaison, par l'intermédiaire de leurs groupes terminaux.
De tels polymères dits pontés ou « bridged » entrent dans la définition des polymères hyperbranchés selon la présente invention.
Les dendrimères ou arbre moléculaire sont des macromolécules constituées de monomères qui s'associent

suivant un processus arborescent autour d'un coeur central plurifonctionnel. Les dendrimères présentent donc une structure fractale (ou molécule fractale), constituée d'un coeur, un nombre déterminé de génération de branches (ou fuseaux), de cavités internes provenant desdites ramifications de la molécule, et de fonctions terminales. Les dendrimères sont structurellement des polymères et oligomères hautement ramifiés ayant une structure chimique bien définie.

Les générations de branches sont constituées d'unités structurelles, qui sont identiques pour une même génération de branches et qui peuvent être identiques ou différentes pour des générations de branches différentes. L'ensemble des points de jonction des branches de mêmes génération sont situés à une égale distance du coeur correspond à une génération.

Les générations de branches s'étendent radialement en une progression géométrique à partir du coeur. Les groupes terminaux d'un dendrimère de la $N^{ième}$ génération sont les groupes fonctionnels terminaux des branches de la $N^{ième}$ génération ou appelé génération terminale.

La définition des dendrimères donnée supra inclut des molécules à ramifications symétriques ; elle inclut également des molécules à ramification non symétrique, comme par exemple les dendrimères dont les branches sont des groupements lysine, dans lesquels le branchement d'une génération de fuseaux sur la précédente se fait sur les amines $\alpha$ et $\varepsilon$ de la lysine, ce qui conduit à une différence dans la longueur des fuseaux des différentes ramifications.

Les polymères denses en étoiles, ou « dense star polymer », les polymères éclatés en étoile, ou « starburst polymer », les dendrimères en baguette, ou « rod-shaped dendrimer », sont inclus dans la présente définition des dendrimères. Les molécules dénommées arborols et molécules en cascade entrent également dans la définition des dendrimères selon la présente invention.

Par ailleurs, plusieurs dendrimères peuvent être associés entre eux, par une liaison covalente ou un autre type de liaison, par l'intermédiaire de leurs groupes terminaux pour donner des entités connues sous le nom de « dendrimères pontés », « agrégats de dendrimères » ou « bridged dendrimer ». De telles entités sont incluses dans la définition des dendrimères selon la présente invention.

Des dendrimères peuvent se présenter sous la forme d'un ensemble de molécules de même génération, ensembles dits monodisperses ; ils peuvent également se présenter sous la forme d'ensembles de générations différentes, dits polydisperses. La définition des dendrimères selon la présente invention inclut des ensembles monodisperses aussi bien que polydisperses de dendrimères.

A titre d'exemple de polymères hyperbranchés et dendrimères comportant des groupements fonctionnels thiols on citera notamment la demande de brevet français FR97-04085, au nom de la demanderesse, qui décrit des polymères comportant des groupements fonctionnels répondant à la formule (II) suivante :

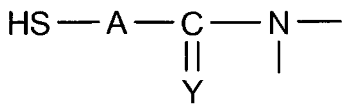

(II)

Formule (II) dans laquelle :

- Y représente l'atome d'oxygène ou un groupement NH,
- A représente un groupe alcane di-yle en $C_1$-$C_{12}$, linéaire, ramifié ou cyclique, saturé

ou insaturé; ce groupe étant éventuellement interrompu par un ou plusieurs hétéroatomes et/ou substitué par une fonction choisie parmi amino, acylamino, acide carboxylique et ester.

Suivant un autre mode préféré de l'invention, les polymères thiolés sont tels que décrits dans le document FR2761691 Ce sont des polymères hyperbranchés et les dendrimères comportant des groupements fonctionnels répondant à la formule (III) :

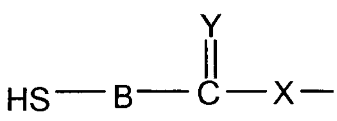

(III)

formule (III) dans laquelle :

■ Y représente l'atome d'oxygène ou un groupement NH,

■ B représente un groupement alkylène en $C_1$-$C_{12}$, linéaire, ramifié ou cyclique, saturé

ou insaturé ; ledit groupement alkylène peut être éventuellement interrompu par un ou plusieurs hétéroatomes, comme O ou N ; de plus ledit groupement alkylène peut éventuellement être substitué par une fonction

- amino : $-NH_2$, éventuellement sous forme d'un sel d'un acide minéral ou organique,
- acylamino : -NH-C(O)R, dans lequel R représente un groupement alkyle en $C_1$-$C_{10}$ linéaire, ramifié ou cyclique, saturé ou insaturé,
- acide carboxylique,
- ester en $C_1$-$C_{10}$ ;

■ X représente un atome d'oxygène ou un groupement -NR'- dans lequel R' est choisi parmi un atome d'hydrogène ; un groupement alkyle en $C_1$-$C_6$, linéaire ou ramifié, saturé ou insaturé ; un groupement mono- ou poly-hydroxyalkyle en $C_1$-$C_6$, linéaire ou ramifié, saturé ou insaturé ; un groupement aminoalkyle en $C_1$-$C_6$ ou un groupement polyalkylèneimine.

Par exemple, B peut être un groupement méthylène, éthylène, propylène, méthylpropylène, éthylpropylène, tétraméthylène, pentaméthylène, hexaméthylène, phénylène.

Avantageusement, B représente un radical répondant à l'une des formules (a) à (d) suivantes :

$-CHR^1-CHR^2-CHR^3-$ (a)

$-CHR'^1-CHR'^2-CHR'^3-CHR'^4-$ (b)

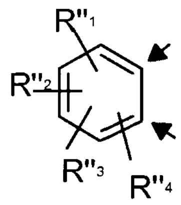

(c)

$-(CHR'''^1)_k-(CHR'''^2)-CH(CO_2H)-NH-$ (d)

Formules (a), (b), (c) et (d) dans lesquelles $R^1$, $R^2$, $R^3$, $R'^1$, $R'^2$, $R'^3$ et $R'^4$, $R'''^1$ $R'''^2$, identiques ou différents représentent : l'atome d'hydrogène, un radical alkyle en $C_1$-$C_6$, linéaire, ramifié ou cyclique, saturé ou insaturé, un radical amino $-NH_2$, un radical acide carboxylique -COOH , un radical alkyl amino en $C_1$-$C_{10}$; $R''^1$, $R''^2$, $R''^3$ et $R''^4$, identiques ou différents représentent : l'atome d'hydrogène, un radical alkyle en $C_1$-$C_4$, linéaire ou ramifié, saturé ou insaturé, les flèches indiquant les positions des substitutions dans la formule (c), k est un entier, préférentiellement k=0 ou 1. Préférentiellement B est choisi parmi :

$-CH_2-CH(CO_2H)-NH-$ et Y représente un atome d'oxygène ;

$-(CH_2)_2-(CH_3CONH)CH-$ et Y représente un atome d'oxygène ;

$-(CH_2)_3-$ et Y représente un atome d'oxygène ou un groupement NH.

De façon particulière, B est le radical propylène $-CH_2-CH_2-CH_2-$, et Y représente un atome d'oxygène, le composé selon l'invention répondant alors à la formule (IV) suivante :

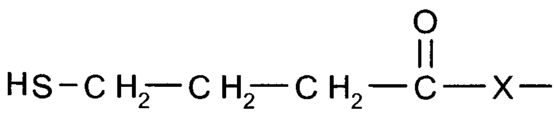 (IV)

Formule (IV) dans laquelle :

X représente un groupement nucléophile, préférentiellement un atome d'oxygène ou un groupement -NR'- dans lequel R' est choisi parmi un atome d'hydrogène ; un groupement alkyle en $C_1$-$C_6$, linéaire ou ramifié, saturé ou insaturé ; un groupement mono- ou poly-hydroxyalkyle en $C_1$-$C_6$, linéaire ou ramifié, saturé ou insaturé; un groupement aminoalkyle en $C_1$-$C_6$ ou un groupement polyalkylèneimine.

Préférentiellement, selon l'invention, X de la formule (II) et (IV) est choisi parmi l'atome d'oxygène et un groupement NH.

Suivant l'un des modes préférés de l'invention, les polymères thiolés sont tels que décrits dans le document FR2853533, il s'agit de poly N-α- et N-ε- lysine et ornithine de formule I, à fonction thiol, qui peuvent être obtenues à partir de poly N-α- et N-ε- lysine et ornithine par réaction avec une thiolactone telle que, par exemple la thiobutyrolactone (dihydrothiophèn-2(3H)-one).

Selon un mode de réalisation préféré de l'invention les polymères hyperbranchés et les dendrimères utiles à l'invention comportent des groupements fonctionnels répondant à la formule (V) :

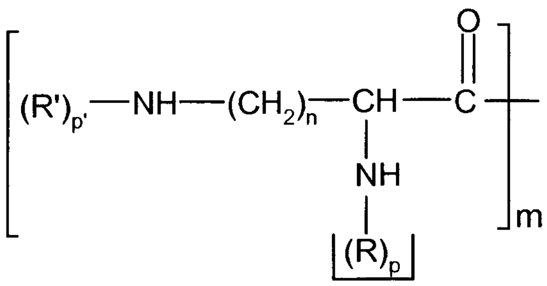 (V)

Formule (V) dans laquelle:

- p différent de p' et p, p'valent 0 ou 1 ;
- n vaut 3 ou 4 ;
- si p' vaut 0, alors le NH voisin est engagé dans une polymérisation N-ε ;
- si p vaut 0, alors le NH voisin est engagé dans une polymérisation N-α ;
- si p ou p' vaut 1, alors R ou R' représente -B-SH, avec
    - B représentant une chaîne hydrocarbonée en $C_1$-$C_{30}$, saturée ou non, linéaire ou ramifiée pouvant être interrompue par un ou plusieurs hétéroatomes ou fonctions, seuls ou en combinaisons, tels que :

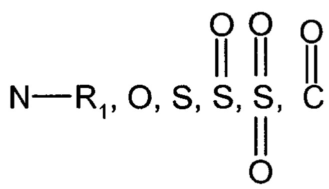

ou/et par un ou plusieurs aryle, hétéroaryle, cycloalkyle ou hétérocycloalkyle à 5, 6 ou 7 chaînons et pouvant être substituée par un ou plusieurs groupements : COOH, OH, $NH_2$, (di)($C_1$-$C_8$)alkylamino, ($C_1$-$C_8$)acylamino, ($C_1$-$C_8$)acyloxy, ($C_1$-$C_8$)alkyloxycarbonylamino, ($C_1$-$C_8$)alkylamino-carbonyloxy, halogéno, ($C_1$-$C_8$)alkyl aminocarbonyle ; sachant que R ou R' peuvent, en partie seulement, également représenter un atome d'hydrogène, ou/et

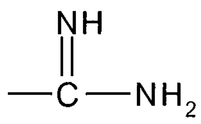

et ses sels ou/et

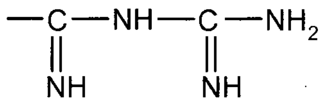

et ses sels.

- $R_1$ représente un atome d'hydrogène ou un groupment $(C_1-C_8)$alkyle, $(C_1-C_8)$acyle, $(C_1-C_8)$alkyloxycarbonyle, $(C_1-C_8)$alkyl amino-carbonyle, halogéno.
- B peut également représenter un groupement aryle, hétéroaryle, cycloalkyle, ou hétérocycloalkyle à 5, 6 ou 7 chaînons, éventuellement substitués ;
- m représente un entier compris inclusivement de 3 à 10000.

De préférence, le taux de greffage de fonction thiol sera supérieur ou égal à 1%.

Avantageusement, les poly N-$\tilde{\alpha}$ et N-ε- lysine et ornithine répondant à la formule (V) présentent: $5<m<1000$.

Le "taux théorique de greffage de fonction thiol" représente le pourcentage théorique en unité lysine ou ornithine porteuse de la fonction thiol dans le composé de formule (V).

A titre d'exemple de polymères hyperbranchés on peut citer tout particulièrement les polyéthylèneimines thiolés hyperbranchés, tels que ceux décrits dans la demande EP103759, de masse molaire moléculaire comprise entre $30.10^4$ et $50.10^4$.

La concentration en polymères thiolés est comprise entre 0,001 % et 40 % de la composition, de préférence comprise entres 0,1 % et 15 %, de préférence entre 0,4 et 10 %, ce pourcentage s'exprimant en poids pour poids.

*3. Le milieu :*

Le milieu approprié pour la teinture, appelé aussi support de teinture, est un milieu cosmétique généralement constitué par de l'eau ou par un mélange d'eau et d'au moins un solvant organique ou un mélange de solvant organique.

Par solvant organique, on entend une substance organique capable de dissoudre une autre substance sans la modifier chimiquement.

*3.1 Les Solvants organiques:*

A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

*3.2 Les Adjuvants:*

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères non thiolés anioniques, cationiques, non-ioniques, amphotères, zwitterioniques ou leurs mélanges, des agents épaississants minéraux ou organiques, et en

particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiant.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

*3.3 Les Colorants additionnels:*

La composition tinctoriale peut en outre contenir un ou plusieurs colorants directs additionnels. Ces colorants directs sont par exemple choisis parmi ceux classiquement utilisés en coloration directe, et parmi lesquels on peut citer tous les colorants aromatiques et/ou non aromatiques d'utilisation courante tels que les colorants directs nitrés benzéniques neutres, acides ou cationiques, les colorants directs azoïques neutres, acides ou cationiques, les colorants directs naturels, les colorants directs quinoniques et en particulier anthraquinoniques neutres, acides ou cationiques, les colorants directs aziniques, triarylméthaniques, indoaminiques, les méthines, les styriles, les porphyrines, métalloporphyrines, les phtalocyanines, les cyanines méthiniques, et les colorants fluorescents.

Parmi les colorants directs naturels, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Selon l'invention, le ou les colorants directs utilisés selon l'invention représentent de préférence, de 0.001 % à 10% en poids environ du poids total de la composition tinctoriale et encore plus préférentiellement de 0.05% à 5% en poids environ.

La composition tinctoriale peut également contenir une ou plusieurs bases d'oxydation et/ou un ou plusieurs coupleurs conventionnellement utilisés pour la teinture de fibres kératiniques en présence d'agent oxydant.

Parmi les bases d'oxydation, on peut citer les para-phénylènediamines, les bis-phénylalkylènediamines, les para-aminophénols, les bis-para-aminophénols, les orthoaminophénols, les bases hétérocycliques et leurs sels d'addition

Parmi ces coupleurs, on peut notamment citer les méta-phénylènediamines, les méta-aminophénols, les méta-diphénols, les coupleurs naphtaléniques, les coupleurs hétérocycliques et leur sels d'addition.

L'agent oxydant peut être choisi par exemple parmi le peroxyde d'hydrogène, le peroxyde d'urée, les bromates de métaux alcalins tel que le bromate de sodium, les persels tels que les perborates et persulfates, et les enzymes telles que les peroxydases et les oxydo-réductases à deux ou à quatre électrons comme les uricases, et les oxydases à 4 électrons comme les laccases. L'utilisation du peroxyde d'hydrogène est particulièrement préférée.

Le ou les coupleurs sont chacun généralement présents en quantité comprise entre 0,001 et 10 % en poids du poids total de la composition tinctoriale, de préférence entre 0,005 et 6 %.

La ou les bases d'oxydation présentes dans la composition tinctoriale sont en général présentes chacune en quantité comprise entre 0,001 à 10 % en poids du poids total de la composition tinctoriale, de préférence entre 0,005 et 6 % en poids.

La teneur en agent oxydant est en général comprise entre 1 et 40 % en poids, par rapport au poids de la composition de préférence entre 1 et 20 % en poids par rapport au poids de la composition.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que les hydroxydes de métal alcalin comme la soude, la potasse, l'ammoniaque, les amines ou les alcanolamines.

Selon un mode de réalisation particulier, la composition de la présente invention contient au moins une base d'oxydation et éventuellement au moins un coupleur tels que défini ci-dessus.

*3.4 Le pH :*

Le pH de la composition tinctoriale comprenant le ou les colorants est généralement compris entre 2 et 12 environ, et de préférence entre 3 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Le pH de la composition selon l'invention est préférentiellement compris entre 6 et 9.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (a) suivante :

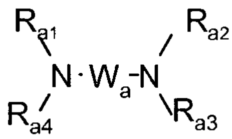
(α)

dans laquelle $W_a$ est un reste propylène éventuellement substitué par un groupement hydroxy ou un radical alkyle en $C_1$-$C_4$ ; $R_{a1}$, $R_{a2}$, $R_{a3}$ et $R_{a4}$, identiques ou différents, représentent un atome d'hydrogène, un radical alkyle en $C_1$-$C_4$ ou hydroxyalkyle en $C_1$-$C_4$.

Selon un mode de réalisation particulier de l'invention la composition tinctoriale contient des agents alcalins dont au moins la monoéthanolamine.

*3.5 Formes de la composition :*

La composition tinctoriale peut se présenter sous des formes galéniques diverses, telles que sous forme de liquide, de lotion, de crème, de gel, ou sous toute autre forme appropriée pour réaliser une teinture des fibres kératiniques. Elle peut également être conditionnée sous pression en flacon aérosol en présence d'un agent propulseur et former une mousse.

*4 Le procédé de teinture*

L'invention a aussi pour objet un procédé de teinture directe qui comprend l'application sur les matières kératiniques, en particulier les fibres kératiniques telles que les cheveux foncés, d'une composition tinctoriale contenant au moins un colorant direct à fonction disulfure et au moins un polymère à fonction thiol de poids moléculaire supérieur à 500 notamment supérieur à 1000. Après un temps de pose, les fibres kératiniques sont rincées laissant apparaître des fibres colorées.

Selon un mode de réalisation particulier dans le procédé de l'invention le temps de pose après l'application de la composition contenant au moins un colorant direct à fonction disulfure et au moins un polymère à fonction thiol de poids moléculaire supérieur à 500 notamment supérieur à 1000, est fixé entre 5 minutes et 1 heure. Préférentiellement entre 15 minutes et 1 heure.

Selon une variante, le procédé de coloration selon l'invention consiste à appliquer sur les matières kératiniques, en particulier les fibres kératiniques telles que les cheveux foncés, i) une composition tinctoriale, comprenant dans un milieu cosmétique au moins un colorant disulfure, thiol ou thiol protégé de formule **(I),** tel que défini précédemment, et ii) une composition comprenant au moins un polymère à fonction thiol de poids moléculaire supérieur à 500 notamment supérieur à 1000. L'agent réducteur polymère à fonction thiol de poids moléculaire supérieur à 500 notamment supérieur à 1000 peut être appliqué de façon extemporanée ou en post-traitement.

La durée du post traitement avec un polymère à fonction thiol de poids moléculaire supérieur à 500 notamment supérieur à 1000 peut être courte, par exemple de 1 seconde à 30 minutes de préférence de 1 minute à 15 minutes.

Lorsqu'un colorant thiol ou thiol protégé de formule **(I)** comprend un groupement Y protecteur de la fonction thiol, le procédé de l'invention peut être précédé d'une étape de déprotection visant à restituer *in-situ* la fonction SH.

A titre d'exemple il est possible de déprotéger la fonction S-Y des colorants de l'invention avec Y groupement protecteur en ajustant le pH comme suit :

| **Y : groupement protecteur** | **déprotection** |
|---|---|
| alkylcarbonyle, | pH>9 |
| arylcarbonyle, | pH>9 |
| alcoxycarbonyle, | pH>9 |

(suite)

| Y : groupement protecteur | déprotection |
|---|---|
| aryloxycarbonyle, | pH>9 |
| arylalcoxycarbonyle | pH>9 |
| (di)(alkyl)aminocarbonyle, | pH>9 |
| (alkyl)arylaminocarbonyle | pH>9 |
| aryle éventuellement substitué tel que le phényle, | pH>9 |
| hétéroaryle monocyclique à 5, 6 ou 7 chaînons tel que l'oxazolium ; | pH>9 |
| hétéroaryle bicyclique à 8 à 11 chaînons tels que benzoimidazolium, ou benzoxazolium | pH>9 |

L'étape de déprotection peut également être réalisée au cours d'une étape de prétraitement des cheveux comme par exemple, le prétraitement réducteur des cheveux.

Un traitement avec un agent oxydant peut éventuellement être associé. On pourra utiliser n'importe quel type d'agent oxydant classique dans le domaine. Ainsi, il peut être choisi parmi le peroxyde d'hydrogène, le peroxyde d'urée, les bromates de métaux alcalins, les persels tels que les perborates et les persulfates, ainsi que les enzymes parmi lesquelles on peut citer les peroxydases, les oxydo-réductases à 2 électrons tels que les uricases et les oxygénases à 4 électrons comme les laccases. L'utilisation du peroxyde d'hydrogène est particulièrement préférée. La durée du post-traitement éventuel avec un agent oxydant est comprise entre 1 seconde et 40 minutes. De préférence entre 1 et 10 minutes.

L'application de la composition tinctoriale selon l'invention est généralement effectuée à température ambiante. Elle peut cependant être réalisée à des températures variant de 20 à 80°C, préférentiellement entre 20 et 60°C.

L'application de la composition peut être effectuée sur cheveux secs ou être précédée de l'humidification des cheveux.

L'invention a aussi pour objet un dispositif à plusieurs compartiments ou "kit" de teinture dans lequel un premier compartiment renferme une composition tinctoriale comprenant au moins un colorant disulfure, thiol ou thiol protégé de formule **(I),** et un deuxième compartiment renferme un polymère à fonction thiol de poids moléculaire supérieur à 500 notamment supérieur à 1000. Ledit dispositif peut contenir un troisième compartiment comprenant un agent oxydant.

Les exemples qui suivent servent à illustrer l'invention sans toutefois présenter un caractère limitatif.

Les colorants directs disulfures utiles dans la présente invention sont des composés connus et peuvent être préparés selon des méthodes connues de l'homme de l'art, notamment à partir des méthodes décrites dans la demande EP 1 647 580.

**EXEMPLE DE COLORATION**

**<u>Exemple 1</u> : Synthèse du dichlorure de 2,2'-{disulfanediylbis[éthane-2,1-diylimino-4,1-phénylènediazène-2,1-diyl]}bis(1,3-diméthyl-1H-imidazol-3-ium) [1]**

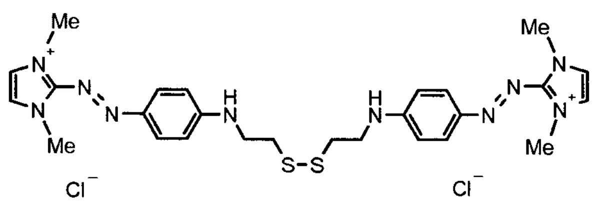

[1]

Schéma de synthèse

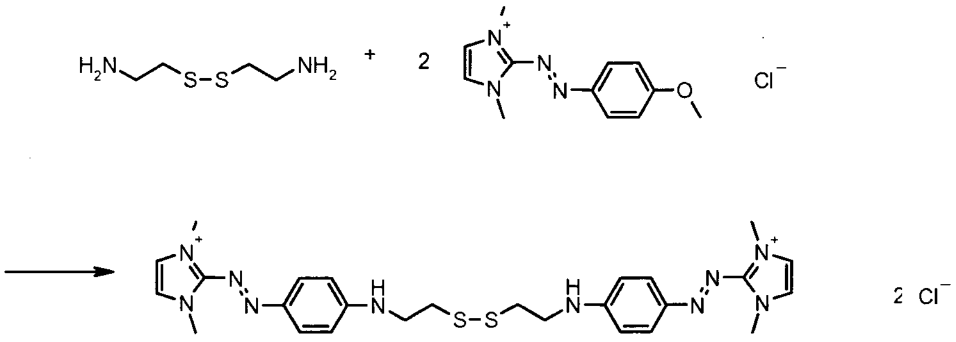

La cystamine base (552.2 mg ; 3.62 mmol) obtenue à partir de dichlorhydrate de cystamine par addtion de soude et extraction par l'acétate d'éthyle, est solubilisée dans 2 ml de pentanol. On ajoute le chlorure de 2-[(4-méthoxyphenyl)diazenyl]-1,3-dimethyl-1H-imidazol-3-ium (2.42 m ; 9.1 mmol), en suspension dans 80 ml de dichlorométhane. Le mélange est porté à 50°C et maintenu agité 1 heure. Il est concentré sous vide (élimination du dichlorométhane), on ajoute 20 ml d'eau et le mélange réactionnel est maintenu une heure supplémentaire à 50°C. Il est ensuite refroidi et versé sur 50mL de pentanol ; un précipité rouge apparaît, qui est filtré et lavé par de l'acétone puis séché sous vide. On obtient ainsi 1.1g de poudre rouge foncée conforme à la structure ci-dessus.

**<u>Exemple 2:</u> Synthèse du diméthoxysulfate de 4,4'-idisulfanediylbis[éthane-2,1-diyl-(méthylimino)-4,1-phénylène éthène-2,1-diyl]}bis(1-méthylpyridinium) [2]**

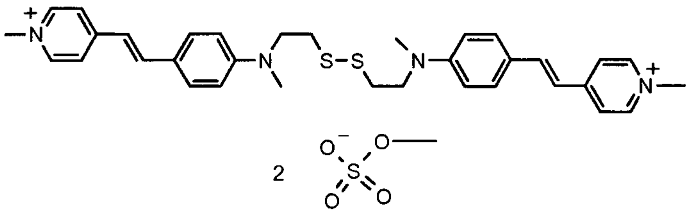

**[2]**

## Schéma de synthèse :

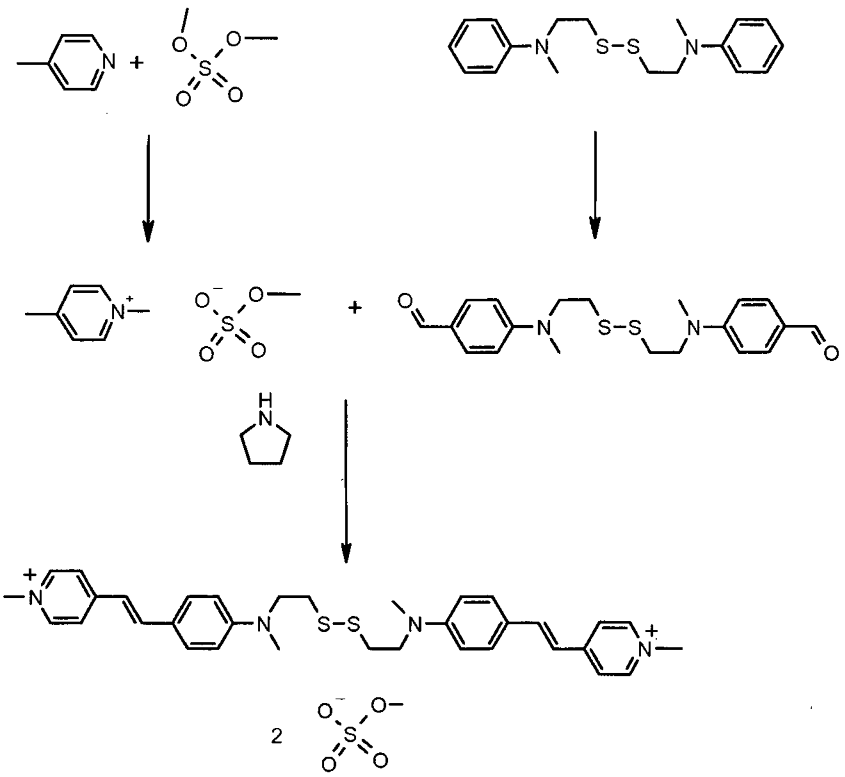

**[2]**

**Mode opératoire :**

**Etape 1 : 4,4'-{disulfanediylbis[éthane-2,1-diyl(méthylimino)]}dibenzaldéhyde**

82,3 g d'oxychlorure de phosphore sont ajoutés à 500 mL de DMF à 0 °C. Après 30 min d'agitation à 0 °C une solution de 47 g de *N,N*-(disulfanediyldiéthane-2,1-diyl)bis(N-méthylaniline) sont ajoutés goutte à goutte. Le mélange est agité 90 min. à 0 °C puis 75 min. à 10°C et 105 min. à 40 °C. Il est ensuite versé sur 2,5 L d'eau glacée, 700 mL de soude 5N sont ajoutés. Le précipité jaune obtenu est filtré sur célite, solubilisé dans 200 mL de dichlorométhane et la solution obtenue est lavée par 200 mL de solution aqueuse saturée en chlorure de sodium. Après séchage sur sulfate de magnésium et évaporation du dichlorométhane, le résidu jaune (80 g) est purifié par chromatographie sur gel de silice. Après séchage, une poudre jaune clair est recueillie. Les analyses indiquent que le produit est conforme à la structure attendue.

**Diméthoxysulfate de 4,4'-{disulfanediylbis[éthane-2,1-diyl(méthylimino)-4,1-phénylène-éthène-2,1-diyl]}bis(1-méthylpyridinium) [2]**

2,62 g de 4-picoline sont dilués dans 25 mL de dichlorométhane, 3 mL de sulfate de diméthyle sont ajoutés à la solution, dont la température s'élève au reflux (40 °C). Après 40 min. d'agitation, 50 mL d'isopropanol sont ajoutés, et le mélange est concentré par distillation du dichlorométhane (mélange chauffé à 60 °C). 1,83 g de pyrrolidine sont introduits dans le mélange suivis de 4,99 g de 4,4'-{disulfanediylbis[éthane-2,1-diyl(méthylimino)]}dibenzaldéhyde. Après 2 h d'agitation à 65 °C, le mélange réactionnel est refroidi à température ambiante, le précipité formé est filtré, lavé par 3 fois 100 mL d'isopropanol. La pâte rouge obtenue est dispersée dans 200 mL d'isopropanol, le mélange ainsi obtenu est porté à reflux puis refroidi. Le précipité rouge formé est filtré puis séché. 8,94 g de poudre rouge sont recueillis. Les analyses indiquent que le produit est conforme à la structure de [2] et pur. RMN $^{1}H$ (400 MHz, DMSO-$d_6$) 2.96 (t, 4 H), 3.02 (s, 6 H), 3.36 (s, 6 H), 3.72 (t, 4 H), 4.16 (s, 6 H), 6.81 (d, 4 H), 7.15 (d, 2 H), 7.57 (d, 4 H), 7.87 (d, 2 H), 8.02 (d, 4 H), 8.66 (d, 4 H).

Compositions 1 à 3 :

Les compositions on été réalisée avec les teneurs en ingrédients données dans le tableau ci dessous

| Composition | Composition 1 | Composition 2 (Comparative) | Composition 3 |
|---|---|---|---|
| colorants | Colorant 1 0.62g% | Colorant 1 0.62g% | Colorant 2 0.5g% |
| LUPASOL: polymère thiolé obtenu par réaction du Poly(éthylèneimine) (PM=25000) et du N-Acétyl-D,L-homocystéine thiolactone dans un rapport molaire de 1/80* | 1 g % (MA) | - | 1 g % (MA) |
| hydroxyethylcellulose | 0,72g | 0,72g | 0,72 g |
| Mélange de p-hydroxybenzoates de méthyle/ éthyle/ propyle/ isobutyle (7/57/22/14). | 0,06 g | 0,06 g | 0,06 g |
| Alkyl (C8/C10 50/50) polygluocosde (2) en solution aqueuse à 60 % tamponnée | 5 g | 5 g | 5 g |
| Alcool benzylique | 4 g | 4 g | 4 g |
| Polyéthylène glycol (8 OE) | 6 g | 6 g | 6 g |
| 511 | Qsp 100 | Qsp 100 | Qsp 100 |
| *(*) le polymère de l'exemple* a *été obtenu par réaction entre la polyéthylène imine et une thiolactone. L'ouverture du cycle thiolactone entraînant d'une part la connexion de la chaîne (formation d'une liaison amido) et la libération de la fonction thiol (conformément aux explications de la demande de* brevet US2002/108188*).* | | | |

Mode opératoire

Application sur mèches de cheveux 90% blancs naturels/ blancs permanentés (BN/BP), sur cheveux sensibilisés SA 42% et uniquement pour la composition 3 sur cheveux châtains.

Pour les compositions 1 et 3 :

Les compositions colorantes sont appliquées sur les cheveux suivies d'un temps de pause 20 minutes à température ambiante.

Pour la composition 2 (comparative) :

Les mèches de cheveux sont immergées dans une solution aqueuse contenant 0,05 M d'acide thioglycolique pendant 10 minutes, suivi d'un rinçage à l'eau et de shampooings. Sur les mèches ainsi traitées on applique alors la composition colorante 2 suivi d'un temps de pause 20 minutes à température ambiante.

Résultats colorimétriques

La couleur des mèches avant et après coloration a été évaluée dans le système L*a*b* au moyen d'un spectrophotomètre CM2600D MINOLTA®.

Dans le système L* a* b*, les trois paramètres désignent respectivement l'intensité (L*), la nuance (a*) et la saturation (b*). Selon ce système, plus la valeur de L est élevée, plus la couleur est claire ou peu intense. Inversement, plus la valeur de L est faible, plus la couleur est foncée ou très intense. a* et b* indiquent deux axes de couleurs, a* indique l'axe de couleur vert/rouge et b* l'axe de couleur bleu/jaune.

Les mèches obtenues sont colorées en rouge de façon puissante et homogène. Les colorations sont très tenaces aux lavages.

Les mèches sont évaluées avant et après la teinture dans le système L*a*b*.

Les mèches colorées sont soumises à 12 shampooings selon un cycle qui comprend le mouillage des mèches à l'eau, le lavage aux shampooings, un rinçage à l'eau suivi d'un séchage.

La couleur des mèches avant et après les 12 lavages a été évaluée dans le système L*a*b*. La variation de

la couleur avant et après lavages a été mesurée par ΔE selon l'équation ci dessus à partir des valeurs de Lo*ao*bo* des mèches colorées et des valeurs de L*a*b* obtenues après 12 shampooings.

$$\Delta E = \sqrt{(L^* - L_o{}^*)^2 + (a^* - a_o{}^*)^2 + (b^* - b_o{}^*)^2}$$

Plus la valeur de ΔE est importante, plus la différence de couleur avant et après lavages est importante, et dans le cas présent, moins la coloration est résistante aux shampooings.

Les résultats sont regroupés dans les tableaux ci-dessous :

**Exemple 1 :**

| mèche BN traitée avec composition 1, 2 ou 3 / après 12 shampoings | L* (D65) | a* (D65) | b* (D65) | ΔE*ab (D65) | Dégradation lavage (ΔE) | % Dégradation lavage |
|---|---|---|---|---|---|---|
| BN + composition **1** | 28,98 | 32,39 | 17,68 | 46,26 | - | - |
| BN + composition **1** + 12 shampooings | 31,2 | 36,21 | 18,94 | 47,45 | 4,59 | 9,9 |
| BN + composition **2** | 28,4 | 30,62 | 15,7 | 45,49 | - | - |
| BN + composition **2** + 12 shampooings | 30,29 | 37,55 | 19,41 | 49,09 | 8,08 | 17,8 |
| mèche BP traitée avec composition 1, 2 ou 3 / après 12 shampoings | L* (D65) | a* (D65) | b* (D65) | ΔE*ab (D65) | Dégradation lavage (ΔE) | % Dégradation lavage |
| BP + composition **1** | 26,87 | 28,51 | 15,37 | 40,92 | | |
| BP + composition **1** + 12 shampooings | 28,33 | 33,49 | 19,07 | 43,67 | 6,37 | 15,6 |
| BP + composition **2** | 26,43 | 26,55 | 14,42 | 39,97 | | |
| BP + composition **2** + 12 shampooings | 28,68 | 34,09 | 18,08 | 43,77 | 8,68 | 21,7 |
| mèche SA traitée avec composition 1, 2 ou 3 / après 12 shampoings | L* (D65) | a* (D65) | b* (D65) | ΔE*ab (D65) | Dégradation lavage (ΔE) | % Dégradation lavage |
| SA42 + composition **1** | 24,41 | 16,93 | 10,42 | 42,44 | | |
| SA42 + composition **1** + 12 shampooings | 23,65 | 22,07 | 13,59 | 42,91 | 6,09 | 14,3 |
| SA42 + composition **2** | 26,49 | 11,8 | 10,36 | 40,06 | | |
| SA42 + composition **2** + 12 shampooings | 24,15 | 24,7 | 12,13 | 43,94 | 13,23 | 33,0 |

**Exemple 2 :**

| mèche châtain traitée avec composition 3 / après 12 shampoings | L* (D65) | a* (D65) | b* (D65) | ΔE*ab (D65) | Dégradation lavage (ΔE) | % Dégradation lavage |
|---|---|---|---|---|---|---|
| Châtain HT4 avant traitement | 20,18 | 2,92 | 2,71 | | | |
| Châtain + composition **3** | 22,51 | 7,61 | 7,15 | 6,87 | | |
| Châtain + composition **3** + 12 shampooings | 21,9 | 7,12 | 7,13 | 6,34 | 0,78 | 11,4 |

Il apparaît à partir des résultats des exemples 1 et 2 de coloration, que les fibres kératiniques sont colorées de façon chromatique. De plus les colorations obtenues à partir de la composition et du procédé selon l'invention sont plus tenace vis-à-vis des shampoings successifs par rapport au comparatif.

## Revendications

1. Composition tinctoriale, comprenant dans un milieu cosmétique approprié, i) un ou plusieurs colorants choisis parmi les colorants directs à fonction(s) disulfure(s), et fonction(s) thiol(s) protégé(s) et ii) au moins un ou plusieurs polymère(s) à fonction(s) thiol(s) de poids moléculaire supérieur à 500.

2. Composition selon la revendication précédente dans laquelle le ou les colorants sont de formule **(I)** :

$$\mathbf{A\text{-}(X)_p\text{-}C_{sat}\text{-}S\text{-}U} \qquad \mathbf{(I)}$$

ses sels d'acide organique ou minéral, ses isomères optiques, géométriques et leurs solvates tels que les hydrates, formule **(I),** dans laquelle :

• **U** représente un radical choisi parmi :

**i) - S - $C'_{sat}$ - $(X')_{p'}$ - A'** ;
**ii) - S - $C'_{sat}$ - $(X')_{p'}$ - D** ; et
**iii) - Y** ;

• **A** et **A**', identiques ou différents, représentent un radical contenant un ou plusieurs chromophore(s) cationique(s) ou non ;
• **Y** représente ; a) un métal alcalin ; b) un métal alcalino-terreux ; c) un groupement ammonium : $N^+R^\alpha R^\beta R^\gamma R^\delta$, $An''^-$ ou un groupement phosphonium : $P^+R^\alpha R^\beta R^\gamma R^\delta$, $An''^-$ avec $R^\alpha$, $R^\beta$, $R^\gamma$ et $R^\delta$, identiques ou différents, représentant un atome d'hydrogène ou un groupement $(C_1\text{-}C_4)$alkyle et , $An''^-$ représentant un contre-ion anionique ; ou d) un groupement protecteur de fonction thiol ;
• **X** et **X**', identiques ou différents, représentent :

a) une chaîne hydrocarbonée en $C_1$-$C_{30}$, linéaire ou ramifiée, saturée ou insaturée, éventuellement substituée, éventuellement interrompue et/ou éventuellement terminée à une ou aux deux extrémités de ladite chaine par un ou plusieurs groupes divalents ou leur combinaisons choisis parmi :

i)-N(R)-; $-N^+(R)(R')-$, $Q^-$; -O- ; -S- ; -CO- ; $-SO_2-$ avec R, R', identiques ou différents, sont choisis parmi un hydrogène, un radical alkyle en $C_1$-$C_4$, hydroxyalkyle et aminoalkyle, $Q^-$ représente un contre-ion anionique ; et ii) un radical (hétéro)cyclique, aromatique ou non, saturé ou insaturé, condensé ou non, comprenant éventuellement un ou plusieurs hétéroatomes identiques ou non, éventuellement substitué ;

ou
b) un ou plusieurs groupes divalents ou leur combinaisons choisis parmi :

$-SO_2-$ ; -O- ; -S- ; -N(R)- ; -N+(R)(R°)- ; -CO- ; avec R, R°, identiques ou différents, représentant un atome d'hydrogène, un radical alkyle en $C_1$-$C_4$, hydroxyalkyle en $C_1$-$C_4$ ou un aryl($C_1$-$C_4$)alkyle ; et un radical hétérocycloalkyle ou hétéroaryle, cationique ou non;

• **p** et **p',** identiques ou différents, valent 0 ou 1 ;
• **$C_{sat}$** et **$C'_{sat}$,** identiques ou différents représentent une chaîne alkylène en $C_1$-$C_{18}$, linéaire ou ramifiée, éventuellement substituée, éventuellement cyclique ;
• **D** correspond à un radical choisi parmi les radicaux hydroxy, hydroxyalkyle, alkoxy, carboxyliques, carboxylates, amino, alkylamino, et dialkylamino.

3. Composition selon la revendication précédente dans laquelle **$C_{sat}$** et **$C'_{sat}$,** représentent une chaîne $-(CH_2)_k-$ avec k entier, compris inclusivement entre 1 et 8.

**4.** Composition selon une quelconque des revendications 2 ou 3, dans laquelle p est égal à 1, X et X', identiques ou différents, représentent la séquence suivante :

$$-(T)_t-(Z)_z-(T)_{t'}-$$

ladite séquence étant reliée dans les formules (I) ou (II) de façon symétrique comme suit :

$$- C_{sat} \text{ (ou } C'_{sat})-(T)_t-(Z)_z-(A \text{ ou } A') ;$$

dans laquelle :

**T** et **T',** identiques ou différents, représentent un ou plusieurs radicaux ou leurs combinaisons choisis parmi : -O- ; -S- ; -N(R)- ; $-N^+(R)(R°)-$ ;-SO- ; $-SO_2-$ ; et -CO- ; avec R, R°, identiques ou différents, représentant un atome d'hydrogène, un radical alkyle en $C_1$-$C_4$, hydroxyalkyle en $C_1$-$C_4$ ou un aryl($C_1$-$C_4$)alkyle ; et un radical hétérocycloalkyle ou hétéroaryle, cationique ou non, préférentiellement monocyclique, contenant préférentiellement deux hétéroatomes;

**t** et **t',** identiques ou différents, valent 0 ou 1 ;

**Z** représente :

- $-(CH_2)_m-$ avec m entier compris entre 1 et 8
- $-(CH_2CH_2O)_q-$ ou $-(OCH_2CH_2)_q-$ dans lesquelles q est un entier compris entre 1 et 15
- un radical aryle, alkylaryle ou arylalkyle dont le radical alkyle est en $C_1$-$C_4$ et le radical aryle est de préférence en $C_6$, étant éventuellement substitué par au moins un groupement $SO_3M$ avec M représentant un atome d'hydrogène, un métal alcalin ou un groupement ammonium ammonium substitué par un ou plusieurs radicaux alkyle, identiques ou non, linéaires ou ramifiés, en $C_1$-$C_{18}$ éventuellement porteurs d'au moins un hydroxy

**z** vaut 0 ou 1.

**5.** Composition selon une quelconque des revendications précédentes dans laquelle le colorant direct à fonction disulfure est choisi parmi les colorants de formule **(III)** à **(XIX) :**

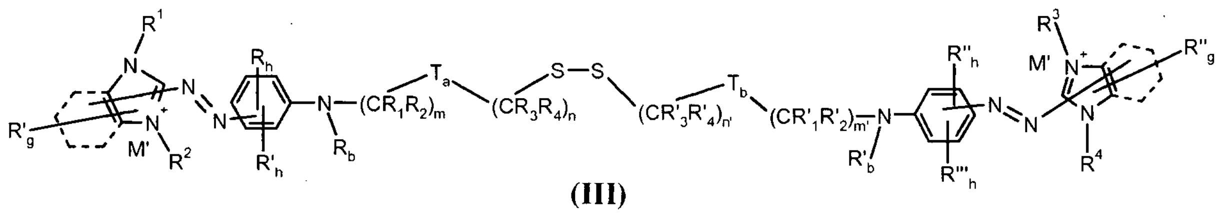

**(III)**

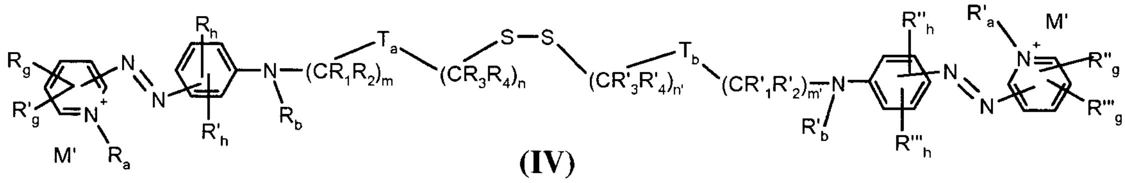

**(IV)**

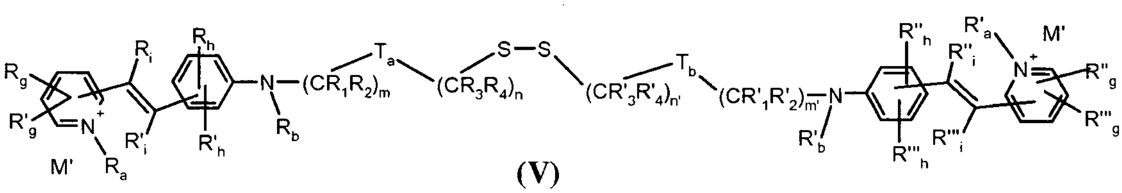

**(V)**

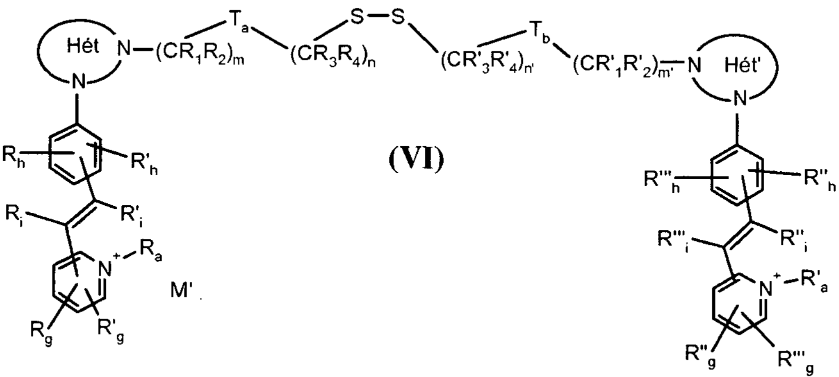

(VI)

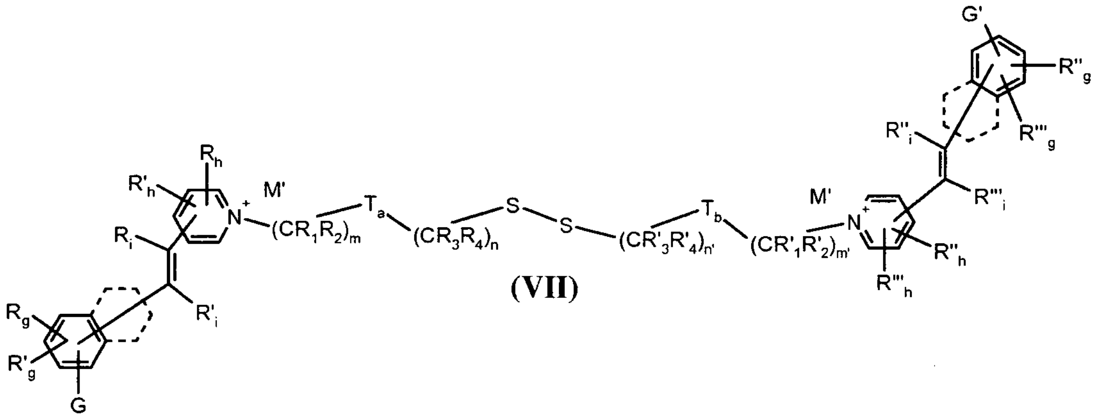

(VII)

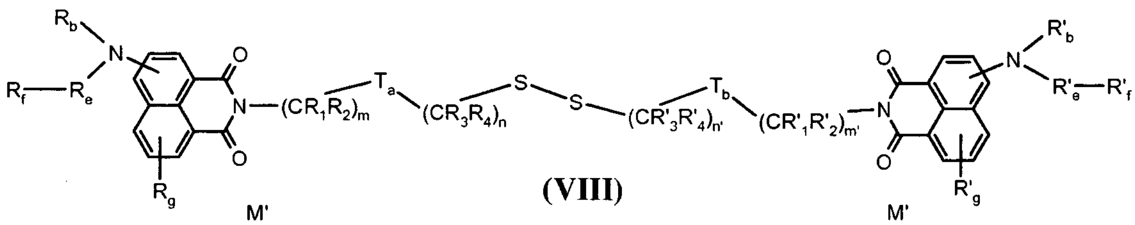

(VIII)

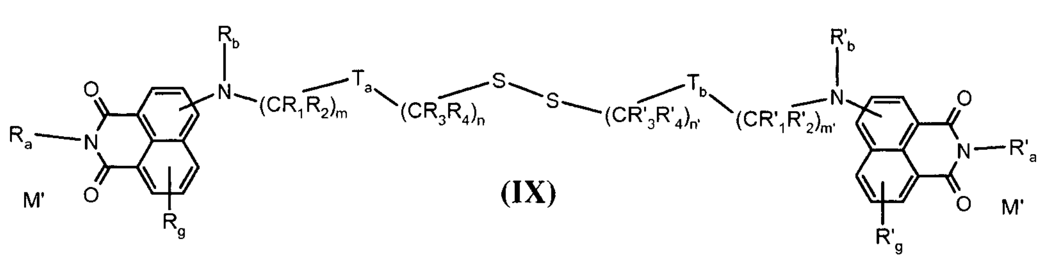

(IX)

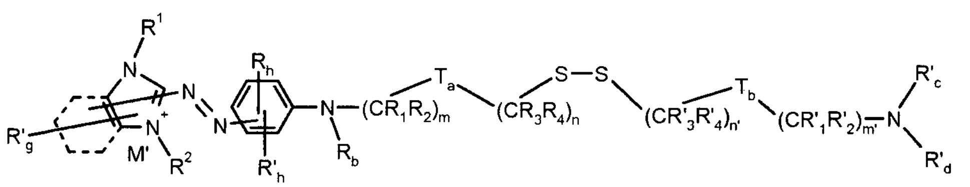

(X)

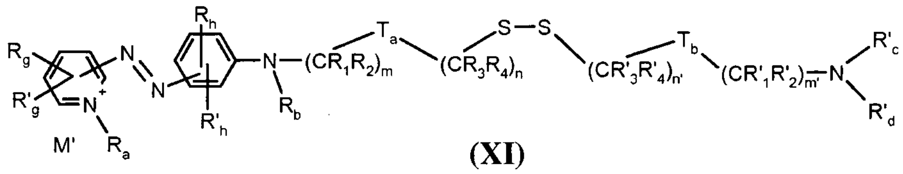

(XI)

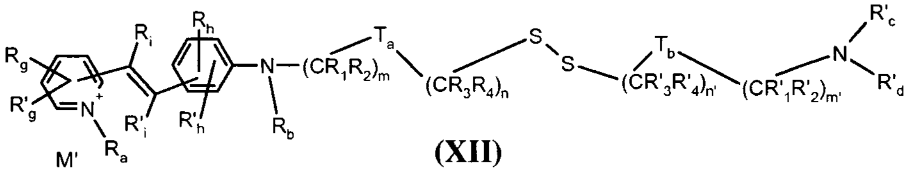

(XII)

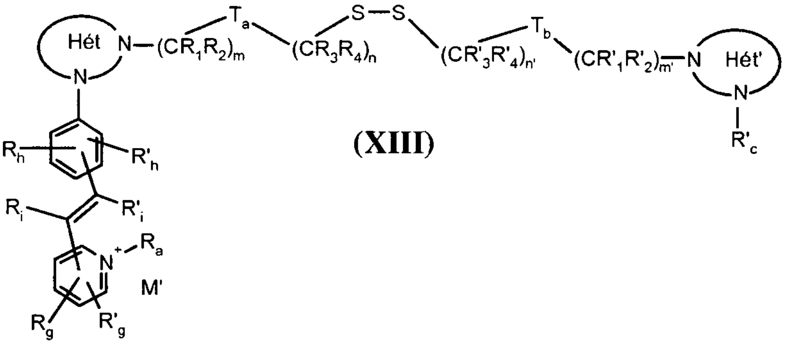

(XIII)

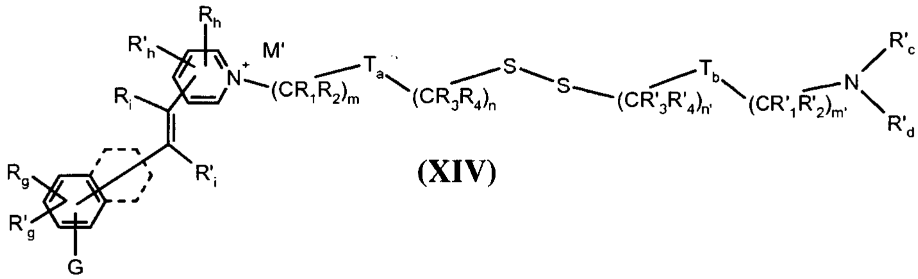

(XIV)

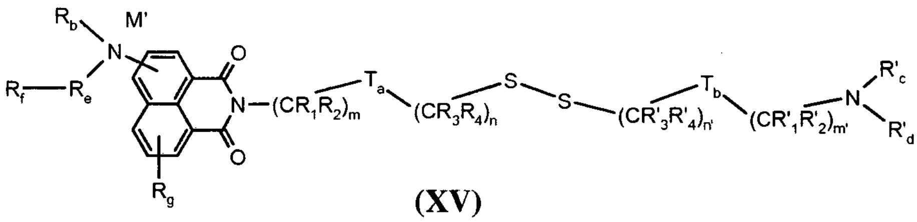

(XV)

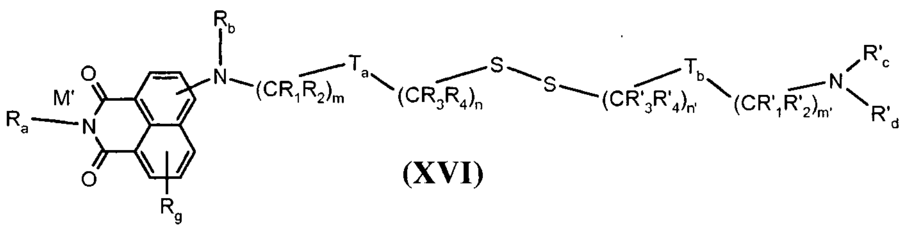

(XVI)

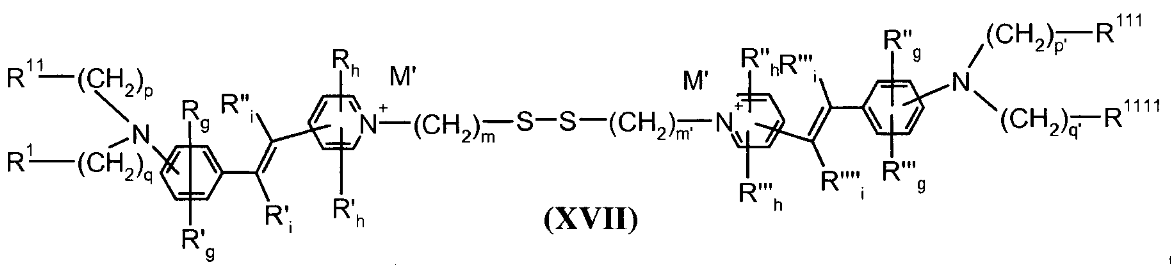

(XVII)

et

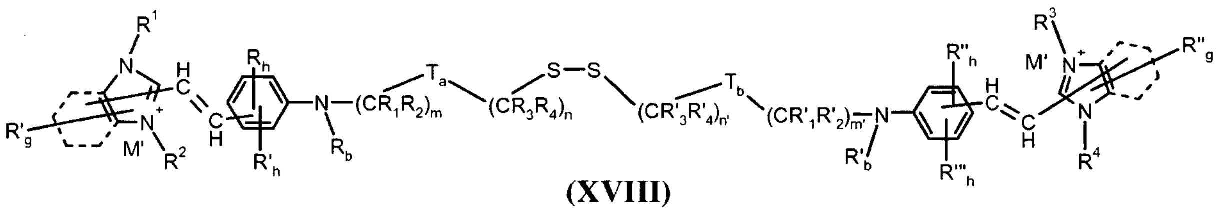

(XVIII)

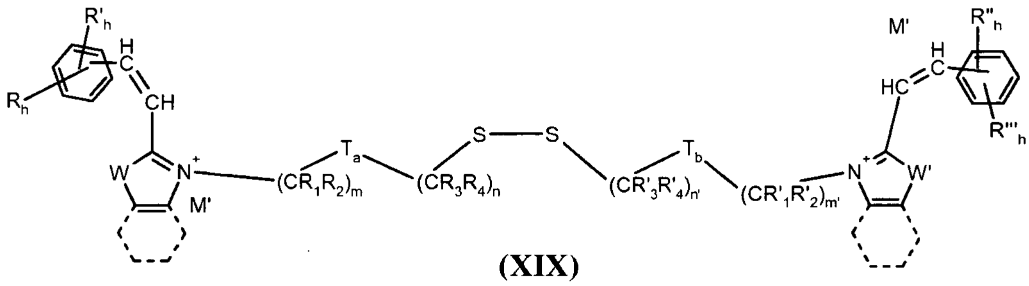

(XIX)

dans lequel

- G et G', identiques ou différents, représentent un groupement $-NR_cR_d$, $-NR'_cR'_d$, ou $C_1$-$C_6$ alkoxy éventuellement substitué, préférentiellement non substitué ; préférentiellement G et G' représentent un groupement $-NR_cR_d$ et $-NR'_cR'_d$ respectivement ;
- W et W', identiques ou différents, représentent un atome d'oxygène ou un groupement N-R1, avec R1 tel que défini ci après ;
- $R^1$, $R^2$, $R^3$ et $R^4$, identiques ou différents, représentent un atome d'hydrogène ou ub groupement alkyle $C_1$-$C_6$ alkyle ;
- $R_a$ et $R'_a$, identiques ou différents, représentent un groupement aryl($C_1$-$C_4$)alkyle ou un groupement $C_1$-$C_6$ alkyle éventuellement substitué par un groupement hydroxy ou amino, $C_1$-$C_4$ alkylamino, $C_1$-$C_4$ dialkylamino, lesdits radicaux alkyles pouvant former avec l'atome d'azote qui les portent un hétérocycle comprenant de 5 à 7 chaînons, comprenant éventuellement un autre hétéroatome différent ou non de l'azote ; préférentiellement $R_a$ et $R'_a$ représentent un groupement $C_1$-$C_3$ alkyle éventuellement substitué par un groupement hydroxy, ou un groupement benzyle ;
- $R_b$, $R'_b$, identiques ou différents, représentent un atome d'hydrogène, un groupement aryl($C_1$-$C_4$)alkyle ou un groupement $C_1$-$C_6$ alkyle éventuellement substitué; préférentiellement $R_b$, $R'_b$ représentent un atome d'hydrogène ou un groupement $C_1$-$C_3$ alkyle ou benzyle ;
- $R_c$, $R'_c$, $R_d$ et $R'_d$, identiques ou différents, représentent un atome d'hydrogène, un groupement aryl($C_1$-$C_4$)alkyle, $C_1$-$C_6$ alkoxy ou un groupement $C_1$-$C_6$ alkyle éventuellement substitué ; $R_c$, $R'_c$, $R_d$ et $R'_d$ représentent préférentiellement un atome d'hydrogène, un groupement hydroxy, $C_1$-$C_3$ alkoxy, amino, $C_1$-$C_3$ (di)alkylamino, ou un groupement $C_1$-$C_3$ alkyle éventuellement substitué par i) un groupement hydroxy, ii) amino, iii) $C_1$-$C_3$ (di)alkylamino, ou iv) ammonium quaternaire (R")(R"')(R"")$N^+$ -; ou alors deux radicaux adjacents $R_c$ et $R_d$, $R'_c$ et $R'_d$ portés par le même atome d'azote forment ensemble un groupe hétérocyclique ou hétéroaryle ; préférentiellement l'hétérocycle ou l'hétéroaryle est monocyclique et comprend entre 5 et 7 chaînons ; plus préférentiellement les groupements sont choisis parmi l'imidazolyle et le pyrrolidinyle ;

• $R_e$ et $R'_e$, identiques ou différents, représentent une chaîne hydrocarbonée en $C_1$-$C_6$ alkylènyle divalente, linéaire ou ramifiée, éventuellement insaturée ;

• $R_f$ et $R'_f$, identiques ou différents, représentent un groupement ammonium quaternaire (R'')(R''')(R'''')$N^+$ - où R'', R''' et R'''', identiques ou différents, représentent un atome d'hydrogène ou un groupement $C_1$-$C_4$ alkyle ou alors (R'')(R''')(R'''')$N^+$ - représente un groupement hétéroaryle cationique éventuellement substitué préférentiellement un groupement imidazolinium éventuellement substitué par un groupement $C_1$-$C_3$ alkyle ;

• $R_g$, $R'_g$, $R''_g$, $R'''_g$, $R_h$, $R'_h$, $R''_h$, et $R'''_h$, identiques ou différents, représentent un atome d'hydrogène, un atome d'halogène, un groupement amino, $C_1$-$C_4$ alkylamino, $C_1$-$C_4$ dialkylamino, cyano, carboxy, hydroxy, trifluorométhyle, un radical acylamino, alcoxy en $C_1$-$C_4$, (poly)hydroxyalcoxy en $C_2$-$C_4$, alkylcarbonyloxy alcoxycarbonyle, alkylcarbonylamino, un radical acylamino, carbamoyle, alkylsulfonylamino, un radical amino-sulfonyle, ou un radical $C_1$-$C_{16}$ alkyle éventuellement substitué par un groupement choisi parmi $C_1$-$C_{12}$ alcoxy, hydroxy, cyano, carboxy, amino, $C_1$-$C_4$ alkylamino et $C_1$-$C_4$ dialkylamino, ou alors les deux radicaux alkyles portés par l'atome d'azote du groupement amino forment un hétérocycle comprenant de 5 à 7 chaînons et comprenant éventuellement un autre hétéroatome identique ou différent à celui de l'atome d'azote ; préférentiellement $R_g$, $R'_g$, $R''_g$, $R'''_g$, $R_h$, $R'_h$, $R''_h$, et $R'''_h$ représentent un atome d'hydrogène, d'halogène ou un groupement $C_1$-$C_3$ alkyle ;

• ou alors deux groupements $R_g$ et $R'_g$ ; $R''_g$ et $R'''_g$ ; $R_h$, et $R'_h$ ; $R''_h$ et $R'''_h$ portés par deux atomes de carbone adjacents, forment ensembles une cycle benzo, indéno, un groupement hétérocycloalkyle fusionné ou hétéroaryle fusionné; le cycle benzo, indéno, hétérocycloalkyle ou hétéroaryle étant éventuellement substitué par un atome d'halogène, un groupement amino, $C_1$-$C_4$ alkylamino, $C_1$-$C_4$ dialkylamino, nitro, cyano, carboxy, hydroxy, trifluorométhyle, un radical acylamino, alcoxy en $C_1$-$C_4$, (poly)hydroxyalcoxy en $C_2$-$C_4$, alkylcarbonyloxy alcoxycarbonyle, alkylcarbonylamino, un radical acylamino, carbamoyle , alkylsulfonylamino, un radical amino-sulfonyle, ou un radical $C_1$-$C_{16}$ alkyle éventuellement substitué par : un groupement choisi parmi $C_1$-$C_{12}$ alcoxy, hydroxy, cyano, carboxy, amino, $C_1$-$C_4$ alkylamino, $C_1$-$C_4$ dialkylamino, ou alors les deux radicaux alkyles portés par l'atome d'azote du groupement amino forment un hétérocycle comprenant de 5 à 7 chaînons et comprenant éventuellement un autre hétéroatome identique ou différent à celui de l'atome d'azote ; préférentiellement $R_g$ et $R'_g$ ; $R''_g$ et $R'''_g$ forment ensemble un groupement benzo ;

• ou alors lorsque G représente -$NR_cR_d$ et G' représente -$NR'_cR'_d$ deux groupements $R_c$ et $R'_g$ ; $R'_c$ et $R''_g$ ; $R_d$ et $R_g$ ; $R'_d$ et $R'''_g$ forment ensemble un hétéroaryle ou hétérocycle saturé, éventuellement substitué par un ou plusieurs groupement $C_1$-$C_6$ alkyle, préférentiellement un l'hétérocycle contenant un ou deux hétéroatomes choisis parmi l'azote et l'oxygène et comprenant entre 5 et 7 chaînons ; plus préférentiellement l'hétérocycle est choisi parmi les groupement morpholinyle, pypérazinyle, pypéridinyle et pyrrolidinyle ;

• $R_i$, $R'_i$, $R''_i$, et $R'''_i$, identiques ou différents, représentent un atome d'hydrogène, ou un groupement $C_1$-$C_4$ alkyle ;

• $R_1$, $R_2$, $R_3$, $R_4$, $R'_1$, $R'_2$, $R'_3$, et $R'_4$, identiques ou différents, représentent un atome d'hydrogène ou un groupement $C_1$-$C_4$ alkyle, $C_1$-$C_{12}$ alkoxy, hydroxy, cyano, carboxy, amino, $C_1$-$C_4$ alkylamino, $C_1$-$C_4$ dialkylamino, lesdits radicaux alkyles pouvant former avec l'atome d'azote qui les portent un hétérocyle comprenant de 5 à 7 chaînons, comprenant éventuellement un autre hétéroatome différent ou non de l'azote ; préférentiellement $R_1$, $R_2$, $R_3$, $R_4$, $R'_1$, $R'_2$, $R'_3$, et $R'_4$ sont des atomes d'hydrogène ou un groupement amino ; plus préférentiellement $R_1$, $R_2$, $R_3$, $R_4$, $R'_1$, $R'_2$, $R'_3$, et $R'_4$ représentent un atome d'hydrogène;

• $T_a$, $T_b$, identiques ou différents, représentent i) soit une liaison covalente $\sigma$, ii) soit un ou plusieurs radicaux ou leurs combinaisons choisis parmi -$SO_2$-, -O-, -S-,-N(R)-, -$N^+$(R)(R°), -CO-, avec R, R°, identiques ou différents, représentant un atome d'hydrogène, un radical alkyle en $C_1$-$C_4$, hydroxyalkyle en $C_1$-$C_4$; ou un aryl($C_1$-$C_4$)alkyle, préférentiellement $T_a$ est identique à $T_b$ et représentent une liaison covalente $\sigma$ ou un groupement choisi parmi -N(R)-, -C(O)-N(R)-, -N(R)-C(O)-, -O-C(O)-, -C(O)-O- et -$N^+$(R)(R°), avec R, R° identiques ou différents représentant un atome d'hydrogène ou un groupement $C_1$-$C_4$ alkyle ; plus préférentiellement $T_a$ et $T_b$ représentent une liaison $\sigma$ ; iii) soit un radical hétérocycloalkyle ou hétéroaryle, cationique ou non, préférentiellement monocycliques, préférentiellement identiques, contenant préférentiellement deux hétéroatomes (plus préférentiellement deux atomes d'azote) et comportant préférentiellement de 5 à 7 chaînons tel que l'imidazolium;

•

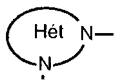

ou

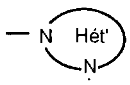

identiques ou différents, représentent une groupement hétérocyclique éventuellement substitué ; préférentiellement les hétérocycles sont identiques, monocycliques, saturés, et comprennent au total deux atomes d'azote et de 5 à 8 chaînons ;

•

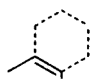

représentent une groupement aryle ou hétéroaryle fusionné au cycle imidazolium ou phényle ; ou alors est absent du cycle imidazolium ou phényle;

• $R^1$ et $R^{1111}$, identiques ou différents, représentent un groupement hydroxy, un groupement amino ($NR_aR_b$) ou ammonium ($N^+R_aR_bR_c$), $An^-$ ; avec $R_a$, $R_b$ et $R_c$, identiques ou différents, représentant un atome d'hydrogène ou un groupement ($C_1$-$C_4$)alkyle ; ou alors deux groupes alkyle $R_a$ et $R_b$ du groupement amino ou ammonium forment un hétérocycle comprenant de 5 à 7 chaînons et comprenant éventuellement un autre hétéroatome identique ou différent à celui de l'atome d'azote tel que morpholinyle, pipérazinyle, pipéridinyle, pyrrolyle, morpholinium, pipérazinium, pipéridinium, pyrrolinium, et $An^-$ représentant un contre-ion anionique ;

• $R^{11}$ et $R^{111}$, identiques ou différents, représentent un atome d'hydrogène ou un groupement tel que défini pour $R^1$ et $R^{1111}$ respectivement ;

• m, m', n et n', identiques ou différents, représentent un entier compris inclusivement entre 0 et 6 avec m+n, m'+n', identiques ou différents, représente un entier compris inclusivement entre 1 et 10; préférentiellement m+n=m'+n'=un entier compris inclusivement entre 2 et 4 ; plus préférentiellement m+n=m'+n'= un entier égal à 2;

M' représentant contre-ion ou un sel d'acide organique ou minéral.

**6.** Composition selon une quelconque des revendications précédentes dans laquelle le colorant direct à fonction disulfure ou thiol protégé est choisi parmi :

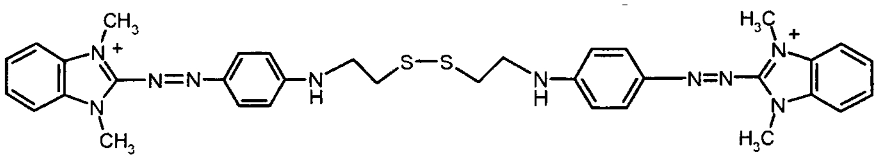

2M'

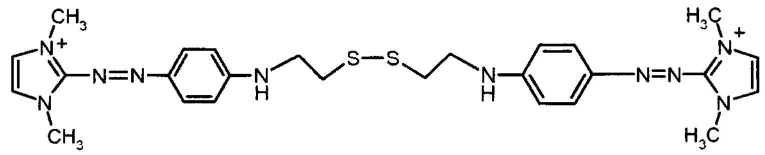

2M'

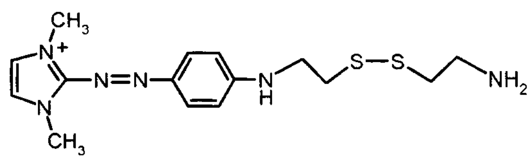

M'

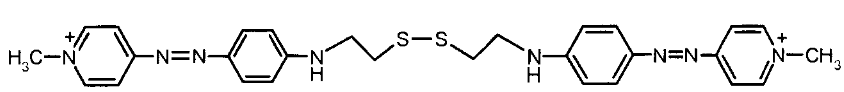

2M'

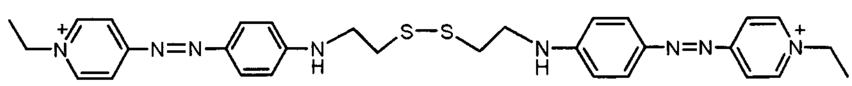

2M'

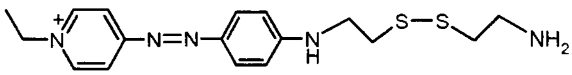

M'

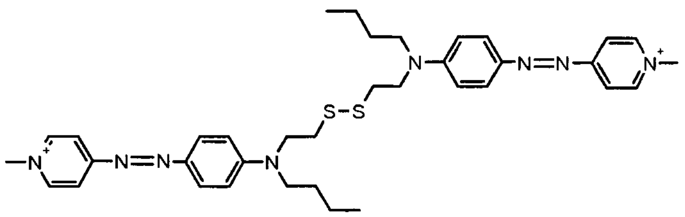

2M'

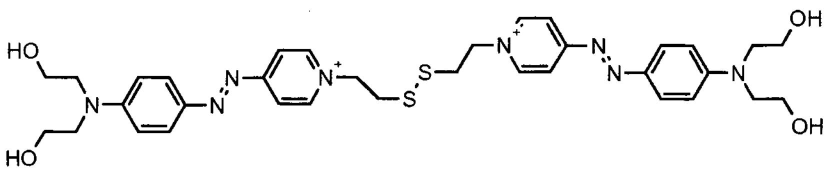

2M'

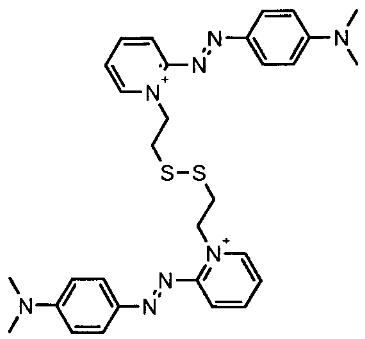

2M'

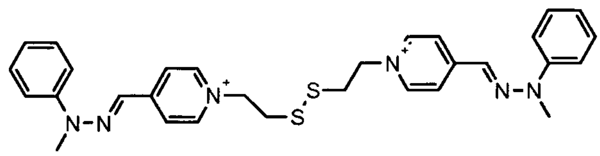

2M'

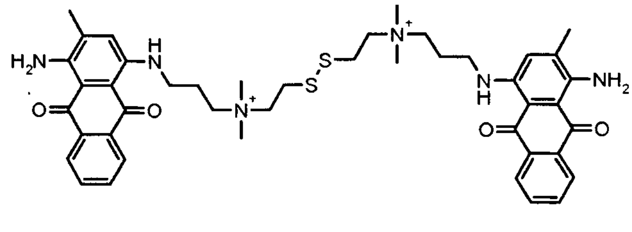

2M'

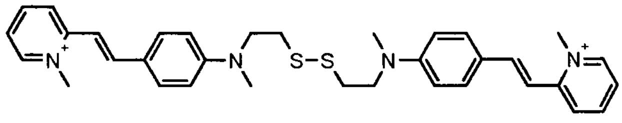

2M'

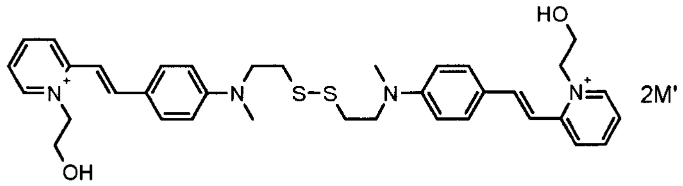

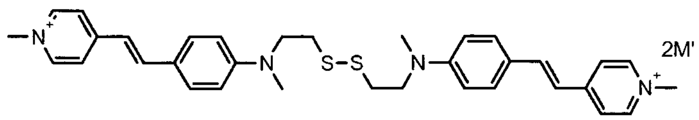

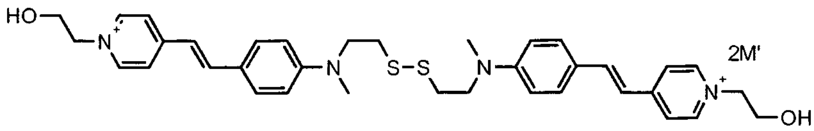

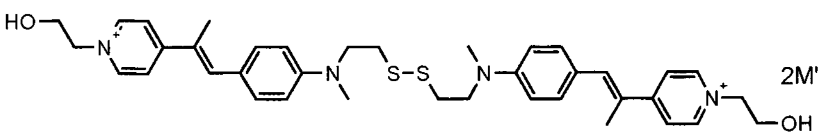

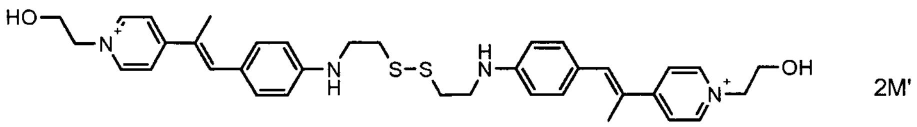

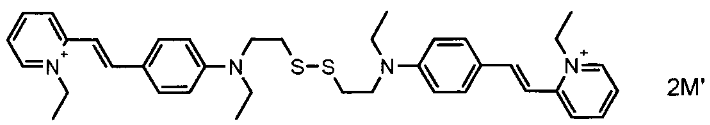

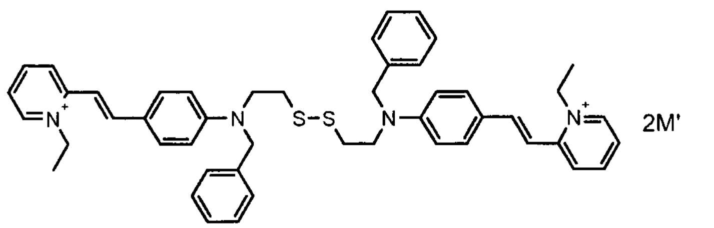

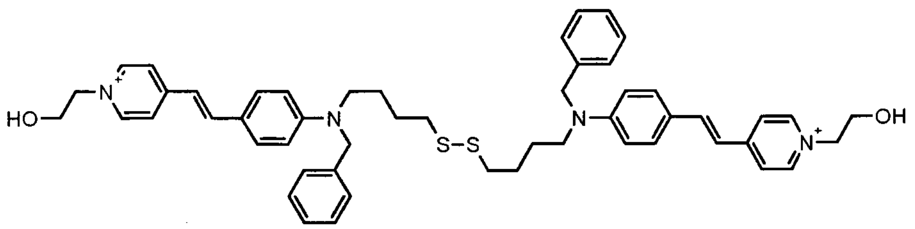

2M'

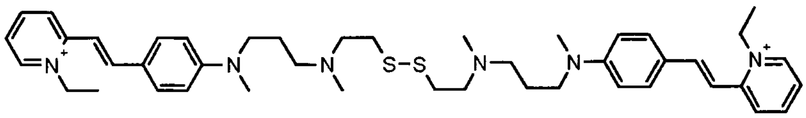

2M'

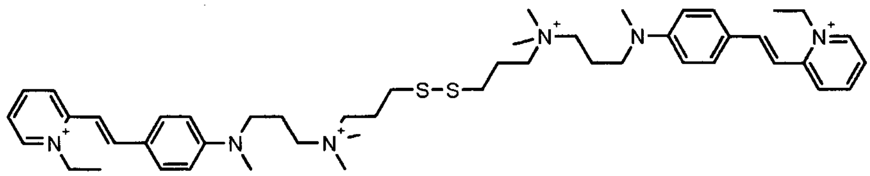

4M'

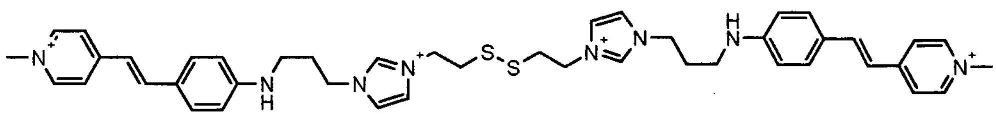

4M'

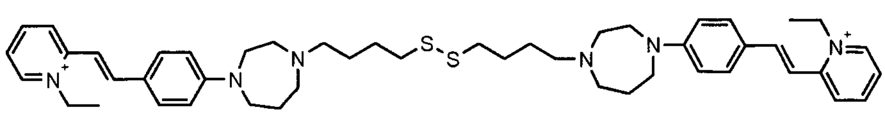

2M'

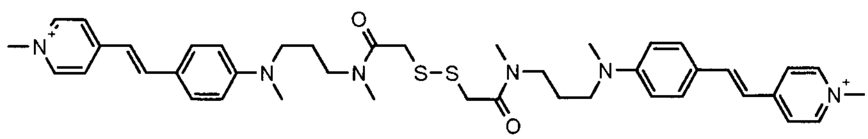

2M'

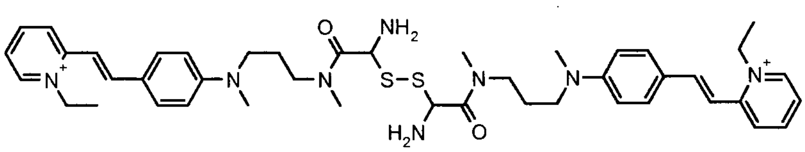

2M'

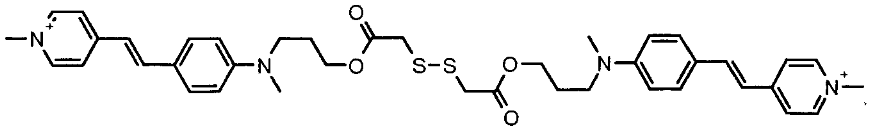

2M'

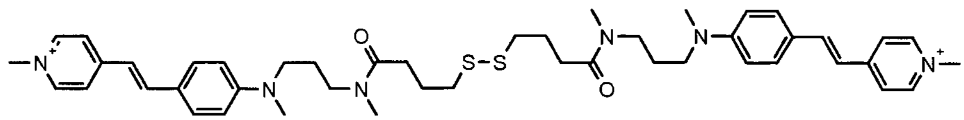

2M'

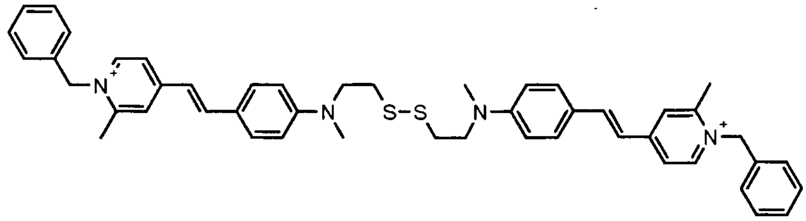

2M'

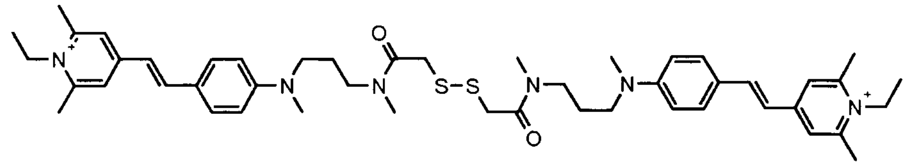

2M'

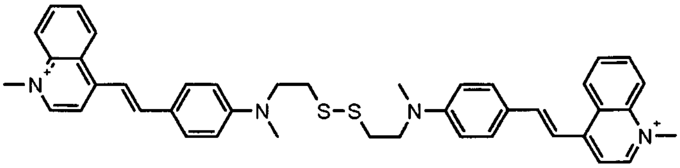

2M'

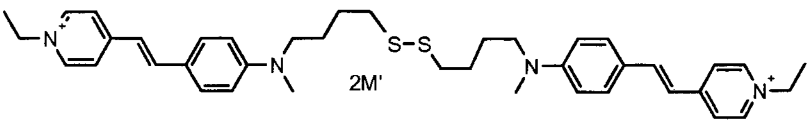

2M'

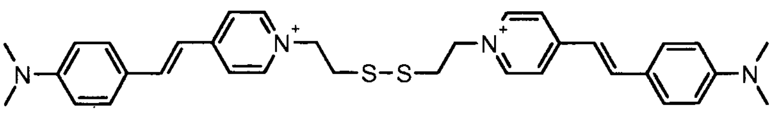

2M'

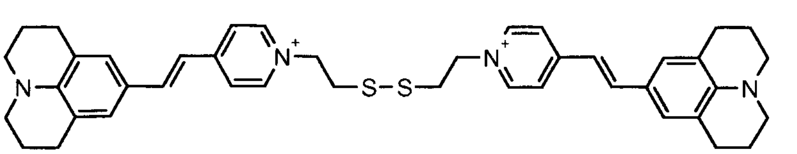

2M'

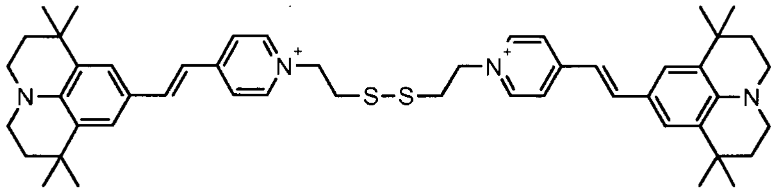

2M'

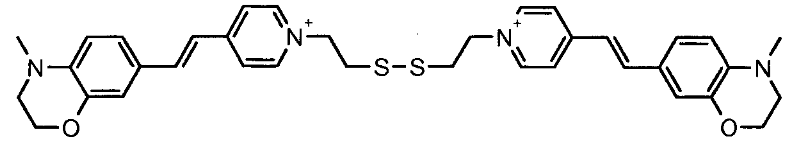

2M'

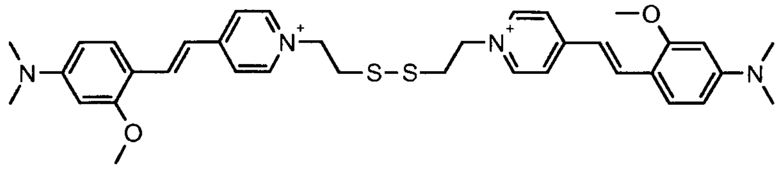

2M'

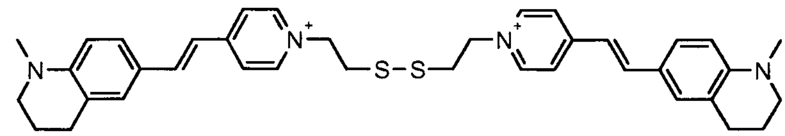

2M'

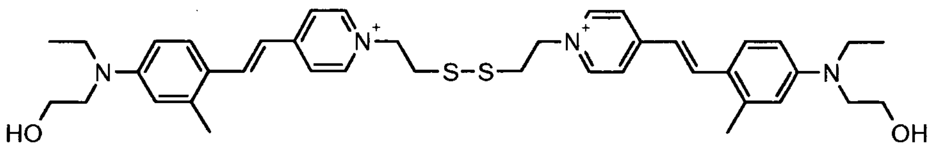

2M'

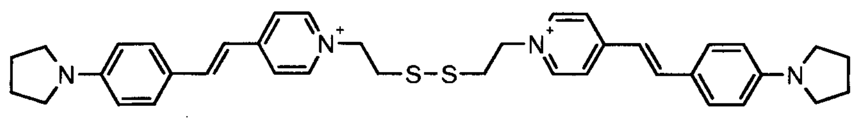

2M'

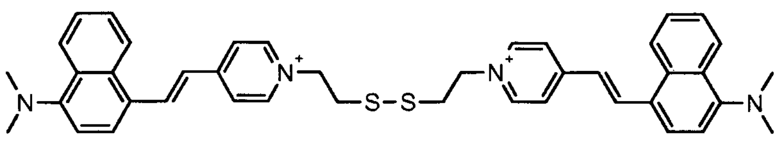

2M'

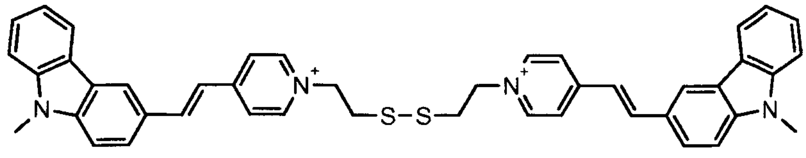

2M'

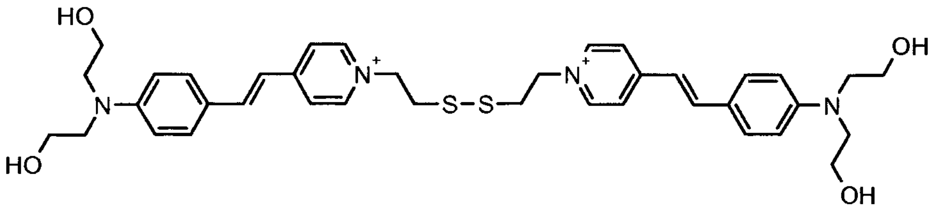

2M'

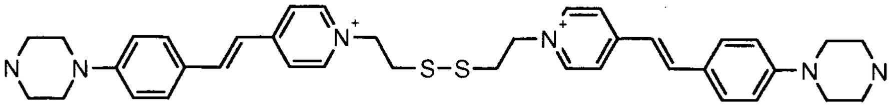

2M'

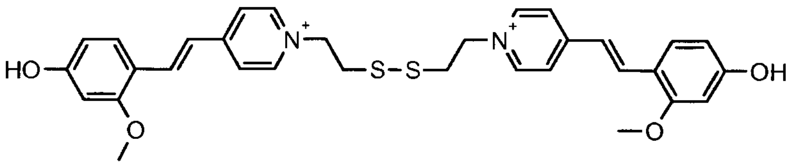

2M'

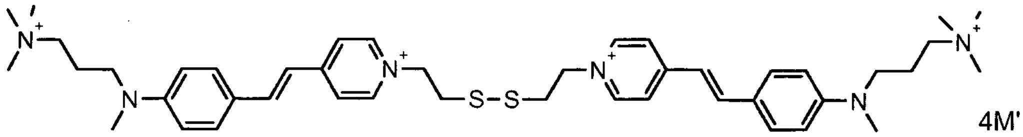

4M'

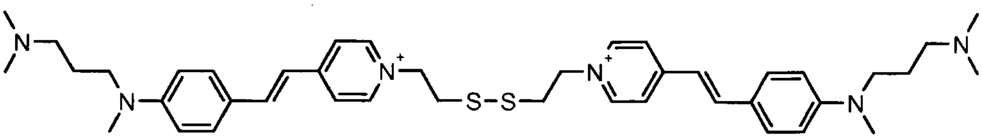

2M'

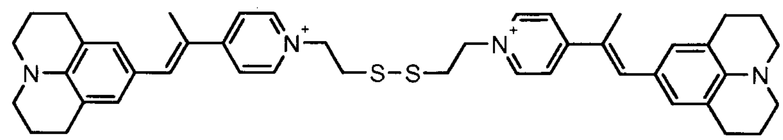

2M'

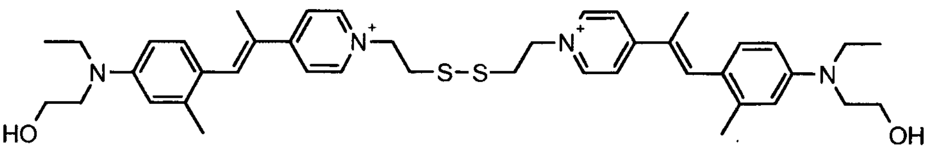

2M'

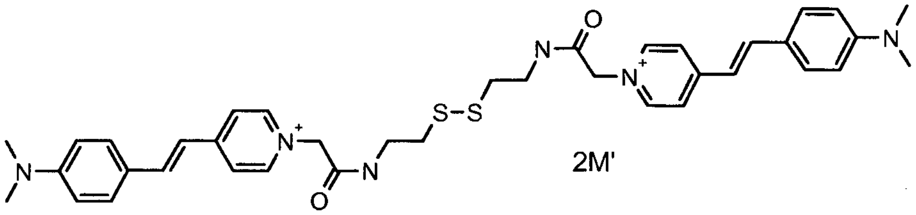

2M'

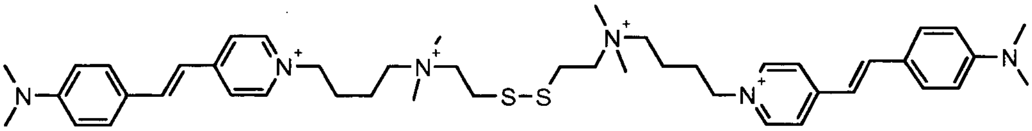

4M'

2M'

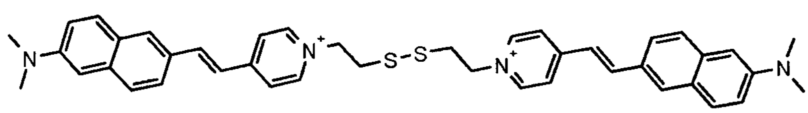

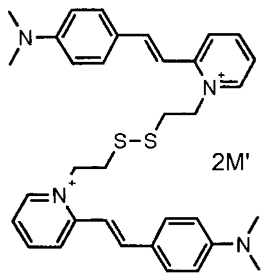

2M'

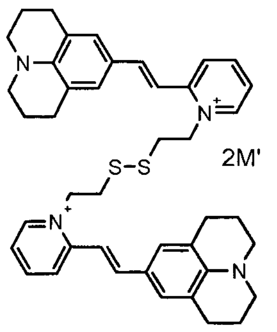

2M'

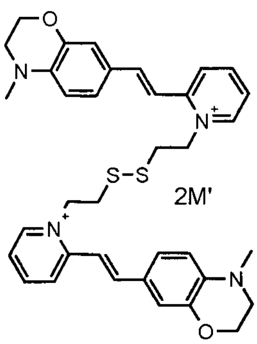

2M'

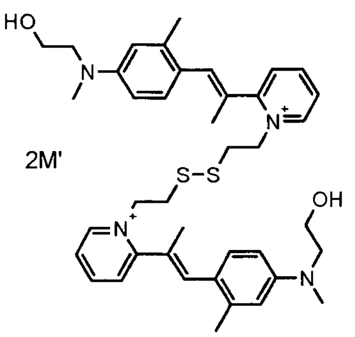

2M'

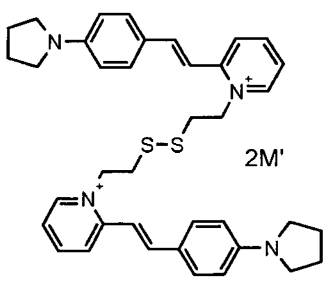

2M'

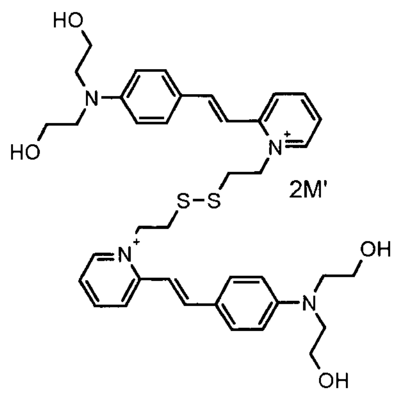

2M'

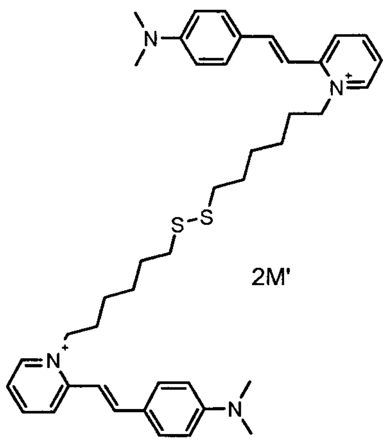

2M'

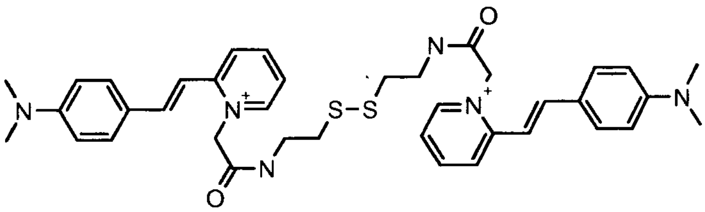

2M'

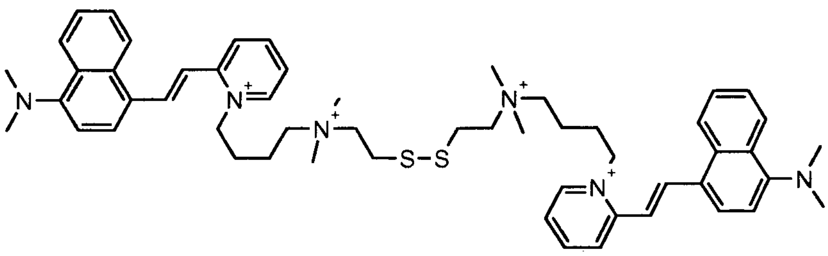

4M'

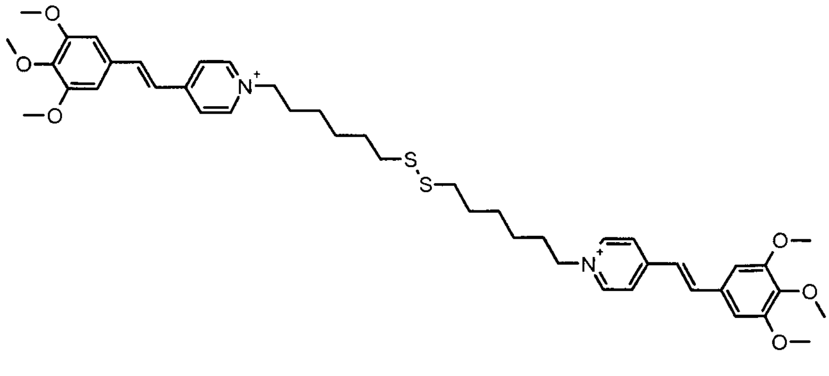

An⁻

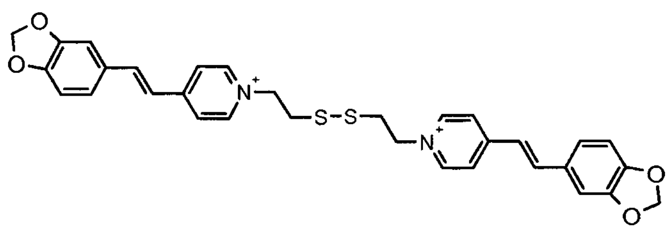

An⁻

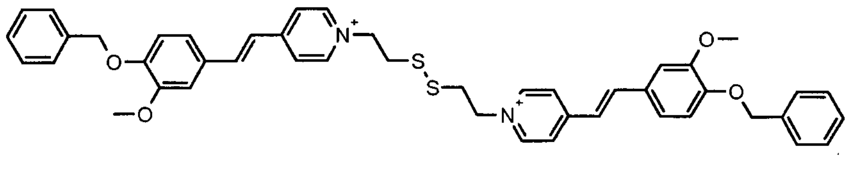

An⁻

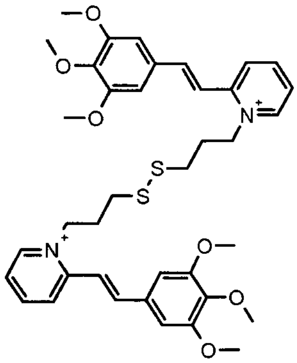

An⁻

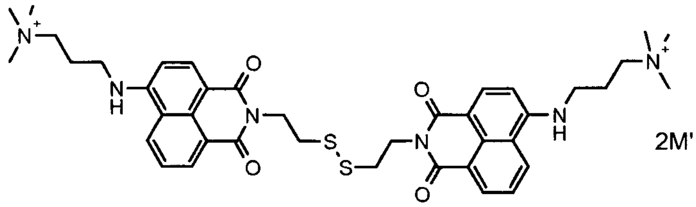

2M'

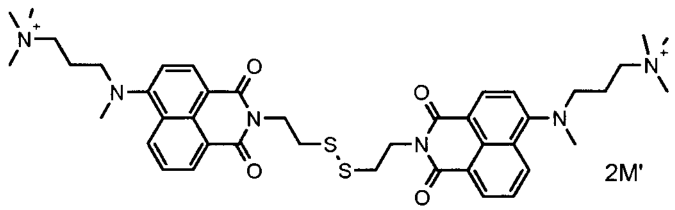

2M'

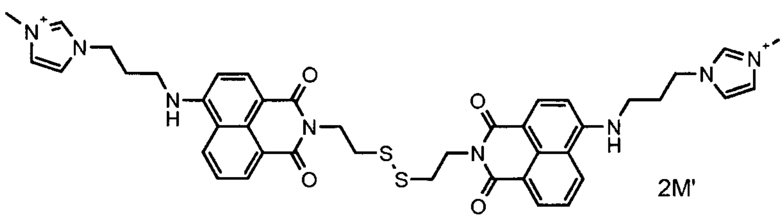

2M'

M'

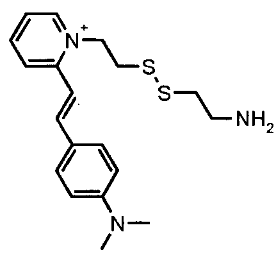

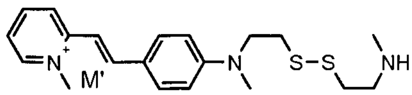

M'

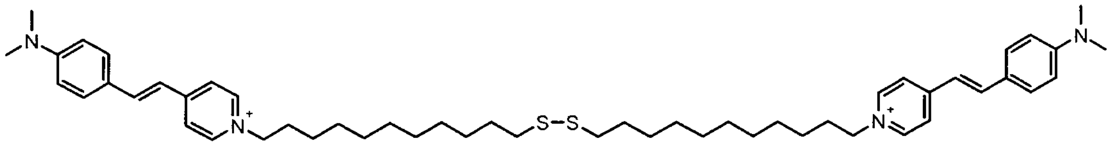

2M'

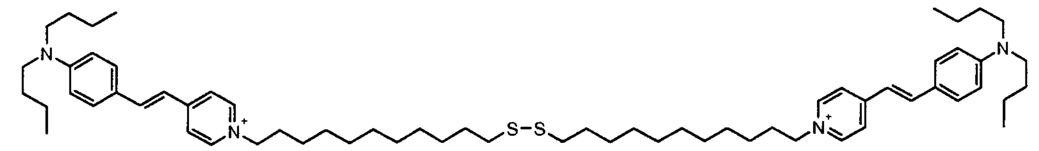

2M'

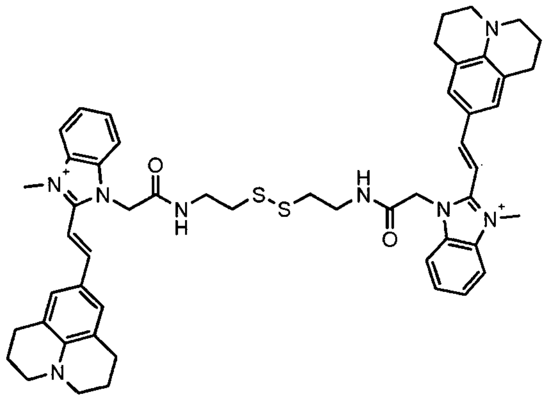

2M'

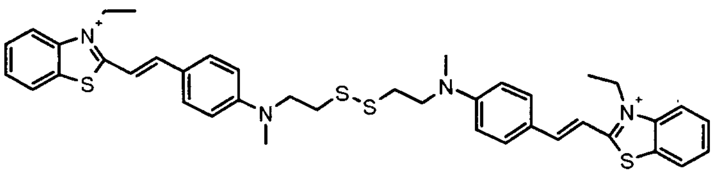

2M'

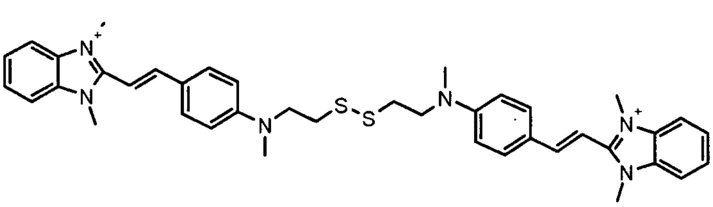

2M'

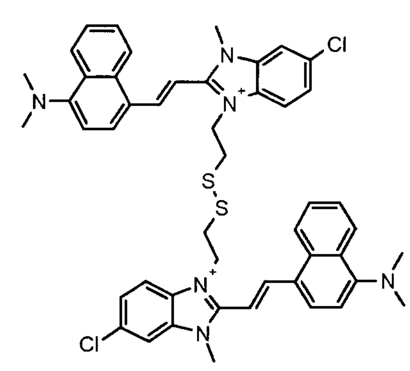

2M'

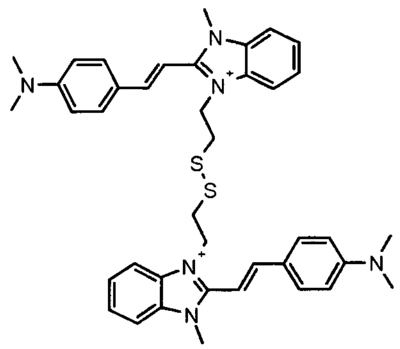

2M'

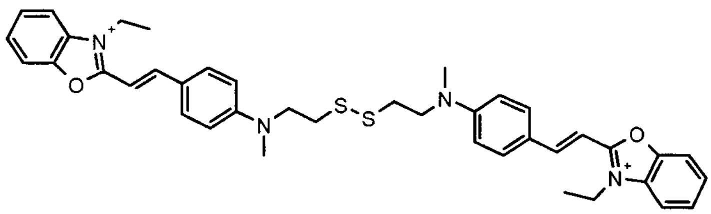

2M'

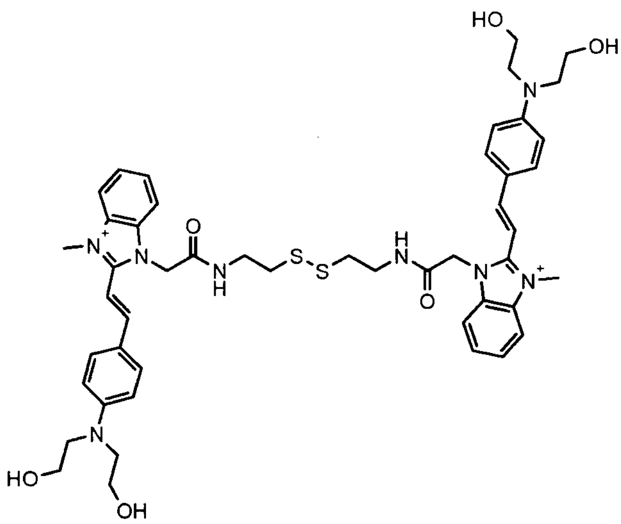

2M'

$Cl^-$

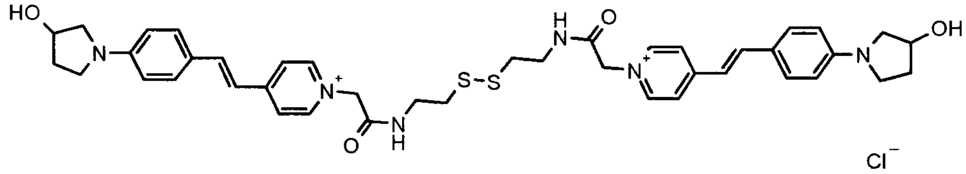

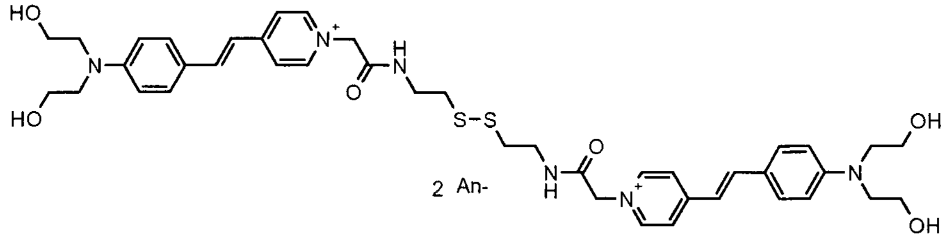

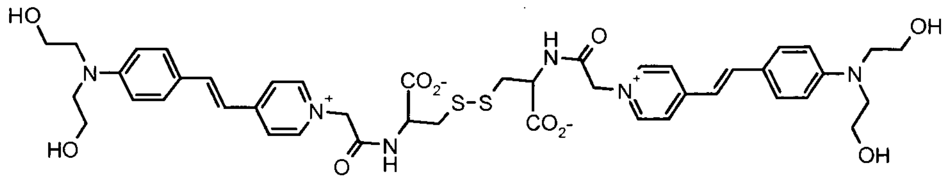

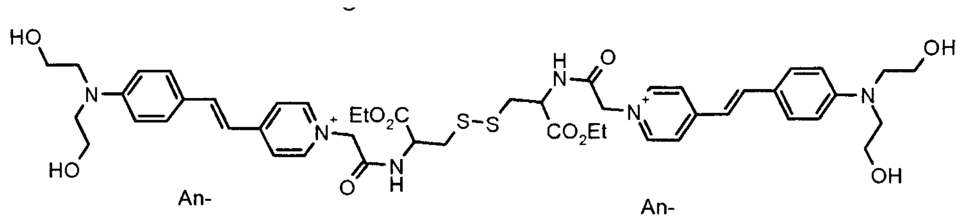

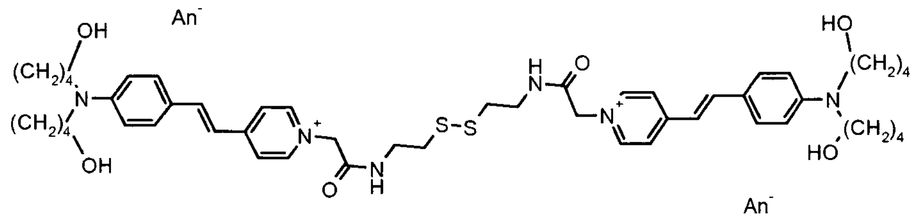

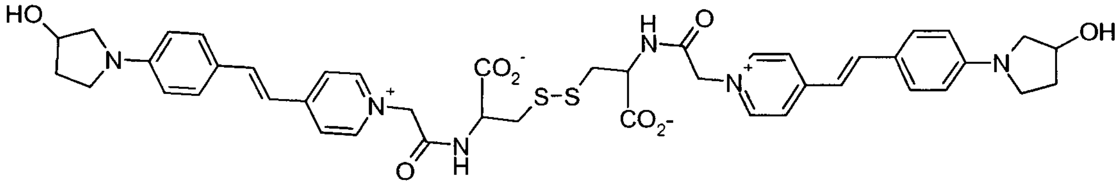

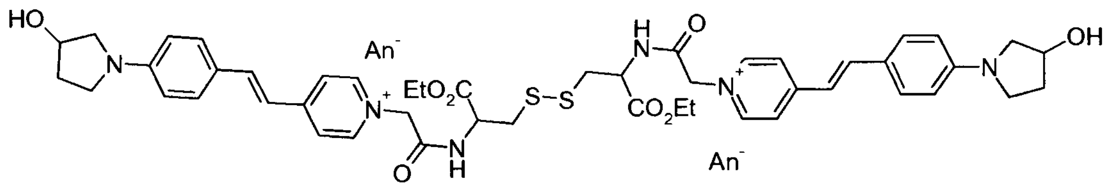

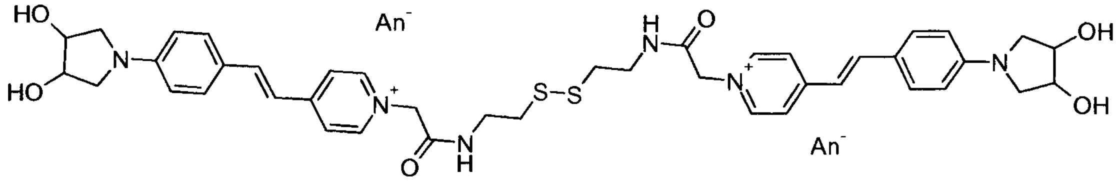

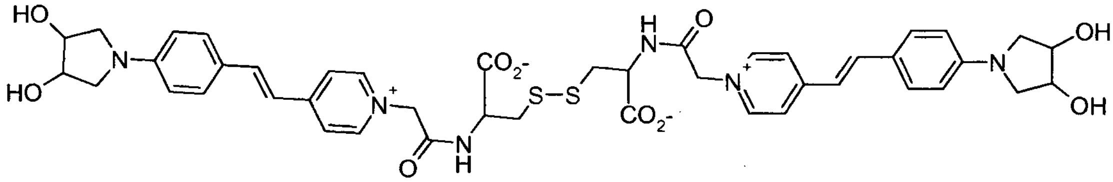

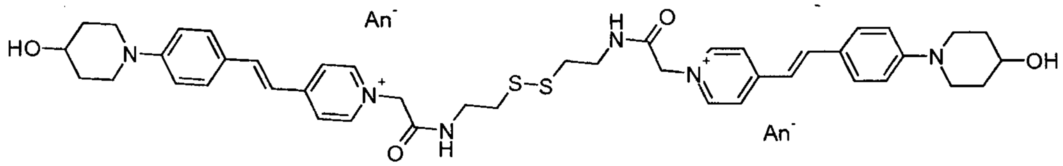

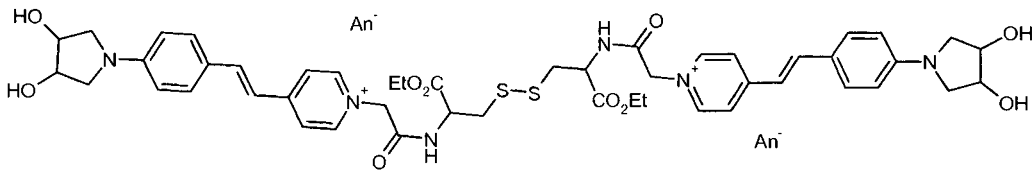

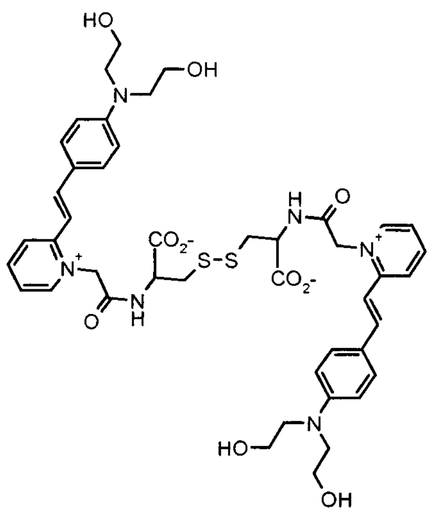

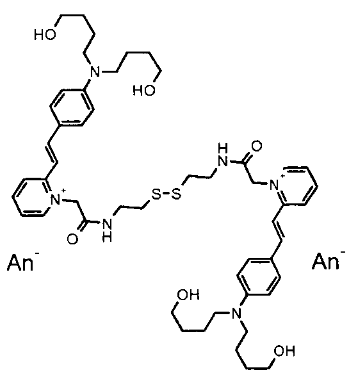

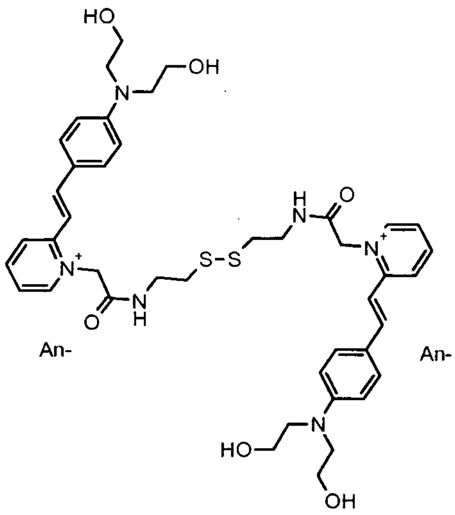

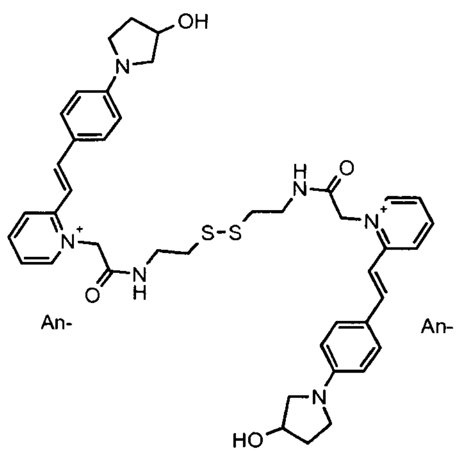

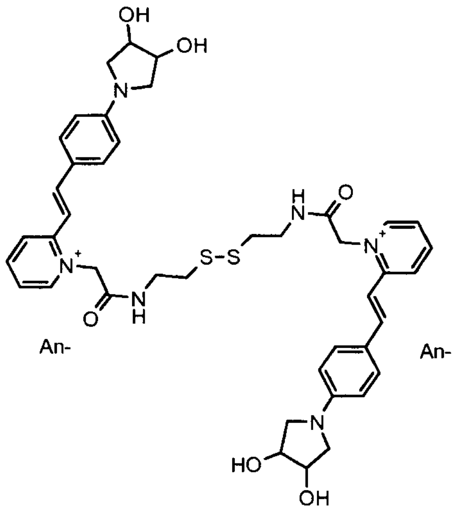

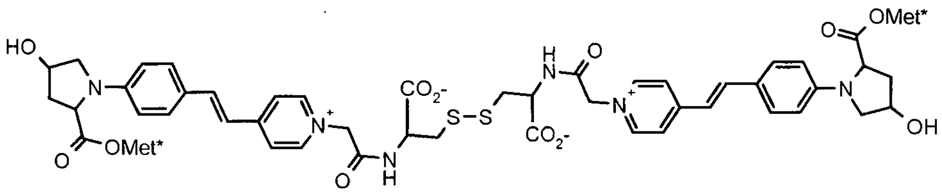

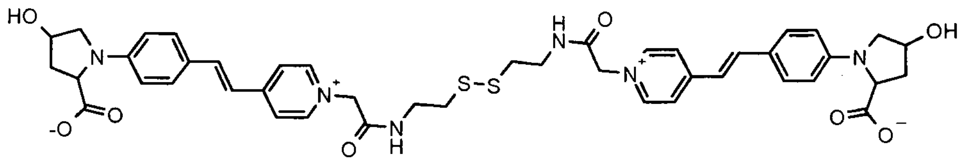

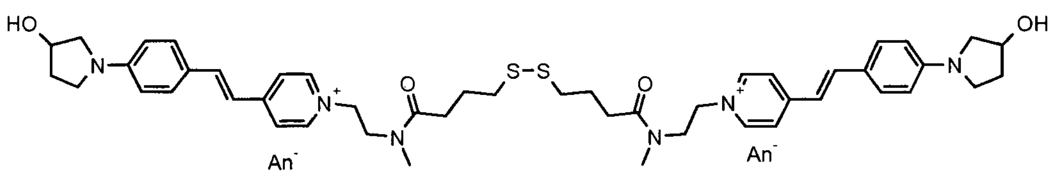

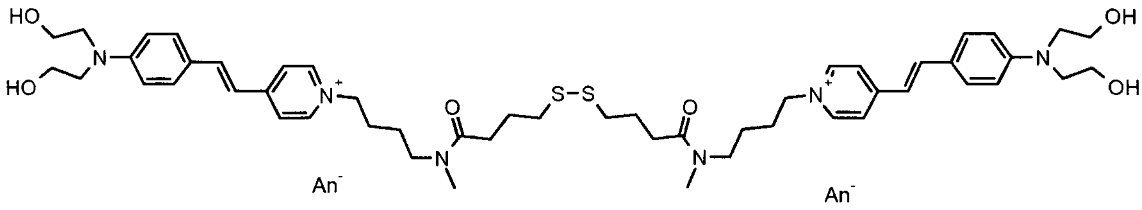

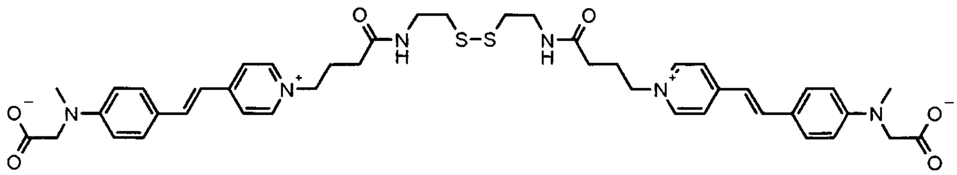

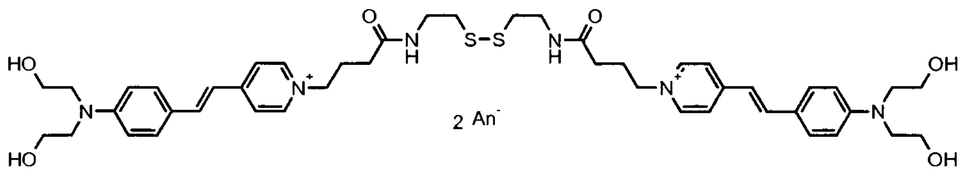

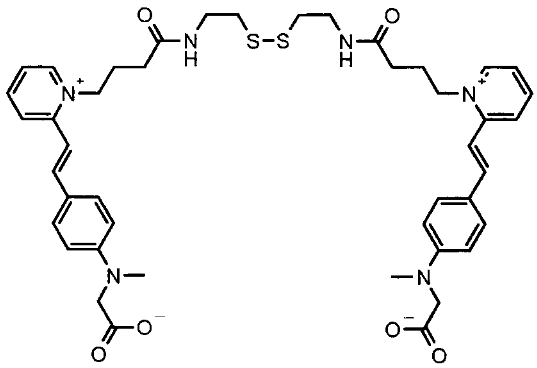

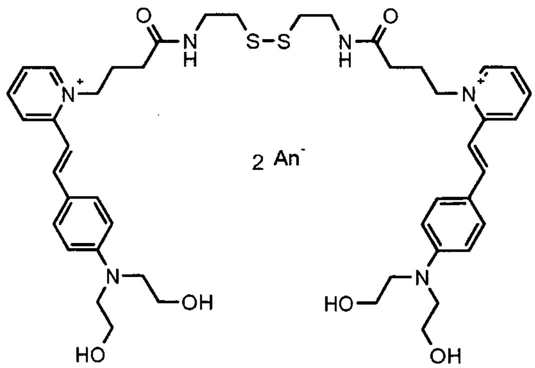

avec An$^-$, M' représentant des contre-ions anioniques.

7. Composition selon une quelconque des revendications précédentes dans laquelle le polymère à fonction thiol possède une masse molaire moléculaire comprise entre $8.10^2$ et $5.10^6$.

8. Composition selon une quelconque des revendications précédentes dans laquelle le polymère à fonction thiol est un polymère linéaire ou réticulé.

9. Composition selon une quelconque des revendications 1 à 7 dans laquelle le polymère à fonction thiol est un dendrimère.

10. Composition selon une quelconque des revendications 1 à 7 et 8 dans laquelle le polymère à fonction thiol est un polyéthylèneimine.

11. Procédé de coloration des fibres kératiniques consistant à appliquer sur les fibres une composition tinctoriale telle que définie dans une quelconque des revendications précédentes.

12. Procédé de coloration selon la revendication précédente dans lequel les fibres kératiniques sont foncées et possèdent une hauteur de ton inférieure à 6, de préférence inférieure ou égale à 4.

**13.** Utilisation pour la coloration des fibres kératiniques, notamment les cheveux, d'au moins un colorant direct à fonction disulfure et d'au moins un polymère à fonction(s) thiol(s).

**14.** Utilisation selon la revendication précédente pour l'éclaircissement des fibres kératiniques foncées possédant une hauteur de ton inférieure à 6, de préférence inférieure ou égale à 4 ; d'au moins au moins un colorant direct à fonction disulfure et au moins un polymère à fonction(s) thiol(s).

## Patentansprüche

**1.** Färbezusammensetzung, die in einem geeigneten kosmetischen Medium i) ein oder mehrere Farbstoffe, die aus Direktfarbstoffen mit einer oder mehreren Disulfidfunktionen und einer oder mehreren geschützten Thiolfunktionen ausgewählt sind, und ii) mindestens ein oder mehrere Polymere mit einer oder mehreren Thiolfunktionen und einem Molekulargewicht von mehr als 500 umfasst.

**2.** Zusammensetzung nach dem vorhergehenden Anspruch, bei dem der Farbstoff bzw. die Farbstoffe die Formel **(I)** aufweist bzw. aufweisen:

$$\mathbf{A\text{-}(X)_p\text{-}C_{sat}\text{-}S\text{-}U} \qquad \mathbf{(I)}$$

organische oder anorganische Säuresalze davon, optische und geometrische Isomere davon und Solvate davon wie Hydrate,
wobei in Formel (I):

- **U** für einen aus:

  i) **-S- C'$_{sat}$ - (X')$_{p'}$ - A'**;
  ii) **-S- C'$_{sat}$ - (X')$_{p'}$ - D** und
  iii) **-Y**

  ausgewählten Rest steht;
- **A** und **A'** gleich oder verschieden sind und für einen Rest mit einem oder mehreren kationischen oder nichtkationischen Chromophoren stehen;
- **Y** für Folgendes steht: a) ein Alkalimetall; b) ein Erdalkalimetall; c) eine Ammoniumgruppe: $N^+R^\alpha R^\beta R^\gamma R^\delta$, An"$^-$ oder eine Phosphoniumgruppe : $P^+R^\alpha R^\beta R^\gamma R^\delta$, An"$^-$, wobei $R^\alpha$, $R^\beta$, $R^\gamma$ und $R^\delta$ gleich oder verschieden sind und für ein Wasserstoffatom oder eine ($C_1$-$C_4$)-Alkylgruppe stehen und An"$^-$ für ein anionisches Gegenion steht; oder d) eine Schutzgruppe für die Thiolfunktion;
- **X** und **X'** gleich oder verschieden sind und für Folgendes stehen:

  a) eine gesättigte oder ungesättigte, lineare oder verzweigte $C_1$-$C_{30}$-Kohlenwasserstoffkette, die gegebenenfalls durch eine oder mehrere zweiwertige Gruppen oder Kombinationen davon, die aus:

    i) -N(R)-; -$N^+$(R)(R')-; $Q^-$; -O-; -S-; -CO- und -$SO_2$-, wobei R und R' gleich oder verschieden sind und aus einem Wasserstoff und einem $C_1$-$C_4$-Alkyl-, Hydroxyalkyl- und Aminoalkylrest ausgewählt sind und $Q^-$ für ein anionisches Gegenion steht; und ii) einem kondensierten oder nichtkondensierten, gesättigten oder ungesättigten, aromatischen oder nichtaromatischen (hetero)cyclischen Rest, der gegebenenfalls ein oder mehrere gleiche oder verschiedene Heteroatome enthält und gegebenenfalls substituiert ist,
    ausgewählt sind, substituiert, unterbrochen und/oder an einem oder beiden ihrer Enden durch diese Gruppen oder Kombinationen davon terminiert ist;

  oder
  b) eine oder mehrere zweiwertige Gruppen oder Kombinationen davon, die aus:

    -$SO_2$-; -O-; -S-; -N(R)-; -$N^+$(R)(R°) - und -CO-, wobei R und R° gleich oder verschieden sind und für ein Wasserstoffatom oder einen $C_1$-$C_4$-Alkyl-, $C_1$-$C_4$-Hydroxyalkyl- oder Aryl ($C_1$-$C_4$-alkyl) rest stehen; und einem kationischen oder nichtkationischen Heterocycloalkyl- oder Heteroarylrest ausgewählt sind;

• **p** und **p'** gleich oder verschieden sind und für 0 oder 1 stehen;
• $\mathbf{C_{sat}}$ und $\mathbf{C'_{sat}}$ gleich oder verschieden sind und für eine gegebenenfalls cyclische, gegebenenfalls substituierte, lineare oder verzweigte $C_1$-$C_{18}$-Alkylenkette stehen;
• **D** einem aus Hydroxy-, Hydroxyalkyl-, Alkoxy-, Carboxyl-, Carboxylat-, Amino-, Alkylamino- und Dialkylaminoresten ausgewählten Rest entspricht.

**3.** Zusammensetzung nach dem vorhergehenden Anspruch, wobei $\mathbf{C_{sat}}$ und $\mathbf{C'_{sat}}$ für eine - $(CH_2)_k$-Kette stehen, wobei k für eine ganze Zahl zwischen 1 und 8 inklusive steht.

**4.** Zusammensetzung nach einem der Ansprüche 2 oder 3, wobei p gleich 1 ist und X und X' gleich oder verschieden sind und für die folgende Sequenz stehen:

$$-(T)_t-(Z)_z-(T')_{t}'-$$

wobei die Sequenz in den Formeln (I) oder (II) folgendemaßen symmetrisch gebunden ist:

$$- C_{sat}\ (\text{oder } C'_{sat})-(T)_t-(Z)_z-\ (\text{A oder A'})\ ;$$

worin:

**T** und **T'** gleich oder verschieden sind und für einen oder mehrere Reste oder Kombinationen davon, die aus -O-; -S-; -N(R)-; $-N^+(R)(R°)$ -; -SO-; $-SO_2$-und -CO-; wobei R und R° gleich oder verschieden sind und für ein Wasserstoffatom oder einen $C_1$-$C_4$-Alkyl-, $C_1$-$C_4$-Hydroxyalkyl- oder Aryl ($C_1$-$C_4$-alkyl)-rest stehen; und einem vorzugsweise monocyclischen kationischen oder nichtkationischen Heterocycloalkyl- oder Heteroarylrest, der vorzugsweise zwei Heteroatome enthält; ausgewählt sind, stehen;
**t** und **t'** gleich oder verschieden sind und für 0 oder 1 stehen;
**Z** für Folgendes steht:

- $-(CH_2)_m$-, wobei m für eine ganze Zahl zwischen 1 und 8 steht,
- $-(CH_2CH_2O)_q$- oder - (OCH2CH2) q-, worin q für eine ganze Zahl zwischen 1 und 15 steht,
- einen Aryl-, Alkylaryl- oder Arylalkylrest, dessen Alkylrest $C_1$-$C_4$ ist und dessen Arylrest vorzugsweise $C_6$ ist und der gegebenenfalls durch mindestens eine $SO_3M$-Gruppe substituiert ist, wobei M für ein Wasserstoffatom, ein Alkalimetall oder eine durch einen oder mehrere gleiche oder verschiedene lineare oder verzweigte $C_1$-$C_{18}$-Alkylreste, die gegebenenfalls mindestens ein Hydroxy tragen, substituierte Ammoniumgruppe steht;

**z** für 0 oder 1 steht.

**5.** Zusammensetzung nach einem der vorhergehenden Ansprüche, wobei der Direktfarbstoff mit Disulfidfunktion aus den Farbstoffen der Formel **(III)** bis **(XIX)** ausgewählt ist:

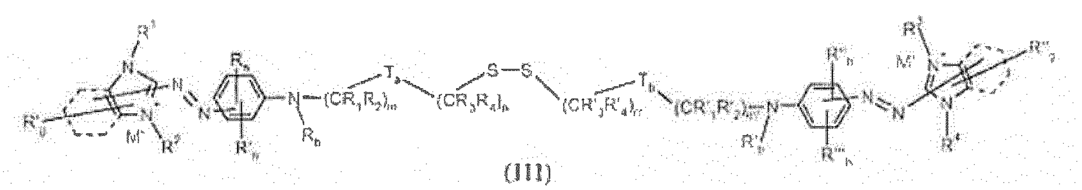

(III)

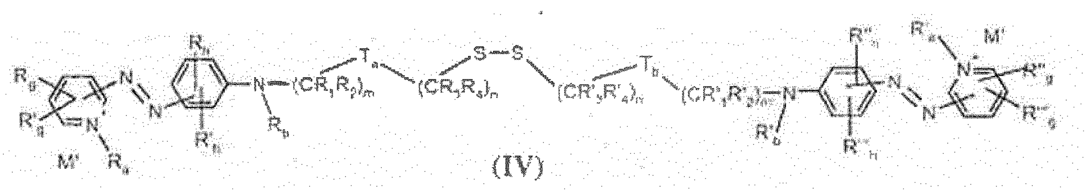

(IV)

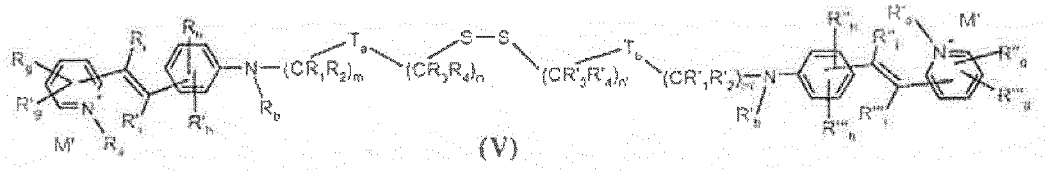

(V)

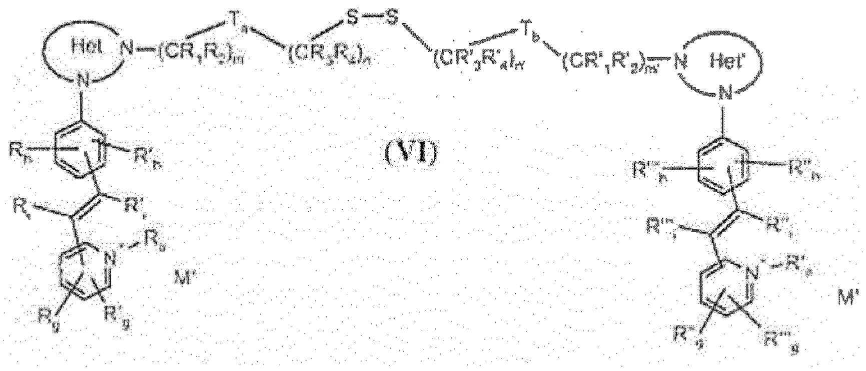

(VI)

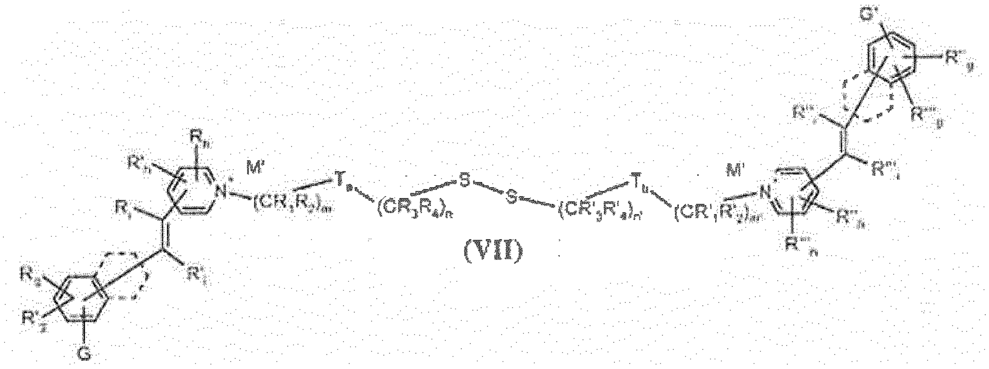

(VII)

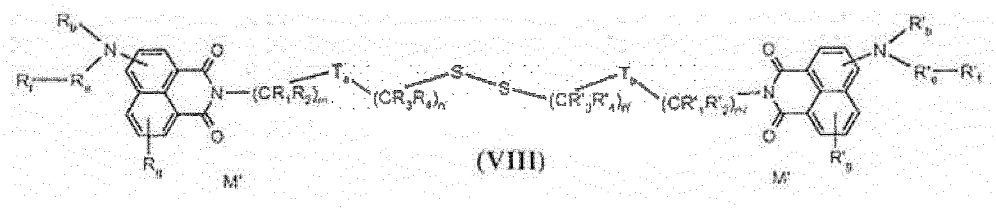

(VIII)

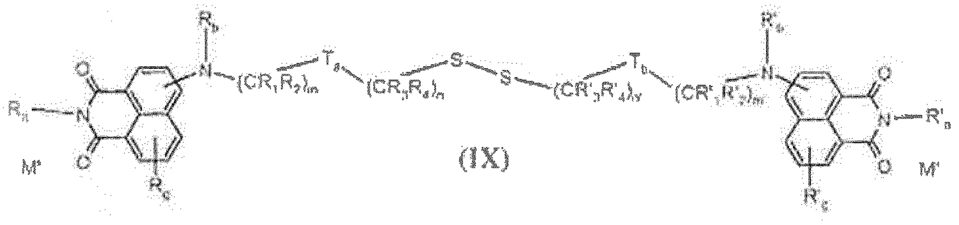

(IX)

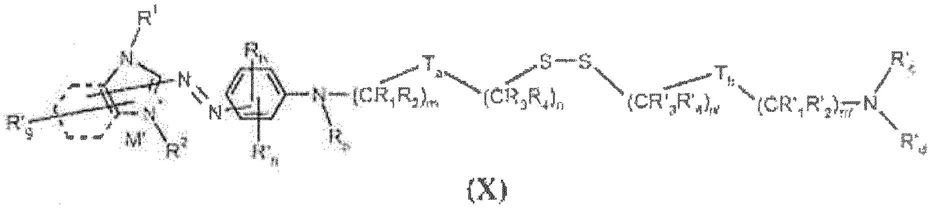

(X)

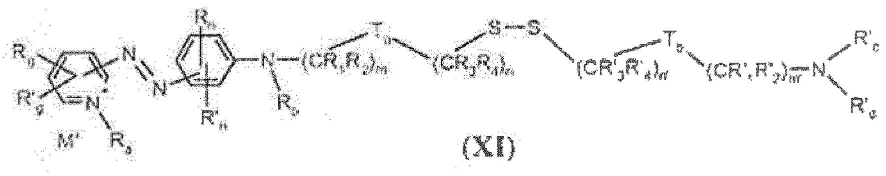

(XI)

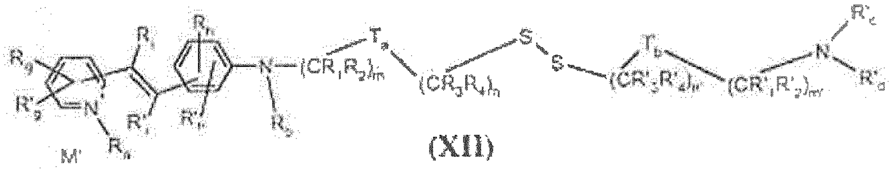

(XII)

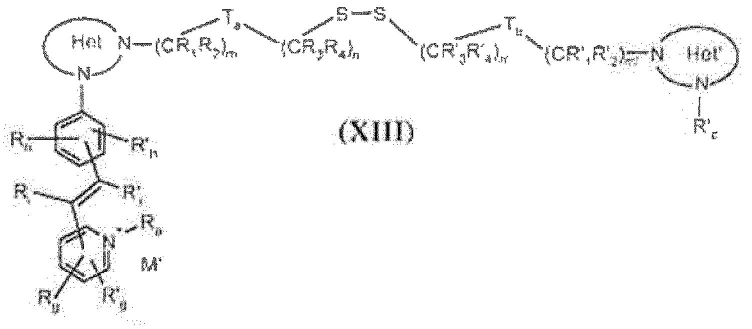

(XIII)

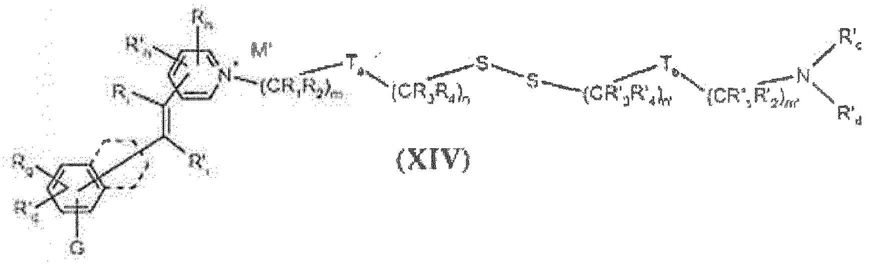

(XIV)

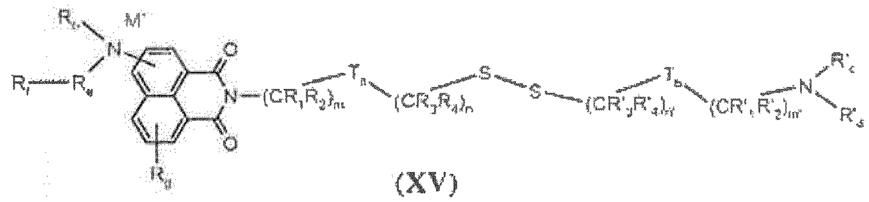

(XV)

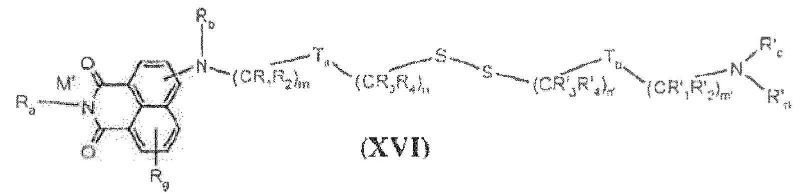

(XVI)

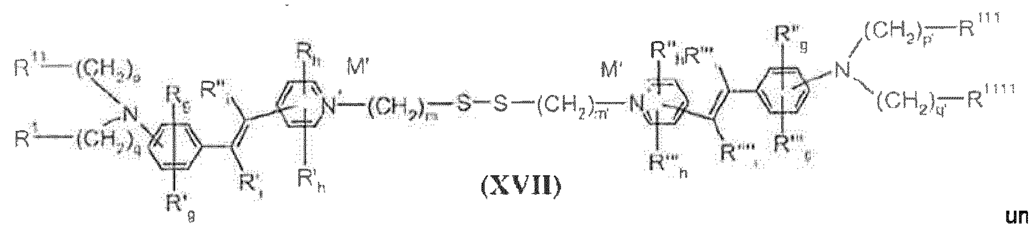

(XVII)

un

und

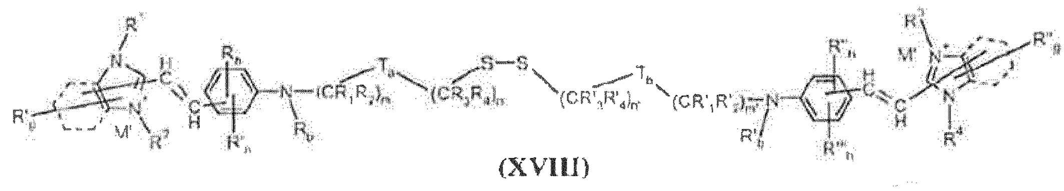

(XVIII)

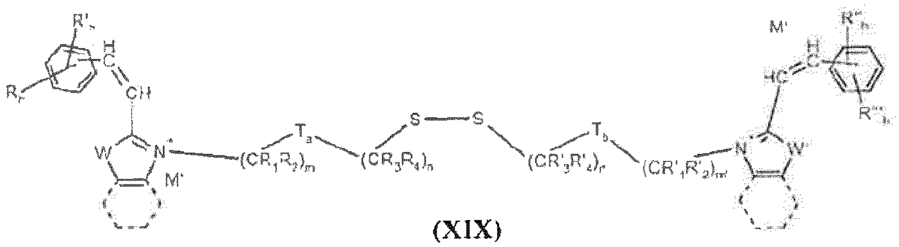

(XIX)

worin

- G und G' gleich oder verschieden sind und für eine $-NR_cR_d-$, $-NR'_cR'_d-$ oder $C_1$-$C_6$-Alkoxygruppe, die gegebenenfalls substituiert und vorzugsweise unsubstituiert ist, stehen; G und G' vorzugsweise für eine $-NR_cR_d$- bzw. $-NR'_cR'_d$-Gruppe stehen;
- W und W' gleich oder verschieden sind und für ein Sauerstoffatom oder eine -N-R1-Gruppe stehen, wobei R1 wie nachstehend definiert ist;
- $R^1$, $R^2$, $R^3$ und $R^4$ gleich oder verschieden sind und für ein Wasserstoffatom oder eine $C_1$-$C_6$-Alkylgruppe stehen;
- $R_a$ und $R'_a$ gleich oder verschieden sind und für eine Aryl($C_1$-$C_4$)alkylgruppe oder eine $C_1$-$C_6$-Alkylgruppe, die gegebenenfalls durch eine Hydroxygruppe oder eine Amino-, $C_1$-$C_4$-Alkylamino- oder $C_1$-$C_4$-Dialkylaminogruppe substituiert ist, stehen, wobei die Alkylreste mit dem Stickstoffatom, an das sie gebunden sind, einen 5- bis 7-gliedrigen Heterocyclus bilden können, der gegebenenfalls ein weiteres Heteroatom, das mit Stickstoff identisch oder davon verschieden ist, enthält; $R_a$ und $R'_a$ vorzugsweise für eine $C_1$-$C_3$-Alkylgruppe, die gegebenenfalls durch eine Hydroxygruppe substituiert ist, oder eine Benzylgruppe stehen;
- $R_b$ und $R'_b$ gleich oder verschieden sind und für ein Wasserstoffatom, eine Aryl($C_1$-$C_4$)alkylgruppe oder eine $C_1$-$C_6$-Alkylgruppe, die gegebenenfalls substituiert ist, stehen; $R_b$ und $R'_b$ vorzugsweise für ein Wasserstoffatom oder eine $C_1$-$C_3$-Alkyl- oder Benzylgruppe stehen;
- $R_c$, $R'_c$, $R_d$ und $R'_d$ gleich oder verschieden sind und für ein Wasserstoffatom, eine Aryl($C_1$-$C_4$)alkylgruppe, eine $C_1$-$C_6$-Alkoxygruppe oder eine $C_1$-$C_6$-Alkylgruppe, die gegebenenfalls substituiert ist, stehen; $R_c$, $R'_c$, $R_d$ und $R'_d$ vorzugsweise für ein Wasserstoffatom, eine Hydroxy-, $C_1$-$C_3$-Alkoxy-, Amino-, $C_1$-$C_3$-(Di)alkylamino-

gruppe oder eine $C_1$-$C_3$-Alkylgruppe, die gegebenenfalls durch i) eine Hydroxygruppe, ii) eine Aminogruppe, iii) eine $C_1$-$C_3$-(Di)alkylaminogruppe oder iv) eine quaternäre Ammoniumgruppe (R'') (R''') (R'''') $N^+$-substituiert ist, stehen;

oder auch zwei benachbarte Reste $R_c$ und $R'_d$ oder $R'_c$ und $R'_d$, die an dasselbe Stickstoffatom gebunden sind, eine heterocyclische Gruppe oder Heteroarylgruppe bilden; der Heterocyclus oder das Heteroaryl vorzugsweise monocyclisch ist und zwischen 5 und 7 Glieder umfasst; die Gruppen weiter bevorzugt aus Imidazolyl und Pyrrolidinyl ausgewählt sind;

• $R_e$ und $R'_e$ gleich oder verschieden sind und für eine gegebenenfalls ungesättigte, lineare oder verzweigte, zweiwertige $C_1$-$C_6$-Alkylenyl-Kohlenwasserstoffkette stehen;

• $R_f$ und $R'_f$ gleich oder verschieden sind und für eine quaternäre Ammoniumgruppe (R") (R"') (R"") $N^+$- stehen, wobei R", R"' und R"" gleich oder verschieden sind und für ein Wasserstoffatom oder eine $C_1$-$C_4$-Alkylgruppe stehen oder (R") (R"') (R"")$N^+$- für eine gegebenenfalls substituierte kationische Heteroarylgruppe, vorzugsweise eine gegebenenfalls durch eine $C_1$-$C_3$-Alkylgruppe substituierte Imidazoliniumgruppe, steht;

• $R_g$, $R'_g$, $R''_g$, $R'''_g$, $R_h$, $R'_h$, $R''_h$ und $R'''_h$ gleich oder verschieden sind und für ein Wasserstoffatom, ein Halogenatom, eine Amino-, $C_1$-$C_4$-Alkylamino-, $C_1$-$C_4$-Dialkylamino-, Cyano-, Carboxy-, Hydroxy- oder Trifluormethylgruppe, einen Acylamino-, $C_1$-$C_4$-Alkoxy-, (Poly)hydroxy-($C_2$-$C_4$)alkoxy-, Alkylcarbonyloxy-, Alkoxycarbonyl- oder Alkylcarbonylaminorest, einen Acylamino-, Carbamoyl- oder Alkylsulfonylaminorest, einen Aminosulfonylrest oder einen $C_1$-$C_{16}$-Alkylrest, der gegebenenfalls durch eine aus $C_1$-$C_{12}$-Alkoxy, Hydroxy, Cyano, Carboxy, Amino, $C_1$-$C_4$-Alkylamino und $C_1$-$C_4$-Dialkylamino ausgewählte Gruppe substituiert ist, stehen oder auch die beiden Alkylreste, die an das Stickstoffatom der Aminogruppe gebunden sind, einen 5- bis 7-gliedrigen Heterocyclus bilden, der gegebenenfalls ein weiteres Heteroatom, das mit demjenigen des Stickstoffatoms identisch oder davon verschieden ist, enthält; $R_g$, $R'_g$, $R''_g$, $R'''_g$, $R_h$, $R'_h$, $R''_h$ und $R'''_h$ vorzugsweise für ein Wasserstoffatom, ein Halogenatom oder eine $C_1$-$C_3$-Alkylgruppe stehen;

• oder auch zwei Gruppen Rg und $R'_g$; $R''_g$ und $R'''_g$; $R_h$ und $R'_h$; $R''_h$ und $R'''_h$, die an zwei benachbarte Kohlenstoffatome gebunden sind, zusammen einen Benzo- oder Indenoring oder eine kondensierte Heterocycloalkyl- oder kondensierte Heteroarylgruppe bilden; wobei der Benzo-, Indeno-, Heterocycloalkyl- oder Heteroarylring gegebenenfalls durch ein Wasserstoffatom, ein Halogenatom, eine Amino- , $C_1$-$C_4$-Alkylamino- , $C_1$-$C_4$-Dialkylamino-, Nitro-, Cyano-, Carboxy-, Hydroxy- oder Trifluormethylgruppe, einen Acylamino-, $C_1$-$C_4$-Alkoxy-, (Poly)hydroxy($C_2$-$C_4$)alkoxy-, Alkylcarbonyloxy-, Alkoxycarbonyl-oder Alkylcarbonylaminorest, einen Acylamino-, Carbamoyl- oder Alkylsulfonylaminorest, einen Aminosulfonylrest oder einen $C_1$-$C_{16}$-Alkylrest, der gegebenenfalls durch eine aus $C_1$-$C_{12}$-Alkoxy, Hydroxy, Cyano, Carboxy, Amino, $C_1$-$C_4$-Alkylamino und $C_1$-$C_4$-Dialkylamino ausgewählte Gruppe substituiert ist, substituiert ist oder auch die beiden Alkylreste, die an das Stickstoffatom der Aminogruppe gebunden sind, einen 5- bis 7-gliedrigen Heterocyclus bilden, der gegebenenfalls ein weiteres Heteroatom, das mit demjenigen des Stickstoffatoms identisch oder davon verschieden ist, enthält; $R_g$ und $R'_g$; $R''_g$ und $R'''_g$ vorzugsweise zusammen eine Benzogruppe bilden;

• oder auch dann, wenn G für $-NR_cR_d$ steht und G' für $-NR'_cR'_d$ steht, zwei Gruppen $R_c$ und $R'_g$; $R'_c$ und $R''_g$; $R_d$ und Rg; $R'_d$ und $R'''_g$ zusammen ein Heteroaryl oder einen gesättigten Heterocyclus, das bzw. der gegebenenfalls durch ein oder mehrere $C_1$-$C_6$-Alkylgruppen substituiert ist, vorzugsweise einen Heterocyclus, der ein oder zwei aus Stickstoff und Sauerstoff ausgewählte Heteroatome enthält und zwischen 5 und 7 Glieder umfasst, bilden; der Heterocyclus weiter bevorzugt aus Morpholinyl-, Piperazinyl-, Piperidinyl- und Pyrrolidinylgruppen ausgewählt ist;

• $R_i$, $R'_i$, $R''_i$ und $R'''_i$ gleich oder verschieden sind und für ein Wasserstoffatom oder eine $C_1$-$C_4$-Alkylgruppe stehen;

• $R_1$, $R_2$, $R_3$, $R_4$, $R'_1$, $R'_2$, $R'_3$ und $R'_4$ gleich oder verschieden sind und für ein Wasserstoffatom oder eine $C_1$-$C_4$-Alkyl-, $C_1$-$C_{12}$-Alkoxy-, Hydroxy-, Cyano-, Carboxy-, Amino-, $C_1$-$C_4$-Alkylamino- oder $C_1$-$C_4$-Dialkylaminogruppe stehen, wobei die Alkylreste mit dem Stickstoffatom, an das sie gebunden sind, einen 5- bis 7-gliedrigen Heterocyclus bilden können, der gegebenenfalls ein weiteres Heteroatom, das mit Stickstoff identisch oder davon verschieden ist, enthält; $R_1$, $R_2$, $R_3$, $R_4$, $R'_1$, $R'_2$, $R'_3$ und $R'_4$ vorzugsweise Wasserstoffatome oder eine Aminogruppe sind; $R_1$, $R_2$, $R_3$, $R_4$, $R'_1$, $R'_2$, $R'_3$ und $R'_4$ weiter bevorzugt für ein Wasserstoffatom stehen;

• $T_a$ und $T_b$ gleich oder verschieden sind und für i) eine kovalente $\sigma$-Bindung, ii) einen oder mehrere Reste oder Kombinationen davon, die aus $-SO_2$-, -O-, -S-, -N(R) -, $-N^+(R)$ ($R^o$) - und -CO-, wobei R und $R^o$ gleich oder verschieden sind und für ein Wasserstoffatom oder einen $C_1$-$C_4$-Alkyl-, $C_1$-$C_4$-Hydroxyalkyl- oder Aryl ($C_1$-$C_4$-alkyl) rest stehen, ausgewählt sind, $T_a$ vorzugsweise mit $T_b$ identisch ist und für eine kovalente $\sigma$-Bindung oder eine aus -N(R)-, -C(O)-N(R)-, -N(R)-C(O)-, -O-C(O)-, -C(O)-O- und $-N^+(R)(R^o)$-, wobei R und $R^o$ gleich oder verschieden sind und für ein Wasserstoffatom oder eine $C_1$-$C_4$-Alkylgruppe stehen, ausgewählte Gruppe steht; $T_a$ und $T_b$ weiter bevorzugt für eine 6-Bindung stehen; iii) oder einen kationischen oder nichtkationischen Heterocycloalkyl- oder Heteroarylrest, der vorzugsweise monocyclisch ist, vorzugsweise identisch ist, vorzugsweise zwei Heteroatome (weiter bevorzugt zwei Stickstoffatome) enthält und vorzugsweise 5 bis 7 Glieder

umfasst, wie Imidazolium, stehen;

•

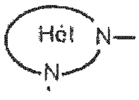 oder 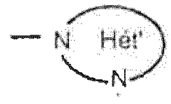

gleich oder verschieden sind und für eine gegebenenfalls substituierte heterocyclische Gruppe stehen; die Heterocyclen vorzugsweise identisch, monocyclisch und gesättigt sind und insgesamt zwei Stickstoffatome und 5 bis 8 Glieder umfassen;

•

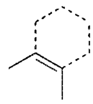

für eine mit einem Imidazolium- oder Phenylring kondensierte Aryl- oder Heteroarylgruppe steht oder auch an dem Imidazolium- oder Phenylring fehlt;

• $R^1$ und $R^{1111}$ gleich oder verschieden sind und für eine Hydroxygruppe, eine Aminogruppe ($NR_aR_b$) oder eine Ammoniumgruppe ($N^+R_aR_bR_c$), $An^-$ stehen; wobei $R_a$, $R_b$ und $R_c$ gleich oder verschieden sind und für ein Wasserstoffatom oder eine ($C_1$-$C_4$)-Alkylgruppe stehen; oder auch zwei Alkylgruppen $R_a$ und $R_b$ der Amino- oder Ammoniumgruppe einen 5- bis 7-gliedrigen Heterocyclus bilden, der gegebenenfalls ein weiteres Heteroatom, das mit demjenigen des Stickstoffatoms identisch oder davon verschieden ist, enthält, wie Morpholinyl, Piperazinyl, Piperidinyl, Pyrrolyl, Morpholinium, Piperazinium, Piperidinium, Pyrrolinium, und $An^-$ für ein anionisches Gegenion steht;

• $R^{11}$ und $R^{111}$ gleich oder verschieden sind und für ein Wasserstoffatom oder eine wie für $R^1$ bzw. $R^{1111}$ definierte Gruppe stehen;

• m, m', n und n' gleich oder verschieden sind und für eine ganze Zahl zwischen 0 und 6 inklusive stehen, wobei m+n, m'+n' gleich oder verschieden sind und für eine ganze Zahl zwischen 1 und 10 inklusive stehen; vorzugsweise m+n = m'+n' = eine ganze Zahl zwischen 2 und 4 inklusive; weiter bevorzugt m+n = m'+n' = eine ganze Zahl mit einem Wert von 2;

wobei M' für ein Gegenion oder ein organisches oder anorganisches Säuresalz steht.

**6.** Zusammensetzung nach einem der vorhergehenden Ansprüche, wobei der Direktfarbstoff mit Disulfidfunktion oder geschützter Thiolfunktion ausgewählt ist aus:

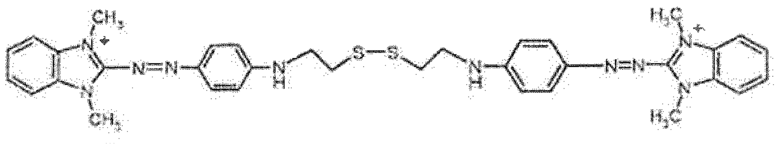

2M'

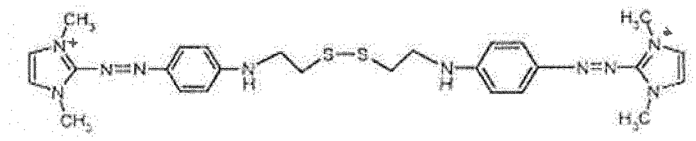

2M'

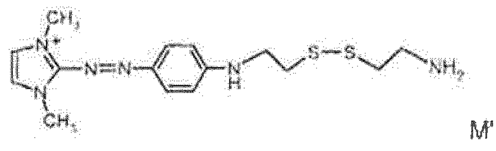

M'

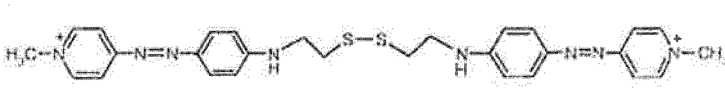

2M'

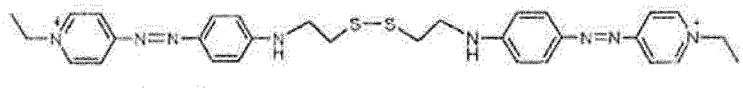

2M'

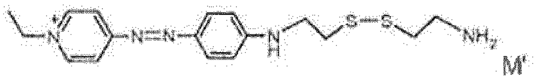

M'

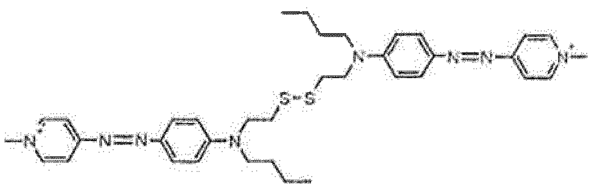

2M'

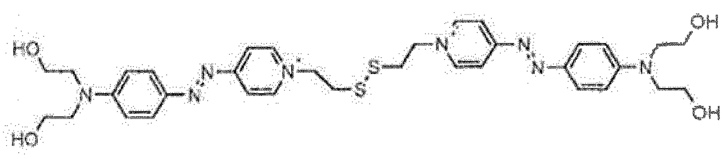

2M'

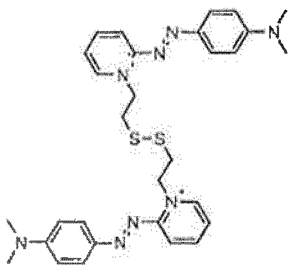

2M'

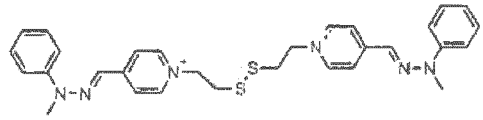

2M'

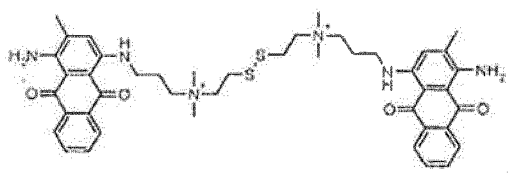

2M'

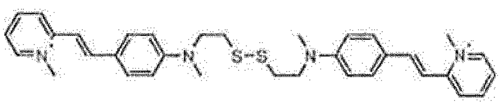

2M'

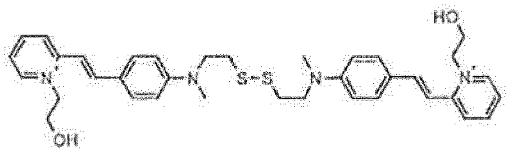
2M'

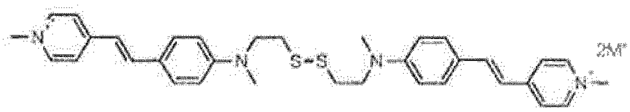
2M'

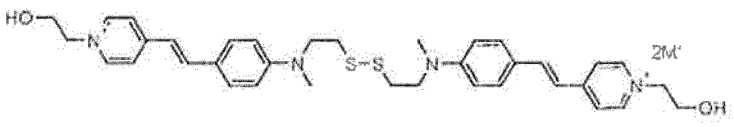
2M'

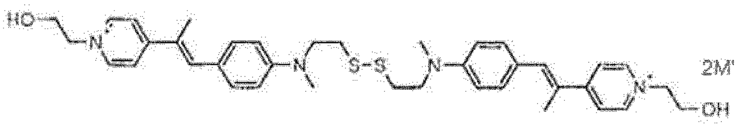
2M'

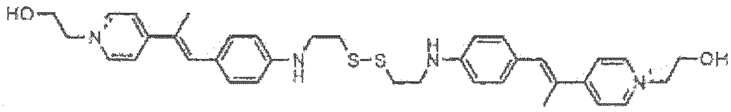
2M'

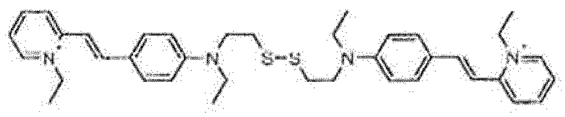
2M'

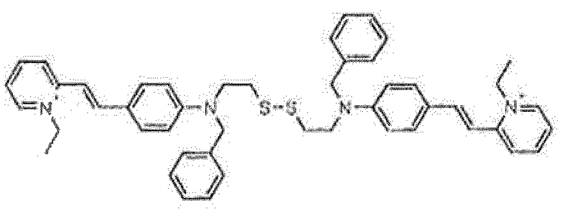
2M'

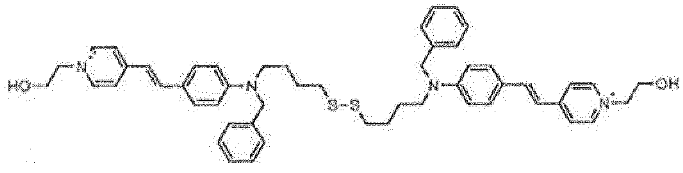
2M'

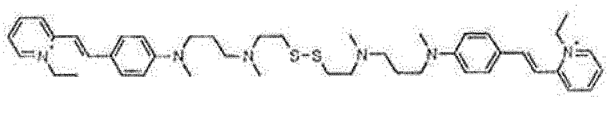
2M'

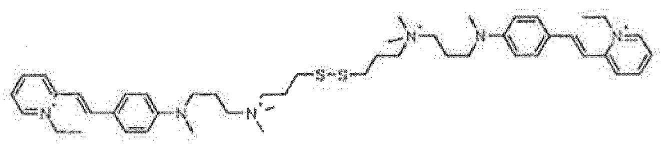

4M'

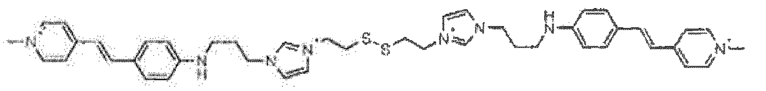

4M'

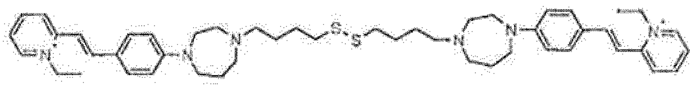

2M'

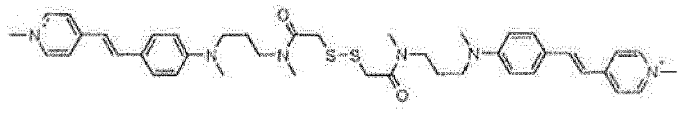

2M'

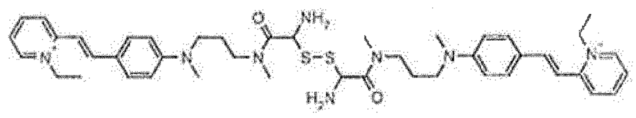

2M'

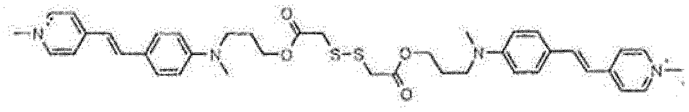

2M'

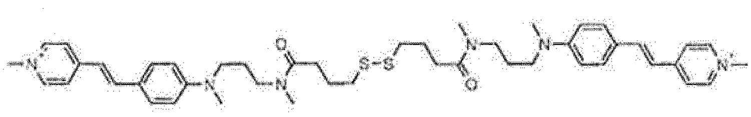

2M'

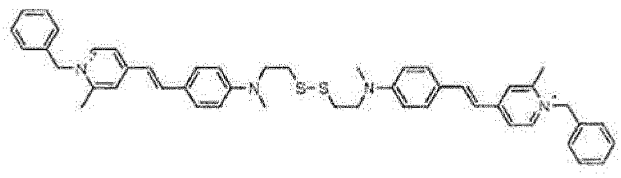

2M'

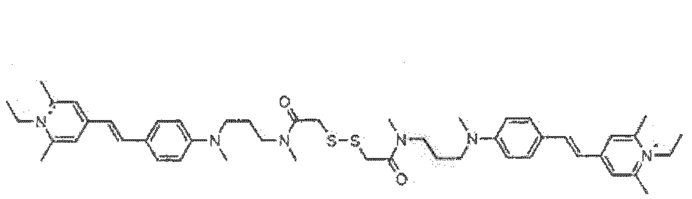

2M'

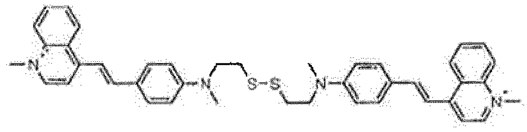 2M'

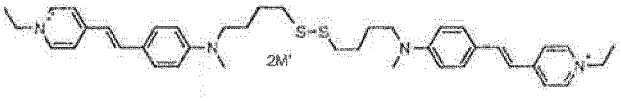

2M'

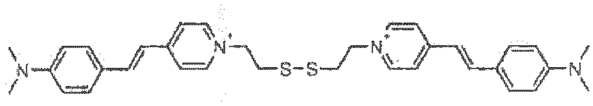 2M'

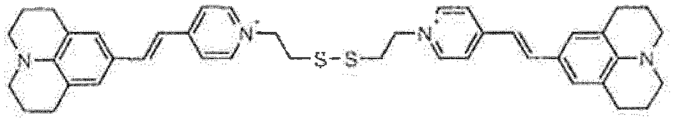 2M'

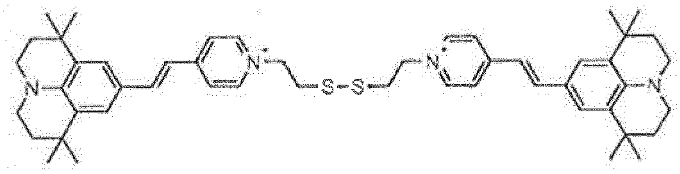 2M'

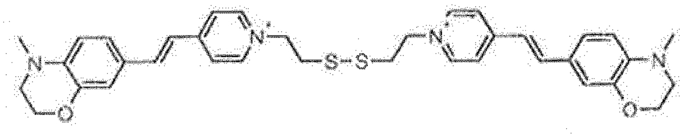 2M'

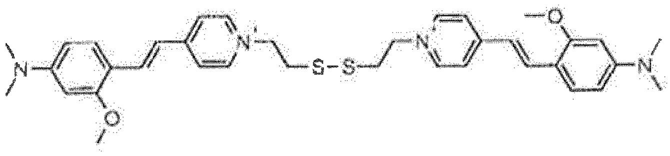 2M'

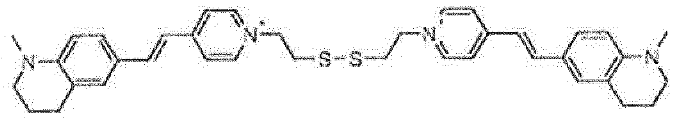 2M'

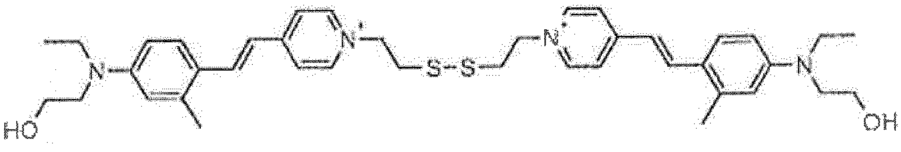 2M'

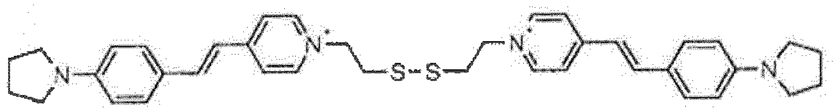

2M'

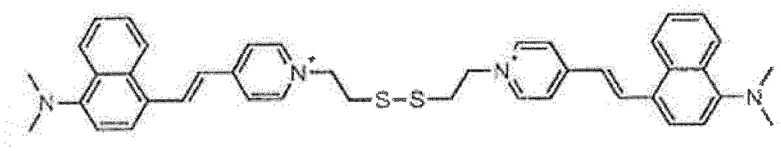

2M'

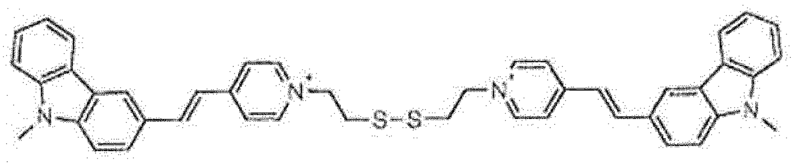

2M'

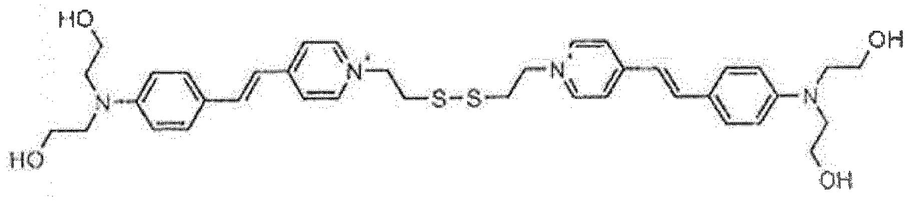

2M'

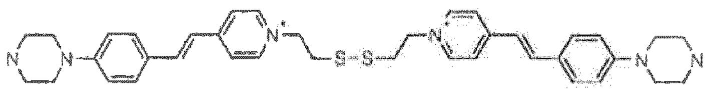

2M'

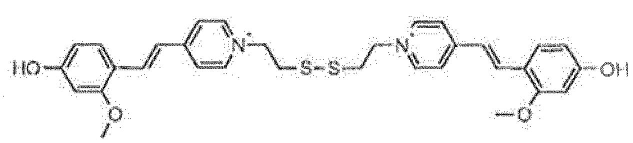

2M'

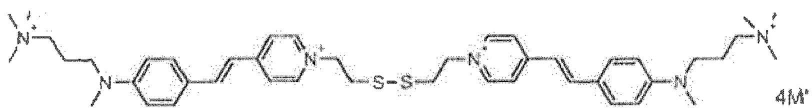

4M'

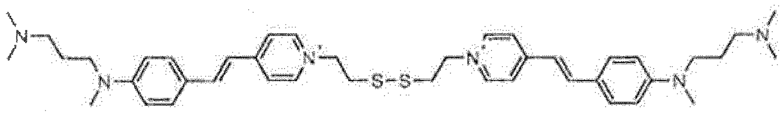

2M'

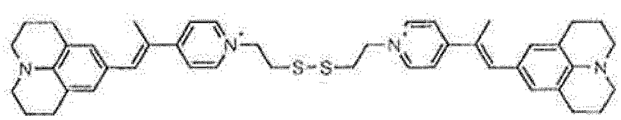

2M'

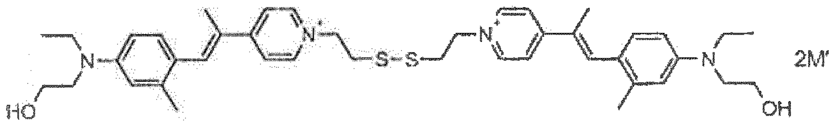

2M'

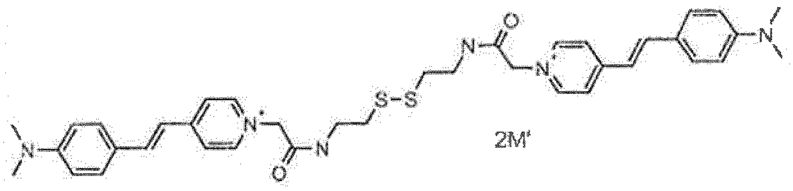

2M'

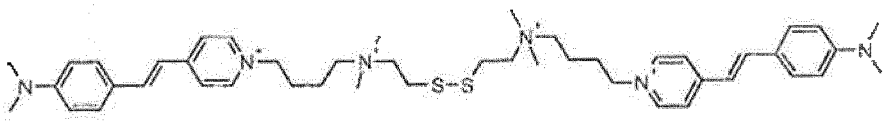

4M'

2M'

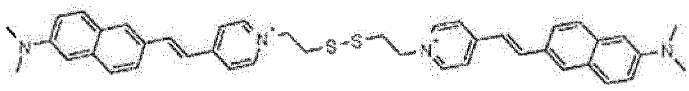

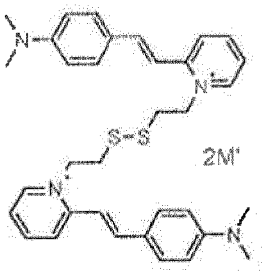

2M'

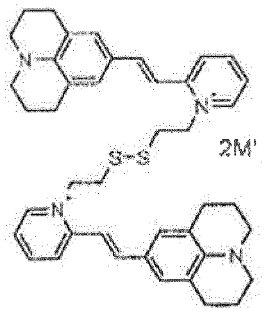

2M'

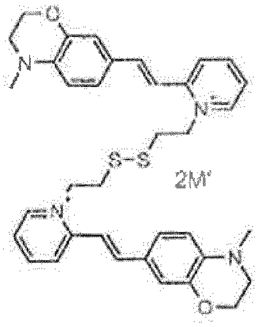

2M'

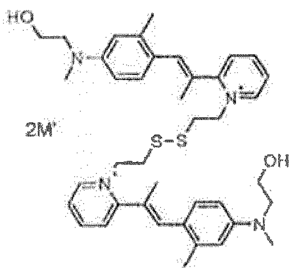

2M'

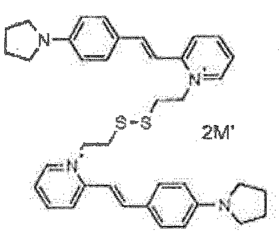

2M'

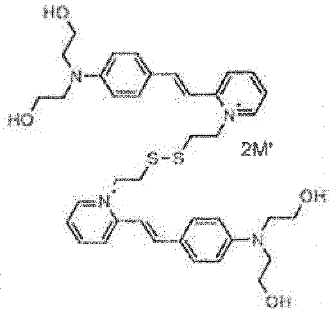

2M'

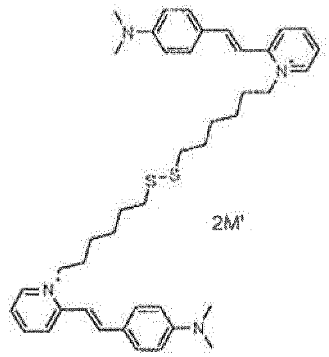

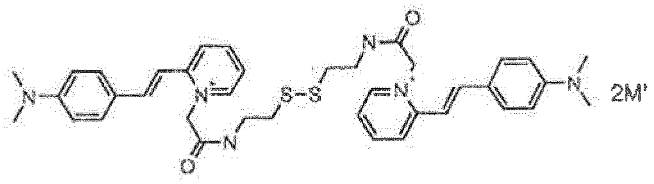

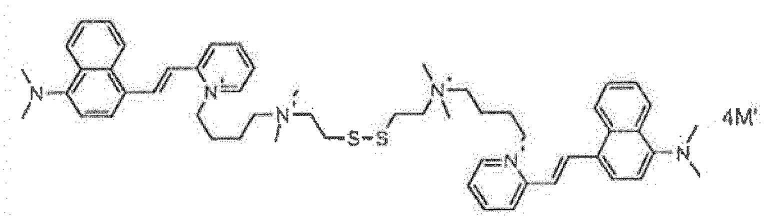

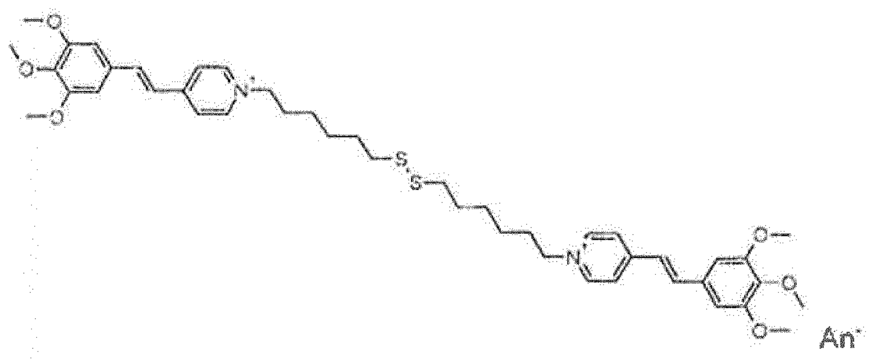

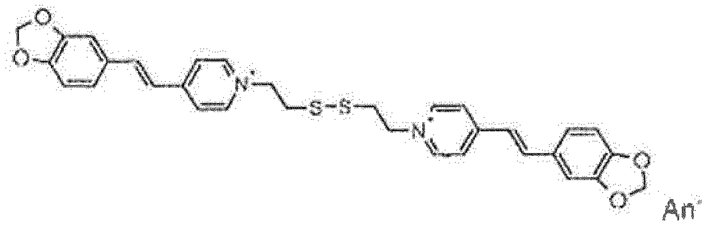

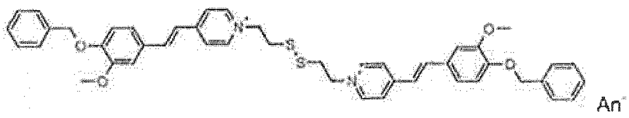

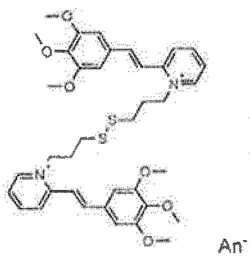

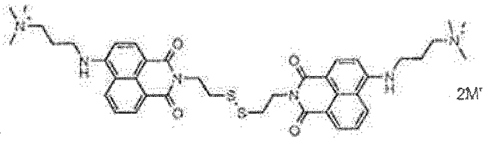

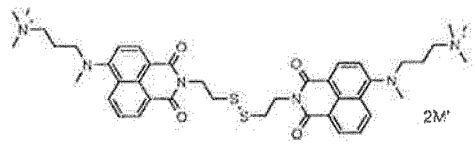

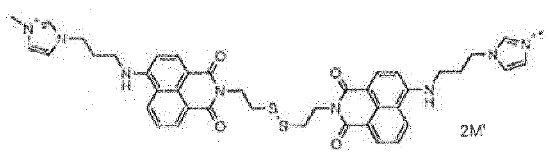

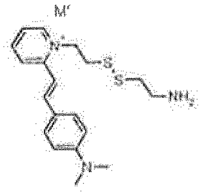

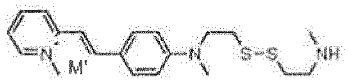

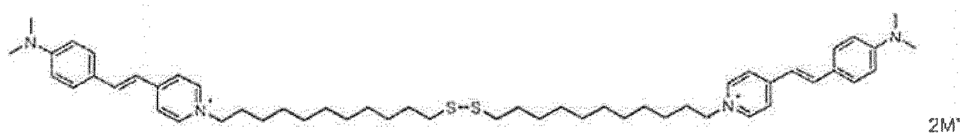

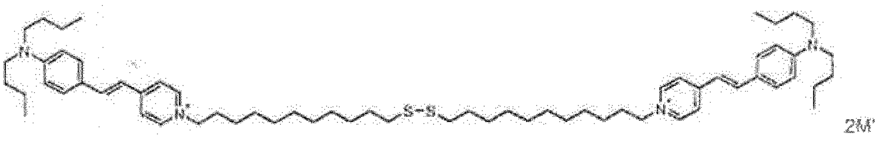

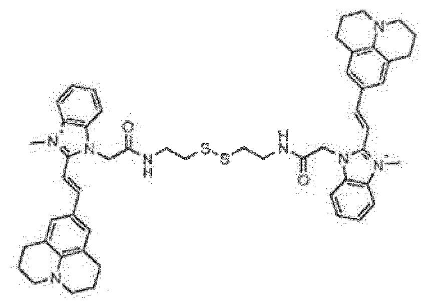

2M'

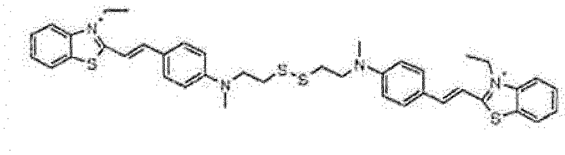

2M'

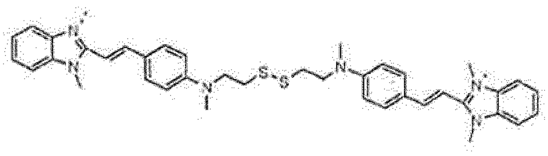

2M'

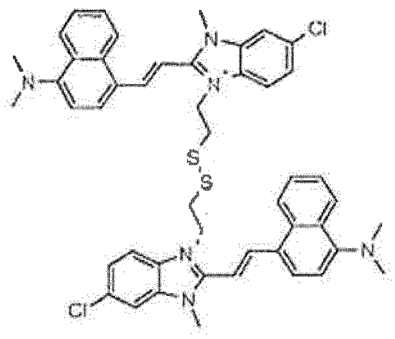

2M'

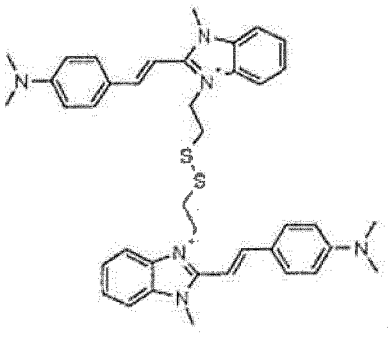

2M'

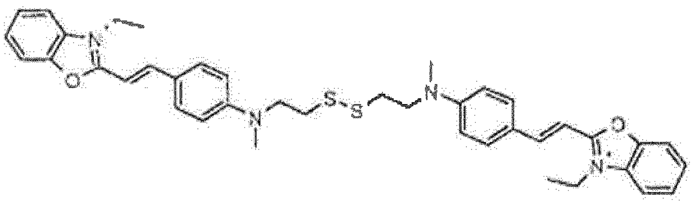

2M'

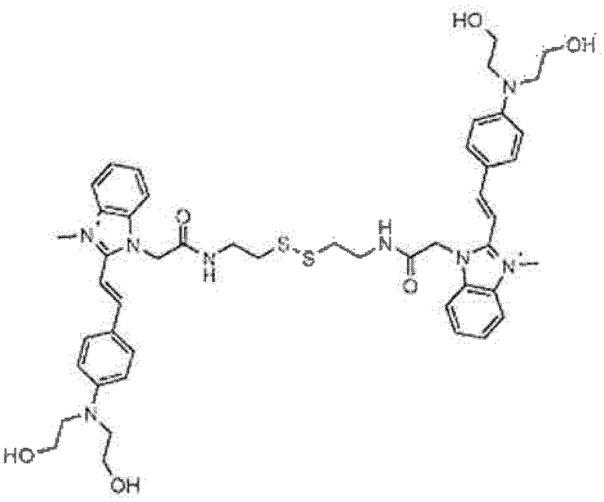

2M'

Cl⁻

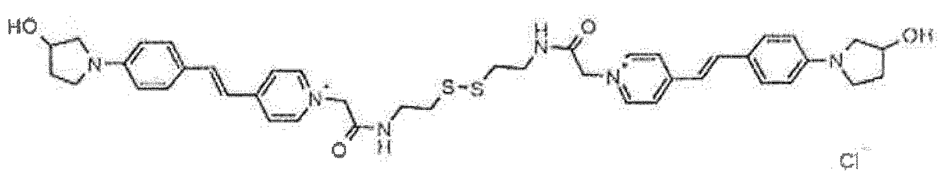

Cl⁻

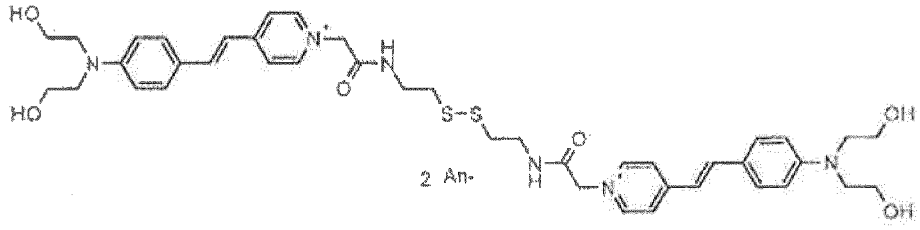

2 An-

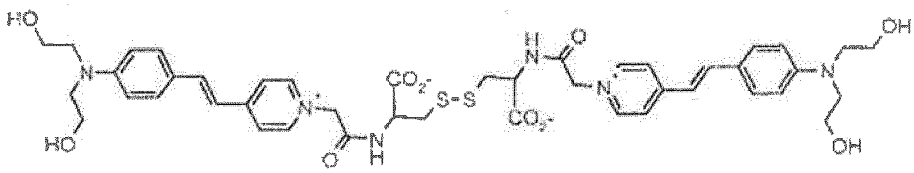

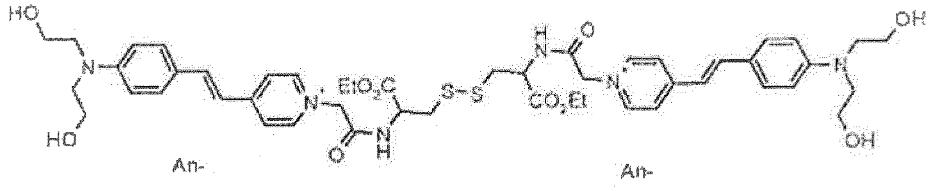

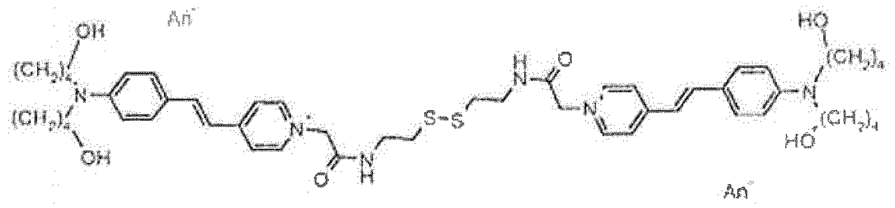

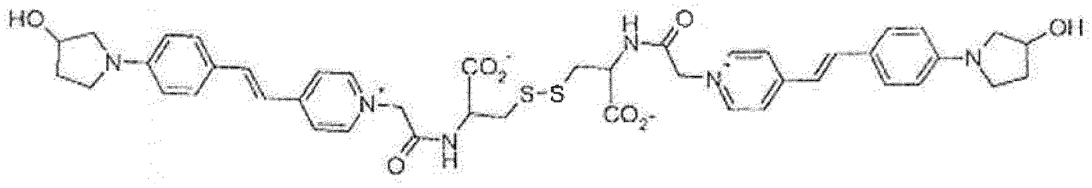

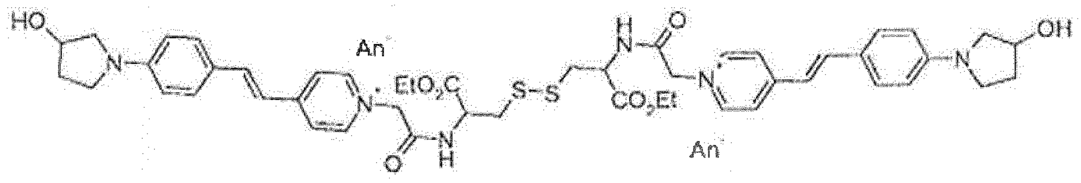

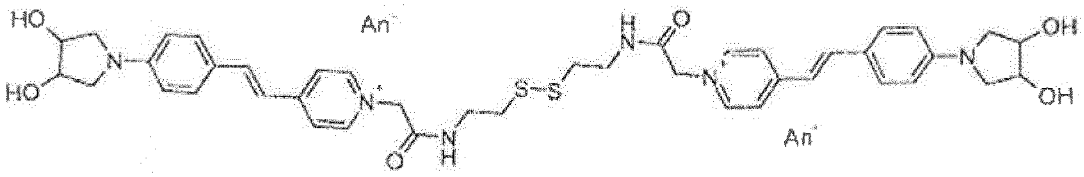

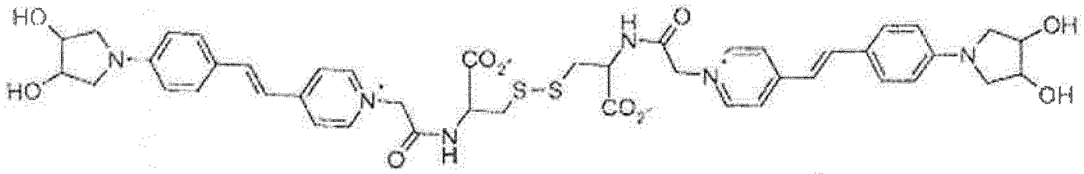

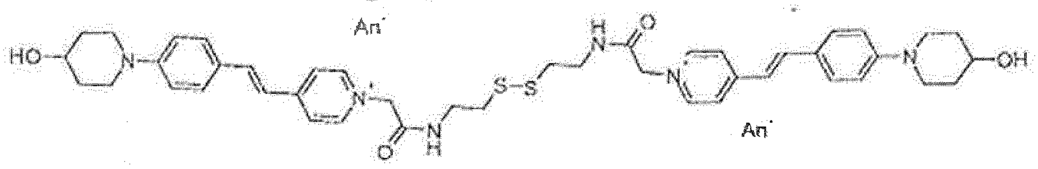

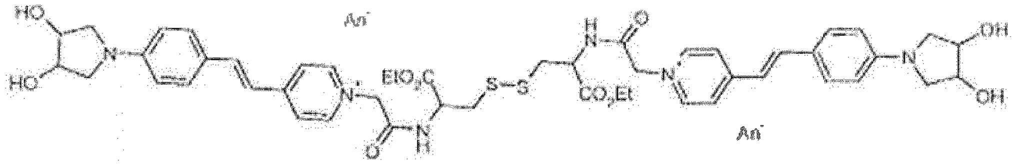

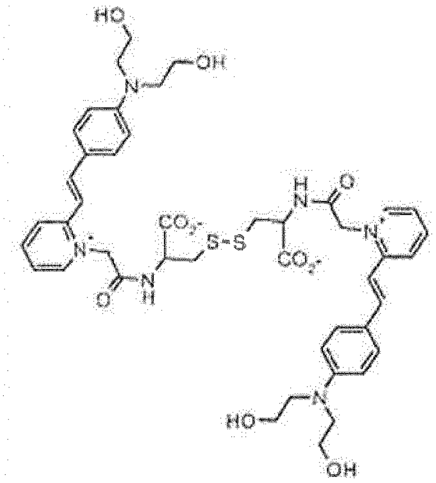

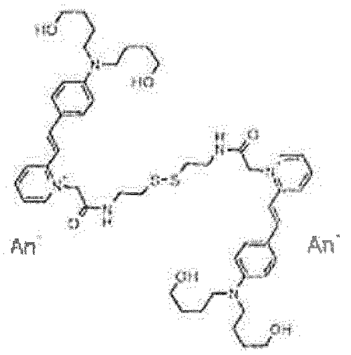

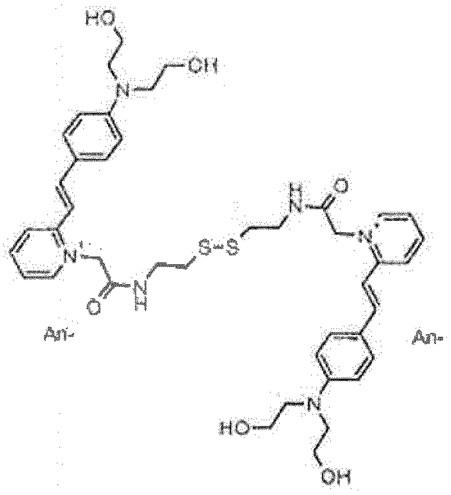

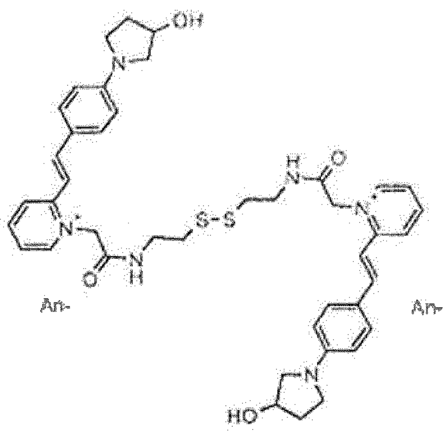

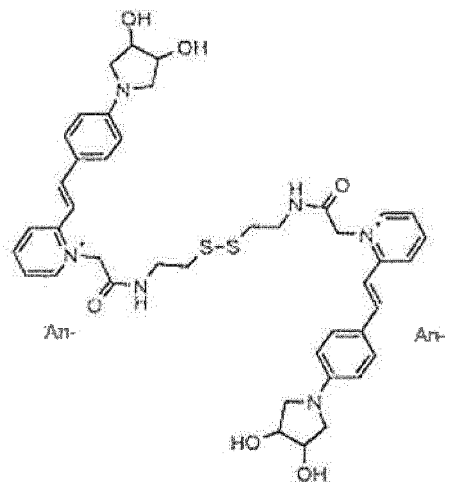

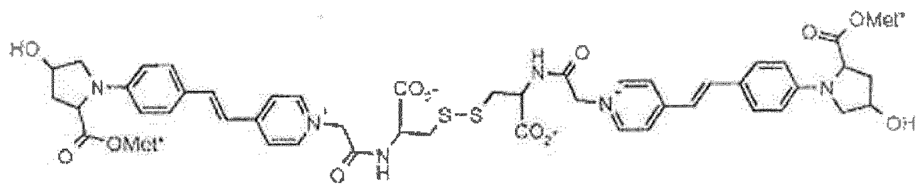

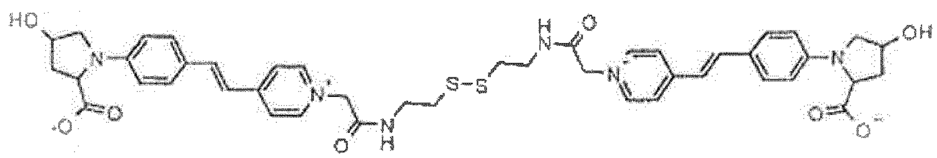

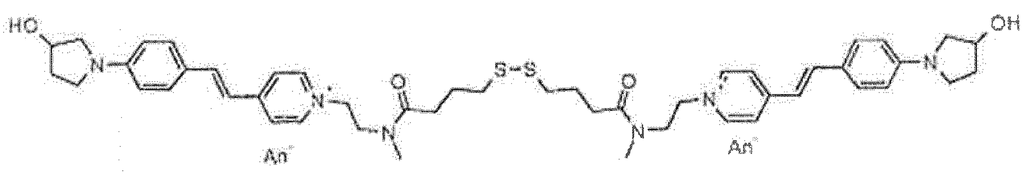

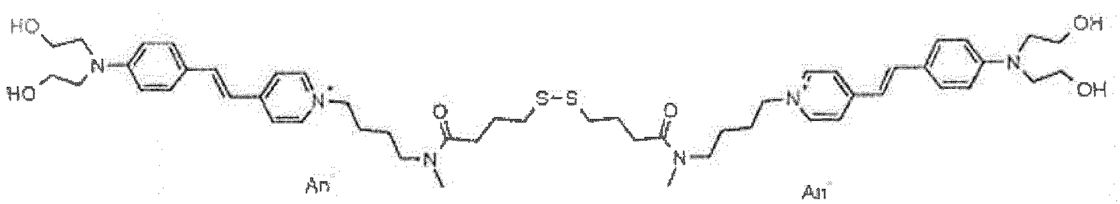

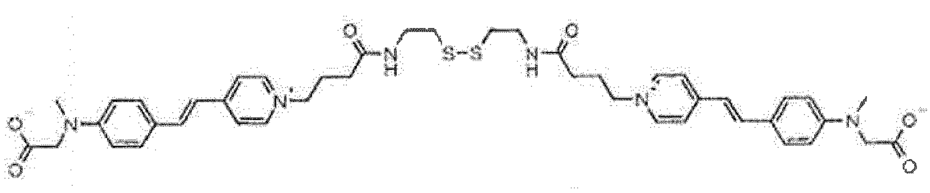

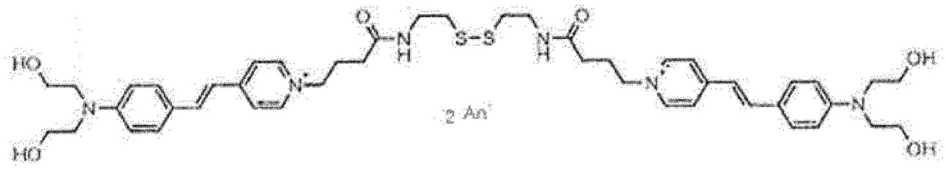

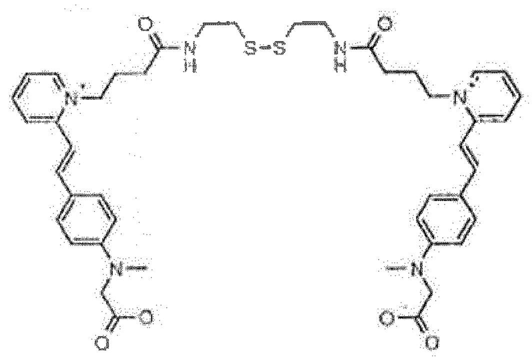

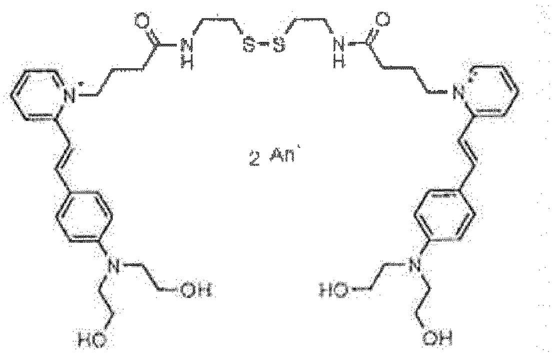

wobei $An^-$ und M' für anionische Gegenionen stehen.

**7.** Zusammensetzung nach einem der vorhergehenden Ansprüche, wobei das Polymer mit Thiolfunktion eine Molmasse zwischen $8.10^2$ und $5.10^6$ aufweist.

**8.** Zusammensetzung nach einem der vorhergehenden Ansprüche, wobei es sich bei dem Polymer mit Thiolfunktion um ein lineares oder vernetztes Polymer handelt.

**9.** Zusammensetzung nach einem der Ansprüche 1 bis 7, wobei es sich bei dem Polymer mit Thiolfunktion um ein Dendrimer handelt.

**10.** Zusammensetzung nach einem der Ansprüche 1 bis 7 und 8, wobei es sich bei dem Polymer mit Thiolfunktion um ein Polyethylenimin handelt.

**11.** Verfahren zum Färben von Keratinfasern, bei dem man auf die Fasern eine Färbezusammensetzung gemäß einem der vorhergehenden Ansprüche aufbringt.

**12.** Färbeverfahren nach dem vorhergehenden Anspruch, bei dem die Keratinfasern dunkel sind und eine Tonhöhe kleiner 6 und vorzugsweiser kleiner gleich 4 aufweisen.

**13.** Verwendung mindestens eines Direktfarbstoffs mit Disulfidfunktion und mindestens eines Polymers mit einer oder mehreren Thiolfunktionen zum Färben von Keratinfasern, insbesondere Haaren.

**14.** Verwendung nach dem vorhergehenden Anspruch mindestens eines Direktfarbstoffs mit Disulfidfunktion und mindestens eines Polymers mit einer oder mehreren Thiolfunktionen zum Aufhellen von dunklen Keratinfasern mit einer Tonhöhe kleiner 6 und vorzugsweise kleiner gleich 4.

## Claims

**1.** Dye composition comprising, in a suitable cosmetic medium, i) one or more dyes chosen from direct dyes comprising a disulfide function or functions and a protected-thiol function or functions, and ii) at least one or more polymer(s) comprising a thiol function or functions and having a molecular weight of greater than 500.

**2.** Composition according to the preceding claim, in which the dye(s) is (are) of formula **(I)**:

$$\mathbf{A\text{-}\ (X)p\ \text{-}\ C_{sat}\ \text{-}\ S\text{-}U} \qquad \mathbf{(I)}$$

the organic or inorganic acid salts thereof, the optical and geometric isomers thereof and the solvates thereof such as hydrates,
in which formula (**I**):

- U represents a radical chosen from:

  **i) -S- $C'_{sat}$ - $(X')_{p'}$ - A';**
  **ii) -S- $C'_{sat}$ - $(X')_{p'}$ - D**; and
  **iii) -Y**;

- **A** and **A'**, which may be identical or different, represent a radical containing one or more cationic or noncationic chromophore(s);
- **Y** represents a) an alkali metal; b) an alkaline-earth metal; c) an ammonium group: $N^+R^\alpha R^\beta R^\gamma R^\delta$, An"- or a phosphonium group: $P^+R^\alpha R^\beta R^\gamma R^\delta$, An"- with $R^\alpha$, $R^\beta$, $R^\gamma$ and $R^\delta$, which may be identical or different, representing a hydrogen atom or a $(C_1-C_4)$ alkyl group and An"- representing an anionic counterion; or d) a thiol-function-protecting group;
- **X** and **X'**, which may be identical or different, represent:

  a) a linear or branched, saturated or unsaturated, optionally substituted, $C_1-C_{30}$ hydrocarbon-based chain, optionally interrupted and/or optionally terminated at one or both ends of said chain with one or more divalent groups or combinations thereof chosen from:

    i) -N(R)-; $-N^+(R)(R')$ -, $Q^-$; -O-; -S-; -CO-; $-SO_2-$ with R, R', which may be identical or different, chosen from a hydrogen and a $C_1-C_4$ alkyl, hydroxyalkyl and aminoalkyl radical, $Q^-$ represents an anionic counterion; and
    ii) a condensed or noncondensed, saturated or unsaturated, aromatic or nonaromatic (hetero)cyclic radical optionally comprising one or more heteroatoms, which may be identical or different, and optionally substituted;

  or
  b) one or more divalent groups or a combination thereof, chosen from:

    $-SO_2-$ -O-; -S-; -N(R)-; $-N^+(R)(R°)$ -; -CO-; with R and R°, which may be identical or different, representing a hydrogen atom, a $C_1-C_4$ alkyl radical, a $C_1-C_4$ hydroxyalkyl radical or an aryl$(C_1-C_4)$alkyl; and a heterocycloalkyl or heteroaryl radical, which is cationic or noncationic;

- **p** and **p'**, which may be identical or different, are 0 or 1;
- **$C_{sat}$** and **$C'_{sat}$**, which may be identical or different, represent a linear or branched, optionally substituted, optionally cyclic $C_1-C_{18}$ alkylene chain;
- **D** corresponds to a radical chosen from hydroxyl, hydroxyalkyl, alkoxy, carboxylic, carboxylate, amino, alkylamino and dialkylamino radicals.

**3.** Composition according to the preceding claim, in which **$C_{sat}$** and **$C'_{sat}$** represent a - $-(CH_2)_k$-chain with k being an integer between 1 and 8, limits included.

**4.** Composition according to either one of Claims 2 and 3, in which p is equal to 1, X and X', which may be identical or different, represent the following sequence:

$$-(T)_t-(Z)_z-(T')_{t'}-$$

said sequence being linked in the formulae (I) of (II) symmetrically as follows:

$$-C_{sat}\ (\text{or } C'_{sat})-(T)_t-(Z)_z-(A \text{ or } A');$$

in which:

**T** and **T'**, which may be identical or different, represent one or more radicals or combinations thereof chosen

from: -O-; -S-; -N(R)-; $-N^+(R)(R^o)-$; -SO-; $-SO_2-$; and -CO-; with R and $R^o$, which may be identical or different, representing a hydrogen atom, or a $C_1$-$C_4$ alkyl, $C_1$-$C_4$ hydroxyalkyl or aryl ($C_1$-$C_4$) alkyl radical; and a cationic or noncationic, preferably monocyclic, heterocycloalkyl or heteroaryl radical, preferably containing two heteroatoms;

**t** and **t'**, which may be identical or different, are 0 or 1;

**Z** represents:

- $-(CH_2)_m-$ with m being an integer between 1 and 8;
- $-(CH_2CH_2O)_q-$ or $-(OCH_2CH_2)_q-$ in which q is an integer between 1 and 15;
- an aryl, alkylaryl or arylalkyl radical of which the alkyl radical is $C_1$-$C_4$ and the aryl radical is preferably $C_6$, being optionally substituted with at least one $SO_3M$ group with M representing a hydrogen atom, an alkali metal or an ammonium group substituted with one or more identical or different, linear or branched, $C_1$-$C_{18}$ alkyl radicals, optionally bearing at least one hydroxyl;

**z** is 0 or 1.

**5.** Composition according to any one of the preceding claims, in which the direct dye comprising a disulfide function is chosen from the dyes of formulae **(III)** to **(XIX) :**

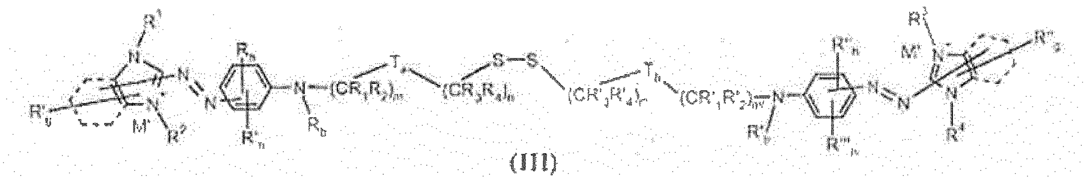

(III)

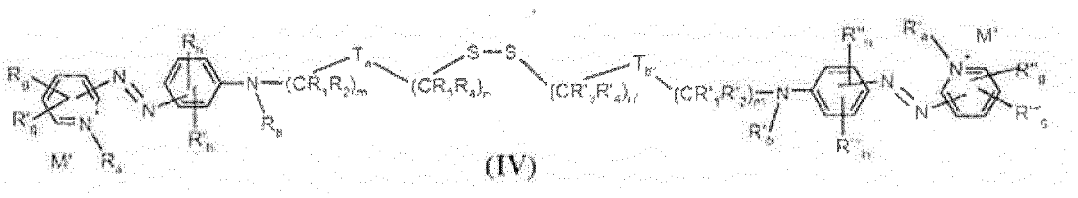

(IV)

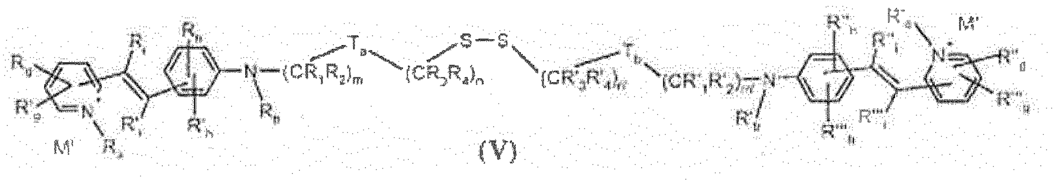

(V)

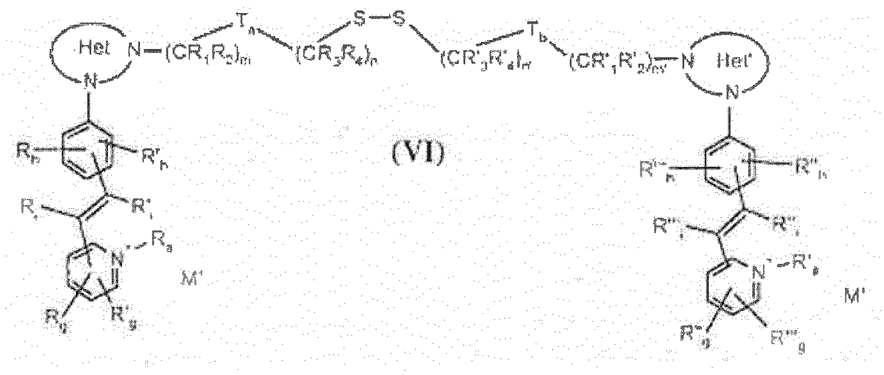

(VI)

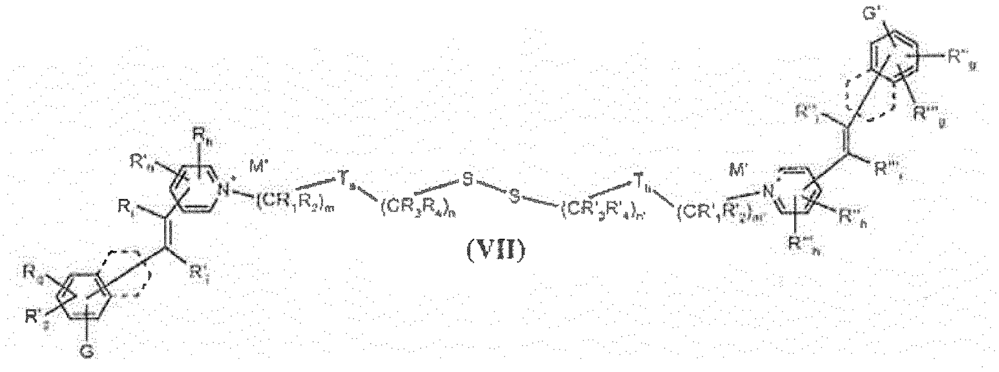

(VII)

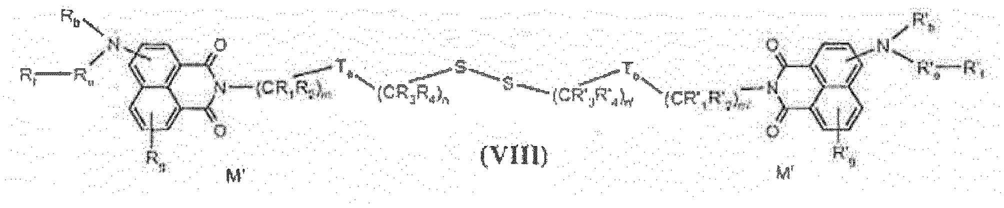

(VIII)

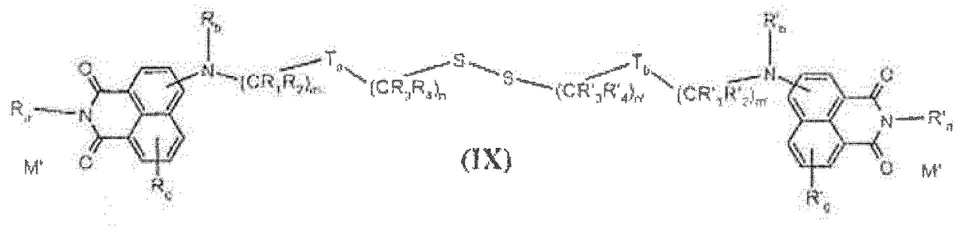

(IX)

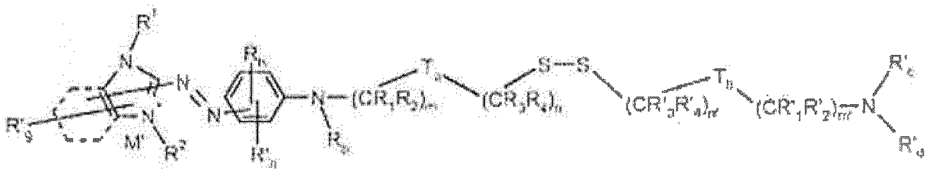

(X)

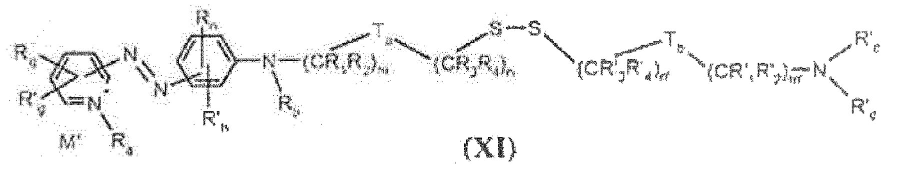

(XI)

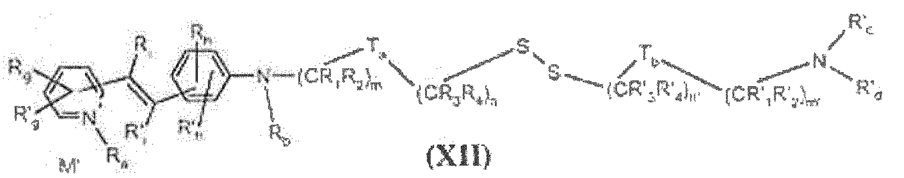

(XII)

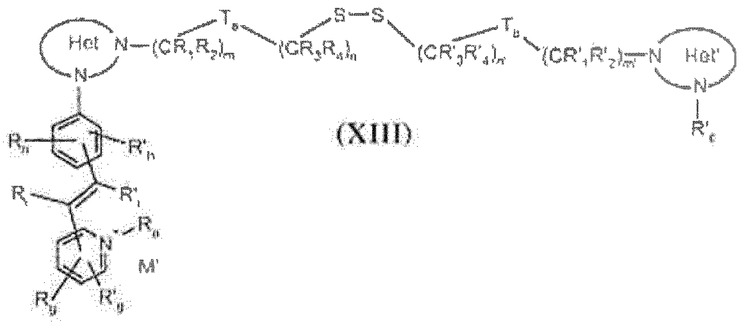

(XIII)

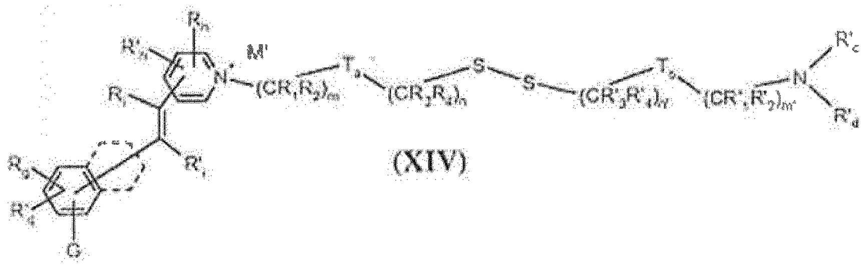

(XIV)

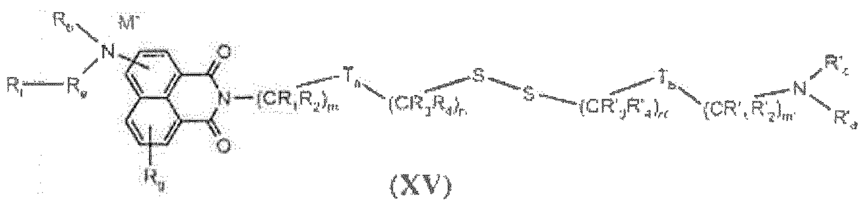

(XV)

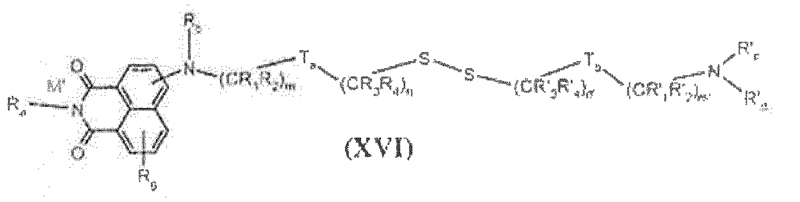

(XVI)

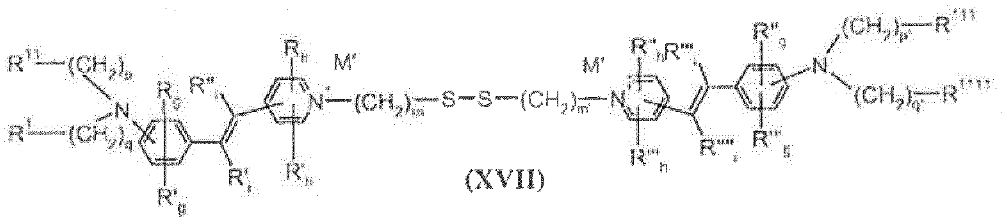

(XVII)

and

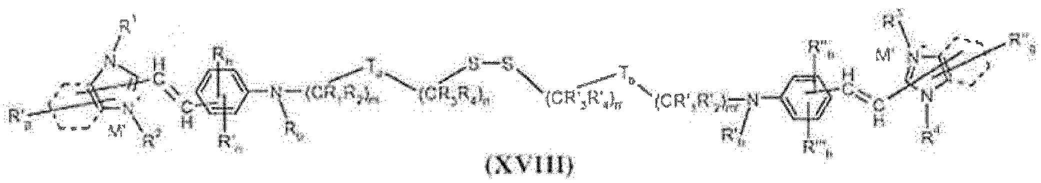

(XVIII)

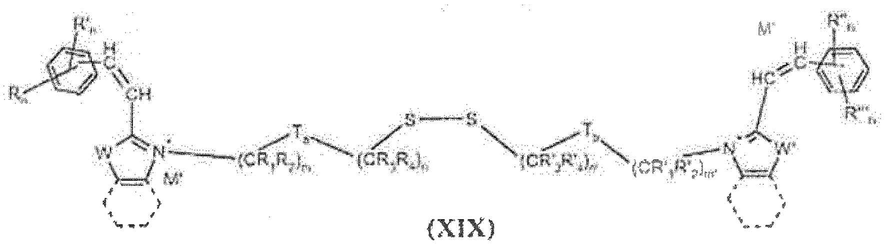

(XIX)

in which

- G and G', which may be identical or different, represent an $-NR_cR_d$ group, an $-NR'_cR'_d$ group or a $C_1$-$C_6$ alkoxy group which is optionally substituted, preferably unsubstituted; preferably, G and G' represent an $-NR_cR_d$ group and an $-NR'_cR'_d$ group, respectively;
- W and W', which may be identical or different, represent an oxygen atom or an N-R1 group, with R1 as defined hereinafter;
- $R^1$, $R^2$, $R^3$ and $R^4$, which may be identical or different, represent a hydrogen atom or a $C_1$-$C_6$ alkyl group;
- $R_a$ and $R'_a$, which may be identical or different, represent an aryl ($C_1$-$C_4$) alkyl group or a $C_1$-$C_6$ alkyl group optionally substituted with a hydroxyl group or an amino, $C_1$-$C_4$ alkylamino or $C_1$-$C_4$ dialkylamino group, it being possible for said alkyl radicals to form, with the nitrogen atom which bears them, a heterocycle comprising from 5 to 7 ring members, optionally comprising another heteroatom identical to or different from nitrogen; preferably, $R_a$ and $R'_a$ represent a $C_1$-$C_3$ alkyl group optionally substituted with a hydroxyl group, or a benzyl group;
- $R_b$ and $R'_b$, which may be identical or different, represent a hydrogen atom, or an aryl($C_1$-$C_4$)alkyl group or a $C_1$-$C_6$ alkyl group which is optionally substituted; preferably, $R_b$ and $R'_b$ represent a hydrogen atom or a $C_1$-$C_3$ alkyl or benzyl group;
- $R_c$, $R'_c$, $R_d$ and $R'_d$, which may be identical or different, represent a hydrogen atom, an aryl ($C_1$-$C_4$) alkyl group, a $C_1$-$C_6$ alkoxy group or a $C_1$-$C_6$ alkyl group which is optionally substituted; $R_c$, $R'_c$, $R_d$ and $R'_d$ preferably represent a hydrogen atom, a hydroxyl group, a $C_1$-$C_3$ alkoxy group, an amino group, a $C_1$-$C_3$ (di)alkylamino group, or a $C_1$-$C_3$ alkyl group which is optionally substituted with i) a hydroxyl group, ii) an amino group, iii) a $C_1$-$C_3$ (di)alkylamino group, or iv) a quaternary ammonium group (R") (R"') (R"")$N^+$-;
or else two adjacent radicals $R_c$ and $R_d$, $R'_c$ and $R'_d$, borne by the same nitrogen atom, together form a heterocyclic or heteroaryl group; preferably, the heterocycle or the heteroaryl is monocyclic and comprises between 5 and 7 ring members; more preferably, the groups are chosen from imidazolyl and pyrrolidinyl;
- $R_e$ and $R'_e$, which may be identical or different, represent a linear or branched, optionally unsaturated, divalent, $C_1$-$C_6$ alkylenyl hydrocarbon-based chain;
- $R_f$ and $R'_f$, which may be identical or different, represent a quaternary ammonium group (R") (R"')(R"")$N^+$- where R", R"' and R"", which may be identical or different, represent a hydrogen atom or a $C_1$-$C_4$ alkyl group, or else (R") (R"')(R"")$N^+$- represents an optionally substituted cationic heteroaryl group, preferably an imidazolinium group optionally substituted with a $C_1$-$C_3$ alkyl group;
- $R_g$, $R'_g$, $R''_g$, $R'''_g$, $R_h$, $R'_h$, $R''_h$ and $R'''_h$, which may be identical or different, represent a hydrogen atom, a halogen atom, an amino, $C_1$-$C_4$ alkylamino, $C_1$-$C_4$ dialkylamino, cyano, carboxyl, hydroxyl or trifluoromethyl group, an acylamino, $C_1$-$C_4$ alkoxy, (poly) hydroxy ($C_2$-$C_4$) alkoxy, alkylcarbonyloxy, alkoxycarbonyl or alkylcarbonylamino radical, an acylamino, carbamoyl or alkylsulfonylamino radical, an aminosulfonyl radical, or a $C_1$-$C_{16}$ alkyl radical optionally substituted with a group chosen from $C_1$-$C_{12}$ alkoxy, hydroxyl, cyano, carboxyl, amino, $C_1$-$C_4$ alkylamino and $C_1$-$C_4$ dialkylamino, or else the two alkyl radicals borne by the nitrogen atom of the amino group form a heterocycle comprising from 5 to 7 ring members and optionally comprising another heteroatom identical to or different from that of the nitrogen atom; preferably, $R_g$, $R'_g$, $R''_g$, $R'''_g$, $R_h$, $R'_h$, $R''_h$ and $R'''_h$ represent a hydrogen atom, a halogen atom or a $C_1$-$C_3$ alkyl group;
- or else two groups Rg and $R'_g$; $R''_g$ and $R'''_g$; $R_h$, and $R'_h$; $R''_h$ and $R'''_h$, borne by two adjacent carbon atoms, together form a benzo or indeno ring or a fused heterocycloalkyl or fused heteroaryl group; the benzo, indeno, heterocycloalkyl or heteroaryl ring being optionally substituted with a halogen atom, an amino, $C_1$-$C_4$ alkylamino, $C_1$-$C_4$ dialkylamino, nitro, cyano, carboxyl, hydroxyl or trifluoromethyl group, an acylamino, $C_1$-$C_4$ alkoxy, (poly)hydroxy($C_2$-$C_4$)alkoxy, alkylcarbonyloxy, alkoxycarbonyl or alkylcarbonylamino radical, an acylamino, carbamoyl or alkylsulfonylamino radical, an aminosulfonyl radical, or a $C_1$-$C_{16}$ alkyl radical optionally substituted with: a group chosen from $C_1$-$C_{12}$ alkoxy, hydroxyl, cyano, carboxyl, amino, $C_1$-$C_4$ alkylamino and $C_1$-$C_4$ dialkylamino, or else the two alkyl radicals borne by the nitrogen atom of the amino group form a heterocycle comprising from 5 to 7 ring members and optionally comprising another heteroatom identical to or different from

that of the nitrogen atom; preferably, Rg and $R'_g$; $R''_g$ and $R'''_g$ together form a benzo group;

- or else, when G represents $-NR_cR_d$ and G' represents $-NR'_cR'_d$, two groups $R_c$ and $R'_g$; $R'_c$ and $R''_g$; $R_d$ and Rg; $R'_d$ and $R'''_g$ together form a saturated heteroaryl or heterocycle optionally substituted with one or more $C_1$-$C_6$ alkyl groups, preferably a heterocycle containing one or two heteroatoms chosen from nitrogen and oxygen and comprising between 5 and 7 ring members; more preferably, the heterocycle is chosen from morpholinyl, piperazinyl, piperidinyl and pyrrolidinyl groups;
- $R_i$, $R'_i$, $R''_i$ and $R'''_i$, which may be identical or different, represent a hydrogen atom or a $C_1$-$C_4$ alkyl group;
- $R_1$, $R_2$, $R_3$, $R_4$, $R'_1$, $R'_2$, $R'_3$ and $R'_4$, which may be identical or different, represent a hydrogen atom or a $C_1$-$C_4$ alkyl, $C_1$-$C_{12}$ alkoxy, hydroxyl, cyano, carboxyl, amino, $C_1$-$C_4$ alkylamino or $C_1$-$C_4$ dialkylamino group, it being possible for said alkyl radicals to form, with the nitrogen atom which bears them, a heterocycle comprising from 5 to 7 ring members, optionally comprising another heteroatom identical to or different from nitrogen; preferably, $R_1$, $R_2$, $R_3$, $R_4$, $R'_1$, $R'_2$, $R'_3$ and $R'_4$ are hydrogen atoms or an amino group; more preferably, $R_1$, $R_2$, $R_3$, $R_4$, $R'_1$, $R'_2$, $R'_3$ and $R'_4$ represent a hydrogen atom;
- $T_a$ and $T_b$, which may be identical or different, represent i) either a covalent □ bond, ii) or one or more radicals or combinations thereof chosen from $-SO_2-$, -O-, -S-, -N(R)-, $-N^+(R)(R°)$ - and -CO-, with R and R°, which may be identical or different, representing a hydrogen atom, a $C_1$-$C_4$ alkyl or $C_1$-$C_4$ hydroxyalkyl radical, or an aryl-($C_1$-$C_4$) alkyl; preferably, $T_a$ is identical to $T_b$ and they represent a covalent □. bond or a group chosen from -N(R)-, -C(O)-N(R)-, -N(R)-C(O)-, -O-C(O) -, -C(O) -O- and $-N^+(R)(R^o)-$, with R and $R^o$, which may be identical or different, representing a hydrogen atom or a $C_1$-$C_4$ alkyl group; more preferably, $T_a$ and $T_b$ represent a □. bond; iii) or a cationic or noncationic, preferably monocyclic, preferably identical heterocycloalkyl or heteroaryl radical, preferably containing two heteroatoms (more preferably two nitrogen atoms) and preferably comprising from 5 to 7 ring members, such as imidazolium;
-

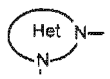 or 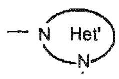

which may be identical or different, represent an optionally substituted heterocyclic group; preferably, the heterocycles are identical, monocyclic and saturated, and comprise in total two nitrogen atoms and from 5 to 8 ring members;

-

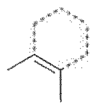

represents an aryl or heteroaryl group fused to the imidazolium or phenyl ring; or else is absent from the imidazolium or phenyl ring;

- $R^1$ and $R^{1111}$, which may be identical or different, represent a hydroxyl group, or a group amino ($NR_aR_b$) or ammonium ($N^+R_aR_bR_c$), $An^-$; with $R_a$, $R_b$ and $R_c$, which may be identical or different, representing a hydrogen atom or a ($C_1$-$C_4$)alkyl group; or else two alkyl groups $R_a$ and $R_b$ of the amino or ammonium group form a heterocycle comprising from 5 to 7 ring members and optionally comprising another heteroatom which may be identical to or different from that of the nitrogen atom, such as morpholinyl, piperazinyl, piperidinyl, pyrrolyl, morpholinium, piperazinium, piperidinium or pyrrolinium, and $An^-$ representing an anionic counterion;
- $R^{11}$ and $R^{111}$, which may be identical or different, represent a hydrogen atom or a group as defined for $R^1$ and $R^{1111}$ respectively;
- m, m', n and n', which may be identical or different, represent an integer between 0 and 6, limits included, with m+n, m'+n', which may be identical or different, representing an integer between 1 and 10, limits included; preferably, m+n=m'+n' = an integer between 2 and 4, limits included; more preferably, m+n=m'+n' = an integer equal to 2;

M' representing a counterion or an organic or inorganic acid salt.

**6.** Composition according to any one of the preceding claims, in which the direct dye comprising a disulfide or protected-thiol function is chosen from:

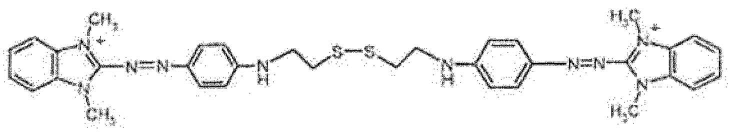

2M'

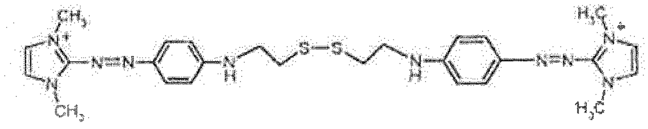

2M'

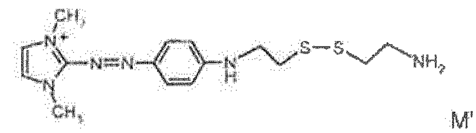

M'

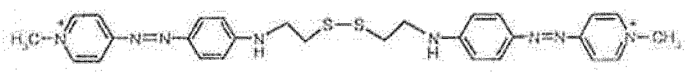

2M'

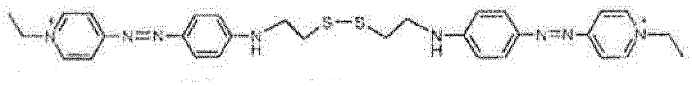

2M'

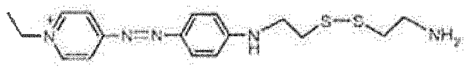

M'

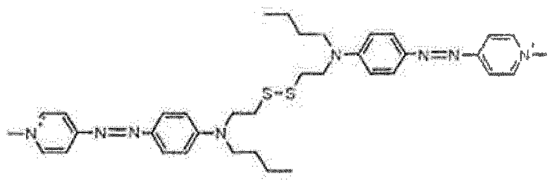

2M'

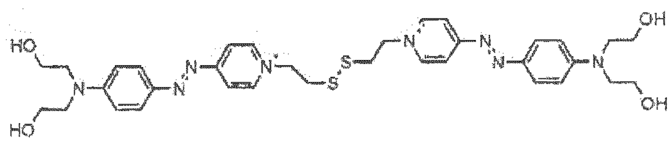

2M'

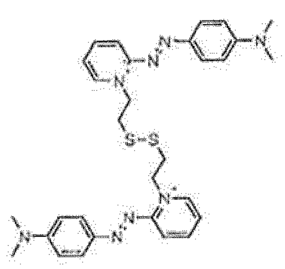

2M'

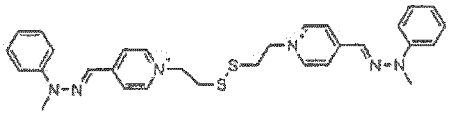

2M'

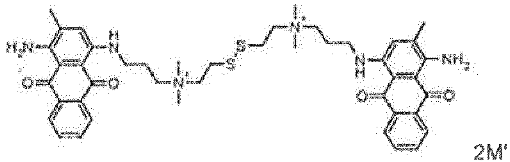

2M'

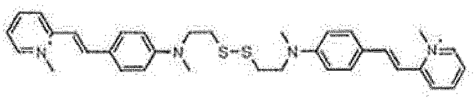

2M'

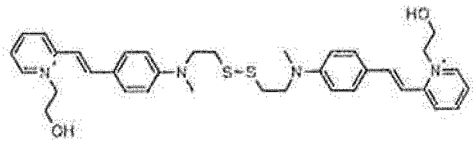

2M'

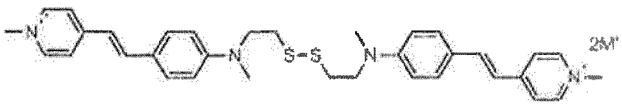

2M'

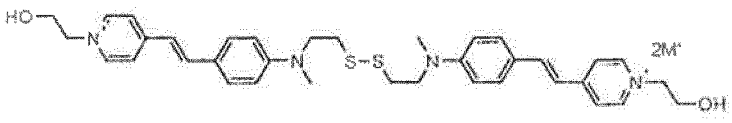

2M'

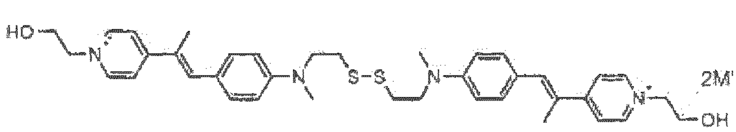

2M'

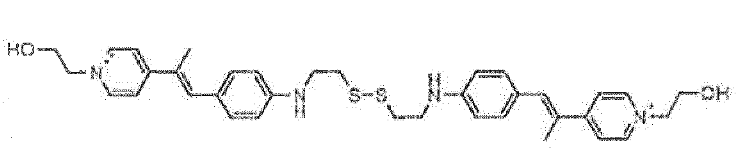

2M'

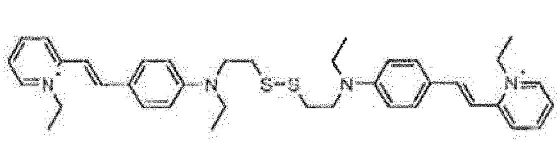

2M'

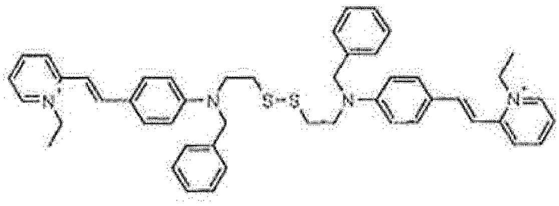

2M'

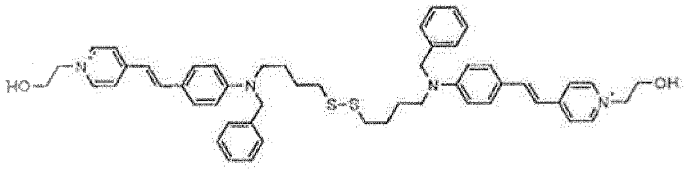

2M'

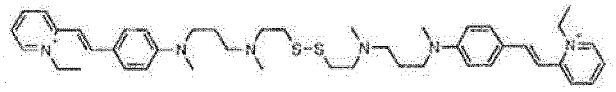

2M'

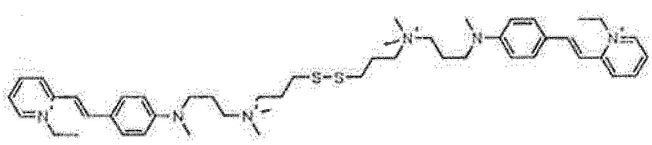

4M'

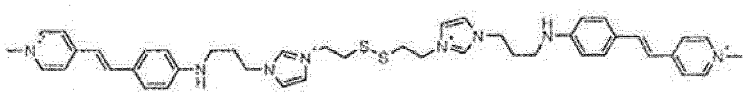

4M'

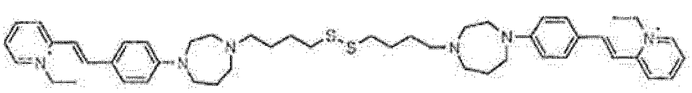

2M'

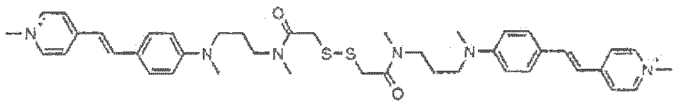

2M'

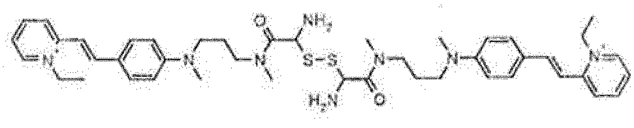

2M'

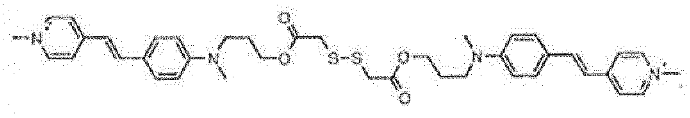

2M'

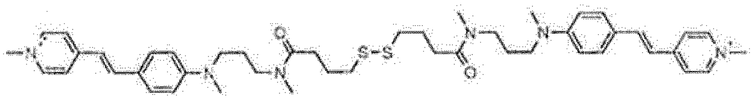

2M'

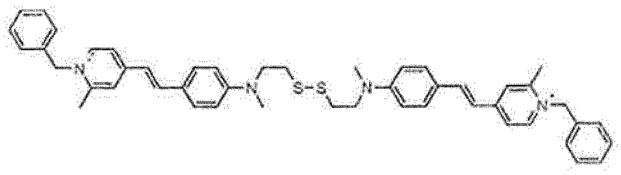

2M'

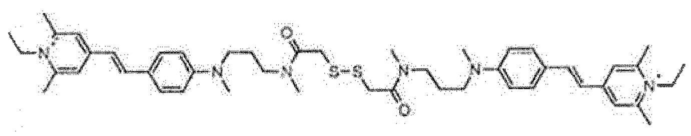

2M'

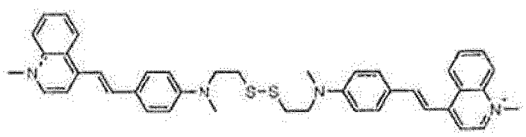

2M'

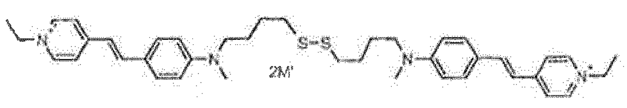

2M'

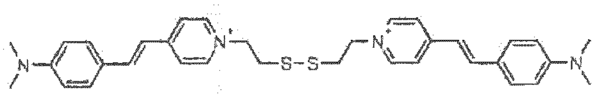

2M'

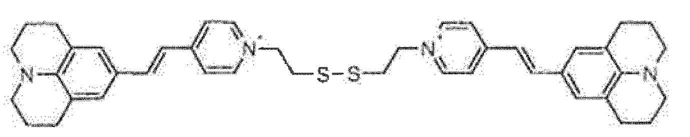

2M'

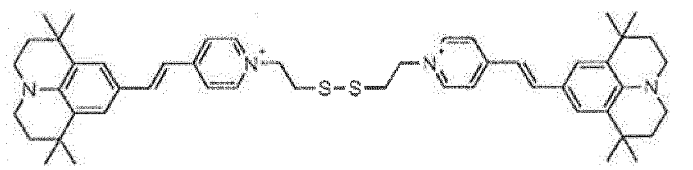

2M'

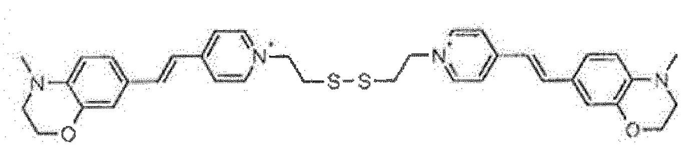

2M'

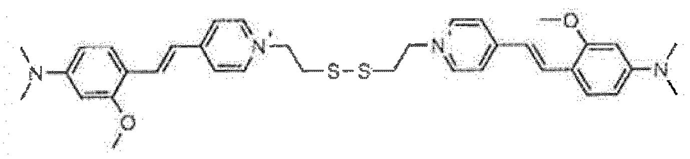

2M'

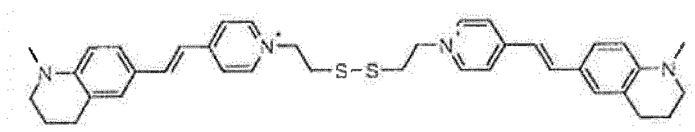

2M'

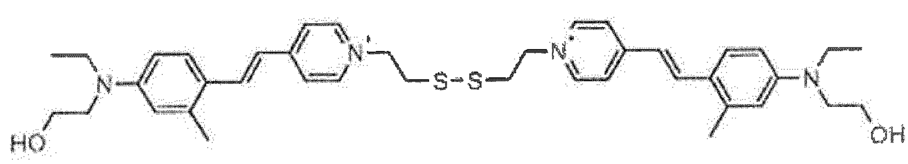

2M'

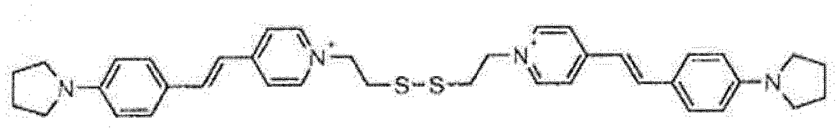

2M'

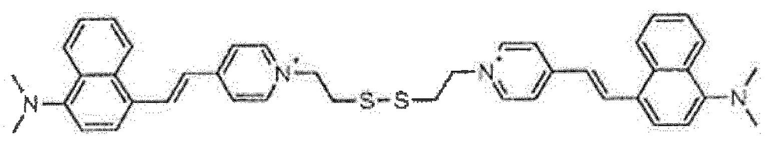

2M'

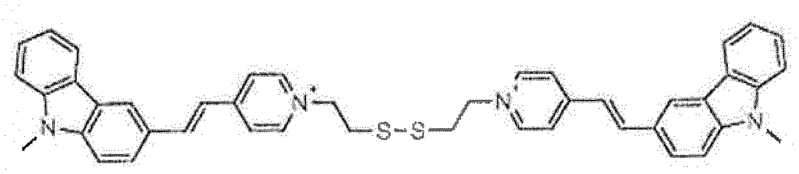

2M'

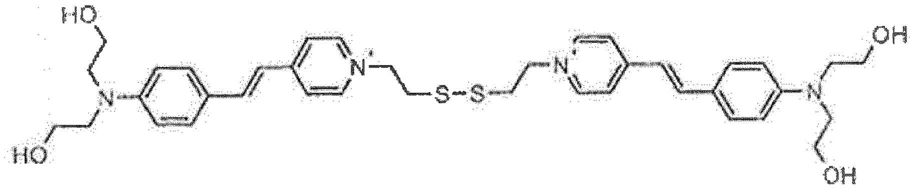

2M'

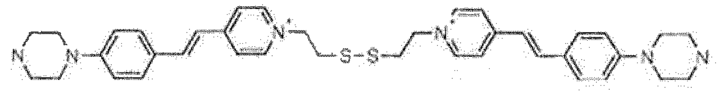

2M'

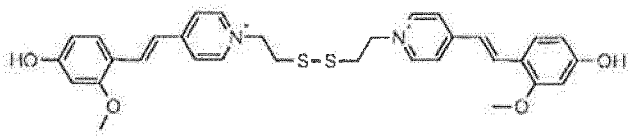

2M'

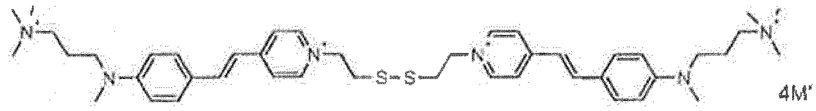

4M'

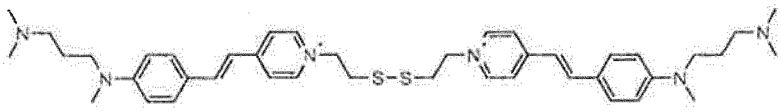

2M'

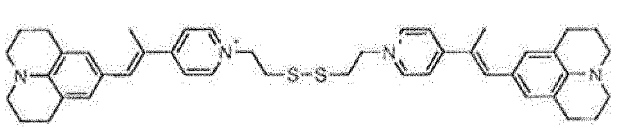

2M'

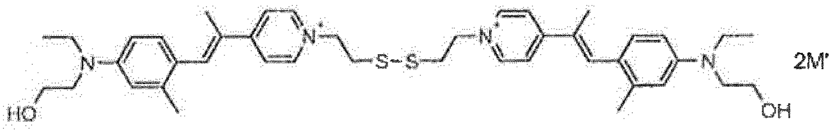

2M'

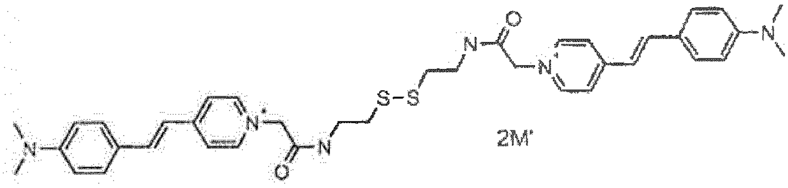

2M'

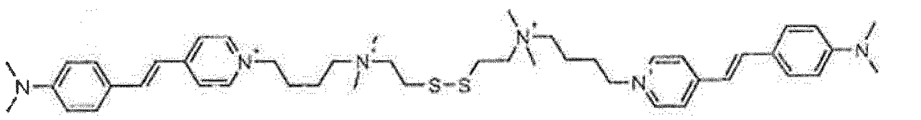

4M'

2M'

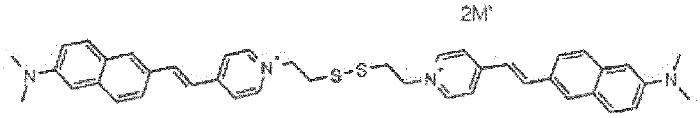

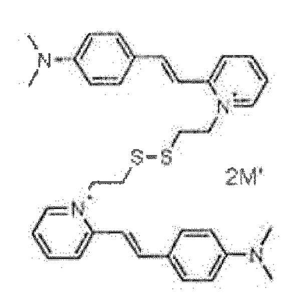

2M'

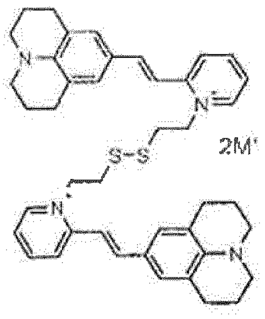

2M'

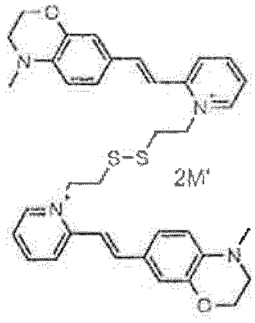

2M'

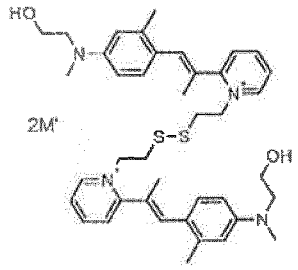

2M'

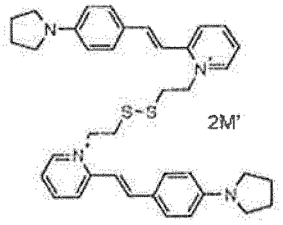

2M'

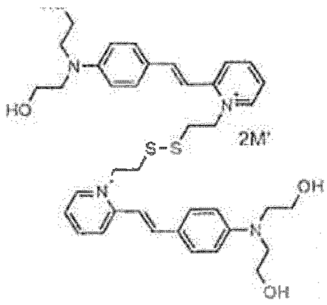

2M'

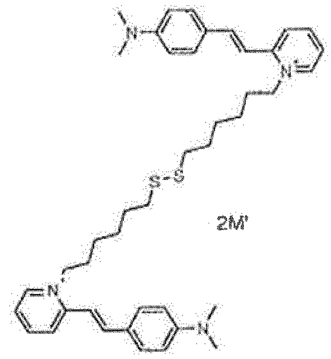

2M'

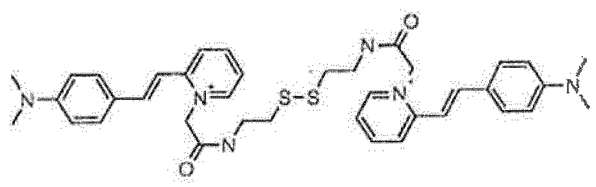

2M'

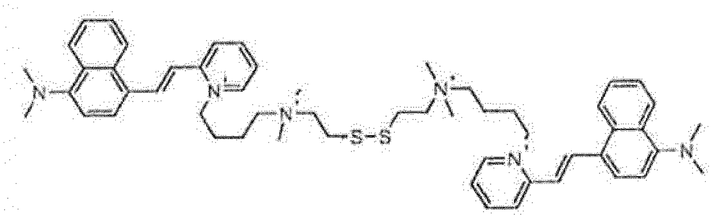

4M'

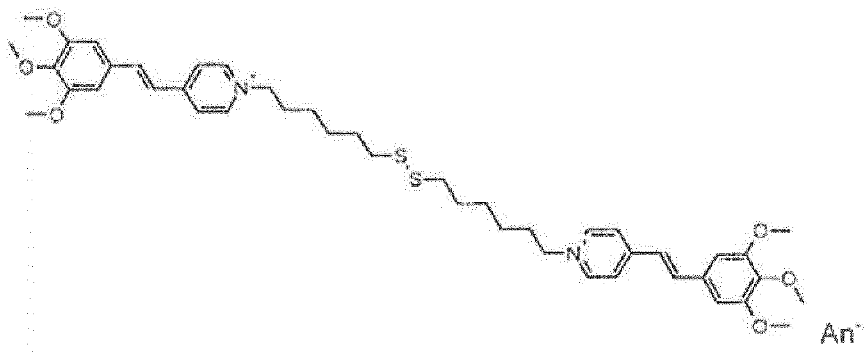

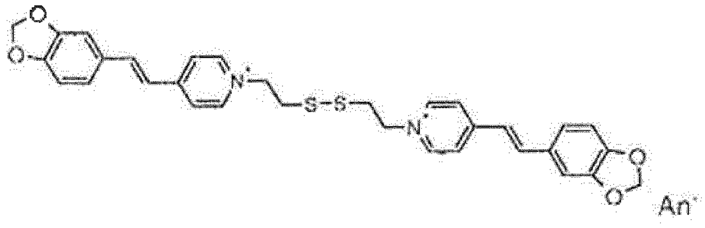

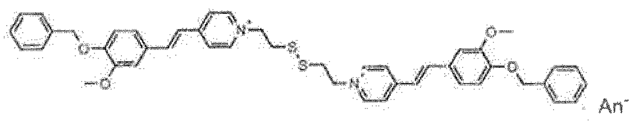

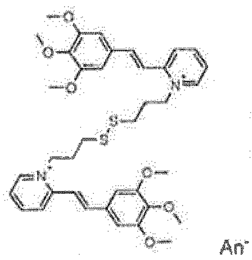

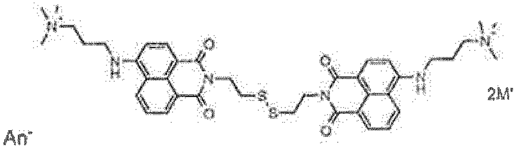

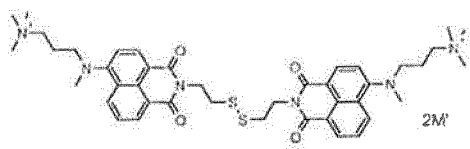

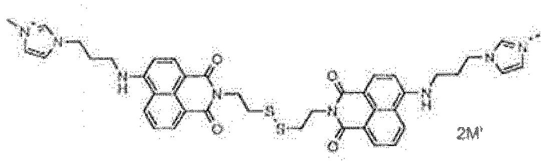

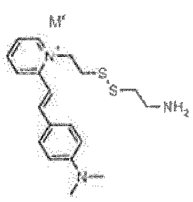

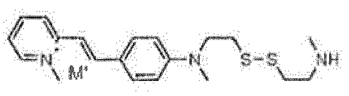

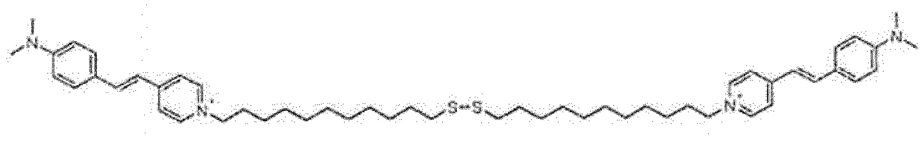

2M'

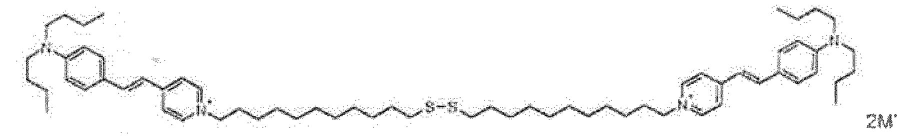

2M'

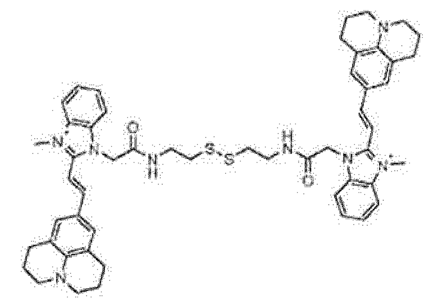

2M'

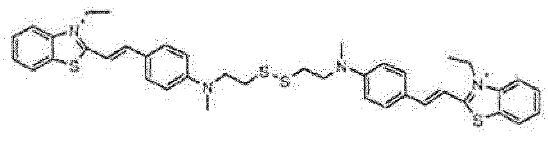

2M'

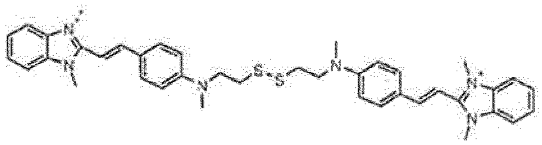

2M'

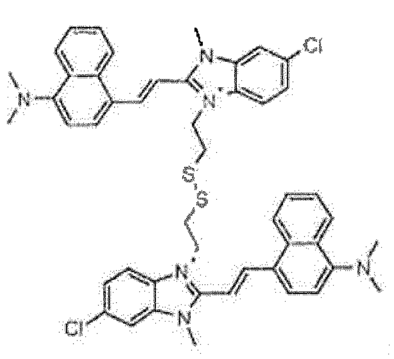

2M'

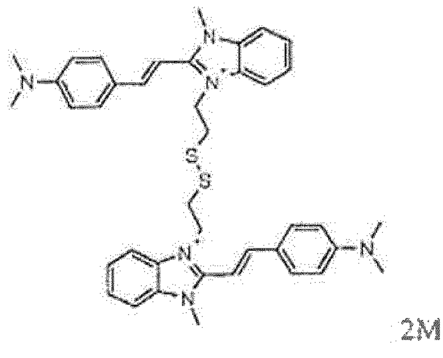

2M'

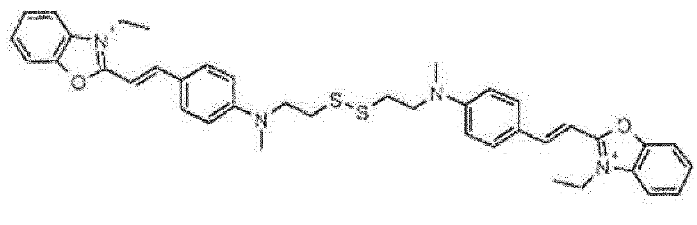

2M'

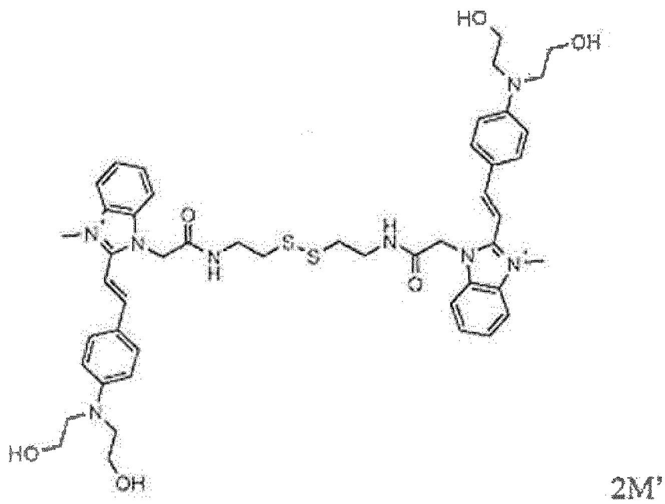

2M'

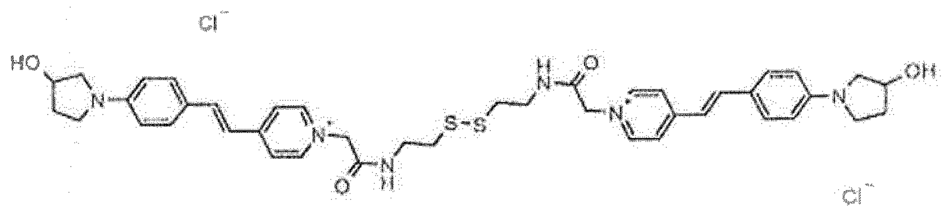

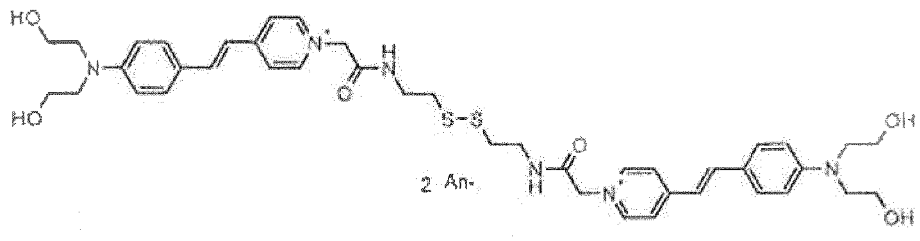

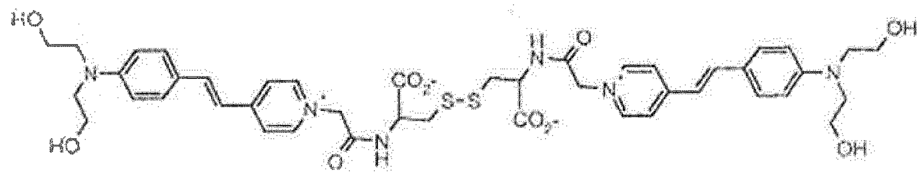

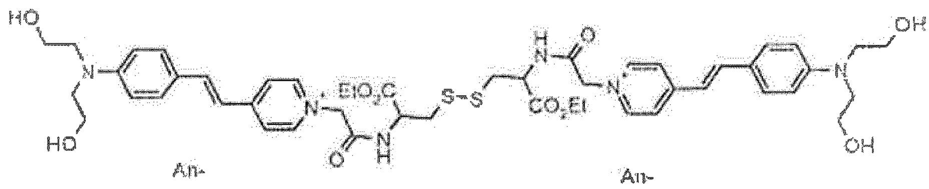

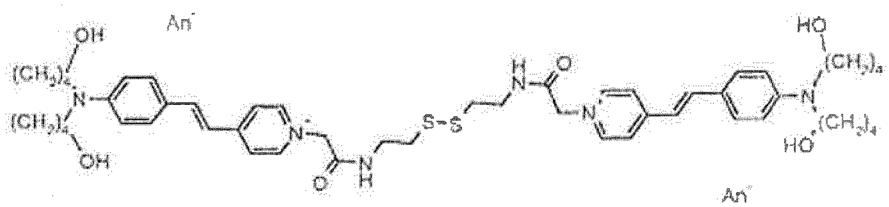

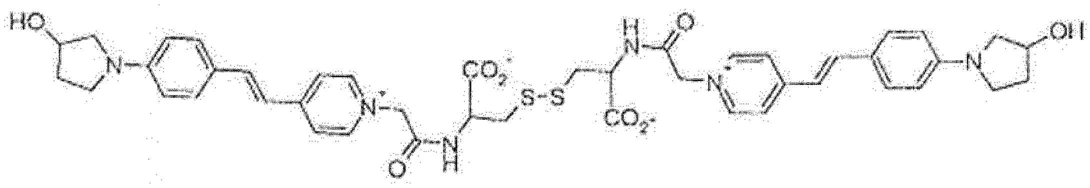

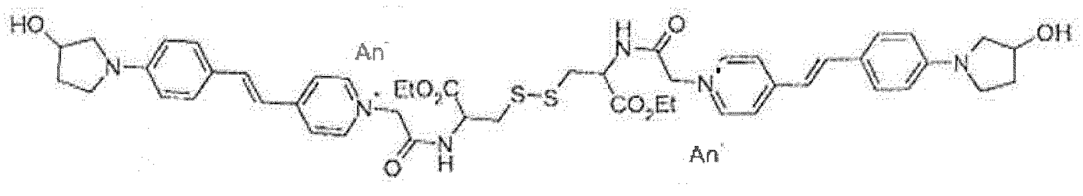

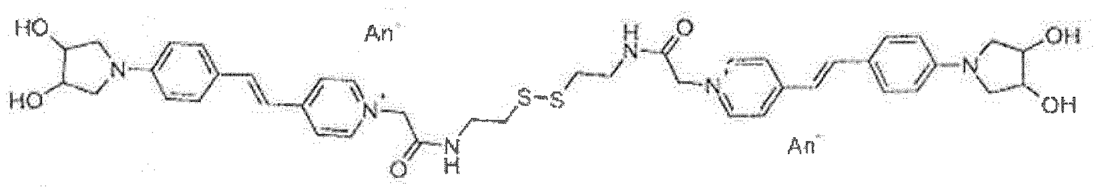

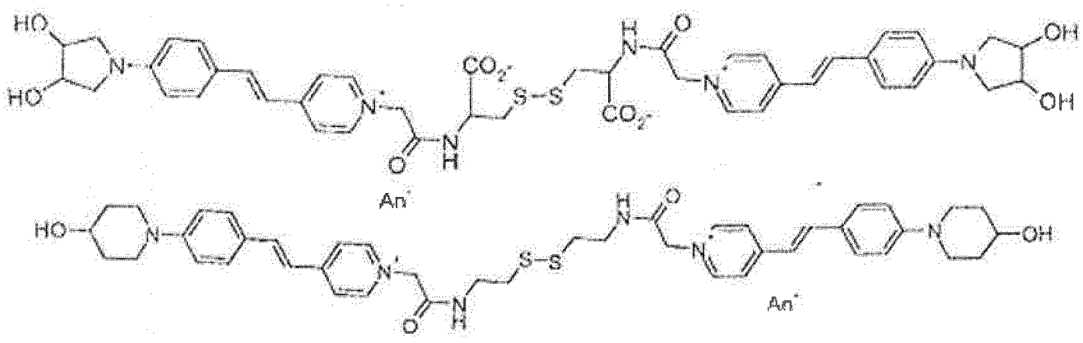

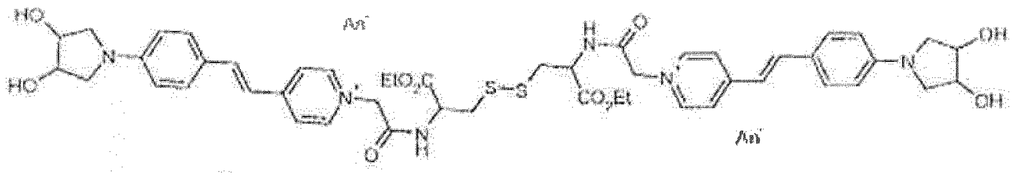

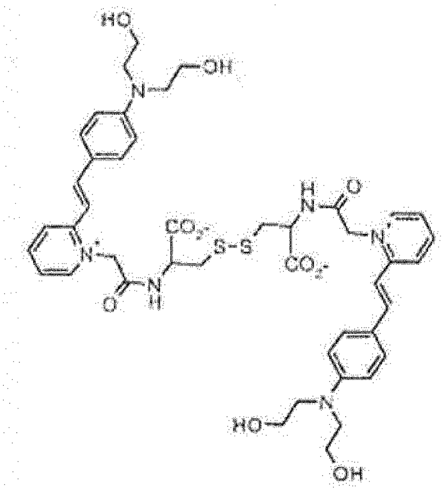

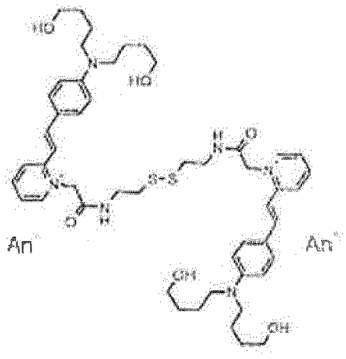

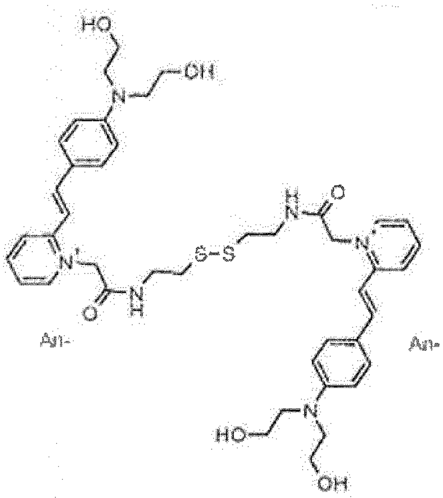

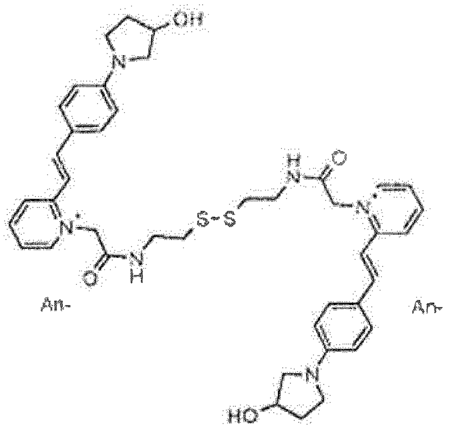

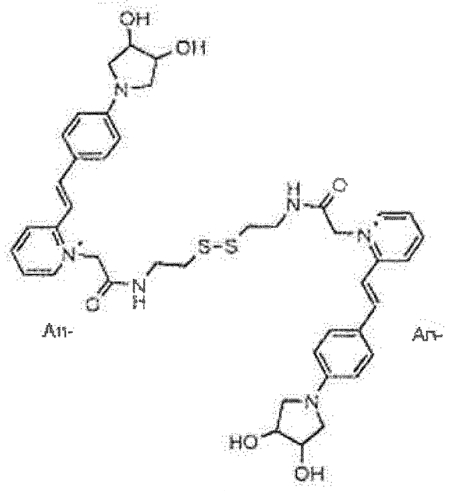

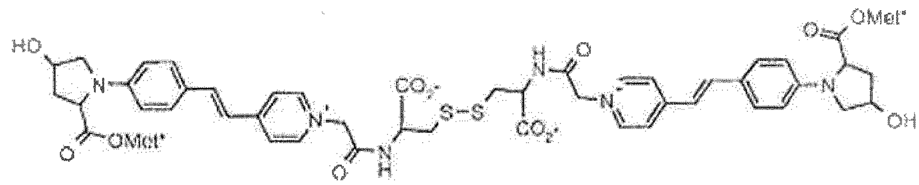

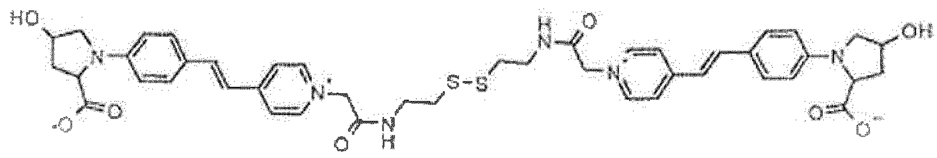

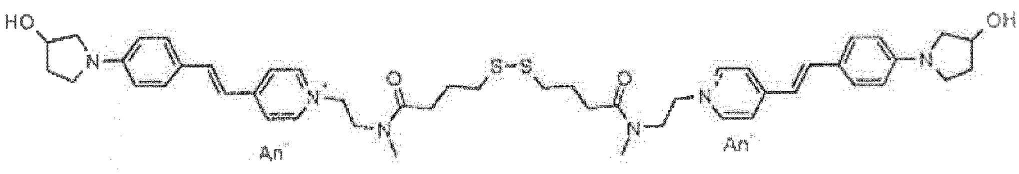

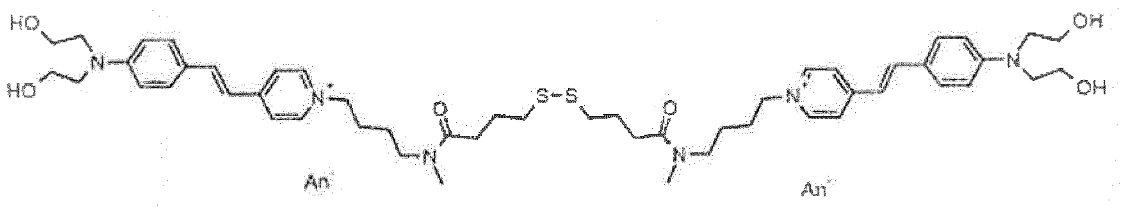

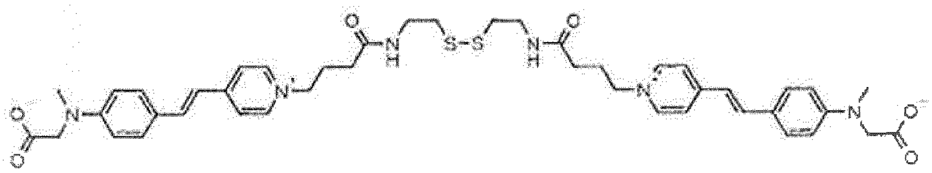

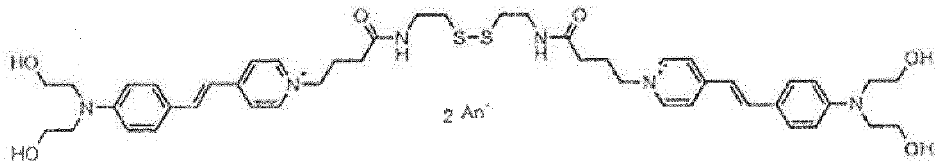

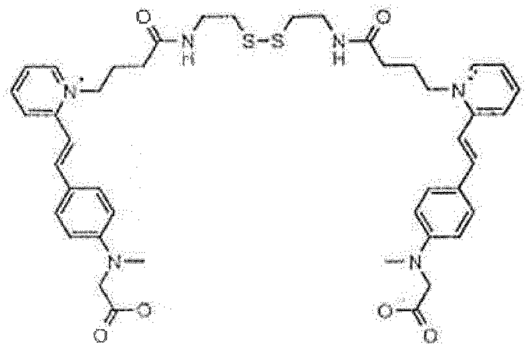

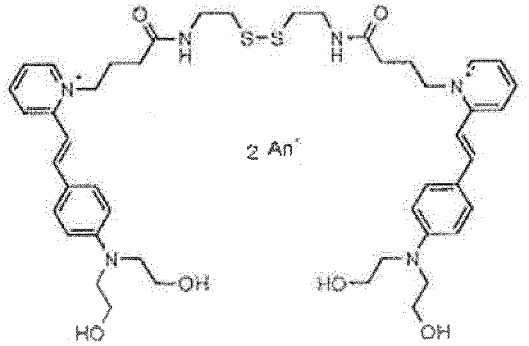

with $An^-$, M' representing anionic counterions.

7. Composition according to any one of the preceding claims, in which the polymer comprising a thiol function has a molecular molar mass of between $8\times10^2$ and $5\times10^6$.

8. Composition according to any one of the preceding claims, in which the polymer comprising a thiol function is a linear or crosslinked polymer.

9. Composition according to any one of Claims 1 to 7, in which the polymer comprising a thiol function is a dendrimer.

10. Composition according to any one of Claims 1 to 7 and 8, in which the polymer comprising a thiol function is a polyethyleneimine.

11. Method of dyeing keratin fibres which comprises applying, to the fibres, a dye composition as defined in any one of the preceding claims.

12. Dyeing method according to the preceding claim, in which the keratin fibres are dark and have a tone height of less than 6, preferably less than or equal to 4.

13. Use of at least one direct dye comprising a disulfide function and of at least one polymer comprising a thiol function or functions, for dyeing keratin fibres and in particular the hair.

14. Use according to the preceding claim, for lightening dark keratin fibres having a tone height of less than 6, preferably

less than or equal to 4, of at least one direct dye comprising a disulfide function and of at least one polymer comprising a thiol function or functions.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1647580 A
- WO 2005097051 A
- WO 2006136617 A
- WO 2006134043 A
- WO 2007039527 A
- US 2002108188 A
- EP 1133975 A
- WO 03029359 A
- EP 860636 A
- WO 9501772 A
- WO 9515144 A
- EP 714954 A
- US 5888252 A
- EP 318294 A
- EP 850636 A
- FR 2788433
- EP 920856 A
- WO 9948465 A
- FR 2757385
- EP 850637 A
- EP 918053 A
- WO 9744004 A
- FR 2570946
- FR 2285851
- DE 2538363
- FR 2189006
- FR 1560664
- FR 1540423
- FR 1567219
- FR 1516943
- FR 1221122
- DE 4220388
- DE 4137005
- WO 0166646 A
- US 5708151 A
- WO 515144 A
- GB 1195386 A
- US 3524842 A
- US 5879413 A
- EP 1062940 A
- EP 1133976 A
- GB 738585 A
- DE 2527638
- FR 2275462
- GB 197427645 A
- EP 682059 A
- WO 9614346 A
- WO 9614345 A
- WO 9612754 A
- FR 9704085
- FR 2761691
- FR 2853533
- EP 103759 A


### Littérature non-brevet citée dans la description


- Protective Groups in Organic Synthesis. 1981, 193-217
- **P. KOCIENSKI.** Protecting Groups. Thieme, 2005
- **T. W. GREENE.** Protective Groups in Organic Synthesis. 1981, 193-217
- The chemistry of synthetic dye. **DE K. VENKATARAMAN.** Kirk Othmer. Academic press, 1952, vol. 1, 7
- Dye intermediate. ULLMANN's ENCYCLOPEDIA of Industrial chemistry. Wiley and sons, 1993
- Labeling Technologies. Molecular Probes/Invitrogen. Oregon, 2005
- The chemistry of synthetic dye. **DE K. VENKATARAMAN.** Kirk Othmer. Academic press, 1952, vol. 1-7
- *Acta Histochem.,* 1978, vol. 61 (1), 48-52
- *Tsitologiya,* 1968, vol. 10 (3), 403-5
- *Zh. Obshch. Khim.,* 1970, vol. 40 (1), 195-202
- *Ann. Chim. (Rome,* 1975, vol. 65 (5-6), 305-14
- *Journal of the Chinese Chemical Society (Taipei,* 1988, vol. 45 (1), 209-211
- *Rev. Roum. Chim.,* 1988, vol. 33 (4), 377-83
- *Text. Res. J.,* 1984, vol. 54 (2), 105-7
- *Chim. Ind. (Milan,* 1974, vol. 56 (9), 600-3
- *Khim. Tekhnol.,* 1979, vol. 22 (5), 548-53
- *Ger. Monatsh. Chem.,* 1975, vol. 106 (3), 643-8
- *MRL Bull. Res. Dev.,* 1992, vol. 6 (2), 21-7
- **LIHUA JIANYAN.** *Huaxue Fence,* 1993, vol. 29 (4), 233-4
- *Dyes Pigm.,* 1992, vol. 19 (1), 69-79
- *Dyes Pigm.,* 1989, vol. 11 (3), 163-72

- The Synthesis and Characterization of Dendritic Molecules. **H.M. JANSSEN ; E.W. MEIJER.** Material Science and Treatment, Synthesis of polymers. Wiley-VCH Verlag GmbH, 1999
- **D. A. TOMALIA et al.** *Angew. Chem. Int. Engl.,* 1990, vol. 29, 138-175
- **N. ARDOIN ; D. ASTRUC.** *Bull. Soc. Chim. Fr.,* 1995, vol. 132, 875-909
- **B. I. VOIT.** *Acta Polymer,* 1995, vol. 46, 87-99
- **B.I.VOIT.** *Acta Polymer.,* 1995, vol. 46, 87-99